# EUROPEAN PATENT SPECIFICATION

(11) **EP 1 198 456 B1**
(45) Date of publication and mention of the grant of the patent: **19.09.2007**
(21) Application number: 00955327.2
(22) Date of filing: 02.08.2000
(51) Int. Cl.: C07D 213/74, C07C 335/18, C07C 335/16, C07C 279/28, C07C 279/30, C07D 209/08, C07D 235/06, C07D 401/12, C07D 239/42, C07D 401/04, C07D 233/42, C07D 407/12, C07C 275/30, C07D 215/38, A61K 31/47, A61K 31/44, A61K 31/17, A61K 31/155

(54) **POTASSIUM CHANNEL OPENERS**
KALIUM KANALAKTIVATOREN
AGENTS D'OUVERTURE DES CANAUX POTASSIQUES

(30) Priority: 03.08.1999 US 364771; 21.07.2000 US 621154
(43) Date of publication of application: 24.04.2002
(73) Proprietor: ABBOTT LABORATORIES, Abbott Park, IL 60064-6050 (US)
(72) Inventor: ALTENBACH, Robert, J., Chicago, IL 60631 (US); BAI, Hao, Libertyville, IL 60048 (US); BRIONI, Jorge, D., Vernon Hills, IL 60061 (US); CARROLL, William, A., Evanston, IL 60201 (US); GOPALAKRISHNAN, Murali, Libertyville, IL 60048 (US); GREGG, Robert, J., Libertyville, IL 60048 (US); HOLLADAY, Mark, W., Tucson, AZ 85750 (US); HUANG, Peggy, P., Lake Bluff, IL 60044 (US); KINCAID, John, F., Newbury Park, CA 91320 (US); KORT, Michael, E., Lake Bluff, IL 60044 (US); KYM, Philip, R., Grayslake, IL 60030 (US); LYNCH, John, K., Kenosha, WI 53142 (US); PEREZ-MEDRANO, Arturo, Grayslake, IL 60031 (US); ZHANG, Henry, Q., Grayslake, IL 60030 (US)
(74) Representative: Modiano, Micaela Nadia
(86) International application number: PCT/US2000/021054
(87) International publication number: WO 2001/009096

(56) References cited:
- EP-A- 0 381 504
- EP-A- 0 401 010
- EP-A- 0 501 797
- EP-A- 0 503 627
- EP-A- 0 656 350
- WO-A-99/07672
- FR-A- 2 023 940
- FR-A- 2 340 927
- DATABASE CHEMABS [Online] CHEMICAL ABSTRACTS SERVICE, COLUMBUS, OHIO, US; EYADA, H. A.: "Synthesis of some 4,5,6,7-tetrachlorophthalimido containing benzoxazole, benzimidazole, benzoxazine, and carbamate moieties" retrieved from STN Database accession no. 124:289398 XP002151933 & AL-AZHAR J. PHARM. SCI. (1994), 13, 93-103,
- DATABASE CHEMABS [Online] CHEMICAL ABSTRACTS SERVICE, COLUMBUS, OHIO, US; MOHAMED, Y. A.: "Synthesis and reactions of some new 5,6-dichlorophthalimidoacetic acid derivatives" retrieved from STN Database accession no. 118:38720 XP002151934 & J. SERB. CHEM. SOC. (1992), 57(9), 555-62,
- DATABASE CHEMABS [Online] CHEMICAL ABSTRACTS SERVICE, COLUMBUS, OHIO, US; ESSAWY, SAMY A. ET AL: "Synthesis and reactions of phthalimido aliphatic acid azides" retrieved from STN Database accession no. 116:173943 XP002151936 & POL. J. CHEM. (1991), 65(7-8), 1243-50,
- DATABASE CHEMABS [Online] CHEMICAL ABSTRACTS SERVICE, COLUMBUS, OHIO, US; BOYER, JOSEPH H. ET AL: "Alkylation of nitrocyanamide. A new synthesis of isocyanates" retrieved from STN Database accession no. 110:94646 XP002151938 & J. CHEM. SOC., PERKIN TRANS. 1 (1988), (8), 2137-40,
- DATABASE CHEMABS [Online] CHEMICAL ABSTRACTS SERVICE, COLUMBUS, OHIO, US; ALY, N. F., MRS. ET AL: "Acid azides: part IX - synthesis and reactions of phthalimidoacetic acid azide and its derivatives" retrieved from STN Database accession no. 101:110675 XP002151939 & INDIAN J. CHEM., SECT. B (1984), 23B(2), 121-4,
- DATABASE CHEMABS [Online] CHEMICAL ABSTRACTS SERVICE, COLUMBUS, OHIO, US; BOEHME, H. ET AL: "N-(.alpha.-Haloalkyl)carboxamides. 20. Imidomethyl thiocyanates and isothiocyanates" retrieved from STN Database accession no. 82:3924 XP002151941 & ARCH. PHARM. (WEINHEIM, GER.) (1974), 307(10), 775-9,
- DATABASE CHEMABS [Online] CHEMICAL ABSTRACTS SERVICE, COLUMBUS, OHIO, US; MOFFATT, J. G. ET AL: "Carbodiimide-sulfoxide reactions. XI. Reactions of carboxylic acids, hydroxamic acids, and amides" retrieved from STN Database accession no. 76:3528 XP002151943 & J. ORG. CHEM. (1971), 36(22), 3391-400,

## Description

Novel aminal compounds and their derivatives can open potassium channels and are useful for treating a variety of medical conditions.

### Background of Invention

Potassium channels play an important role in regulating cell membrane excitability. When the potassium channels open, changes in the electrical potential across the cell membrane occur and result in a more polarized state. A number of diseases or conditions may be treated with therapeutic agents that open potassium channels; see for example (K. Lawson, Pharmacol. Ther., v. 70, pp. 39-63 (1996)); (D.R. Gehlert et al., Prog. Neuro-Psychopharmacol & Biol. Psychiat., v. 18, pp. 1093-1102 (1994)); (M. Gopalakrishnan et al., Drug Development Research, v. 28, pp. 95-127 (1993)); (J.E. Freedman et al., The Neuroscientist, v. 2, pp. 145-152 (1996)); (D. E. Nurse et al., Br. J. Urol., v. 68 pp. 27-31 (1991)); (B. B. Howe et al., J. Pharmacol. Exp. Ther., v. 274 pp. 884-890 (1995)); (D. Spanswick et al., Nature, v. 390 pp. 521-25 (December 4, 1997)); (Dompeling Vasa. Supplementum (1992) 3434); (WO9932495); (Grover, J Mol Cell Cardiol. (2000) 32, 677); and (Buchheit, Pulmonary Pharmacology & Therapeutics (1999) 12, 103). Such diseases or conditions include asthma, epilepsy, male sexual dysfunction, female sexual dysfunction, pain, bladder overactivity, stroke, diseases associated with decreased skeletal blood flow such as Raynaud's phenomenon and intermittent claudication, eating disorders, functional bowel disorders, neurodegeneration, benign prostatic hyperplasia (BPH), dysmenorrhea, premature labor, alopecia, cardioprotection, coronary artery disease, angina and ischemia.

Bladder overactivity is a condition associated with the spontaneous, uncontrolled contractions of the bladder smooth muscle. Bladder overactivity thus is associated with sensations of urgency, urinary incontinence, pollakiuria, bladder instability, nocturia, bladder hyerreflexia, and enuresis (Resnick, The Lancet (1995) 346, 94-99; Hampel, Urology (1997) 50 (Suppl 6A), 4-14; Bosch, BJU International (1999) 83 (Suppl 2), 7-9). Potassium channel openers (KCOs) act as smooth muscle relaxants. Because bladder overactivity and urinary incontinence can result from the spontaneous, uncontrolled contractions of the smooth muscle of the bladder, the ability of potassium channel openers to hyperpolarize bladder cells and relax bladder smooth muscle may provide a method to ameliorate or prevent bladder overactivity, pollakiuria, bladder instability, nocturia, bladder hyperreflexia, urinary incontinence, and enuresis (Andersson, Urology (1997) 50 (Suppl 6A), 74-84; Lawson, Pharmacol. Ther., (1996) 70, 39-63; Nurse., Br. J. Urol., (1991) 68, 27-31; Howe, J. Pharmacol. Exp. Ther., (1995) 274, 884-890; Gopalakrishnan, Drug Development Research, (1993) 28, 95-127).

The irritative symptoms of BPH (urgency, frequency, nocturia and urge incontinence) have been shown to be correlated to bladder instability (Pandita, The J. of Urology (1999) 162, 943). Therefore the ability of potassium channel openers to hyperpolarize bladder cells and relax bladder smooth muscle may provide a method to ameliorate or prevent the symptoms associated with BPH. (Andersson; Prostate (1997) 30: 202-215).

The excitability of corpus cavemosum smooth muscle cells is important in the male erectile process. The relaxation of corporal smooth muscle cells allows arterial blood to build up under pressure in the erectile tissue of the penis leading to erection (Andersson, Pharmacological Reviews (1993) 45, 253). Potassium channels play a significant role in modulating human corporal smooth muscle tone, and thus, erectile capacity. By patch clamp technique, potassium channels have been characterized in human corporal smooth muscle cells (Lee, Int. J. Impot. Res. (1999) 11(4),179-188). Potassium channel openers are smooth muscle relaxants and have been shown to relax corpus cavemosal smooth muscle and induce erections (Andersson, Pharmacological Reviews (1993) 45, 253; Lawson, Pharmacol. Ther., (1996) 70, 39-63, Vick, J. Urol. (2000) 163: 202). Potassium channel openers therefore may have utility in the treatment of male sexual dysfunctions such as male erectile dysfunction, impotence and premature ejaculation.

The sexual response in women is classified into four stages: excitement, plateau, orgasm and resolution. Sexual arousal and excitement increase blood flow to the genital area, and lubrication of the vagina as a result of plasma transudation. Topical application of KCOs like minoxidil and nicorandil have been shown to increase clitoral blood flow (J.J. Kim, J.W. Yu, J.G. Lee, D.G. Moon, "Effects of topical K-ATP channel opener solution on clitoral blood flow", J. Urol. (2000) 163 (4): 240). KCOs may be effective for the treatment of female sexual dysfunction including clitoral erectile insufficiency, vaginismus and vaginal engorgement (I. Goldstein and J.R. Berman., "Vasculogenic female sexual dysfunction: vaginal engorgement and clitoral erectile insufficiency syndromes"., Int. J. Impotence Res. (1998) 10:S84-S90), as KCOs can increase blood flow to female sexual organs.

Potassium channel openers may have utility as tocolytic agents to inhibit uterine contractions to delay or prevent premature parturition in individuals or to slow or arrest delivery for brief periods to undertake other therapeutic measures (Sanborn, Semin. Perinatol. (1995) 19, 31-40; Morrison, Am. J. Obstet. Gynecol. (1993) 169(5), 1277-85). Potassium channel openers also inhibit contractile responses of human uterus and intrauterine vasculature. This combined effect would suggest the potential use of KCOs for dysmenhorrea (Kostrzewska, Acta Obstet. Gynecol. Scand. (1996) 75(10), 886-91). Potassium channel openers relax uterine smooth muscle and intrauterine vasculature and therefore may have utility in the treatment of premature labor and dysmenorrhoea (Lawson, Pharmacol. Ther., (1996) 70, 39-63).

Potassium channel openers relax gastrointestinal smooth tissues and therefore may be useful in the treatment of functional bowel disorders such as irritable bowel syndrome (Lawson, Pharmacol. Ther., (1996) 70, 39-63).

Potassium channel openers relax airway smooth muscle and induce bronchodilation. Therefore potassium channel openers may be useful in the treatment of asthma and airways hyperreactivity (Lawson, Pharmacol. Ther., (1996) 70, 39-63; Buchheit, Pulmonary Pharmacology & Therapeutics (1999) 12, 103; Gopalakrishnan, Drug Development Research, (1993) 28, 95-127).

Neuronal hyperpolarization can produce analgesic effects. The opening of potassium channels by potassium channel openers and resultant hyperpolarization in the membrane of target neurons is a key mechanism in the effect of opioids. The peripheral antinociceptive effect of morphine results from activation of ATP-sensitive potassium channels, which causes hyperpolarization of peripheral terminals of primary afferents, leading to a decrease in action potential generation (Rodrigues, Br J Pharmacol (2000) 129(1), 110-4). Opening of K_{ATP} channels by potassium channel openers plays an important role in the antinociception mediated by alpha-2 adrenoceptors and mu opioid receptors. KCOs can potentiate the analgesic action of both morphine and dexmedetomidine via an activation of K_{ATP} channels at the spinal cord level (Vergoni, Life Sci. (1992) 50(16), PL135-8; Asano, Anesth. Analg. (2000) 90(5), 1146-51).. Thus, potassium channel openers can hyperpolarize neuronal cells and have shown analgesic effects. Potassium channel openers therefore may be useful as analgesics in the treatment of various pain states including but not limited to migraine and dyspareunia (Lawson, Pharmacol. Ther., (1996) 70, 39-63; Gopalakrishnan, Drug Development Research, (1993) 28, 95-127; Gehlert, Prog. Neuro-Psychopharmacol. & Biol. Psychiat., (1994) 18, 1093-1102).

Epilepsy results from the propagation of nonphysiologic electrical impulses. Potassium channel openers hyperpolarize neuronal cells and lead to a decrease in cellular excitability and have demonstrated antiepileptic effects. Therefore potassium channel openers may be useful in the treatment of epilepsy (Lawson, Pharmacol. Ther., (1996) 70, 39-63; Gopalakrishnan, Drug Development Research, (1993) 28, 95-127; Gehlert, Prog. Neuro-Psychopharmacol. & Biol. Psychiat., (1994) 18, 1093-1102).

Neuronal cell depolarization can lead to excitotoxicity and neuronal cell death. When this occurs as a result of acute ischemic conditions, it can lead to stroke. Long-term neurodegeneration can bring about conditions such as Alzheimer's and Parkinson's diseases. Potassium channel openers can hyperpolarize neuronal cells and lead to a decrease in cellular excitability. Activation of potassium channels has been shown to enhance neuronal survival. Therefore potassium channel openers may have utility as neuroprotectants in the treatment of neurodegenerative conditions and diseases such as cerebral ischemia, stroke, Alzheimer's disease and Parkinson's disease (Lawson, Pharmacol. Ther., (1996) 70, 39-63; Gopalakrishnan, Drug Development Research, (1993) 28, 95-127; Gehlert, Prog. Neuro-Psychopharmacol & Biol. Psychiat., (1994) 18, 1093-1102; Freedman, The Neuroscientist (1996) 2, 145).

Potassium channel openers may have utility in the treatment of diseases or conditions associated with decreased skeletal muscle blood flow such as Raynaud's syndrome and intermittent claudication (Lawson, Pharmacol. Ther., (1996) 70, 39-63; Gopalakrishnan, Drug Development Research, (1993) 28, 95-127; Dompeling Vasa. Supplementum (1992) 3434; and WO9932495).

Potassium channel openers may be useful in the treatment of eating disorders such as obesity (Spanswick, Nature, (1997) 390, 521-25; Freedman, The Neuroscientist (1996) 2, 145).

Potassium channel openers have been shown to promote hair growth therefore potassium channel openers have utility in the treatment of hair loss and baldness also known as alopecia (Lawson, Pharmacol. Ther., (1996) 70, 39-63; Gopalakrishnan, Drug Development Research, (1993) 28, 95-127).

Potassium channel openers possess cardioprotective effects against myocardial injury during ischemia and reperfusion. (Garlid, Circ. Res. (1997) 81(6), 1072-82). Therefore, potassium channel openers may be useful in the treatment of heart diseases (Lawson, Pharmacol. Ther., (1996) 70, 39-63; Grover, J. Mol. Cell Cardiol. (2000) 32, 677).

Potassium channel openers, by hyperpolarization of smooth muscle membranes, can exert vasodilation of the collateral circulation of the coronary vasculature leading to increase blood flow to ischemic areas and could be useful for the coronary artery disease (Lawson, Pharmacol. Ther., (1996) 70, 39-63, Gopalakrishnan, Drug Development Research, (1993) 28, 95-127).

US 3,636,105 discloses a group of 1-fluoroacetylamino-2,2,2-trichloroethyl urea rodenticide agents. US 4,146,646 discloses a group of bis-amide fungicide agents. US 5,140,031 and US 5,278,169 disclose a group of cyanoguanidine cardiovascular agents. US 4,057,636 discloses a group of pyridylcyanoguanidine hypotensive agents. US 5,547,966 discloses a group of urea, thiourea, and cyanoguanidine agents for treating ischemia. ZA 695324 discloses a group of thioureas useful as insecticide, acaricidal,.and rodenticide agents. WO 92/04045 discloses a group of carbamate cholecystokinin receptor antagonists. WO 97/14417 discloses a group of peptide mimetic agents useful as fibrinogen receptor antagonists. WO 98/57940 discloses a group of oxazolidinone and imidazolidinone agents useful as α_{1A} receptor antagonists. WO 99/28291 discloses a group of bis(hydroxyureas) useful as inhibitors of 5-lipoxygenase.

Compounds of the present invention are novel, hyperpolarize cell membranes, open potassium channels, relax smooth muscle cells, inhibit bladder contractions and may be useful for treating diseases that can be ameliorated by opening potassium channels.

### Summary of the Invention

In its principal embodiment, the present invention discloses compounds having formula I: 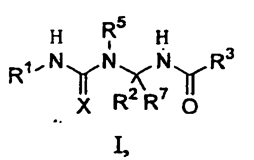 or a pharmaceutically acceptable salt thereof wherein,
X is selected from O, S, CHCN, C(CN)₂, CHNO₂, and NR⁸;
R⁸ is selected from hydrogen, alkoxy, alkyl, alkylsulfonyl, arylalkoxy, aryloxy, arylsulfonyl, cyano, haloalkylsulfonyl, heterocyclealkoxy, heterocycleoxy, hydroxy, nitro, and sulfamyl;
R¹ is selected from aryl, arylalkyl, heterocycle, and heterocyclealkyl;
R² is selected from hydrogen, alkenyl, alkenyloxyalkyl, alkenyloxy(alkenyloxy)alkyl, alkoxyalkyl, alkoxycarbonyl, alkoxycarbonylalkyl, alkoxycarbonyl(halo)alkyl, alkoxy(halo)alkyl, alkyl, alkylcarbonyl, alkylcarbonylalkyl, alkylcarbonyl(halo)alkyl, alkylcarbonyloxyalkyl, alkylsulfinylalkyl, alkylsulfonylalkyl, alkylthioalkyl, alkynyl, amido, amidoalkyl, aryl, arylalkoxyalkyl; arylalkoxycarbonyl, arylalkoxycarbonylalkyl, arylalkyl, arylcarbonyl, arylcarbonylalkyl arylcarbonyloxyalkyl, aryl(halo)alkyl, aryloxyalkyl, aryloxycarbonyl, aryloxycarbonylalkyl, arylalkylthioalkyl, arylsulfonylalkyl, carboxy, carboxyalkyl, carboxy(halo)alkyl, cyanoalkyl, cyano(halo)alkyl, cycloalkenyl, cycloalkenylalkyl, cycloalkyl, cycloalkylalkoxyalkyl, cycloalkylalkyl, cycloalkylcarbonyl, cycloalkyloxyalkyl, cycloalkylalkylthioalkyl, formyl, haloalkenyl, haloalkyl, haloalkylcarbonyl, haloalkynyl, heterocycle, heterocyclealkoxyalkyl, heterocyclealkyl, heterocyclecarbonyl, heterocycleoxyalkyl, heterocyclealkylthioalkyl, hydroxyalkyl, mercaptoalkyl, sulfamylalkyl, sulfamyl(halo)alkyl, and (NR⁹R¹⁰)alkyl wherein R⁹ and R¹⁰ are independently selected from hydrogen, alkyl, alkylcarbonyl, aryl, arylalkyl, arylcarbonyl, formyl, and S(O)₂R¹¹, wherein R¹¹ is selected from alkyl, aryl, and arylalkyl;
R³ is selected from alkyl, aryl, arylalkyl, heterocycle, and heterocyclealkyl;
R⁵ is selected from hydrogen, alkyl and OR¹²;
R¹² is selected from hydrogen, alkyl and arylalkyl; and
R⁷ is selected from hydrogen, haloalkyl and lower alkyl; or
R⁷ and R² taken together with the carbon atom to which they are attached, together form a 5 or 6 membered carbocyclic ring wherein the 5 or 6 membered carbocyclic ring is optionally substituted with 1 or 2 substituents independently selected from alkenyl, alkoxy, alkyl, alkynyl, halogen, haloalkoxy, and haloalkyl;
provided that when X is O; R² is -CCl₃; R³ is alkyl or phenyl; and R⁵ and R⁷ are hydrogen; then R¹ is other than phenyl;
further provided that
   N-[[[[(2-methylphenyl)amino]carbonyl]amino]phenylmethyl]-benzamide;
   N-[[[[(4-chlorophenyl)amino]carbonyl]amino]phenylmethyl]-benzamide;
   N-[2,2-dichloro-1-[[phenylamino)thioxomethyl]amino]ethyl]-benzamide;
   N-[2,2-dichloro-1-[[phenylamino)thioxomethyl]amino]ethyl]-4-methyl-benzamide;
   N-[2,2-dichloro-1-[[[4-methylphenyl)amino)thioxomethyl]amino]ethyl]-benzamide;
   N-[2,2-dichloro-1-[[[4-methylphenyl)amino)thioxomethyl]amino]ethyl]-4-methyl-benzamide;
   N-[2,2-dichloro-1-[[[4-chlorophenyl)amino)thioxomethyl]amino]ethyl]-benzamide;
   N-[2,2-dichloro-1-[[[4-chlorophenyl)amino)thioxomethyl]amino]ethyl]-4-methyl-benzamide;
   N-[[[(phenylamino)carbonyl]amino]methyl]-3-pyridinecarboxamide;
   N-[[[[(2-methylphenyl)amino)carbonyl]amino]methyl]-3-pyridinecarboxamide;
   N-[[[[(4-methylphenyl)amino)carbonyl]amino]methyl]-3-pyridinecarboxamide;
   N-[[[[(3-ethoxyphenyl)amino)carbonyl]amino]methyl]-3-pyridinecarboxamide;
   N-[[[[(4-nitrophenyl)amino)carbonyl]amino]methyl]-3-pyridinecarboxamide;
   N-[[[[(3-methylphenyl)amino)carbonyl]amino]methyl]-3-pyridinecarboxamide;
   N-[[[(phenylamino)carbonyl]amino]methyl]-benzamide;
   N-[[[(phenylamino)thioxomethyl]amino]methyl]-benzamide;
   N-[[[(phenylamino)carbonyl]amino]methyl]-4-chloro-benzamide;
   N-[[[(phenylamino)thioxomethyl]amino]methyl]-4-cbloro-benzamide;
   1-(1-acetamido-2,2,2-trichloroethyl)-3-phenyl-2-thio-urea;
   1-phenyl-2-thio-3-(2,2,2-trichloro-1-propionamidoethyl)-urea; and
   1-(1-butyramido-2,2,2-trichloroethyl)-3-phenyl-2-thio-urea are excluded.

### Detailed Description of the Invention

In its principal embodiment, the present invention discloses compounds having formula I as defined above.

In another embodiment of the present invention, compounds have formula I wherein X is selected from O, S, CHCN, C(CN)₂, CHNO₂, and NR⁸; R⁸ is selected from alkoxy, alkylsulfonyl, arylalkoxy, arylsulfonyl, cyano, haloalkylsulfonyl, hydroxy, and nitro; R¹ is selected from the aryl, arylalkyl, heterocycle, and heterocyclealkyl; R² is selected from hydrogen, alkenyl, alkenyloxyalkyl, alkenyloxy(alkenyloxy)alkyl, alkoxyalkyl, alkoxycarbonylalkyl, alkyl, alkylcarbonyl, alkylsulfonylalkyl, alkylthioalkyl, aryl, arylalkyl, arylsulfonylalkyl, cyanoalkyl, cycloalkenyl, cycloalkenylalkyl, cycloalkyl, cycloalkylalkyl, haloalkyl, haloalkylcarbonyl, heterocycle, heterocyclealkyl, hydroxyalkyl, sulfamylalkyl, and (NR⁹R¹⁰)alkyl; R³ is selected from aryl, arylalkyl, and heterocycle; R⁵ is selected from hydrogen and alkyl; R⁷ is selected from hydrogen, haloalkyl, and lower alkyl; or R⁷ and R² taken together with the carbon atom to which they are attached, together form a 5 or 6 membered carbocyclic ring wherein the 5 or 6 membered carbocyclic ring is optionally substituted with 1 or 2 substituents independently selected from alkyl, halo, haloalkoxy, and haloalkyl; and R⁹ and R¹⁰ are as defined in formula I.

In another embodiment of the present invention, compounds have formula I wherein X is selected from O, S, CHCN, C(CN)₂, CHNO₂, and NR⁸; R⁸ is selected from alkoxy, alkylsulfonyl, haloalkylsulfonyl, cyano, hydroxy, nitro, arylalkoxy wherein the aryl portion of arylalkoxy is phenyl, and arylsulfonyl wherein the aryl portion of arylsulfonyl is phenyl; R¹ is selected from heterocycle and aryl wherein heterocycle is selected from pyridine, pyrimidine and quinoline wherein pyridine, pyrimidine and quinoline are optionally substituted with 1, 2, or 3 substituents independently selected from alkoxy, alkyl, halo, haloalkyl, nitro, phenylsulfonyl and sulfamyl, and wherein aryl is phenyl optionally substituted with 1, 2, or 3 substituents independently selected from alkoxy, alkyl, halo, haloalkyl, nitro, phenylsulfonyl, and sulfamyl; R² is selected from hydrogen, alkenyl, alkenyloxyalkyl, alkenyloxy(alkenyloxy)alkyl, alkoxyalkyl, alkoxycarbonylalkyl, alkyl, alkylcarbonyl, alkylsulfonylalkyl, aryl wherein aryl is phenyl, arylalkyl wherein the aryl portion of arylalkyl is phenyl, arylsulfonylalkyl wherein the aryl portion of arylsulfonylalkyl is phenyl, cyanoalkyl, cycloalkenylalkyl, cycloalkyl, haloalkyl, haloalkylcarbonyl, hydroxyalkyl, sulfamylalkyl, (NR⁹R¹⁰)alkyl and heterocycle wherein heterocycle is selected from 1,3-dioxane, pyrrolidine and thiophene; R³ is selected from aryl wherein aryl is phenyl and arylalkyl wherein the aryl portion of arylalkyl is phenyl; R⁵ is hydrogen; R⁷ is selected from hydrogen, haloalkyl, and lower alkyl; or R⁷ and R² taken together with the carbon atom to which they are attached, together form a 5 or 6 membered carbocyclic ring wherein the 5 or 6 membered carbocyclic ring is optionally substituted with 1 or 2 substituents independently selected from alkyl, halo, haloalkoxy, and haloalkyl; and R⁹ and R¹⁰ are as defined in formula I.

In another embodiment of the present invention, compounds have formula I wherein X is selected from O, S, CHNO₂, C(CN)₂, and NR⁸; R⁸ is selected from arylsulfonyl, cyano, haloalkylsulfonyl, nitro and sulfamyl; R¹ is selected from aryl, arylalkyl, heterocycle and heterocyclalkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is selected from hydrogen and alkyl; R⁷ is hydrogen; and R⁹ and R¹⁰ are as defined in formula I.

In another embodiment of the present invention, compounds have formula I wherein X is selected from O, S, CHNO₂, C(CN)₂, and NR⁸; R⁸ is selected from arylsulfonyl wherein the aryl portion of arylsulfonyl is phenyl, cyano, haloalkylsulfonyl, nitro and sulfamyl; R¹ is selected from aryl wherein aryl is phenyl, arylalkyl wherein the aryl portion of arylalkyl is phenyl, heterocycle wherein heterocycle is selected from quinoline, pyridine and pyrimidine, and heterocyclalkyl
wherein the heterocycle portion of heterocyclealkyl is pyridine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is selected from aryl wherein aryl is phenyl and arylalkyl wherein the aryl portion of arylalkyl is phenyl; R⁵ is selected from hydrogen and alkyl; R⁷ is hydrogen; and R⁹ arid R¹⁰ are as defined in formula I.

In another embodiment of the present invention, compounds have formula I wherein X is NR⁸; R⁸ is cyano; R¹ is selected from heterocycle and heterocyclealkyl; R³ is selected from heterocycle and heterocyclealkyl; R⁵ is hydrogen; and R² and R⁷ are as defined in formula I.

In another embodiment of the present invention, compounds have formula I wherein X is NR⁸; R⁸ is cyano; R¹ is selected from heterocycle and heterocyclealkyl; R³ is selected from aryl and arylalkyl; R⁵ is hydrogen; and R² and R⁷ are as defined in formula I.

In another embodiment of the present invention, compounds have formula I wherein X is NR⁸; R⁸ is cyano; R¹ is selected from heterocycle and heterocyclealkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is NR⁸; R⁸ is cyano; R¹ is heterocyclealkyl wherein the heterocycle portion of heterocyclealkyl is pyridine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰alkyl and heterocycle wherein heterocycle is selected from 1,3-dioxane, pyrrolidine and thiophene; R³ is aryl wherein aryl is phenyl; R⁵ is hydrogen; and R⁷, R⁹ and R¹⁰ are as defined in formula I.

In another embodiment of the present invention, compounds have formula I wherein X is NR⁸; R⁸ is cyano; R¹ is heterocycle wherein heterocycle is selected from quinoline and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is NR⁸; R⁸ is cyano; R¹ is heterocycle wherein heterocycle is pyridine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is NR⁸; R⁸ is cyano; R¹ is heterocycle; R² is haloalkyl; R³ is aryl; R⁵ is hydrogen; and R⁷ is hydrogen.

In another embodiment of the present invention, compounds have formula I wherein X is NR⁸; R⁸ is cyano; R¹ is heterocycle wherein heterocycle is pyridine; R² is haloalkyl; R³ is aryl wherein aryl is phenyl; R⁵ is hydrogen ; and R⁷is hydrogen.

In another embodiment of the present invention, compounds have formula I wherein X is NR⁸; R⁸ is cyano; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is alkyl; R⁷ is hydrogen; and R⁹ and R¹⁰ are as defined in formula I.

In another embodiment of the present invention, compounds have formula I wherein X is NR⁸; R⁸ is cyano; R¹ is selected from heterocycle and heterocyclealkyl; R³ is alkyl; R⁵ is hydrogen; and R² and R⁷are as defined in formula I.

In another embodiment of the present invention, compounds have formula I wherein X is NR⁸; R⁸ is cyano; R¹ is selected from aryl and arylalkyl; R³ is selected from heterocycle and heterocyclealkyl; R⁵ is hydrogen; and R² and R⁷ are as defined in formula I.

In another embodiment of the present invention, compounds have formula I wherein X is NR⁸; R⁸ is cyano; R¹ is selected from aryl and arylalkyl; R³ is selected from aryl and arylalkyl; R⁵ is hydrogen; and R² and R⁷ are as defined in formula I.

In another embodiment of the present invention, compounds have formula I wherein X is NR⁸; R⁸ is cyano; R¹ is selected from aryl and arylalkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is NR⁸; R⁸ is cyano; R¹ is arylalkyl wherein the aryl portion of arylalkyl is phenyl; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is NR⁸; R⁸ is cyano; R¹ is aryl wherein aryl is phenyl; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is NR⁸; R⁸ is cyano; R¹ is selected from aryl and arylalkyl; R³ is alkyl; R⁵ is hydrogen; and R² and R⁷ are as defined in formula I.

In another embodiment of the present invention, compounds have formula I wherein X is NR⁸; R⁸ is selected from hydrogen, alkoxy, alkyl, alkylsulfonyl, arylalkoxy, aryloxy, arylsulfonyl, haloalkylsulfonyl, heterocyclealkoxy, heterocycleoxy, hydroxy, nitro, and sulfamyl; R⁵ is hydrogen; and R¹, R², R³ and R⁷ are as defined in formula I.

In another embodiment of the present invention, compounds have formula I wherein X is NR⁸; R⁸ is nitro; R⁵ is hydrogen; and R¹, R², R³ and R⁷ are as defined in formula I.

In another embodiment of the present invention, compounds have formula I wherein X is NR⁸; R⁸ is nitro; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; and R⁷, R⁹ and R¹⁰ are as defined in formula I.

In another embodiment of the present invention, compounds have formula I wherein X is NR⁸; R⁸ is selected from arylsulfonyl, haloalkylsulfonyl and sulfamyl; R⁵ is hydrogen; and R¹, R², R³, and R⁷ are as defined in formula I.

In another embodiment of the present invention, compounds have formula I wherein X is NR⁸; R⁸ is selected from arylsulfonyl wherein the aryl portion of arylsulfonyl is phenyl, haloalkylsulfonyl and sulfamyl; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; and R⁷, R⁹ and R¹⁰ are as defined in formula I.

In another embodiment of the present invention, compounds have formula I wherein X is S; R⁵ is hydrogen; and R¹, R², R³ and R⁷ are as defined in formula I.

In another embodiment of the present invention, compounds have formula I wherein X is S; R¹ is selected from heterocycle and heterocyclealkyl; R³ is selected from aryl and arylalkyl; R⁵ is hydrogen; and R² and R⁷ are as defined in formula I.

In another embodiment of the present invention, compounds have formula I wherein X is S; R¹ is selected from heterocycle and heterocyclealkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is S; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is S; R¹ is heterocycle; R² is haloalkyl; R³ is aryl; R⁵ is hydrogen; and R⁷ is hydrogen.

In another embodiment of the present invention, compounds have formula I wherein X is S; R¹ is heterocycle wherein heterocycle is pyridine; R² is haloalkyl; R³ is aryl wherein aryl is phenyl; R⁵ is hydrogen; and R⁷ is hydrogen.

In another embodiment of the present invention, compounds have formula I wherein X is S; R¹ is selected from aryl and arylalkyl; R³ is selected from aryl and arylalkyl; R⁵ is hydrogen; and R² and R⁷ are as defined in formula I.

In another embodiment of the present invention, compounds have formula I wherein X is S; R¹ is selected from aryl and arylalkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is S; R¹ is aryl wherein aryl is phenyl; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is S; R¹ is aryl wherein aryl is phenyl; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is O; R¹ is selected from heterocycle and heterocyclealkyl; R³ is selected from aryl and arylalkyl; R⁵ is hydrogen; and R² and R⁷ are as defined in formula I.

In another embodiment of the present invention, compounds have formula I wherein X is O; R¹ is selected from heterocycle and heterocyclealkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is O; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is O; R¹ is heterocycle; R² is haloalkyl; R³ is aryl; R⁵ is hydrogen; and R⁷ is hydrogen.

In another embodiment of the present invention, compounds have formula I wherein X is O; R¹ is heterocycle wherein heterocycle is pyridine; R² is haloalkyl; R³ is aryl wherein aryl is phenyl; R⁵ is hydrogen; and R⁷ is hydrogen.

In another embodiment of the present invention, compounds have formula I wherein X is O; R¹ is selected from heterocycle and heterocyclealkyl; R³ is selected from heterocycle, and heterocyclealkyl; R⁵ is hydrogen; and R² and R⁷ are as defined in forumula I.

In another embodiment of the present invention, compounds have formula I wherein X is O; R¹ is selected from aryl and arylalkyl; R³ is selected from aryl and arylalkyl; R⁵ is hydrogen; and R² and R⁷ are as defined in formula I.

In another embodiment of the present invention, compounds have formula I wherein X is O; R¹ is selected from aryl and arylalkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is O; R¹ is aryl wherein aryl is phenyl; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; and R⁷, R⁹ and R¹⁰ are as defined in formula I.

In another embodiment of the present invention, compounds have formula I wherein X is selected from CHCN and CHNO₂; R¹ is selected from heterocycle and heterocyclealkyl; R³ is selected from heterocycle and heterocyclealkyl; R⁵ is hydrogen; and R² and R⁷ are as defined in formula I.

In another embodiment of the present invention, compounds have formula I wherein X is selected from CHCN and CHNO₂; R¹ is selected from heterocycle and heterocyclealkyl; R³ is selected from aryl and arylalkyl; R⁵ is hydrogen; and R² and R⁷ are as defined in formual I.

In another embodiment of the present invention, compounds have formula I wherein X is selected from CHCN and CHNO₂; R¹ is selected from heterocycle and heterocyclealkyl; R² is selected from hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; R³ is selected from aryl and arylalkyl; R⁵ is hydrogen; R⁷ is hydrogen; and R⁹ and R¹⁰ are as defined in formula I.

In another embodiment of the present invention, compounds have formula I wherein X is selected from CHCN and CHNO₂; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is selected from CHCN and CHNO₂; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; R⁷ is hydrogen; and R⁹ and R¹⁰ are as defined in formula I.

In another embodiment of the present invention, compounds have formula I wherein X is selected from CHCN and CHNO₂; R¹ is heterocycle; R² is haloalkyl; R³ is aryl; R⁵ is hydrogen; and R⁷ is hydrogen.

In another embodiment of the present invention, compounds have formula I wherein X is selected from CHCN and CHNO₂; R¹ is heterocycle wherein heterocycle is pyridine; R² is haloalkyl; R³ is aryl wherein aryl is phenyl; R⁵ is hydrogen; and R⁷ is hydrogen.

In another embodiment of the present invention, compounds have formula I wherein X is selected from CHCN and CHNO₂; R¹ is selected from heterocycle and heterocyclealkyl; R³ is alkyl; R⁵ is hydrogen; and R² and R⁷ are as defined in formula I.

In another embodiment of the present invention, compounds have formula I wherein X is selected from CHCN and CHNO₂; R¹ is selected from aryl and arylalkyl; R³ is selected from heterocycle and heterocyclealkyl; R⁵ is hydrogen; and R² and R⁷ are as defined in formula I.

In another embodiment of the present invention, compounds have formula I wherein X is selected from CHCN and CHNO₂; R¹ is selected from aryl and arylalkyl; R³ is selected from aryl and arylalkyl; R⁵ is hydrogen; and R² and R⁷ are as defined in formula I.

In another embodiment of the present invention, compounds have formula I wherein X is selected from CHCN and CHNO₂; R¹ is selected from aryl and arylalkyl; R³ is alkyl; R⁵ is hydrogen; and R² and R⁷ are as defined in formula I:

In another embodiment of the present invention, compounds have formula I wherein X is C(CN)₂; R¹ is selected from heterocycle and heterocyclealkyl; R³ is selected from aryl and arylalkyl; R⁵ is hydrogen; and R² and R⁷ are as defined in formula I.

In another embodiment of the present invention, compounds have formula I wherein X is C(CN)₂; R¹ is heterocycle wherein heterocycle is selected from quinoline, pyridine and pyrimidine; R² is selected from hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from 1,3-dioxane, pyrrolidine and thiophene; R³ is aryl wherein aryl is phenyl; R⁵ is hydrogen; and R⁷, R⁹ and R¹⁰ are as defined in formula I.

In another embodiment of the present invention, compounds have formula VI: 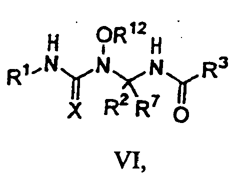 or a pharmaceutically acceptable salt thereof wherein X, R¹, R², R³, R⁷ and R¹² are as defined in formula I.

In another embodiment of the present invention, compounds have formula VI wherein X is NR⁸; R⁸ is cyano; R¹, R², R³, R⁷ and R¹² are as defined in formula I.

In another embodiment of the present invention, compounds have formula VI wherein X is NR⁸; R⁸ is selected from hydrogen, alkoxy, alkyl, alkylsulfonyl, arylalkoxy, aryloxy, arylsulfonyl, haloalkylsulfonyl, heterocyclealkoxy, hydroxy, nitro, and sulfamyl; and R¹, R², R³, R⁷ and R¹² are as defined in formula I.

In another embodiment of the present invention, compounds have formula VI wherein X is S; and R¹, R², R³, R⁷ and R¹² are as defined in formula I.

In another embodiment of the present invention, compounds have formula VI wherein X is O; and R¹, R², R³, R⁷ and R¹² are as defined in formula I.

In another embodiment of the present invention, compounds have formula VI wherein X is S; X is selected from CHCN and CHNO₂; and R¹, R², R³, R⁷ and R¹² are as defined in formula I.

Another embodiment of the invention relates to the use of a compound of formula VII: 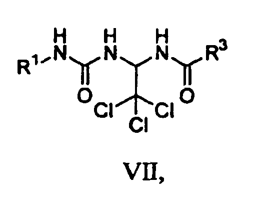 or a pharmaceutically acceptable salt thereof wherein R¹ is phenyl; and R³ is selected from alkyl and phenyl for the manufacture of a medicament for treating or preventing a disease which is prevented or ameliorated with potassium channel openers in a host mammal in need of such treatment by administration of a therapeutically effective amount.

Another embodiment of the present invention relates to pharmaceutical compositions comprising a therapeutically effective amount of a compound of formula I, VI or VII or a pharmaceutically acceptable salt thereof in combination with a pharmaceutically acceptable carrier wherein formula I is as defined in appended Claim 83.

The pharmaceutical compounds disclosed herein may be used in a method of treating male sexual dysfunction including male erectile dysfunction and premature ejaculation, comprising administering a therapeutically effective amount of a compound of formula I, VI or VII or a pharmaceutically acceptable salt thereof.

The pharmaceutical compounds disclosed herein may be used in a method of treating female sexual dysfunction including female anorgasmia, clitoral erectile insufficiency, vaginal engorgement, dyspareunia, and vaginismus comprising administering a therapeutically effective amount of a compound of formula I, VI or VII or a pharmaceutically acceptable salt thereof.

The pharmaceutical compounds disclosed herein may further be used in a method of treating asthma, epilepsy, Raynaud's syndrome, intermittent claudication, migraine, pain, bladder overactivity, pollakiuria, bladder instability, nocturia, bladder hyperreflexia, eating disorders, urinary incontinence, enuresis, functional bowel disorders, neurodegeneration, benign prostatic hyperplasia (BPH), dysmenorrhea, premature labor, alopecia, cardioprotection, and ischemia comprising administering a therapeutically effective amount of a compound of formula I, VI or VII or a pharmaceutically acceptable salt thereof.

The use of compounds of formula I or VII for the manufacture of a medicament for treating or preventing a disease which is prevented or ameliorated with potassium channel openers in a host mammal in need of such treatment by administration of a therapeutically effective amount is included in the present invention as claimed in appended Claims 84-93 and 95-102.

Compounds of the present invention for use as a therapeutic agent are claimed in appended Claim 103.

### Definition of Terms

As used throughout this specification and the appended claims, the following terms have the following meanings.

The term "alkenyl," as used herein, refers to a straight or branched chain hydrocarbon containing from 2 to 10 carbons and containing at least one carbon-carbon double bond formed by the removal of two hydrogens. Representative examples of alkenyl include ethenyl, 2-propenyl, 2-methyl-2-propenyl, 3-butenyl, 1,1-dimethyl-3-butenyl, 4-pentenyl, 5-hexenyl, 2-heptenyl, 2-methyl-1-heptenyl, and 3-decenyl,

The term "alkenyloxy," as used herein, refers to an alkenyl group, as defined herein, appended to the parent molecular moiety through an oxy moiety, as defined herein. Representative examples of alkenyloxy include allyloxy, 2-butenyloxy, and 3-butenyloxy.

The term "alkenyloxyalkyl," as used herein, refers to a alkenyloxy group, as defined herein, appended to the parent molecular moiety through an alkyl group, as defined herein. Representative examples of alkenyloxyalkyl include (allyloxy)methyl, (2-butenyloxy)methyl and (3-butenyloxy)methyl.

The term "alkenyloxy(alkenyloxy)alkyl," as used herein, refers to 2 independent alkenyloxy groups, as defined herein, appended to the parent molecular moiety through an alkyl group, as defined herein. Representative examples of alkenyloxy(alkenyloxy)alkyl include 1,2-bis(allyloxy)ethyl and 1, 1-bis[(allyloxy)methyl]propyl,

The term "alkoxy," as used herein, refers to an alkyl group, as defined herein, appended to the parent molecular moiety through an oxy moiety, as defined herein. Representative examples of alkoxy include methoxy, ethoxy, propoxy, 2-propoxy, butoxy, and tert-butoxy.

The term "alkoxyalkyl," as used herein, refers to an alkoxy group, as defined herein, appended to the parent molecular moiety through an alkyl group, as defined herein. Representative examples of alkoxyalkyl include tert-butoxymethyl, 2-ethoxyethyl, 2-methoxyethyl, methoxymethyl, and 1,1-dimethyl-3-(methoxy)propyl.

The term "alkoxycarbonyl," as used herein, refers to an alkoxy group, as defined herein, appended to the parent molecular moiety through a carbonyl group, as defined herein. Representative examples of alkoxycarbonyl include methoxycarbonyl, ethoxycarbonyl, and tert-butoxycarbonyl.

The term "alkoxycarbonylalkyl," as used herein, refers to an alkoxycarbonyl group, as defined herein, appended to the parent molecular moiety through an alkyl group, as defined herein. Representative examples of alkoxycarbonylalkyl include methoxycarbonylmethyl, ethoxycarbonylmethyl, tert-butoxycarbonylmethyl, and 1,1-dimethyl-2-(methoxycarbonyl)ethyl.,

The term "alkoxycarbonyl(halo)alkyl," as used herein, refers to an alkoxycarbonyl group and at least one halogen, as defined herein, appended to the parent molecular moiety through an alkyl group, as defined herein. Representative examples of alkoxycarbonyl(halo)alkyl include 1,1-dichloro-2-methoxy-2-oxoethyl, 1,1-difluoro-2-methoxy-2-oxoethyl, 1,1-dichloro-3-methoxy-3-oxopropyl, and 1,1-difluoro-3-methoxy-3-oxopropyl.

The term "alkoxy(halo)alkyl," as used herein, refers to an alkoxy group and at least one halogen, as defined herein, appended to the parent molecular moiety through an alkyl group, as defined herein. Representative examples of alkoxy(halo)alkyl include dichloro(methoxy)methyl, dichloro(ethoxy)methyl, dichloro(tert-butoxy)inethyl, 1,1-dichloro-2-ethoxyethyl, 1,1-dichloro-2-methoxyethyl, 1,1-dichloro-3-methoxypropyl, and 1,2-dichloro-3-methoxypropyl.

The term "alkyl," as used herein, refers to a straight or branched chain hydrocarbon containing from 1 to 10 carbon atoms. Representative examples of alkyl include methyl, ethyl, n-propyl, iso-propyl, n-butyl, sec-butyl, isobutyl, tert-butyl, n-pentyl, isopentyl, neopentyl, n-hexyl, 3-methylhexyl, 1-ethylpropyl, 2,2-dimethylpentyl, 2,3-dimethylpentyl, n-heptyl, n-octyl, n-nonyl, and n-decyl.

The term "alkylcarbonyl," as used herein, refers to an alkyl group, as defined herein, appended to the parent molecular moiety through a carbonyl group, as defined herein. Representative examples of alkylcarbonyl include acetyl, 1-oxopropyl, 2,2-dimethyl-1-oxopropyl, 1-oxobutyl, and 1-oxopentyl.

The term "alkylcarbonylalkyl," as used herein, refers to an alkylcarbonyl group, as defined herein, appended to the parent molecular moiety through an alkyl group, as defined herein. Representative examples of alkylcarbonylalkyl include 2-oxopropyl, 1,1-dimethyl-3-oxobutyl, 3-oxobutyl, and 3-oxopentyl.

The term "alkylcarbonyl(halo)alkyl," as used herein, refers to an alkylcarbonyl group and at least one halogen, as defined herein, appended to the parent molecular moiety through an alkyl group, as defined herein. Representative examples of alkylcarbonyl(halo)alkyl include 1,1-dichloro-2-oxopropyl, 1,1-dichloro-3-oxobutyl, 1,1-difluoro-3-oxobutyl, and 1,1-dichloro-3-oxopentyl.

The term "alkylcarbonyloxy," as used herein, refers to an alkylcarbonyl group, as defined herein, appended to the parent molecular moiety through an oxy moiety, as defined herein. Representative examples of alkylcarbonyloxy include acetyloxy, and ethylcarbonyloxy.

The term "alkylcarbonyloxyalkyl," as used herein, refers to an alkylcarbonyloxy group, as defined herein, appended to the parent molecular moiety through an alkyl group, as defined herein. Representative examples of alkylcarbonyloxyalkyl include acetyloxymethyl, and 2-(ethlylcarbonyloxy)ethy.

The term "alkylene" or "alkylene bridge" refers to a divalent group derived from a straight chain hydrocarbon of from 2 to 6 carbon atoms. The alkylene or alkylene bridge can be optionally substituted with 1 or 2 substituents selected from alkyl and oxo. Representative examples of alkylene or alkylene bridge include CH₂CH₂-, -C(O)CH₂-,-C(O)C(O)-, -CH₂CH₂CH₂-, -CH₂C(CH₃)₂CH₂-,-CH₂CH₂CH₂CH₂-, and-CH₂CH₂CH₂CH₂CH₂-.

The term "alkylsulfinyl," as used herein, refers to an alkyl group, as defined herein, appended to the parent molecular moiety through a sulfinyl group, as defined herein. Representative examples of alkylsulfinyl include methylsulfinyl, and ethylsulfinyl.

The term "alkylsulfinylalkyl," as used herein, refers to an alkylsulfinyl group, as defined herein, appended to the parent molecular moiety through an alkyl group, as defined herein. Representative examples of alkylsulfinylalkyl include methylsulfinylmethyl, and ethylsulfinylmethyl.

The term "alkylsulfonyl," as used herein, refers to an alkyl group, as defined herein, appended to the parent molecular moiety through a sulfonyl group, as defined herein. Representative examples of alkylsulfonyl include methylsulfonyl, and ethylsulfonyl.

The term "alkylsulfonylalkyl," as used herein, refers to an alkylsulfonyl group, as defined herein, appended to the parent molecular moiety through an alkyl group, as defined herein. Representative examples of alkylsulfonylalkyl include methylsulfonylmethyl, and ethylsulfonylmethyl.

The term "alkylthio," as used herein, refers to an alkyl group, as defined herein, appended to the parent molecular moiety through a thio moiety, as defined herein. Representative examples of alkylthio include methylsulfanyl, ethylsulfanyl, propylsulfanyl, 2-propylsulfanyl, and tert-butylsulfanyl.

The term "alkylthioalkyl," as used herein, refers to an alkylthio group, as defined herein, appended to the parent molecular moiety through an alkyl group, as defined herein. Representative examples of alkylthioalkyl include tert-butylsulfanylmethyl, 2-ethylsulfanylethyl, 2-methylsulfanylethyl, and methylsulfanylmethyl.

The term "alkynyl," as used herein, refers to a straight or branched chain hydrocarbon group containing from 2 to 10 carbon atoms and containing at least one carbon-carbon triple bond. Representative examples of alkynyl include acetylenyl, 1-propynyl, 2-propynyl, 3-butynyl, 2-pentynyl, and 1-butynyl.,

The term "amido," as used herein, refers to a -NR⁹R¹⁰ group, as defined herein, appended to the parent molecular moiety through a carbonyl group, as defined herein. Representative examples of amido include aminocarbonyl, dimethylaminocarbonyl, ethylaminocarbonyl, and benzylaminocarbonyl.

The term "amidoalkyl," as used herein, refers to an amido group, as defined herein, appended to the parent molecular moiety through an alkyl group, as defined herein. Representative examples of amidoalkyl include aminocarbonylmethyl, dimethylaminocarbonylmethyl, 2-(ethylaminocarbonyl)ethyl, and 3-(benzylaminocarbonyl)propyl.

The term "aryl," as used herein, refers to a monocyclic carbocyclic ring system or a bicyclic carbocyclic fused ring system having one or more aromatic rings. Representative examples of aryl include, azulenyl, indanyl, indenyl, naphthyl, phenyl, tetrahydronaphthyl.

The aryl groups of this invention can be substituted with 1, 2, 3, 4, or 5 substituents independently selected from alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, pjhenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, -NR^{A}R^{B}, (NR^{A}R^{B})alkyl, benzene, and furan.

The term "arylalkoxy," as used herein, refers to an aryl group, as defined herein, appended to the parent molecular moiety through an alkoxy group, as defined herein. Representative examples of arylalkoxy include 2-phenylethoxy, 3-naphth-2-ylpropoxy, and 5-phenylpentyloxy.

The term "arylalkoxyalkyl," as used herein, refers to an arylalkoxy group, as defined herein, appended to the parent molecular moiety through an alkyl group, as defined herein. Representative examples of arylalkoxyalky include 2-phenylethoxymethyl, 2-(3-naphth-2-ylpropoxy)ethyl, and 5-phenylpentyloxymethyl.

The term "arylalkoxycarbonyl," as used herein, refers to an arylalkoxy group, as defined herein, appended to the parent molecular moiety through a carbonyl group, as defined herein. Representative examples of arylalkoxycarbonyl include benzyloxycarbonyl, and naphth-2-ylmethyloxycarbonyl.

The term "arylalkoxycarbonylalkyl," as used herein, refers to an arylalkoxycarbonyl group, as defined herein, appended to the parent molecular moiety through an alkyl group, as defined herein. Representative examples of arylalkoxycarbonylalkyl include benzyloxycarbonylmethyl, 2-(benzyloxycarbonyl)ethyl, and 2-(naphth-2-ylmethyloxycarbonyl)ethyl.

The term "arylalkyl," as used herein, refers to an aryl group, as defined herein, appended to the parent molecular moiety through an alkyl group, as defined herein. Representative examples of arylalkyl include benzyl, 2-phenylethyl, 1,1-dimethyl-2-phenylethyl, 3-phenylpropyl, and 2-naphth-2-ylethyl.

The term "arylalkylthio," as used herein, refers to an arylalkyl group, as defined herein, appended to the parent molecular moiety through a thio moiety, as defined herein. Representative examples of arylalkylthio include 2-phenylethylthio, 3-naphth-2-ylpropylthio, and 5-phenylpentylthio.

The term "arylalkylthioalkyl," as used herein, refers to an arylalkylthio group, as defined herein, appended to the parent molecular moiety through an alkyl group, as defined herein. Representative examples of arylalkylthioalkyl include 2-phenylethylsulfanylmethyl, 3-naphth-2-ylpropylsulfanylmethyl, and 2-(5-phenylpentylsulfanyl)ethyl.

The term "arylcarbonyl," as used herein, refers to an aryl group, as defined herein, appended to the parent molecular moiety through a carbonyl group, as defined herein. Representative examples of arylcarbonyl include benzoyl, and naphthoyl.

The term "arylcarbonylalkyl," as used herein, refers to an arylcarbonyl group, as defined herein, appended to the parent molecular moiety through an alkyl group, as defined herein. Representative examples of arylcarbonylalkyl include 2-oxo-3-phenylpropyl, and 1,1-dimethyl-3-oxo-4-phenylbutyl.

The term "arylcarbonyloxy," as used herein, refers to an arylcarbonyl group, as defined herein, appended to the parent molecular moiety through an oxy moiety, as defined herein. Representative examples of arylcarbonyloxy include benzoyloxy, and naphthoyloxy.

The term "arylcarbonyloxyalkyl," as used herein, refers to an arylcarbonyloxy group, as defined herein, appended to the parent molecular moiety through an alkyl group, as defined herein. Representative examples of arylcarbonyloxyalkyl include benzoyloxymethyl, 2-(benzoyloxy)ethyl, and 2-(naphthoyloxy)ethyl.

The term "aryl(halo)alkyl," as used herein, refers to an aryl group and at least one halogen, as defined herein, appended to the parent molecular moiety through an alkyl group, as defined herein. Representative examples of aryl(halo)alkyl include dichloro(phenyl)methyl, 1,1-dichloro-2-phenylethyl, 1,1-difluoro-2-phenylethyl, 1,1-dichloro-3-phenylpropyl, and 1,1-difluoro-3-phenylpropyl.

The term "aryloxy," as used herein, refers to an aryl group, as defined herein, appended to the parent molecular moiety through an oxy moiety, as defined herein. Representative examples of aryloxy include phenoxy naphthyloxy, 3-bromophenoxy, 4-chlorophenoxy, 4-methylphenoxy, and 3,5-dimethoxyphenoxy.

The term "aryloxyalkyl," as used herein, refers to an aryloxy group, as defined herein, appended to the parent molecular moiety through an alkyl group, as defined herein. Representative examples of aryloxyalkyl include 2-phenoxyethyl, 3-naphth-2-yloxypropyl, and 3-bromophenoxymethyl.

The term "aryloxycarbonyl," as used herein, refers to an aryloxy group, as defined herein, appended to the parent molecular moiety through an oxy moiety, as defined herein. Representative examples of aryloxycarbonyl include phenoxycarbonyl, and haphthyloxycarbonyl.

The term "aryloxycarbonylalkyl," as used herein, refers to an aryloxycarbonyl group, as defined herein, appended to the parent molecular moiety through an alkyl group, as defined herein. Representative examples of aryloxycarbonylalkyl include phenoxycarbonylmethyl, 2-(phenoxycarbonyl)ethyl, and naphthyloxycarbonyl.

The term "arylsulfonyl," as used herein, refers to an aryl group, as defined herein, appended to the parent molecular moiety through a sulfonyl group, as defined herein. Representative examples of arylsulfonyl include naphthylsulfonyl, phenylsulfonyl, and 4-fluotrophenylsulfonyl.

The term "arylsulfonylalkyl," as used herein, refers to an arylsulfonyl group, as defined herein, appended to the parent molecular moiety through an alkyl group, as defined herein. Representative examples of arylsulfonylalkyl include 1,1-dimethyl-3-(phenylsulfonyl)propyl, naphthylsulfonylmethyl, 2-(phenylsulfonyl)ethyl, phenylsulfonylmethyl, and 4-fluorophenylsulfonylmethyl.

The term "carbonyl," as used herein, refers to a -C(O)- group.

The term "carboxy," as used herein, refers to a -CO₂H group.

The term "carboxyalkyl," as used herein, refers to a carboxy group, as defined herein, appended to the parent molecular moiety through an alkyl group, as defined herein. Representative examples of carboxyalkyl include carboxymethyl, 2-carboxyethyl, 3-carboxypropyl, and 3-carboxy-1,1-dimethylpropyl.

The term "carboxy(halo)alkyl," as used herein, refers to a carboxy group and at least one halogen, as defined herein, appended to the parent molecular moiety through an alkyl group, as defined herein. Representative examples of carboxy(halo)alkyl include carboxy(dichloro)ethyl, carboxy(difluoro)methyl, 2-carboxy-1,1-dichloroethyl, and 2-carboxy-1,1-difluoroethyl.

The term "cyano," as used herein, refers to a -CN group.

The term "cyanoalkyl," as used herein, refers to a cyano group, as defined herein, appended to the parent molecular moiety through an alkyl group, as defined herein. Representative examples of cyanoalkyl include cyanomethyl, 2-cyanoethyl, 3-cyanopropyl, 3-cyano-1,1-dimethylpropyl, and 3-cyano-1,1-diethylpropyl.

The term "cyano(halo)alkyl," as used herein, refers to a cyano group and at least one halogen, as defined herein, appended to the parent molecular moiety through an alkyl group, as defined herein. Representative examples of cyano(halo)alkyl include 3-cyano-1,1-difluoropropyl, 1,1 -dichloro-3-cyanopropyl, and 3-cyano-1,1-bis(trifluoromethyl)propyl.

The term "cycloalkenyl," as used herein, refers to a cyclic hydrocarbon containing from 3 to 8 carbons and containing at least one carbon-carbon double bond formed by the removal of two hydrogens. Representative examples of cycloalkenyl include cyclohexene, 1-cyclohexen-2-yl, 3,3-dimethyl-1-cyclohexene, cyclopentene, and cycloheptene.

The cycloalkenyl groups of this invention can be substituted with 1, 2, 3, 4, or 5 substituents independently selected from alkenyl, alkoxy, alkoxycarbonyl, alkoxycarbonylalkyl, alkyl, alkylcarbonyloxy, alkylcarbonyloxyalkyl, alkynyl, amido, amidoalkyl, halo, haloalkoxy, haloalkyl, hydroxy, hydroxyalkyl, sulfamylalkyl, -NR^{A}R^{B}, and (NR^{A}R^{B}) alkyl.

The term "cycloalkenylalkyl," as used herein, refers to a cycloalkenyl group, as defined herein, appended to the parent molecular moiety through an alkyl group, as defined herein. Representative examples of cycloalkenylalkyl include (2,6,6-trimethyl-1-cyclohexen-1-yl)methyl, 1-cyclohexen-1-ylmethyl, and 2-(2 cyclohepten-1-yl)ethyl.

The term "cycloalkyl," as used herein, refers to a monocyclic, bicyclic, or tricyclic ring system. Monocyclic ring systems are exemplified by a saturated cyclic hydrocarbon group containing from 3 to 8 carbon atoms. Examples of monocyclic ring systems include cyclopropyl, cyclobutyl, cyclopentyl, cyclohexyl, cycloheptyl, and cyclooctyl. Bicyclic ring systems are exemplified by a bridged monocyclic ring system in which two non-adjacent carbon atoms of the monocyclic ring are linked by an alkylene bridge of between one and three additional carbon atoms. Representative examples of bicyclic ring systems include bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Tricyclic ring systems are exemplified by a bicyclic ring system in which two non-adjacent carbon atoms of the bicyclic ring are linked by a bond or an alkylene bridge of between one and three carbon atoms. Representative examples of tricyclic-ring systems include tricyclo[3.3.1.0^{3,7}]nonane and tricyclo[3.3.1.1^{3,7}]decane (adamantane).

The cycloalkyl groups of this invention can be substituted with 1, 2, 3, 4, or 5 substituents independently selected from alkenyl, alkoxy, alkoxycarbonyl, alkoxycarbonylalkyl, alkyl, alkylcarbonylalkyl, alkylcarbonyloxy, alkylcarbonyloxyalkyl, alkylsulfonylalkyl, alkynyl, amido, amidoalkyl, cyanoalkyl, halo, haloalkoxy, haloalkyl, hydroxy, hydroxyalkyl, sulfamylalkyl, - NR^{A}R^{B}, and (NR^{A}R^{B})alkyl.

The term "cycloalkylalkoxy," as used herein, refers to a cycloalkyl group, as defined herein, appended to the parent molecular moiety through an alkoxy group, as defined herein. Representative examples of cycloalkylalkoxy include cyclopropylmethoxy, 2-cyclobutylethoxy, cyclopentylmethoxy, cyclohexylmethoxy, and 4-cycloheptylbutoxy.

The term "cycloalkylalkoxyalkyl," as used herein, refers to a cycloalkylalkoxy group, as defined herein, appended to the parent molecular moiety through an alkyl group, as defined herein. Representative examples of cycloalkylalkoxyalkyl include cyclopropylmethoxymethyl, 2-cyclobutylethoxymethyl, cyclopentylmethoxymethyl, 2-cyclohexylethoxymethyl, and 1-(4-cycloheptylbutoxy)ethyl.

The term "cycloalkylalkyl," as used herein, refers to a cycloalkyl group, as defined herein, appended to the parent molecular moiety through an alkyl group, as defined herein. Representative examples of cycloalkylalkyl include cyclopropylmethyl, 2-cyclobutylethyl, cyclopentylmethyl, cyclohexylmethyl and 4-cycloheptylbutyl.

The term "cycloalkylcarbonyl," as used herein, refers to a cycloalkyl group, as defined herein, appended to the parent molecular moiety through a carbonyl group, as defined herein. Representative examples of cycloalkylcarbonyl include cyclopropylcarbonyl, 2-cyclobutylcarbonyl, and cyclohexylcarbonyl.

The term "cycloalkyloxy," as used herein, refers to a cycloalkyl group, as defined herein, appended to the parent molecular moiety through an oxy moiety, as defined herein. Representative examples of cycloalkyloxy include cyclohexyloxy, and cyclopentyloxy.

The term "cycloalkyloxyalkyl," as used herein, refers to a cycloalkyloxy group, as defined herein, appended to the parent molecular moiety through an alkyl group, as defined herein. Representative examples of cycloalkyloxyalkyl include 4-(cyclohexyloxy)butyl, and cyclohexyloxymethyl.

The term "cycloalkylalkylthio," as used herein, refers to a cycloalkylalkyl group, as defined herein, appended to the parent molecular moiety through a thio moiety, as defined herein. Representative examples of cycloalkylalkylthio include (2 cyclohexylethyl)sulfanyl, and cyclohexylmethylsulfanyl.

The term "cycloalkylalkylthioalkyl," as used herein, refers to a cycloalkylalkylthio group, as defined herein, appended to the parent molecular moiety through an alkyl group, as defined herein. Representative examples of cycloalkylalkylthioalkyl include 2-[(2-cyclohexylethyl)sulfanyl]ethyl, and (2-cyclohexylethyl)sulfanylmethyl.

The term "formyl," as used herein, refers to a -C(O)H group.

The term "halo" or "halogen," as used herein, refers to -Cl, -Br, -I or -F.

The term "haloalkoxy," as used herein, refers to at least one halogen, as defined herein, appended to the parent molecular moiety through an alkoxy group, as defined herein. Representative examples of haloalkoxy include chloromethoxy, 2-fluoroethoxy, 1,2-difluoroethoxy, trifluoromethoxy, and pentafluoroethoxy.

The term "haloalkenyl," as used herein, refers to at least one halogen, as defined herein, appended to the parent molecular moiety through an alkenyl group, as defined herein. Representative examples of haloalkenyl include 2,2-dichloroethenyl, 2,2-difluoroethenyl, and 5-chloropenten-2-yl.

The term "haloalkyl," as used herein, refers to at least one halogen, as defined herein, appended to the parent molecular moiety through an alkyl group, as defined herein. Representative examples of haloalkyl include chloromethyl, trichloromethyl, 1,1-dichloroethyl, 2-fluoroethyl, trifluoromethyl, 2,2,2-trifluoroethyl, 2,2,2-trifluoro-1-(trifluoromethyl)-1-(methyl)ethyl, pentafluoroethyl, and 2-chloro-3-fluoropentyl.

The term "haloalkylcarbonyl," as used herein, refers to a haloalkyl group, as defined herein, appended to the parent molecular moiety through a carbonyl group, as defined herein. Representative examples of haloalkylcarbonyl include chlommethylcarbonyl, trichloromethylcarbonyl, and trifluoromethylcarbonyl.

The term "haloalkylsulfonyl," as used herein, refers to a haloalkyl group, as defined herein, appended to the parent molecular moiety through a sulfonyl group, as defined herein. Representative examples of haloalkylsulfonyl include chloromethylsulfonyl, trichloromethylsulfonyl, and trifluoromethylsulfonyl.

The term "haloalkynyl," as used herein, refers to at least one halogen, as defined herein, appended to the parent molecular moiety through an alkynyl group, as defined herein. Representative examples of haloalkynyl include 4,4,4-trichlorobutyn-2-yl.

The term "heterocycle," as used herein, refers to a monocyclic or a bicyclic ring system. Monocyclic ring systems are any 5 or 6 membered ring containing 1, 2, 3, or 4 heteroatoms independently selected from oxygen, nitrogen and sulfur. The 5-membered ring has from 0-2 double bonds and the 6-membered ring has from 0-3 double bonds. Representative examples of monocyclic ring systems include azetidine, azepine, aziridine, diazepine, 1,3-dioxolane, dioxane, 1,3-dioxane, dithiane, furan, imidazole, imidazoline, imidazolidine, isothiazole, isothiazoline, isothiazolidine, isoxazole, isoxazoline, isoxazolidine, morpholine, oxadiazole, oxadiazoline, oxadiazolidine, oxazole, oxazoline, oxazolidine, piperazine, piperidine, pyran, pyrazine, pyrazole, pyrazoline, pyrazolidine, pyridine, pyrimidine, pyridazine, pyrrole, pyrroline, pyrrolidine, tetrahydrofuran, tetrahydrothiophene, tetrazine, tetrazole, thiadiazole, thiadiazoline, thiadiazolidine, thiazole, thiazoline, thiazolidine, thiophene, thiomorpholine, thiomorpholine sulfone, thiopyran, triazine, triazole, and trithiane. Bicyclic ring systems are any of the above monocyclic ring systems fused to an aryl group as defined herein, a cycloalkyl group as defined herein, or another monocyclic ring system as defined herein. Representative examples of bicyclic ring systems include for example, benzimidazole, benzothiazole, benzothiadiazole, benzothiophene, benzoxadiazole, benzoxazole, benzofuran, benzopyran, benzothiopyran, benzotriazole, benzodioxine, 1,3-benzodioxole, cinnoline, indazole, indole, indoline, indolizine, naphthyridine, isobenzofuran, isobenzothiophene, isoindole, isoindoline, 1-isoindolinone, isoquinoline, 1-isoquinolinone, phthalazine, pyranopyridine, quinoline, quinolizine, quinoxaline, quinazoline, tetrahydroisoquinoline, tetrahydroquinoline, and thiopyranopyridine.

The heterocycle groups of this invention can be substituted with 1, 2, or 3 substituents independently selected from alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, oxo, sulfamyl, sulfamylalkyl, -NR^{A}R^{B}, (NR^{A}R^{B})alkyl, and benzene optionally substituted with 1 or 2 substituents independently selected from alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, -NR^{A}R^{B}, and (NR^{A}R^{B})alkyl.

The term "heterocyclealkoxy," as used herein, refers to a heterocycle group, as defined herein, appended to the parent molecular moiety through an alkoxy group, as defined herein. Representative examples of heterocyclealkoxy include 2-pyrid-3-ylethoxy, 3-quinolin-3-ylpropoxy, and 5-pyrid-4-ylpentyloxy.

The term "heterocyclealkoxyalkyl," as used herein, refers to a heterocyclealkoxy group, as defined herein, appended to the parent molecular moiety through an alkyl group, as defined herein. Representative examples of heterocyclealkoxyalkyl include 2-pyrid-3-ylethoxymethyl, 2-(3-quinolin-3-ylpropoxy)ethyl, and 5-pyrid-4-ylpentyloxymethyl.

The term "heterocyclealkyl," as used herein, refers to a heterocycle, as defined herein, appended to the parent molecular moiety through an alkyl group, as defined herein. Representative examples of heterocyclealkyl include pyrid-3-ylmethyl, and pyrimidin-5-ylmethyl.

The term "heterocyclealkylthio," as used herein, refers to a heterocyclealkyl group, as defined herein, appended to the parent molecular moiety through a thio moiety, as defined herein. Representative examples of heterocyclealkylthio include 2-pyrid-3-ylethysulfanyl, 3-quinolin-3-ylpropysulfanyl, and 5-pyrid-4-ylpentylsulfanyl.

The term "heterocyclealkylthioalkyl," as used herein, refers to a heterocyclealkylthio group, as defined herein, appended to the parent molecular moiety through an alkyl group, as defined herein. Representative examples of heterocyclealkylthioalkyl include 2-pyrid-3-ylethysulfanylmethyl, 2-(3-quinolin-3-ylpropysulfanyl)ethyl, and 5-pyrid-4-ylpentylsulfanylmethyl.

The term "heterocyclecarbonyl," as used herein, refers to a heterocycle, as defined herein, appended to the parent molecular moiety through a carbonyl group, as defined herein. Representative examples of heterocyclecarbonyl include pyrid-3-ylcarbonyl, quinolin-3-ylcarbonyl, and thiophen-2-ylcarbonyl.

The term "heterocycleoxy," as used herein, refers to a heterocycle group, as defined herein, appended to the parent molecular moiety through an oxy moiety, as defined herein. Representative examples of heterocycleoxy include pyrid-3-yloxy, and quinolin-3-yloxy.

The term "heterocycleoxyalkyl," as used herein, refers to a heterocycleoxy group, as defined herein, appended to the parent molecular moiety through an alkyl group, as defined herein. Representative examples of heterocycleoxyalkyl include pyrid-3-yloxymethyl, and 2-quinolin-3-yloxyethyl.

The term "hydroxy," as used herein, refers to an -OH group.

The term "hydroxyalkyl," as used herein, refers to 1 or 2 hydroxy groups, as defined herein, appended to the parent molecular moiety through an alkyl group, as defined herein. Representative examples of hydroxyalkyl include hydroxymethyl, 2-hydroxyethyl, 3-hydroxyproyl, 2,3-dihydroxypropyl, 2-ethyl-4-hydroxyheptyl, 2-hydroxy-1,1-dimethylethyl, and 3-hydroxy-1,1-dimethylpropyl.

The term "Lewis acid," as used herein, refers to a chemical species that has a vacant orbital or can accept an electron pair. Representative examples of Lewis acid include aluminum chloride, boron trifluoride, iron(II) chloride, iron(III) chloride, magnesium bromide, magnesium chloride, magnesium trifluoromethanesulfonate, manganese(II) chloride, titanium(IV) isopropoxide, zinc bromide, zinc chloride, and zirconium (IV) chloride.

The term "lower alkyl," as used herein, is a subset of alkyl as defined herein and refers to a straight or branched chain hydrocarbon group containing from 1 to 6 carbon atoms. Representative examples of lower alkyl include methyl, ethyl, n-propyl, iso-propyl, n-butyl, iso-butyl, and, tert-butyl.

The term "mercapto," as used herein, refers to a -SH group.

The term "mercaptoalkyl," as used herein, refers to a mercapto group, as defined herein, appended to the parent molecular moiety through an alkyl group, as defined herein. Representative examples of mercaptoalkyl include 2-sulfanylethyl, and 3-sulfanylpropyl.

The term "-NR⁹R¹⁰," as used herein, refers to two groups, R⁹ and R¹⁰, which are appended to the parent molecular moiety through a nitrogen atom. R⁹ and R¹⁰ are independently selected from hydrogen, alkyl, alkylcarbonyl, aryl, arylalkyl, arylcarbonyl, formyl, and S(O)₂R¹¹, as defined herein, wherein R¹¹ is selected from alkyl, aryl, and arylalkyl, as defined herein. Representative examples of -NR⁹R¹⁰ include acetylamino, amino, methylamino, (ethylcarbonyl)methylamino, ethylmethylamino, formylamino, methylsulfonylamino, phenylsulfonylamino, and benzylsulfonylamino.

The term "(NR⁹R¹⁰)alkyl," as used herein, refers to a -NR⁹R¹⁰ group, as defined herein, appended to the parent molecular moiety through an alkyl group, as defined herein. Representative examples of (NR⁹R¹⁰)alkyl include acetylaminomethyl, aminomethyl, 2-aminoethyl, 2-(methylamino)ethyl, (ethylcarbonyl)methylaminomethyl, 3-(ethylmethylamino)propyl, 1,1-dimethyl-3-(dimethylamino)propyl, 2-(formylamino)ethyl, methylsulfonylaminomethyl, 2-(phenylsulfonylamino)ethyl, and benzylsulfonylaminomethyl.

The term "-NR^{A}R^{B}," as used herein, refers to two groups, R^{A} and R^{B}, which are appended to the parent molecular moiety through a nitrogen atom. R^{A} and R^{B} are independently selected from hydrogen, alkyl, alkylcarbonyl and formyl, as defined herein. Representative examples of -NR^{A}R^{B} include acetylamino, amino, methylamino, (ethylcarbonyl)methylamino, dimethylamino, ethylmethylamino, and formylamino,

The term "(NR^{A}R^{B})alkyl," as used herein, refers to a -NR^{A}R^{B} group, as defined herein, appended to the parent molecular moiety through an alkyl group, as defined herein. Representative examples of (NR^{A}R^{B})alkyl include acetylaminomethyl, aminomethyl, 2-aminoethyl, 2-(methylamino)ethyl, (ethylcarbonyl)methylaminomethyl, 3-(ethylmethylamino)propyl, 1,1-dimethyl-3-(dimethylamino)propyl, and 2-(formylamino)ethyl.

The term "nitro," as used herein, refers to a -NO₂ group.

The term "oxo," as used herein, refers to a (=O) moiety.

The term "oxy," as used herein, refers to a (-O-) moiety.

The term "sulfamyl," as used herein, refers to a -SO₂NR⁹⁴R⁹⁵ group, wherein R⁹⁴ and R⁹⁵ are independently selected from hydrogen, alkyl, aryl, and arylalkyl, as defined herein. Representative examples of sulfamyl include aminosulfonyl, methylaminosulfonyl, dimethylaminosulfonyl, phenylaminosulfonyl, benzylaminosulfonyl.

The term "sulfamylalkyl," as used herein, refers to a sulfamyl group, as defined herein, appended to the parent molecular moiety through an alkyl group, as defined herein. Representative examples of sulfamylalkyl include (aminosulfonyl)methyl, (dimethylaminosulfonyl)methyl, 2-(aminosulfonyl)ethyl, 3-(minosulfonyl)propyl, and 3-aminosulfonyl-1,1-dimethylpropyl.

The term "sulfamyl(halo)alkyl," as used herein, refers to a sulfamyl group and at least one halogen, as defined herein, appended to the parent molecular moiety through an alkyl group, as defined herein. Representative examples of sulfamyl(halo)alkyl include (aminosulfonyl)dichloromethyl, (aminosulfonyl)difluoromethyl, (dimethylaminosulfonyl)difluoromethyl, 2-(aminosulfonyl)-1,1-dichloroethyl,3-(aminosulfonyl)-1,1-difluoropropyl, 3-aminosulfonyl-1,1-dichloropropyl, and 3-(aminosulfonyl)-1,2-difluoropropyl.

The term "sulfinyl," as used herein, refers to a -S(O)- group.

The term "sulfonyl," as used herein, refers to a -SO₂- group.

The term "tautomer," as used herein, refers to a proton shift from one atom of a molecule to another atom of the same molecule.

The term "thio," as used herein, refers to a (-S-) moiety.

Compounds of the present invention may exist as stereoisomers wherein, asymmetric or chiral centers are present. These stereoisomers are "R" or "S" depending on the configuration of substituents around the chiral carbon atom. The terms "R" and "S" used herein are configurations as defined in IUPAC 1974 Recommendations for Section E, Fundamental Stereochemistry, Pure Appl. Chem., 1976, 45: 13-30. The present invention contemplates various stereoisomers and mixtures thereof and are specifically included within the scope of this invention. Stereoisomers include enantiomers and diastereomers, and mixtures of enantiomers or diastereomers. Individual stereoisomers of compounds of the present invention may be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

Tautomers may exist in the compounds of the present invention and are specifically included within the scope of the present invention. The present invention contemplates tautomers due to proton shifts from one atom to another atom of the same molecule generating two or more compounds that are in equilibrium with each other. An example of tautomers of the present invention includes 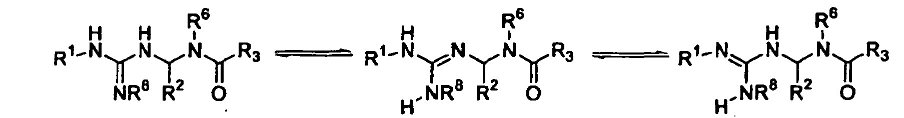 wherein R¹, R², R³, and R⁸ are as defined in formula and R⁶ is H.

Syn and anti geometric isomers and mixtures thereof may also exist in the compounds of the present invention. Syn and anti geometric isomers and mixtures thereof are specifically included within the scope of this invention. An example of syn and anti geometric isomers of the present invention includes 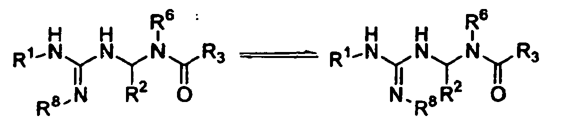 wherein R¹, R², R³, and R⁸ are as defined in formula I and R⁶ is H.

Preferred compounds of formula I include,
4-chloro-N-(1-{[(hydroxyimino)(3-pyridinylamino)methyl]amino}-2,2-dimethylpropyl)benzamide;
4-chloro-N-(1-{[(methoxyimino)(3-pyridinylamino)methyl]amino}-2,2-dimethylpropyl)benzamide;
4-chloro-N-(1-{[{[(4-fluorobenzyl)oxy]imino}(3-pyridinylamino)methyl]amino}-2,2-dimethylpropyl)benzamide;
4-chloro-N-(2,2-dimethyl-1-{[[(methylsulfonyl)imino](3-pyridinylamino)methyl]amino}propyl)benzamide;
3-(4-chlorophenylr)-N-(1-{[(cyanoimino)(3-pyridinylamino)methyl]amino}-2,2-dimethylpropyl)propanamide;
N-(1-{[(cyanoimino)(3-pyridinylamino)methyl]amino}-2,2-dimethylpropyl)-3-phenylpropanamide;
N-(1-{[(cyanoimino)(3-pyridinylamino)methyl]amino)-2,2-dimethylpropyl)-2-phenylacetamide;
4-(aminosulfonyl)-N-(1-{[(cyanoimino)(3-pyridinylamino)methyl]amino}-2,2-dimethylpropyl)-2-fluorobenzamide;
4-chloro-N-[1-({(cyanoimino)[(4-ethyl-3-pyridinyl)amino]methyl}amino)-2,2-dimethylpropyl]benzamide;
N-(1-{[(cyanoimino)(3-pyridinylamino)methyl]amino}-2,2-dimethylpropyl)-4-(trifluoromethoxy)benzamide;
4-chloro-N-[1-({(cyanoimino)[(4-ethyl-3-pyridinyl)amino]methyl}amino)-2,2-dimethylpropyl]-2-fluorobenzamide;
4-chloro-N-(1-{[(cyanoimino)(5-pyrimidinylamino)methyl]amino}-2,2-dimethylpropyl)benzamide;
4-chloro-N-(1-{[(cyanoimino)(5-pyrimidinylamino)methyl]amino}-2,2-dimethylpropyl)-2-fluorobenzamide;
N-(1-{[[(4-bromo-3-pyridinyl)amino](cyanoimino)methyl]amino}-2,2-dimethylpropyl)-4-chlorobenzamide;
4-chloro-2-fluoro-N-[2,2,2-trichloro-1-({(cyanoimino)[(4-ethyl-3-pyridinyl)amino]methyl}amino)ethyl]benzamide;
4-chloro-N-(2,2,2-trichloro-1-{[(cyanoimino)(5-pyrimidinylamino)methyl]amino}ethyl)benzamide;
4-chloro-2-fluoro-N-(2,2,2-trichloro-1-{ [(cyanoimino)(5-pyrimidinylamino)methyl]amino}ethyl)benzamide;
N-(1- {[[(4-bromo-3-pyridinyl)amino](cyanoimino)methyl]amino}-2,2,2-trichloroethyl)-4-chlorobenzamide;
N-(1- {[[(2-bromo-3-pyridinyl)amino](cyanoimino)methyl]amino)-2,2-dimethylpropyl)-4-chlorobenzamide;
4-chloro-N-[1-({(cyanoimino)[(2-ethyl-3-pyridinyl)amino]methyl }amino)-2,2-dimethylpropyl]benzamide;
N-(1-{[[(5-bromo-4-ethyl-3-pyridinyl)amino](cyanoimino)methyl]amino}-2,2-dimethylpropyl)-4-chlorobenzamide;
4-chloro-N-[1-({(cyanoimino)[(4,5-dibromo-3-pyridinyl)amino]methyl}amino)-2,2-dimethylpropyl]benzamide;
4-chloro-N-(1-{[[(5-chloro-3-pyridinyl)amino](cyanoimino)methyl]amino}-2,2-dimethylpropyl)benzamide;
N-(1-{[[(5-bromo-6-chloro-3-pyridinyl)amino](cyanoimino)methyl]amino}-2,2-dimethylpropyl)-4-chlorobenzamide;
N-(1-{[[(5-bromo-3-pyridinyl)amino](cyanoimino)methyl]amino}-2,2-dimethylpropyl)-4-chlorobenzamide;
N-(1-{[[(6-bromo-3-pyridinyl)amino](cyanoimino)methyl]amino}-2,2-dimethylpropyl)-4-chlorobenzamide;
4-chloro-N-(1-{[(cyanoimino)({5-[(4-fluorophenyl)sulfonyl]-3-pyridinyl}amino)methyl]amino}-2,2-dimethylpropyl)benzamide;
N-(1-{[({5-[(aminoperoxy)sulfanyl]-3-pyridinyl}amino)(cyanoimino)methyl]amino}-2,2-dimethylpropyl)-4-chlorobenzamide;
N-(1-{[[(6-bromo-4-fluoro-3-pyridinyl)amino](cyanoimino)methyl]amino}-2,2-dimethylpropyl)-4-chlorobenzamide;
4-chloro-N-[1-{[(cyanoimino)(3-pyridinylamino)methyl]amino}-2,2,2-trifluoro-1-(trifluoromethyl)ethyl]benzamide;
4-chloro-N-(1- {[(cyanoimino)(3-pyridinylamino)methyl]amino}cyclopentyl)benzamide;
4-chloro-N-(1-{ [(cyanoimino)(3-pyridinylamino)methyl]amino}cyclohexyl)benzamide;
4-chloro-N-[{[(cyanoimino)(3-pyridinylamino)methyl]amino}(2,6-dimethylphenyl)methyl]benzamide;
4-chloro-N-[{[(cyanoimino)(3-pyridinylamino)methyl]amino}(3-pyridinyl)methyl]benzamide;
4-chloro-N-[ [{[(cyanoimino)(3-pyridinylamino)methyl]amino}(2-pyridinyl)methyl]benzamide;
4-chloro-N-(1- {[(cyanoimino)(3-pyridinylamino)methyl]amino}-2-methyl-2-phenylpropyl)benzamide;
4-chloro-N-(1- {[(cyanoimino)(3-pyridinylamino)methyl]amino}-3,3-dimethyl-2-oxobutyl)benzamide;
4-chloro-N-(1-{[(cyanoimino)(3-pyridinylamino)methyl]amino}-3,3,3-trifluoro-2-oxopropyl)benzamide;
4-chloro-N-[1-{[(cyanoimino)(3-pyridinylamino)methyl]amino}-3,3,3-trifluoro-2-methyl-2-(trifluoromethyl)propyl]benzamide;
methyl 4-[(4-chlorobenzoyl)amino]-4-{[(cyanoimino)(3-pyridinylamino)methyl]amino}-3,3-dimethylbutanoate;
4-chloro-N-[1-{[(cyanoimino)(3-pyridinylamino)methyl]amino}-4-(dimethylamino)-2,2-dimethylbutyl]benzamide;
4-chloro-N-(4-cyano-1-{[(cyanoimino)(3-pyridinylamino)methyl]amino}-2,2-dimethylbutyl)benzamide;
4-chloro-N -(1-{[(cyanoimino)(3-pyridinylamino)methyl]amino}-4-methoxy-2,2-dimethylbutyl)benzamide;
4-chloro-N-(1-{[(cyanoimino)(3-pyridinylamino)methyl)amino}-4-hydroxy-2,2-dimethylbutyl)benzamide;
N-(4-(aminosulfonyl)-1-{[(cyanoimino)(3-pyridinylamino)methyl]amino}-2,2-dimethylbutyl)-4-chlorobenzamide;
4-chloro-N-[1-{[(cyanoimino)(3-pyridinylamino)methyl]amino}-2,2-dimethyl-4-(phenylsulfonyl)butyl]benzamide;
4-chloro-N-(1-{[(cyanoimino)(3-pyridinylamino)methyl]amino}-3-hydroxy-2,2-dimethylpropyl)benzamide;
N-(4-(aminosulfonyl)-2,2-dichloro-1-{ [(cyanoimino)(3-pyridinylamino)methyl]amino }butyl)-4-chlorobenzamide;
4-chloro-N-[4-cyano-1- {[(cyanoimino)(3-pyridinylamino)methyl]amino} -2,2-bis(trifluoromethyl)butyl]benzamide;
4-chloro-N-(1-{[(cyanoimino)(3-pyridinylamino)methyl]amino) }-2,2-difluoro-4-oxopentyl)benzamide;
4-chloro-N-(1-{[2-cyano-1-(3-pyridinylamino)ethenyl]amino -2,2-dimethylpropyl)benzamide;
4-chloro-N-{ 1-[[(cyanoimino)(3-pyridinylamino)methyl](hydroxy)amino]-2,2-dimethylpropyl}benzamide;
4-chloro-N-(2,2,2-trichloro-1-{[2-nitro-1-(3-pyridinylamino)ethenyl]amino}ethyl)benzamide;
4-chloro-N-(2,2,2-trichloro-1-{ [2-cyano-1-(3-pyridinylamino)ethenyl]amino}ethyl)benzamide and pharmaceutically acceptable salts, amides, esters, or produrgs thereof.

More preferred compounds of formula I include,
4-methyl-N-(2,2,2-trifluoro-1-{[(3-pyridinylamino)carbothioyl]amino }ethyl)benzamide;
4-methyl-N-{2,2,2-trifluoro-1-[(2-toluidinocarbothioyl)amino]ethyl}benzamide;
4-methyl-N-(2,2,2-trifluoro-1-{[(4-fluoroanilino)carbothioyl]amino }ethyl)benzamide;
4-methyl-N-(2,2,2-trifluoro-1-{ [(3-nitroanilino)carbothioyl]amino}ethyl)benzamide;
4-methyl-N-[2,2,2-trifluoro-1-({[2-fluoro-3-(trifluoromethyl)anilino]carbothioyl}amino)ethyl]benzamide;
4-methyl-N-(2,2,2-trifluoro-1-{[(4-methoxyanilino)carbothioyl]amino} ethyl)benzamide;
N-[1-({[(6-chloro-3-pyridinyl)amino]carbothioyl}amino)-2,2,2-trifluoroethyl]-4-methylbenzamide;
4-methyl-N-(2,2,2-trifluoro-1-{[(2-methoxyanilino)carbothioyl]amino}ethyl)benzamide;
N-{1-[(anilinocarbothioyl)amino]-2,2,2-trifluoroethyl}-4-methylbenzamide;
4-methyl-N-{2,2,2-trifluoro-1-[(4-toluidinocarbothioyl)amino]ethyl }benzamide;
4-methyl-N-(2,2,2-trifluoro-1-{[(2-fluoroanilino)carbothioyl]amino}ethyl)benzamide;
4-methyl-N-(2,2,2-trifluoro-1- {[(3-methoxyanilino)carbothioyl]amino}ethyl)benzamide;
4-methyl-N-(2,2,2-trifluoro-1-{ {[(3-fluoroanilino)carbothioyl]amino}ethyl)benzamide;
N-(1- [(2,5-difluoroanilino)carbothioyl]amino }-2,2,2-trifluoroethyl)-4-methylbenzamide;
N-(1-{[(2,4-difluoroanilino)carbothioyl]amino}-2,2,2-trifluoroethyl)-4-methylbenzamide;
4-methyl-N-{2,2,2-trifluoro-1-[(3-toluidinocarbothioyl)amino]ethyl benzamide;
N-(1-{[(2,6-difluoroanilino)carbothioyl]amino}-2,2,2-trifluoroethyl)-4-methylbenzamide;
N-(1-{[(2,3-difluoroanilino)carbothioyl]amino -2,2,2-trifluoroethyl)-4-methylbenzamide;
4-chloro-N-(2,2,2-trifluoro-1-{[(3-pyridinylamino)carbothioyl]amino}ethyl)benzamide;
N- {1-[(anilinocarbothioyl)amino]-2,2,2-trifluoroethyl -4-chlorobenzamide;
4-chloro-N-(2,2,2-trifluoro-1-{[(2-fluoroanilino)carbothioyl]amino}ethyl)benzamide;
N-(2,2-dimethyl-1- {[(3-pyridinylamino)carbothioyl]amino}propyl)-4-methylbenzamide;
N-((1R)-2,2-dimethyl-1-{[(3-pyridinylamino)carbothioyl]amino}propyl)-4-methylbenzamide;
N-((1 S)-2,2-dimethyl-1-{ [(3-pyridinylamino)carbothioyl]amino}propyl)-4-methylbenzamide;
N-(2,2-dimethyl-1- ([(3-nitroanilino)carbothioyl]amino}propyl)-4-methylbenzamide;
N-(2,2-dimethyl-1-{[(3-pyridinylamino)carbothioyl]amino}propyl)-2-methylbenzamide;
4-chloro-N-(2,2-dimethyl-1-{[(3-pyridinylamino)carbothioyl]amino}propyl)benzamide;
N-(2,2-dimethyl-1-{[(3-pyridinylamino)carbothioyl]amino}propyl)benzamide;
4-methyl-N-(1-{[(3-nitroanilino)carbothioyl]amino}ethyl)benzamide;
4-methyl-N-(1-{[(3-nitroanilino)carbothioyl]amino}-2-phenylethyl)benzamide;
N-((1R)-2-(tert-butoxy)-1-{[(3-nitroanilino)carbothioyl]amino}ethyl)-4-methylbenzamide;
N-(2-fluoro-1-{[(3-nitroanilino)carbothioyl]amino}ethyl)-4-methylbenzamide;
4-methyl-N-[{[(3-nitroanilino)carbothioyl]amino}(phenyl)methyl]benzamide;
4-methyl-N-(phenyl { [(3-pyridinylamino)carbothioyl]amino} methyl)benzamide;
4-methyl-N-(2-methyl-1-{ [(3-pyridinylamino)carbothioyl]amino}propyl)benzamide;
4-methyl-N-((1R,2S)-2-methyl-1-{[(3-pyridinylamino)carbothioyl]amino }butyl)benzamide;
4-methyl-N- {2,2,2-trichloro-1-[3-(3-fluorophenyl)-2-thioxo-1-imidazolidinyl]ethyl benzamide;
4-methyl-N-(2,2,2-trichloro-1-{ [(3-pyridinylamino)carbonyl]amino}ethyl)benzamide;
2-methyl-N-(2,2,2-trichloro-1-{[(3-pyridinylamino)carbonyl]amino}ethyl)benzamide;
N-(2,2,2-trichloro-1-{[(3-pyridinylamino)carbonyl]amino}ethyl)benzamide;
4-chloro-N-(2,2,2-trichloro-1-{[(3-pyridinylamino)carbonyl]amino}ethyl)benzamide;
N-{1-[(anilinocarbonyl)amino]-2,2,2-trichloroethyl}-4-methylbenzamide;
4-methyl-N-(2,2,2-trichloro-1-{[(2-fluoroanilino)carbonyl]amino}ethyl)benzamide;
4-methyl-N-(2,2,2-trichloro-1-{[(cyanoimino)(3-pyridinylamino)methyl]amino}ethyl)benzamide;
4-chloro-N-(2,2,2-trichloro-1-{[(cyanoimino)(3-pyridinylamino)methyl]amino}ethyl)benzamide;
N-(1-{[anilino(cyanoimino)methyl]amino) -2,2,2-trichloroethyl)-4-methylbenzamide;
4-methyl-N-(2,2,2-trichloro-1-{[(cyanoimino)(2-fluoroanilino)methyl]amino}ethyl)benzamide;
4-methyl-N-(2,2,2-trichloro-1-{ [(cyanoimino)(5-pyrimidinylamino)methyl]amino}ethyl)benzamide;
N-(2,2,2-trichloro-1-{ [(cyanoimino)(3-pyridinylamino)methyl]amino}ethyl)benzamide;
2-methyl-N-(2,2,2-trichloro-1-{[(cyanoimino)(3-pyridinylamino)methyl]amino}ethyl)benzamide;
N-(1-{[(cyanoimino)(3-pyridinylamino)methyl]amino}-2,2-dimethylpropyl)-4-methylbenzamide;
4-chloro-N-(1-{[(cyanoimino)(3-pyridinylamino)methyl]amino}-2,2-dimethylpropyl)benzamide;
N-(1-{ [(cyanoimino)(3-fluoroanilino)methyl]amino }-2,2-dimethylpropyl)-4-methylbenzamide;
4-chloro-N-[{[(cyanoimino)(3-pyridinylamino)methyl]amino} (cyclopropyl)methyl]benzamide;
N-(1-{[[(6-chloro-3-pyridinyl)amino](cyanoimino)methyl]amino}-2,2-dimethylpropyl)-4-methylbenzamide;
4-chloro-N-[{[(cyanoimino)(3-fluoroanilino)methyl)amino}(3-thienyl)methyl]benzamide;
(-)-N-(1-{[(cyanoimino)(3-pyridinylamino)methyl]amino}-2,2-dimethylpropyl)-4-methylbenzamide;
(+)-N-(1-{[(cyanoimino)(3-pyridinylamino)methyl]amino}-2,2-dimethylpropyl)-4-methylbenzamide;
4-chloro-N-(1-{[(cyanoimino)(3-pyridinylamino)methyl]amino}-2-ethylbutyl)benzamide;
4-chloro-N-(1-{[(cyanoimino)(3-pyridinylamino)methyl]amino}-3-methylbutyl)benzamide;
4-chloro-N-[{[(cyanoimino)(3-pyridinylamino)methyl]amino}(cyclohexyl)methyl]benzamide;
4-chloro-N-(1-{[(cyanoimino)(3-pyridinylamino)methyl]amino}-3,3-dimethylbutyl)benzamide;
4-chloro-N-(1-{[(cyanoimino)(3-pyridinylamino)methyl]amino}-2-methylpropyl)benzamide;
4-chloro-N-(1-{[(cyanoimino)(3-pyridinylamino)methyl]amino}-2,2,2-trifluoroethyl)benzamide;
4-chloro-N-(4-cyano-1- {[(cyanoimino)(3-pyridinylamino)methyl]amino }-2,2-diethylbutyl)benzamide;
4-chloro-N-[1-{[(cyanoimino)(3-pyridinylamino)methyl]amino}-2-(2,6,6-trimethyl-1-cyclohexen-1-yl)ethyl]benzamide;
4-chloro-N-(1-{[(cyanoimino)(3-pyridinylamino)methyl]amino}-2,2-dimethyl-4-pentenyl)benzamide;
4-chloro-N-(2-ethyl-1-{ [2-nitro-1-(3-pyridinylamino)ethenyl]amino}butyl)benzamide;
4-chloro-N-(1-{[(cyanoimino)(2-fluoroanilino)methyl]amino}-2,2-dimethylpropyl)benzamide;
4-chloro-N-(1-{[(cyanoimino)(3-fluoroanilino)methyl]amino}-2,2-dimethylpropyl)benzamide;
4-chloro-N-[1-({(cyanoimino)[3-(trifluoromethyl)anilino]methyl}amino)-2,2-dimethylpropyl]benzamide;
4-chloro-N-(1-{[(cyanoimino)(3,5-difluoroanilino)methyl]amino}-2,2-dimethylpropyl)benzamide;
4-chloro-N-(1-{[(cyanoimino)(2,5-difluoroanilino)methyl]amino }-2,2-dimethylpropyl)benzamide;
4-chloro-N-(1-{[(cyanoimino)(2,6-difluoroanilino)methyl]amino}-2,2-dimethylpropyl)benzamide;
4-chloro-N-(1-{[(cyanoimino)(3-chloroanilino)methyl]amino }-2,2-dimethylpropyl)benzamide;
4-chloro-N-(1-{[(cyanoimino)(3-methoxyanilino)methyl]amino}-2,2-dimethylpropyl)benzamide;
4-chloro-N-(1-{ [[(2-chlorobenzyl)amino](cyanoimino)methyl]amino -2,2-dimethylpropyl)benzamide;
4-chloro-N-(1-{[[(3-chlorobenzyl)amino](cyanoimino)methyl]amino}-2,2-dimethylpropyl)benzamide;
4-chloro-N-(1-{[[(4-chlorobenzyl)amino](cyanoimino)methyl]amino}-2,2-dimethylpropyl)benzamide;
4-chloro-N-[1-({(cyanoimino)[(3-pyridinylmethyl)amino]methyl}amino)-2,2-dimethylpropyl]benzamide;
4-chloro-N-[1-({(cyanoimino)[(4-pyridinylmethyl)amino]methyl}amino)-2,2-dimethylpropyl]benzamide;
4-chloro-N-[1-({(cyanoimino)[(2-pyridinylmethyl)amino]methyl}amino)-2,2-dimethylpropyl]benzamide;
4-chloro-N-(1-{[(cyanoimino)(3-quinolinylamino)methyl]amino}-2,2-dimethylpropyl)benzamide;
4-chloro-N-(1-{[(cyanoimino)(3-pyridinylamino)methyl]amino}propyl)benzamide;
4-chloro-N-({[(cyanoimino)(3-pyridinylamino)methyl]amino)methyl)benzamide;
(-) 4-chloro-N-(4-cyano-1-{[(cyanoimino)(3-pyridinylamino)methyl]amino}-2,2-diethylbutyl)benzamide;
(+) 4-chloro-N-(4-cyano-1-{[(cyanoimino)(3-pyridinylamino)methyl]amino)-2,2-diethylbutyl)benzamide;
(+) 4-chloro-N-[1-{[(cyanoimino)(3-pyridinylamino)methyl]amino}-2-(2,6,6-trimethyl-1-cyclohexen-1-yl)ethyl]benzamide;
(-) 4-chloro-N-[1-{[(cyanoimino)(3-pyridinylamino)methyl]amino}-2-(2,6,6-trimethyl-1-cyclohexen-1-yl)ethyl]benzamide;
(-) 4-chloro-N-(1-{[(cyanoimino)(3-pyridinylamino)methyl]amino}-2,2-dimethyl-4-pentenyl)benzamide;
(+)4-chloro-N-(1-{[(cyanoimino)(3-pyridinylamino)methyl]amino}-2,2-dimethyl-4-pentenyl)benzamide;
4-chloro-N-(1-{[(cyanoimino)(3-pyridinylamino)methyl]amino }-3,3-dimethyl-4-pentenyl)benzamide;
4-chloro-N-(1-{[(cyanoimino)(3-pyridinylamino)methyl]amino }-2-cyclohexyl-2-methylpropyl)benzamide;
4-chloro-N-(1- [(cyanoimino)(3-pyridinylamino)methyl]amino}-2,2-dimethylhexyl)benzamide;
N-(2-(1-adamantyl)-1-[(cyanoimino)(3-pyridinylamino)methyl]amino} ethyl)-4-chlorobenzamide;
N-(2,2-bis[(allyloxy)methyl]-1-{([(cyanoimino)(3-pyridinylamino)methyl]amino}butyl)-4-chlorobenzamide;
4-chloro-N-[1-{[(cyanoimino)(3-pyridinylamino)methyl]amino}-3-(dimethylamino)-2,2-dimethylpropyl]benzamide;
tert-butyl (2R)-2-((R)-[(4-chlorobenzoyl)amino] {[(cyanoimino)(3-pyridinylamino)methyl]amino} methyl)-1-pyrrolidinecarboxylate;
4-chloro-N-[1-{[(cyanoimino)(3-pyridinylamino)methyl]amino}-3-(methylsulfanyl)propyl]benzamide;
N-(1-adamantyl{[(cyanoimino)(3-pyridinylamino)methyl]amino}methyl)-4-chlorobenzamide;
4-chloro-N-[({[(cyanoimino)(3-pyridinylainino)methyl]amino}(5-ethyl-1,3-dioxan-5-yl)methyl]benzamide;
4-chloro-N-(1-{[(cyanoimino)(3-pyridinylamino)methyl]amino) }-2,2-dimethyl-3-phenylpropyl)benzamide;
N-(1-{[(cyanoimino)(3-pyridinylamino)methyl]amino}-2,2-dimethylpropyl)-4-iodobenzamide;
N-(1-{[(cyanoimino)(3-pyridinylamino)methyl]amino}-2,2-dimethylpropyl)-4-(2-furyl)benzamide;
4-bromo-N-(1-{[(cyanoimino)(3-pyridinylamino)methyl]amino}-2,2-dimethylpropyl)benzamide;
4-chloro-N-(1-{[(cyanoimino)(3-pyridinylamino)methyl]amino}-2,2-dimethylpropyl)-2-fluorobenzamide;
N-(1-{[(cyanoimino)(3-pyridinylamino)methyl]amino}-2,2-dimethylpropyl)-4-fluorobenzamide;
N-(1-{[(cyanoimino)(3-pyridinylamino)methyl]amino}-2,2-dimethylpropyl)-3-methylbenzamide;
N-(1-{ [(cyanoimino)(3-pyridinylamino)methyl]amino}-2,2-dimethylpropyl)-2-methylbenzamide;
N-(1-{[(cyanoimino)(3-pyridinylamino)methyl]amino}-2,2-dimethylpropyl)-3,5-difluorobenzamide;
4-chloro-N-{1-[[(cyanoimino)(3-pyridinylamino)methyl](methyl)amino]-2,2-dimethylpropyl}benzamide;
(-) 4-chloro-N-(2,2,2-trichloro-1-{[(cyanoimino)(3-pyridinylamino)methyl]amino}ethyl)benzamide;
(+) 4-chloro-N-(2,2,2-trichloro-1-{[(cyanoimino)(3-pyridinylamino)methyl]amino}ethyl)benzamide;
4-iodo-N-(2,2,2-trichloro-1-{[(cyanoimino)(3-pyridinylamino)methyl]amino}ethyl)benzamide;
4-chloro-N-(2,2-dichloro-1-{[(cyanoimino)(3-pyridinylamino)methyl]amino}pentyl)benzamide;
4-chloro-N-(2,2-dichloro-1- {[(cyanoimino)(3-pyridinylamino)methyl]amino}propyl)benzamide;
(-) 4-chloro-N-(2,2-dichloro-1-{[(cyanoimino)(3-pyridinylamino)methyl]amino}propyl)benzamide;
(+) 4-chloro-N-(2,2-dichloro-1-{[(cyanoimino)(3-pyridinylamino)methyl]amino}propyl)benzamide;
3-chloro-N-(2,2-dichloro-1-{[(cyanoimino)(3-pyridinylamino)methyl]amino }propyl)benzamide;
N-(2,2-dichloro-1-{[(cyanoimino)(3-pyridinylamino)methyl]amino}propyl)-3,5-difluorobenzamide;
4-chloro-N-(1-{[(cyanoimino)(3-pyridinylamino)methyl]amino)}-2,2,3,3,3-pentafluoropropyl)benzamide;
3-chloro-N-(1-{[(cyanoimino)(3-pyridinylamino)methyl]amino}-2,2,3,3,3-pentafluoropropyl)benzamide;
4-chloro-N-(1-{[(nitroimino)(3-pyridinylamino)methyl]amino}-2,2-dimethylpropyl)benzamide;
4-chloro-N-(1-{[(nitroimino)(3-pyridinylamino)methyl]amino}-3,3-dimethylbutyl)benzamide;
(+) 4-chloro-N-(1-{[(nitroimino)(3-pyridinylamino)methyl]amino}-3,3-dimethylbutyl)benzamide;
(-) 4-chloro-N-(1-{[(nitroimino)(3-pyridinylamino)methyl]amino}-3,3-dimethylbutyl)benzamide;
4-chloro-N-(1-{[(nitroimino)(3-pyridinylamino)methyl]amino}-2,2-dimethyl-4-pentenyl)benzamide;
4-chloro-N-(1-{[(nitroimino)(3-pyridinylamino)methyl]amino }-2,2-dimethyl-3-phenylpropyl)benzamide;
4-chloro-N-[1-{[(nitroimino)(3-pyridinylamino)methyl]amino}-2-(2,6,6-trimethyl-1-cyclohexen-1-yl)ethyl]benzamide;
4-chloro-N-(1-{[(nitroimino)(3-pyridinylamino)methyl]amino}-2-cyclohexyl-2-methylpropyl)benzamide;
N-(2,2-bis[(allyloxy)methyl]-1-{[(nitroimino)(3-pyridinylamino)methyl]amino}butyl)-4-chlorobenzamide;
4-chloro-N-(4-cyano-1-{[(nitroimino)(3-pyridinylamino)methyl]amino}-2,2-diethylbutyl)benzamide;
4-chloro-N-(1-{[(nitroimino)(3-pyridinylamino)methyl]amino}-3,3-dimethyl-4-pentenyl)benzamide;
N-(2-(1-adamantyl)-1-{[(nitroimino)(3-pyridinylamino)methyl]amino}ethyl)-4-chlorobenzamide;
N-(1-{[(nitroimino)(3-pyridinylamino)methyl]amino)-2,2-dimethylpropyl)-4-phenylbenzamide;
4-chloro-N-(2,2-dichloro-1- {[(nitroimino)(3-pyridinylamino)methyl]amino}pentyl)benzamide;
4-chloro-N-(2,2-dichloro-1-{[(nitroimino)(3-pyridinylamino)methyl]amino}propyl)benzamide;
3-chloro-N-(2,2-dichloro-1-{[(nitroimino)(3-pyridinylamino)methyl]amino}propyl)benzamide;
4-chloro-N-(2,2-dimethyl-1-{[[phenylsulfonyl)imino](3-pyridinylamino)methyl]amino}propyl)benzamide;
4-chloro-N-(3,3-dimethyl-1-{[[(phenylsulfonyl)imino](3-pyridinylamino)methyl]amino}butyl)benzamide;
4-chloro-N-{2,2-dimethyl-1-[((3-pyridinylamino){[(trifluoromethyl)sulfonyl]imino}methyl)amino]propyl}benzamide;
4-chloro-N-{3,3-dimethyl-1-[((3-pyridinylamino){[(trifluoromethyl)sulfonyl]imino}methyl)amino]butyl}benzamide;
N-(1-{[[(aminosulfonyl)imino](3-pyridinylamino)methyl]amino}-2,2-dimethylpropyl)-4-chlorobenzamide;
N-(1-{([(aminosulfonyl)imino](3-pyridinylamino)methyl]amino}-3,3-dimethylbutyl)-4-chlorobenzamide;
4-chloro-N-(1-{[{[(dimethylamino)sulfonyl]imino}(3-pyridinylamino)methyl]amino}-2,2-dimethylpropyl)benzamide;
4-chloro-N-(1-{[{[(dimethylamino)sulfonyl]imino}(3-pyridinylamino)methyl]amino}-3,3-dimethylbutyl)benzamide;
4-chloro-N-(1-{[(2-fluoroanilino)carbonyl]amino}-2,2-dimethylpropyl)benzamide;
4-iodo-N-(2,2,2-trichloro-1-{[(3-pyridinylamino)carbothioyl]amino}ethyl)benzamide;
3-phenyl-N-(2,2,2-trichloro-1-{[(3-nitroanilino)carbothioyl]amino}ethyl)propanamide;
4-chloro-N-(2,2-dimethyl-1-{[2-nitro-1-(3-pyridinylamino)ethenyl]amino}propyl)benzamide;
4-chloro-N-(2,2-dichloro-1-{[2-nitro-1-(3-pyridinylamino)ethenyl]amino}pentyl)benzamide;
4-chloro-N-(1-{[2,2-dicyano-1-(3-pyridinylamino)vinyl]amino}-2,2-dimethylpropyl)benzamide and pharmaceutically acceptable salts thereof.

### Abbreviations

The following abbreviations are used: (Boc)₂O for di-tert-butyl dicarbonate; DCC for dicyclohexylcarbodiimide; DMF for N,N-dimethylformamide; DMSO for dimethyl sulfoxide; EDCI for 1-ethyl-3-[3-(dimethylamino)propyl]-carbodiimide hydrochloride; Et for ethyl; EtOH for ethanol; Me for methyl; MeOH for methanol; NaHMDS for sodium bis(trimethylsilyl)amide; i-Pr for isopropyl; pyr for pyridine; THF for tetrahydrofuran; and p-TsOH for para-toluenesulfonic acid monohydrate.

### Preparation of Compounds of The Invention

The compounds and processes of the present invention will be better understood in connection with the following synthetic schemes and methods which illustrate a means by which the compounds of the invention can be prepared.

The compounds of this invention may be prepared by a variety of synthetic routes. Representative procedures are shown in Schemes 1-23 in which R⁴ and R⁶ are each.

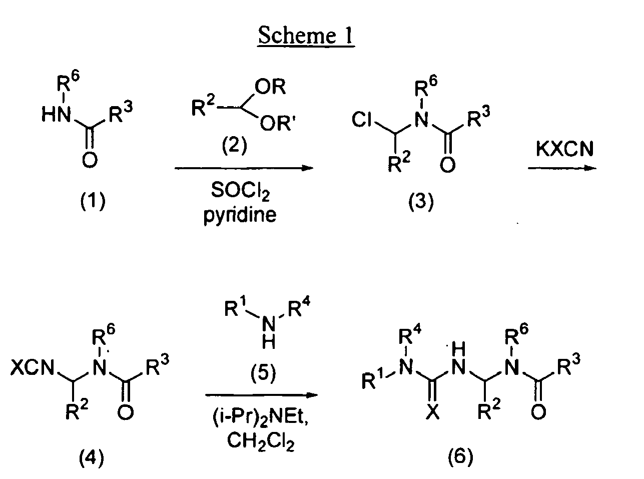

As shown in Scheme 1, urea and thiourea aminals of general formula (6), wherein R¹, R³, R⁴, and R⁶ are as defined in formula I, R² is haloalkyl (such as CCl₃ or CF₃), and X is O or S, may be prepared using the strategy outlined above. Amides of general formula (1) may be treated with α-haloaldehyde hydrates or α-halohemiacetals of general formula (2), wherein R is H and R' is H or alkyl, such as 2,2,2-trichloro-1,1-ethanediol or 1-ethoxy-2,2,2-trifluoro-1-ethanol, followed by addition of a chlorinating agent such as thionyl chloride and a base such as pyridine to provide chloroamides of general formula (3). The chloroamides (3) may be treated with potassium cyanate or potassium thiocyanate to provide isocyanates or isothiocyanates respectively of general formula (4). The isocyanates or isothiocyanates (4) may be treated with amines of general formula (5) in the presence of a base such as diisopropylethylamine to provide urea and thiourea aminals of general formula (6).

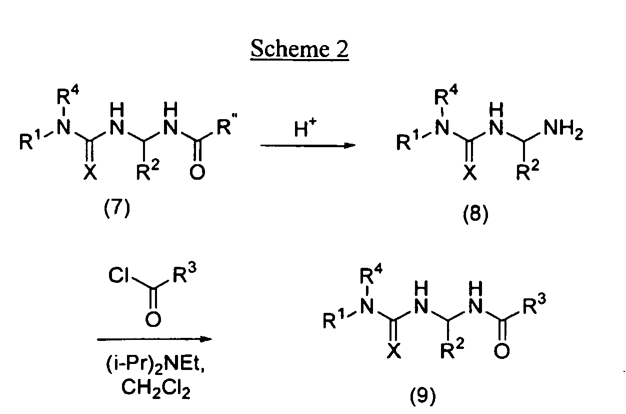

As shown in Scheme 2, urea and thiourea aminal derivatives of general formula (9), wherein R¹, R³, and R⁴, are as defined in formula I, R² is haloalkyl (such as CCl₃ or CF₃), and X is O or S, may be prepared using the above strategy. Urea and thiourea aminals of general formula (7), wherein R" is alkoxy, may be prepared following the strategy described in Scheme 1. Urea and thiourea aminals of general formula (7) may be treated with an acid such as hydrobromic acid to provide primary amines of general formula (8). Amines of general formula (8) may be treated with acid chlorides in the presence of a base such as diisopropylethylamine to provide urea and thiourea aminals of general formula (9).

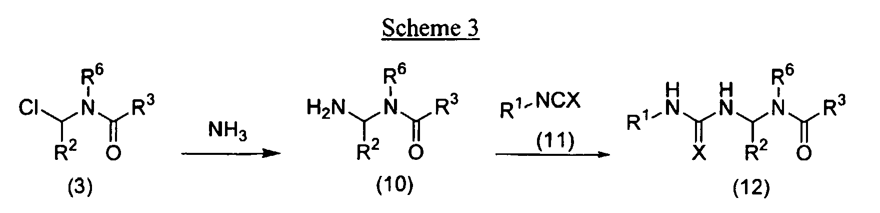

Urea and thiourea aminals of general formula (12), wherein R¹, R³, and R⁶ are as defined in formula I, R² is haloalkyl (such as CCl₃ or CF₃), and X is O or S, may be prepared as described in Scheme 3. Chloroamides of general formula (3) may be treated with ammonia to provide aminoamides of general formula (10). The aminoamides (10) may be treated with an isocyanate or an isothiocyanate of general formula (11), wherein X is O or S, to provide urea and thiourea aminals of general formula (12).

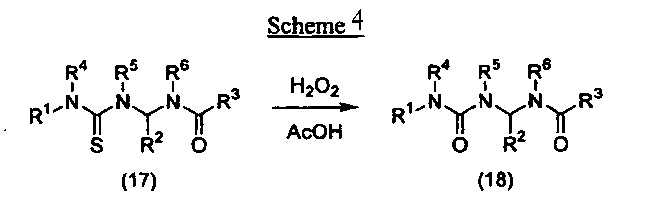

A general method for preparing urea aminals from thiourea aminals is described in Scheme 4. Thiourea aminals of general formula (17) may be treated with an oxidizing agent such as hydrogen peroxide in a protic solvent such as acetic acid to provide urea aminals of general formula (18) wherein R¹, R², R³, R⁴, R⁵, and R⁶ are as defined in formula I.

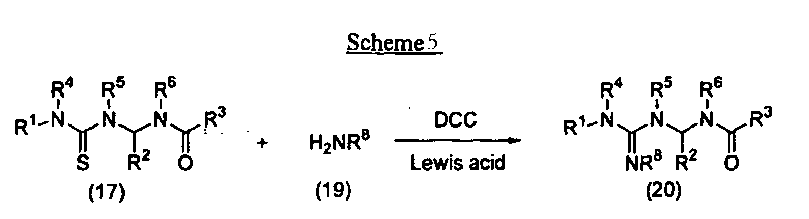

A general method for preparing guanidine aminals from thiourea aminals is described in Scheme 5. Thiourea aminals of general formula (17) may be treated with a dehydrating agent such as DCC followed by addition of amines of general formula (19), prepared as described in (Scharpenberg; Chem. Ber. (1973), 106, 1881), in the presence of a Lewis acid such as titanium isopropoxide to provide guanidine aminals of general formula (20) wherein R¹, R², R³, R⁴, R⁵, R⁶ and R⁸ are as defined in formula I.

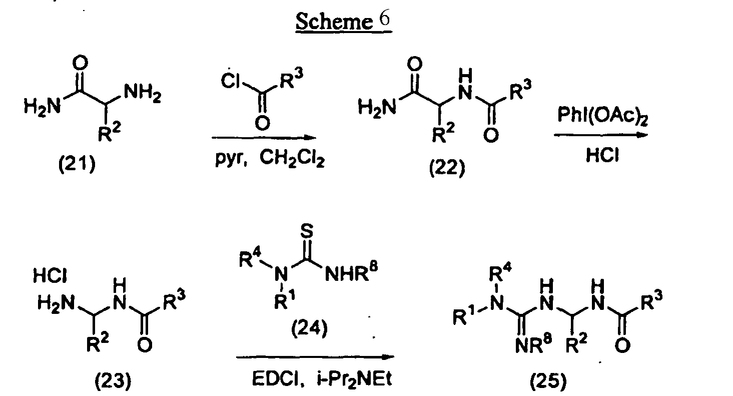

As shown in Scheme 6, guanidine aminals of general formula (25), wherein R¹, R², R³, R⁴, and R⁸ are as defined in formula I, may be prepared by using the above strategy. Aminoacetamides of general formula (21) may be treated with acid chlorides in the presence of a base such as pyridine or triethylamine to provide the corresponding acylaminoamides of general formula (22). The acylaminoamides (22) may undergo a Hofmann rearrangement as described in (Wallis; Lane; Org. React. (1946), 3, 267-306, and references contained therein) with reagents such as iodosobenzene diacetate as described in (Loudon; Radhakrishna; Almond; Blodgett; Boutin; J. Org. Chem. (1984), 49, 4272); (Loudon; Boutin J. Org. Chem. (1984), 49, 4277); (Chan; Pennington; McParland; Whitehead; Coutts; Synth. Commun. (1988), 53, 5158) to provide aminoamides of general formula (23), which may be typically isolated as their hydrochloride salts. The aminoamides (23) may be treated with thioureas of general formula (24), prepared as described in (Solimar, J.Med.Chem. (1979), 22, 321; and Ulrich; Tetrahedron (1966), 22, 1565) to provide guanidine aminals of general formula (25). An alternate approach for preparation of cyanoguanidine aminals of general formula (25), wherein R⁸ is cyano, may be used. Aminoamides of general formula (23) may be treated with cyanothioureas of general formula (24), wherein R⁸ is cyano, in the presence of a base such as diisopropylethylamine and a suitable activating agent such as EDCI to provide cyanoguanidine aminals of general formula (25).

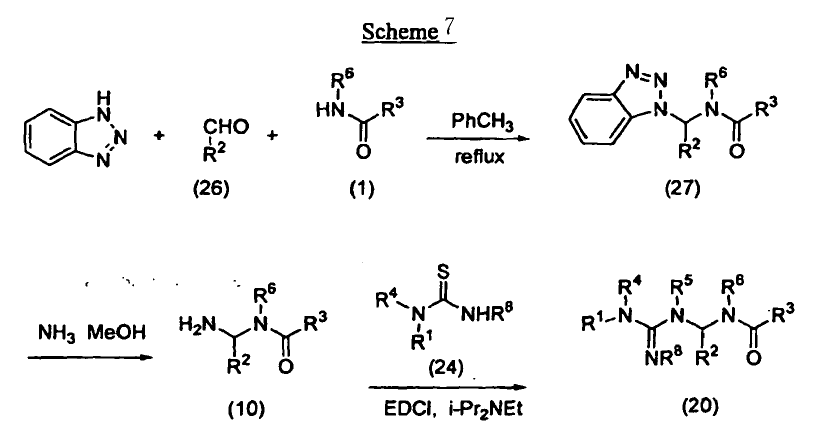

An alternate route to guanidine aminals of general formula (20), wherein R¹, R², R³, R⁴, R⁶ and R⁸ are as defined in formula I and R⁵ is H, is shown in Scheme 7. A three-component condensation including benzotriazole, aldehydes of general formula (26), and amides of general formula (1) in the presence of an acid catalyst such as p-toluenesulfonic acid monohydrate as described in (Katritzky; Urogdi; Mayence; J. Org. Chem. (1990), 55, 2206); (Katritzky; Chem. Rev. (1998), 98, 409); (Katritzky; J. Heterocyclic Chem. (1996), 33, 1935) provides benzotriazole adducts of general formula (27). Nucleophilic displacement of the benzotriazole moiety as described in (Katritzky; Urogdi; Mayence; J. Org. Chem. (1990), 55, 2206); (Katritzky; Chem. Rev. (1998), 98, 409); (Katritzky; J. Heterocyclic Chem. (1996), 33,1935) with ammonia in an alcoholic solvent such as methanol provides aminoamides of general formula (10). The aminoamides (10) may be treated with thioureas of general formula (24) in the presence of a base such as diisopropylethylamine and a suitable activating agent such as EDCI to provide guanidine aminals of general formula (20).

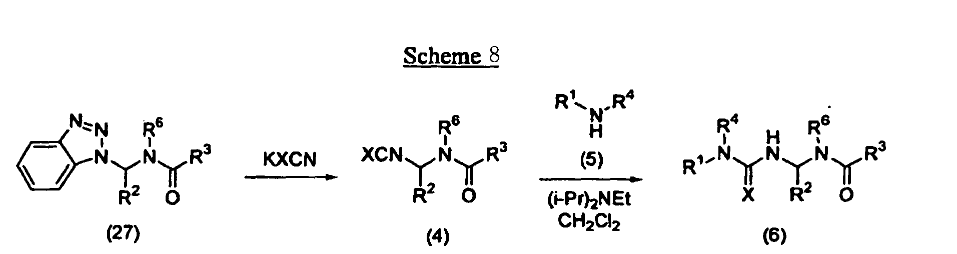

As shown in Scheme 8, urea and thiourea aminals of general formula (6), wherein R¹, R², R³, R⁴ and R⁶ are as defined in formula I and X is O or S, may be prepared by treating benzotriazole adducts of general formula (27) with potassium cyanate or potassium thiocyanate to provide isocyanates or isothiocyantes of general formula (4). Isocyanates or isothiocyanates of general formula (4) may be treated with amines of general formula (5) in the presence of a base such as diisopropylethylamine to provide urea and thiourea aminals of general formula (6).

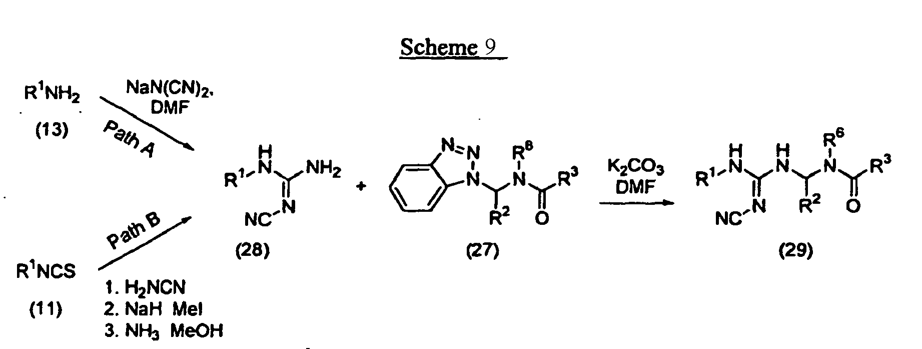

As shown in Scheme 9, cyanoguanidine aminals of general formula (29), wherein R¹, R², R³ and R⁶ are as defined in formula I, may be prepared by using a strategy that employs a two-step sequence. Cyanoguanidines of general formula (28) are first prepared either by Path A or Path B. In path A, amines of general formula (13) are treated with sodium dicyanamide as described in (Tilley; Ramuz; Levitan; Blount; Helv. Chim. Acta. (1980), 63, 841); (Jones; Kuyper; Styles; Caddell; J. Heterocyclic Chem. (1994), 31,1681) to provide cyanoguanidines of general formula (28). In Path B, isothiocyanates of general formula (11) are treated in succession with cyanamide, a sodium base such as sodium hydride, an electrophile such as methyl iodide, and ammonia in a polar aprotic solvent such as methanol as described in (Fairfall; Peak; J. Chem. Soc. (1955), 796) to provide cyanoguanidines of general formula (28). The cyanoguanidines (28) are then treated with benzotriazole adducts of general formula (27) in the presence of a base such as potassium carbonate to provide cyanoguanidine aminals of general formula (29).

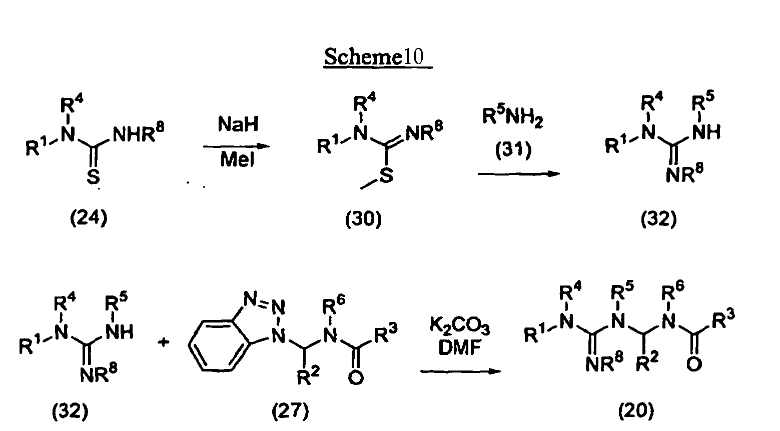

Functionality may be introduced onto the guanidine nitrogen (R⁵) by the synthetic sequence described in Scheme 10. Thioureas of general formula (24) may be treated with a sodium base such as sodium hydride and then alkylated with electrophiles such as methyl iodide to provide methyl carbamimidothioates of general formula (30). Methyl carbamimidothioates (30) may be treated with amines of general formula (31) to provide guanidines of general formula (32) which are then further reacted with benzotriazole adducts of general formula (27) in the presence of a base such as potassium carbonate to provide guanidine aminals of general formula (20) wherein R¹, R², R³, R⁴, R⁵, R⁶, and R⁸ are as defined in formula I.

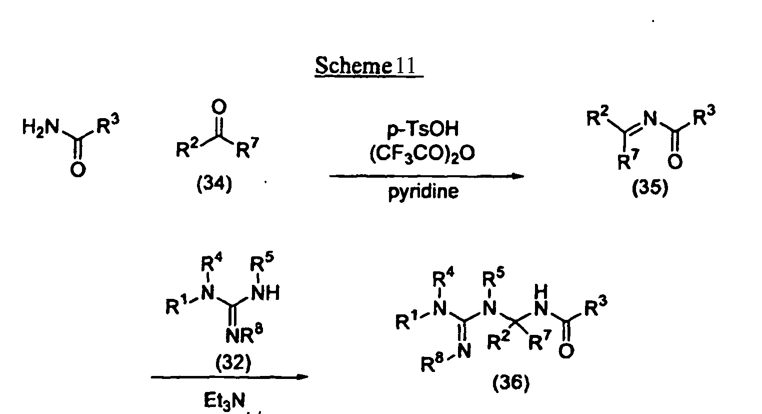

As shown in Scheme 11, geminally-substituted products of general formula (36) wherein R¹, R³, R⁴, R⁵, R⁷, and R⁸ are as defined in formula I and R² is the same as R⁷ or R² and R⁷ taken together with the carbon atom to which they are attached, together form a 5 or 6 membered carbocyclic ring, may be prepared using the above strategy and as described in (Steglich; Chem. Ber. (1974), 107, 1488); (Burger; J. Fluorine Chem. (1982), 20, 813). Optionally substituted primary amides may be treated with symmetrical ketones of general formula (34) in the presence of a dehydrating agent such as trifluoroacetic anhydride and a base such as pyridine to provide symmetrical imines of general formula (35). The symmetrical imines (35) may be treated with guanidines of general formula (32) in the presence of a base such as triethylamine to provide geminally-substituted compounds of general formula (36).

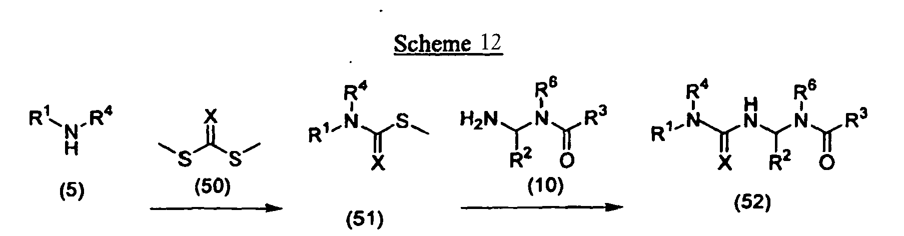

Aminals of general formula (52), wherein R¹, R², R³, R⁴, and R⁶ are as defined in formula I and X is selected from NCN, CHNO₂, and CHCN, may be prepared as illustrated in Schem 12. Bis(methylthio) compounds of general formula (50) are commercially available when X is NCN or CHNO₂ or may be prepared as described in (Hendriksen; Acta Chem. Scand. (1990), 50, 432 and Creemer; Barger; Wagner; Synth. Comm. (1988), 18, 1103) when X is CHCN. Compounds of general formula (50) may be treated with amines of general formula (5) to provide methylthio compounds of general formula (51). Methylthio compounds of general formula (51) may be treated with amines of general formula (10) to provide aminals of general formula (52) wherein X is NCN, CHNO₂, or CHCN.

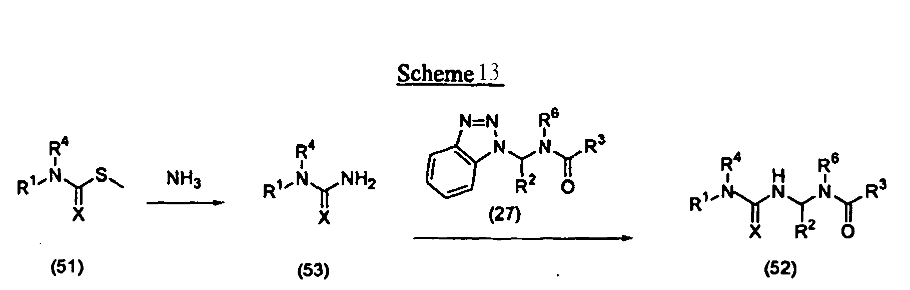

An alternate method of preparing aminals of general formula (52) wherein R¹, R², R³, R⁴, and R⁶ are as defined in formula I and X is selected from NCN, CHNO₂, and CHCN, is shown in Scheme 13. Methylthio compounds of general formula (51) may be treated with ammonia in an alcoholic solvent such as methanol to provide compounds of general formula (53). Compounds of general formula (53) may be treated with benzotriazoles of general formula (27) to provide aminals of general formula (52) wherein X is NCN, CHNO₂, or CHCN.

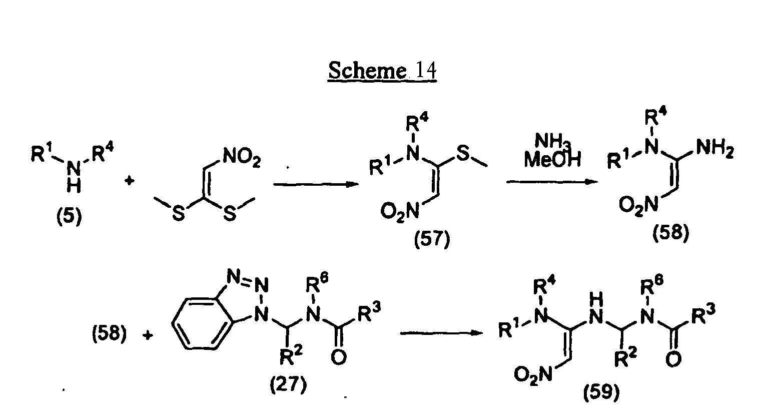

Aminals of general formula (59), wherein R¹, R², R³, R⁴ and R⁶ are as defined in formula I, may be prepared as described in Scheme 14. Amines of general formula (5) may be treated with 1,1-bis(methylsulfanyl)-2-nitroethylene in a solvent such as isopropanol to provide nitroethenyl compounds of general formula (57). Nitroethenyl compounds of general formula (57) may be treated with ammonia and methanol to provide nitroethenediamines of general formula (58). Nitroethenediamines of general formula (58) may be treated with benzotriazole adducts of general formula (27) and a base such as potassium carbonate in a solvent such as DMF to provide aminals of general formula (59).

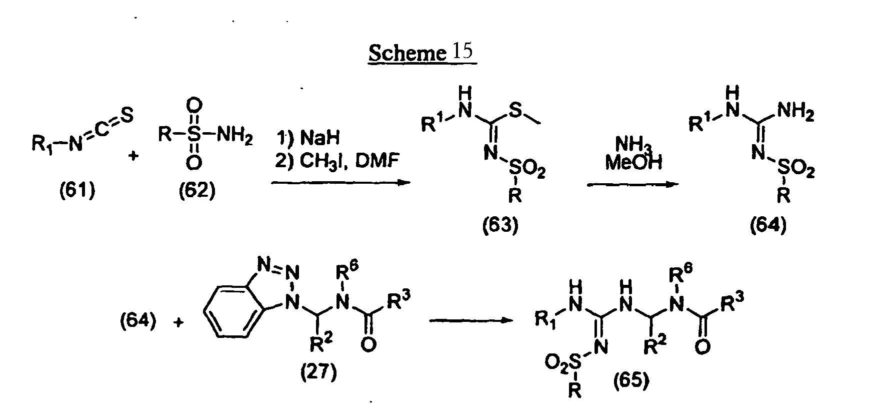

Aminals of general formula (65), wherein R¹, R², R³ and R⁶ are as defined in formula I and R is selected from alkyl, aryl, haloalkyl and NR⁹⁴R⁹⁵ wherein R⁹⁴ and R⁹⁵ are independently selected from hydrogen, alkyl, aryl, and arylalkyl, may be prepared as described in Scheme 15. Isothiocyanates of general formula (61) may be treated with compounds of general formula (62) and sodium hydride followed by treatment with iodomethane in DMF to provide compounds of general formula (63). Compounds of general formula (63) may be treated with ammonia and methanol to provide guanidines of general formula (64). Guanidines of general formula (64) may be treated with benzotriazole adducts of general formula (27) and a base such as potassium carbonate in a solvent such as DMF to provide aminals of general formula (65).

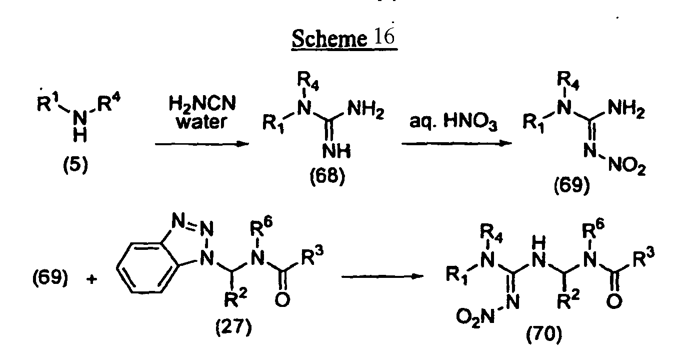

Aminals of general formula (70), wherein R¹, R², R³, R⁴ and R⁶ are as defined in formula I, may be prepared as described in Scheme 16. Amines of general formula (5) may be treated with cyanamide in water to provide guanidines of general formula (68). Guanidines of general formula (68) may be treated with aqueous nitric acid to provide nitroguanidines of general formula (69). Nitroguanidines of general formula (69) may be treated with benzotriazole adducts of general formula (27) and a base such as potassium carbonate in a solvent such as DMF to provide aminals of general formula (70).

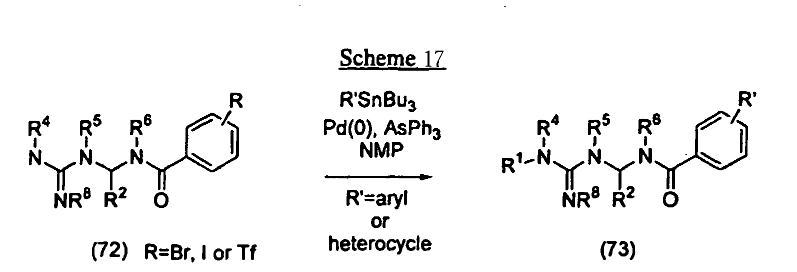

Aminals of general formula (73), wherein R¹, R², R⁴, R⁵, R⁶ and R⁸ are as defined in formula I and R' is selected from alkoxycarbonyl, aryl, carboxy, heterocycle and - NR^{A}R^{B} wherein R^{A} and R^{B} are as defined in formula I, may be prepared as described in Scheme 17. Aminals of general formula (72), wherein R is Br, I or -OS(O)₂CF₃, may be treated with a palladium catalyst, a trialkyltin reagent and triphenylarsine in a solvent such as N-methylpyrrolidin-2-one to provide aminals of general formula (73). Alternatively, cross-coupling reactions (and carbonylations) may be done using Buchwald, Stille, Suzuki or Heck coupling reactions all of which are well known to those skilled in the art of organic chemistry.

### Example 1

### 4-methyl-N-(2,2,2-trifluoro-1-{[(3-pyridinylamino)carbothioy]amino}ethyl)benzamide

### Example 1A

### 4-methyl-N-(2,2,2-trifluoro-1-hydroxyethyl)benzamide

A 500 mL round-bottom flask was charged with trifluoroacetaldehyde ethyl hemiacetal (12.3 g, 85.0 mmol), p-toluamide (10.0 g, 74.0 mmol), and dioxane (150 mL). The thick white slurry was stirred at ambient temperature for 3 hours, then heated at reflux for 44 hours. The homogeneous solution was cooled and concentrated in vacuo to provide a white solid. The crude material was dissolved in ethyl acetate (50 mL), adsorbed onto silica gel (50 g), and eluted through a medium porosity fritted filter funnel (elution with 25% ethyl acetate/hexanes, then ethyl acetate) to provide 14.1 g of the desired product as a white solid.
MS (APCI+) m/z 215 (M-H₂O)⁺.

### Example 1B

### N-(1-chloro-2,2,2-trifluoroethyl)-4-methylbenzamide

A stirred solution of Example 1A (3.48 g, 15.0 mmol) in CH₂Cl₂ (60 mL) at 0 °C was treated with pyridine (1.20 mL). The reaction mixture was treated dropwise with thionyl chloride (1.10 mL, 15.0 mmol), and the reaction flask was equipped with a calcium chloride drying tube. The reaction mixture was stirred at 0 °C for 4 hours, the cooling bath was removed, and the solution was stirred at ambient temperature for an additional 12 hours. Concentration of the reaction mixture provided a white solid which was triturated with diethyl ether (2x200 mL) to provide 2.40 g of the desired product as a white solid.
MS (APCI+) m/z 252 (M+H)⁺.

### Example 1C

### 4-methyl-N-(2,2,2-trifluoro-1-isothiocyanatoethyl)benzamide

A stirred solution of Example 1B (2.00 g, 8.00 mmol) in acetone (35 mL) at ambient temperature was treated with potassium thiocyanate (1.60 g, 16.0 mmol). The reaction mixture was stirred for 12 hours, concentrated, and the crude residue was purified by flash chromatography (elution with 40% ethyl acetate/hexanes) to provide 1.34 g of the desired product as an off-white solid.
MS (APCI+) m/z 275 (M+H)⁺.

### Example 1D

### 4-methyl-N-(2,2,2-trifluoro-1-{[(3-pyridinylamino)carbothioyl]amino}ethyl)benzamide

A solution of 3-aminopyridine (145 mg, 1.54 mmol) in benzene (8 mL) at ambient temperature was treated with a solution of Example 1C (500 mg, 1.54 mmol) in benzene (1.5 mL). The reaction mixture was stirred for 1.5 hours, then concentrated to a nominal volume. The white solids which precipitated from solution were collected by filtration and were washed with diethyl ether. Recrystallization from 25% ethyl acetate/hexanes provided the desired product as a white solid.
mp 185-186 °C;
MS (APCI+) m/z 369 (M+H)⁺;
¹H NMR (DMSO-d₆) δ 10.43 (s, 1H), 9.25 (d, 1H, J=8 Hz), 8.61 (dd, 1H, J=3, 1 Hz), 8.39-8.35 (m, 2H), 8.04-8.00 (m, 1H), 7.79 (m, 2H), 7.43-7.38 (m, 1H), 7.35-7.23 (m, 3H), 2.38 (s, 3H);
Anal. calcd for C₁₆H₁₅F₃N₄OS: C, 52.17; H, 4.10; N, 15.21. Found: C, 52.2; H, 3.96; N, 15.16.

### Example 2

### 4-methyl-N-{2,2,2-trifluoro-1-[(2-toluidinocarbothioyl)amino]ethyl}benzamide

Example 1 C and 2-methylaniline were processed as described in Example 1D to provide the desired product.
mp 210-211 °C;
MS (APCI+) m/z 382 (M+H)⁺;
¹H NMR (DMSO-d₆) δ 10.01 (br s, 1H), 9.09-8.96 (m, 1H), 7.74 (d, 2H, J=8 Hz), 7.39-7.22 (m, 7H), 2.37 (s, 3H);
Anal. calcd for C₁₈H₁₈F₃N₃OS: C, 56.68; H, 4.76; N, 11.02. Found: C, 56.66; H, 4.73; N, 10.84.

### Example 3

### 4-methyl-N-(2,2,2-trifluoro-1- {[(4-fluoroanilino)carbothioyl]amino}ethyl)benzamide

Example 1C and 4-fluoroaniline were processed as described in Example I D to provide the desired product.
mp 208-210 °C;
MS (APCI+) m/z 386 (M+H)⁺;
¹H NMR (DMSO-d₆) δ 10.30 (s, 1H), 9.19 (d, 1H, J=8 Hz), 8.11 (d, 1H, J=10 Hz), 7.77 (d, 2H, J=8 Hz), 7.47 (m, 2H), 7.34 (d, 2H, J=8 Hz), 7.30-7.19 (m, 3H), 2.37 (s, 3H); Anal. calcd for C₁₇Hₗ₅F₄N₃OS: C, 52.98; H, 3.92; N, 10.90. Found: C, 53.08; H, 3.92; N, 10.91.

### Example 4

### 4-methyl-N-(2,2,2-trifluoro-1-{[(3-nitroanilino)carbothioyl]amino}ethyl)benzamide

Example 1C and 3-nitroaniline were processed as described in Example 1D to provide the desired product.
mp 185-186 °C;
MS (APCI+) m/z 416 (M+H)⁺;
¹H NMR (DMSO-d₆) δ 10.72 (s, 1H), 9.32 (d, 1H, J=9 Hz), 8.69 (s, 1H), 8.48 (m, 1H), 8.04 (m, 1H), 7.90 (d, 1H, J=9 Hz), 7.80 (d, 2H, J=9 Hz), 7.65 (t, 1H, J=9 Hz), 7.35 (d, 2H, J=9 Hz), 7.29-7.17 (m, 1H), 2.38 (s, 3H);
Anal. calcd for C₁₇H₁₅F₃N₄O₃S·0.2 H₂O: C, 49.08; H, 3.73; N, 13.47. Found: C, 48.96; H, 3.54; N, 13.38.

### Example 5

### 4-methyl-N-L,2,2-trifluoro-1-({ [2-fluoro-3-(trifluoromethyl)anilino]carbothioyl}amino)ethyl]benzamide

Example 1C and 2-fluoro-3-(trifluoromethyl)aniline were processed as described in Example 1D to provide the desired product.
mp 179-181 °C;
MS (APCI+) m/z 454 (M+H)⁺;
¹H NMR (DMSO-d₆) δ 10.23 (s, 1H), 9.28 (d, 1H, J=8 Hz), 8.62 (d, 1H, J=8 Hz), 8.05 (t, 1H, J=9 Hz), 7.80 (d, 2H, J=8 Hz), 7.67 (t, 1H, J=7 Hz), 7.42 (t, 1H, J=7 Hz), 7.34 (d, 2H, J= Hz), 7.30-7.18 (m, 1H), 2.38 (s, 3H);
Anal. calcd for C₁₈H₁₄F₃N₃OS·0.5 C₄H₈O: C, 49.08; H, 3.71; N, 8.59. Found: C, 49.48; H, 3.75; N, 8.36.

### Example 6

### 4-methyl-N-(2,2,2-trifluoro-1-{[(4-methoxyanilino)carbothioyl]amino}ethyl)benzamide

Example 1C and 4-methoxyaniline were processed as described in Example 1 D to provide the desired product.
mp 193-194 °C;
MS (APCI+) m/z 398 (M+H)⁺;
¹H NMR (DMSO-d₆) δ 10.19 (s, 1H), 9.12 (br s, 1H), 7.8-7.9 (br s, 1H), 7.75 (d, 2H, J=8 Hz), 7.33 (d, 2H, J=8 Hz), 7.32-7.21 (m, 3H), 6.97 (d, 2H, J=9 Hz), 3.76 (s, 3H), 2.37 (s, 3H);
Anal. calcd for C₁₈H₁₈F₃N₃O₂S: C, 54.40; H, 4.57; N, 10.57. Found: C, 54.47; H, 4.49; N, 10.44.

### Example 7

### N-[1-({[(6-chloro-3-pyridinyl)amino]carbothioyl)amino)-2,2,2-trifluoroethyl]-4-methylbenzamide

Example 1C and 5-amino-2-chloropyridine were processed as described in Example I D to provide the desired product.
mp 199-200 °C;
MS (APCI+) m/z 403 (M+H)⁺;
¹H NMR (DMSO-d₆) δ 10.51 (s, 1H), 9.29 (d, 1H, J=8 Hz), 8.55-5.46 (m, 2H), 8.12 (dd, 1H, J=9, 3 Hz), 7.78 (d, 2H, J=8 Hz), 7.53 (d, 1H, J=9 Hz), 7.34 (d, 1H, J=8 Hz), 7.29-7.21 (m, 1H), 2.38 (s, 3H);
Anal. calcd for C₁₆H₁₄F₃N₄OS: C, 47.71; H, 3.50; N, 13.91. Found: C, 47.93; H, 3.53; N, 13.76.

### Example 8

### 4-methyl-N-(2,2,2-trifluoro-1- {[(2-methoxyanilino)carbothioyl]amino}ethyl)benzamide

Example 1C and 2-methoxyaniline were processed as described in Example 1D to provide the desired product.
mp 212-214 °C;
MS (APCI+) m/z 396 (M+H)⁺;
¹H NMR (DMSO-d₆) δ 9.86 (s, 1H), 9.20-9.12 (m, 1H), 7.76 (d, 2H, J=8 Hz), 7.6 (br s, 1H), 7.33 (d, 2H, J=8 Hz), 7.26-7.20 (m, 1H), 7.09 (d, 1H, J=8 Hz), 6.95 (t, 1H, J=8 Hz), 2.37 (s, 3H);
Anal. calcd for C₁₈H₁₈F₃N₃O₂S: C, 54.40; H, 4.57; N, 10.57. Found: C, 54.49; H, 4.62; N,10.55.

### Example 9

### N-{1-[(anilinocarbothioyl)amino]-2,2,2-trifluoroethyl]-4-methylbenzamide

Example 1C and aniline were processed as described in Example 1D to provide the desired product.
mp 205-206 °C;
MS (APCI+) m/z 368 (M+H)⁺;
¹H NMR (DMSO-d₆) δ 10.37 (s, 1H), 9.19 (d, 1H, J=8 Hz), 8.12 (d, 1H, J=10 Hz), 7.76 (d, 2H, J=8 Hz), 7.47-7.28 (m, 4H), 7.21 (t, 1H, J=7 Hz), 2.37 (s, 3H);
Anal. calcd for C₁₇H₁₆F₃N₃OS·0.5 H₂O: C, 54.25; H, 4.55; N, 11.16. Found: C, 54.64; H, 4.08; N, 11.14.

### Example 10

### 4-methyl-N-{2,2,2-trifluoro-1-[(4-toluidinocarbothioyl)amino]ethylbenzamide

Example 1 C and p-toluidine were processed as described in Example 1 D to provide the desired product.
mp 192-193 °C;
MS (APCI+) m/z 382 (M+H)⁺;
¹H NMR (DMSO-d₆) δ 10.28 (s, 1H), 9.18-9.12 (m, 1H), 8.10-7.92 (m, 1H), 7.76 (d, 2H, J=8 Hz), 7.34-7.28 (m, 5H), 7.20 (d, 2H, J=8 Hz), 2.37 (s, 3H), 2.30 (s, 3H);
Anal. calcd for C₁₈H₁₈F₃N₃OS: C, 56.68; H, 4.76; N, 11.02. Found: C, 56.95; H, 4.71; N, 10.87.

### Example 11

### 4-methyl-N-(2,2,2-trifluoro-1- {[(2-fluoroanilino)carbothioyl]amino}ethyl)benzamide

Example 1C and 2-fluoroaniline were processed as described in Example 1D to provide the desired product.
mp 197-198 °C;
MS (APCI+) m/z 386 (M+H)⁺;
¹H NMR (DMSO-d₆) δ 10.11 (s, 1H), 9.22 (d, 1H, J=8 Hz), 8.34 (d, 1H, J=8 Hz), 7.78 (d, 2H, J=8 Hz), 7.69 (t, 1H, J=8 Hz), 7.19-7.35 (m, 6H), 2.38 (s, 3H);
Anal. calcd for C₁₇H₁₅F₄N₃OS: C, 52.98; H, 3.92; N, 10.90. Found: C, 52.89; H, 3.92; N, 10.83.

### Example 12

### 4-methyl-N-(2,2,2-trifluoro-1-{[(3-methoxyanilino)carbothioyl]amino}ethyl)benzamide

Example 1C and 3-methoxyaniline were processed as described in Example 1D to provide the desired product.
mp 206-208 °C;
MS (APCI+) m/z 396 (M+H)⁺;
¹H NMR (DMSO-d₆) δ 10.39 (s, 1H), 9.16 (d, 1H, J=8 Hz), 8.12 (d, 1H, J=9 Hz), 7.76 (d, 2H, J=8 Hz), 7.33-7.21 (m, 4H), 7.14 (br s, 1H), 6.96 (d, 1 H, J=8 Hz), 6.83-6.75 (m, 1H), 2.37 (s, 3H);
Anal. calcd for C₁₈H₁₈F₃N₃O₂S: C, 54.40; H, 4.57; N, 10.57. Found: C, 54.50; H, 4.44; N, 10.55.

### Example 13

### 4-methyl-N-(2,2,2-trifluoro-1-{[(3-fluoroanilino)carbothioyl]amino}ethyl)benzamide

Example 1C and 3-fluoroaniline were processed as described in Example 1D to provide the desired product.
mp 205-206 °C;
MS (APCI+) m/z 386 (M+H)⁺;
¹H NMR (DMSO-d₆) δ 10.51 (s, 1H), 9.25 (d, 1H, J=8 Hz), 8.31 (d, 1H, J=9 Hz), 7.78 (d, 2H, J=8 Hz), 7.64-7.58 (m, 1H), 7.45-7.22 (m, 6H), 7.06-7.01 (m, 1H), 2.38 (s, 3H); Anal. calcd for C₁₇Hₗ₅F₄N₃OS: C, 52.98; H, 3.92; N, 10.90. Found: C, 52.86; H, 3.88; N, 10.76.

### Example 14

### N-(1-{[(2,5-difluoroanilino)carbothioyl]amino}-2,2,2-trifluoroethyl)-4-methylbenzamide

Example 1C and 2,5-difluoroaniline were processed as described in Example 1D to provide the desired product.
mp 183-185 °C;
MS (APCI+) m/z 404 (M+H)⁺;
¹H NMR (DMSO-d₆) δ 10.21 (s, 1H), 9.29 (d, 1H, J=8 Hz), 8.65 (d, 1H, J=8 Hz), 7.89-7.82 (m, 1H), 7.79 (d, 2H, J=8 Hz), 7.40-7.32 (m, 3H), 7.30-7.25 (m, 1H), 7.17-7.12 (m, 1H), 2.38 (s, 3H);
Anal. calcd for C₁₇H₁₄F₅N₃OS: C, 50.62; H, 3.5; N, 10.42. Found: C, 50.58; H, 3.49; N, 10.25.

### Example 15

### N-(1-{[(2,4-difluoroanilino)carbothioyl]amino}-2,2,2-trifluoroethyl)-4-methylbenzamide

Example 1C and 2,4-difluoroaniline were processed as described in Example 1D to provide the desired product.
mp 202-204 °C;
MS (APCI+) m/z 404 (M+H)⁺;
¹H NMR (DMSO-d₆) δ 10.02 (s, 1H), 9.20 (br d, 1H, J=8 Hz), 8.40 (br d, 1H, J=8 Hz), 7.78 (d, 2H, J=9 Hz), 7.57-7.51 (m, 1H), 7.43-7.20 (m, 4H), 7.17-7.11 (m, 1H), 2.38 (s, 3H);
Anal. calcd for C₁₇H₁₄F₅N₃OS: C, 50.62; H, 3.50; N, 10.42. Found: C, 50.74; H, 3.41; N, 10.39.

### Example 16

### 4-methyl-N-{2,2,2-trifluoro-1-[(3-toluidinocarbothioyl)amino]ethyl}benzamide

Example 1C and m-toluidine were processed as described in Example 1D to provide the desired product.
mp 197-198 °C;
MS (APCI+) m/z 382 (M+H)⁺;
¹H NMR (DMSO-d₆) δ 10.31 (s, 1H), 9.16 (d, 1H, J=8 Hz), 8.07 (d, 1H, J=8 Hz), 7.76 (d, 2H, J=8 Hz), 7.34 (d, 2H, J=8 Hz), 7.30-7.21 (m, 4H), 7.03 (d, 1H, J=7 Hz), 2.37 (s, 3H), 2.30 (s, 3H);
Anal. calcd for C₁₈H₁₉F₃N₃OS: C, 56.68; H, 4.76; N, 11.02. Found: C, 56.72; H, 4.64; N, 10.85.

### Example 17

### N-(1-{[(2,6-difluoroanilino)carbothioyl]amino}-2,2,2-trifluoroethyl)-4-methylbenzamide

Example 1C and 2,6-difluoroaniline were processed as described in Example 1D to provide the desired product.
mp 185-186°C;
MS (APCI+) m/z 404 (M+H)⁺;
¹H NMR (DMSO-d₆) δ 9.84 (s, 1H), 9.22 (br s, 1H), 8.40 (br s, 1H), 7.78 (d, 2H, J=8 Hz), 7.48-7..43 (m, 1H), 7.35 (d, 2H, J=8 Hz), 7.30-7.17 (m, 3H), 2.38 (s, 3H);
Anal. calcd for C₁₇H₁₄F₅N₃OS: C, 50.62; H, 3.5; N, 10.42. Found: C, 50.85; H, 3.41; N, 10.38.

### Example 18

### N-(1-{[(2,3-difluoroanilino)carbothioyl]amino}1-2,2,2-trifluoroethyl)-4-methylbenzamide

Example 1C and 2,3-difluoroaniline were processed as described in Example 1D to provide the desired product.
mp 210-211 °C;
MS (APCI+) m/z 404 (M+H)⁺;
¹H NMR (DMSO-d₆) δ 10.20 (s, 1H), 9.23 (d, 1H, J=8 Hz), 8.48 (d, 1H, J=9 Hz), 7.79 (d, 2H, J=8 Hz), 7.53-7.49 (m, 1H), 7.46-7.16 (m, 4H), 2.38 (s, 3H);
Anal. calcd for C₁₇H₁₄F₅N₃OS: C, 50.62; H, 3.50; N, 10.42. Found: C, 50.83; H, 3.44; N, 10.18.

### Example 19

### 4-chloro-N-(2,2,2-trifluoro-1-{[(3-pyridinylamino)carbothioyl]amino}ethyl)benzamide

### Example 19A

### 4-chloro-N-(2,2,2-trifluoro-1-hydroxyethyl)benzamide

Trifluoroacetaldehyde ethyl hemiacetal and 4-chlorobenzamide were processed as described in Example 1 A to provide the desired product.
MS (APCI+) m/z 237 (M-H₂O)⁺.

### Example 19B

### 4-chloro-N-(1-chloro-2,2,2-trifluoroethyl)benzamide

Example 19A, thionyl chloride, and pyridine were processed as described in Example 1B to provide the desired product.
MS (APCI+) m/z 271 (M+H)⁺.

### Example 19C

### 4-chloro-N-(2,2,2-trifluoro-1-isothiocyanatoethyl)benzamide

Example 19B and potassium thiocyanate were processed as described in Example 1C to provide the desired product.
MS (APCI+) m/z 294 (M+H)⁺.

### Example 19D

### 4-chloro-N-(2,2,2-trifluoro-1-{[(3-pyridinylamino)carbothioyl]amino}ethyl)benzamide

Example 19C and 3-aminopyridine were processed as described in Example 1D to provide the desired product.
mp 147-149 °C;
MS (ESI+) m/z 389 (M+H)⁺;
¹H NMR (DMSO-d₆) δ 10.42 (s, 1H), 9.49-9.46 (m, 1H), 8.60 (d, 1H, J=3 Hz), 8.50-8.41 (m, 2H), 8.06-8.01 (m, 1H), 7.92 (d, 2H, J=9 Hz), 7.65 (d, 2H, J=9 Hz), 7.42 (q, 1H, J=3 Hz), 7.29-7.25 (m, 1H);
Anal. calcd for C₁₅H₁₂ClF₃N₄OS: C, 46.34; H, 3.11; N, 14.41. Found: C, 46.32; H, 3.10; N, 14.50.

### Example 20

### N-{ 1-[(anilinocarbothioyl)amino]-2,2,2-trifluoroethyl}-4-chlorobenzamide

Example 19C and aniline were processed as described in Example 1D to provide the desired product.
mp 198-201 °C;
MS (ESI+) m/z 357 (M+H)⁺;
¹H NMR (DMSO-d₆) δ 10.37 (br s, 1H), 9.37 (d, 1H, J=8 Hz), 8.15 (d, 1H, J=9 Hz), 7.91-7.86 (m, 2H), 7.63-7.58 (m, 2H), 7.51-7.45 (m, 2H), 7.42-7.26 (m, 3H), 7.22-7.17 (m, 1H);
Anal. calcd for C₁₆H₁₃ClF₃N₃OS: C, 49.55; H, 3.38; N, 10.84. Found: C, 48.71; H, 3.33; N, 10.86.

### Example 21

### 4-chloro-N-(2,2,2-trifluoro-1-{[(2-fluoroanilino)carbothioyl]amino}ethyl)benzamide

Example 19C and 2-fluoroaniline were processed as described in Example 1D to provide the desired product.
mp 197-200 °C;
MS (ESI+) m/z 357 (M+H)⁺;
¹H NMR (DMSO-d₆) δ 10.04 (br s, 1H), 9.36 (d, 1H, J=8 Hz), 8.35 (br s, 1H), 7.87-7.82 (m, 2H), 7.59-7.55 (m, 2H), 7.29-7.13 (m, 5H);
Anal. calcd for C₁₆H₁₂ClF₄N₃OS: C, 47.36; H, 2.98; N, 10.35. Found: C, 47.58; H, 2.86; N, 10.43.

### Example 22

### N-(2,2-dimethyl-1-{[(3-pyridinylamino)carbothioyl]amino}propyl)-4-methylbenzamide

### Example 22A

### 2-((tert-butoxycarbonyl)amino)-3,3-dimethylbutanoic acid

A stirred solution of racemic tert-butylglycine (2.15 g, 16.4 mmol), di-tert-butyl dicarbonate (4.65 g, 21.3 mmol) in dioxane (30 mL), and water (30 mL) at 5 °C was treated with N-methylmorpholine (2.07 mL, 24.6 mmol). The mixture was allowed to slowly warm to ambient temperature and stirred for 16 hours. The reaction mixture was poured into cold aqueous NaHCO₃ solution (50 mL) and extracted with ethyl acetate (3x50 mL). The aqueous layer was acidified to pH 1 (2M HCl) and extracted with ethyl acetate (3x30 mL). The organic fractions were combined, dried (MgSO₄), and filtered. Removal of solvent provided 2.47 g of the desired product as a white solid.
MS (APCI+) m/z 232 (M+H)⁺.

### Example 22B

### tert-butyl 1-(aminocarbonyl)-2,2-dimethylpropylcarbamate

A stirred solution of Example 22A (1.81 g, 7.83 mmol) in THF (20 mL) at -40 °C was treated with isobutyl chloroformate (1.01 mL, 7.83 mmol) followed by N-methylmorpholine (0.860 mL, 7.83 mmol). After 15 minutes, the milky white reaction mixture was treated dropwise with ammonium hydroxide (6.0 mL of 30% reagent, 15.0 mmol), the reaction flask was warmed to -15 °C, stirred for 45 minutes, treated with brine (20 mL), and the clear homogeneous mixture was extracted with ethyl acetate (2x40 mL). The organic portions were combined, washed with aqueous KHSO₄ solution (15 mL), aqueous NaHCO₃ solution (15 mL), and brine (15 mL), and dried (MgSO₄). Filtration and removal of solvent provided 1.68 g of the desired product as a white solid.
MS (APCI+) m/z 231 (M+H)⁺.

### Example 22C

### 2-amino-3,3-dimethylbutanamide mono(trifluoroacetate)

Example 22B (1.49 g, 6.47 mmol) was dissolved in CH₂Cl₂ (20 mL), cooled to 0 °C, treated with trifluoroacetic acid (5 mL), and the reaction was stirred for 3 hours at 0 °C. Solvents were removed under reduced pressure, the residue was taken up in brine (10 mL), and extracted with ethyl acetate (25 mL). The aqueous layer was basified with 2M aqueous K₂CO₃ solution and extracted with 25% isopropanol/chloroform (3x20 mL). The organic portions were combined and solvents were removed to provide 516 mg of the analytically pure desired product as a white solid.
MS (APCI+) m/z 131 (M+H)⁺.

### Example 22D

### N-(1-(aminocarbonyl)-2,2-dimethylpropyl)-4-methylbenzamide

A solution of Example 22C (489 mg, 3.76 mmol) and p-toluoyl chloride (0.560 mL, 4.14 mmol) in CH₂Cl₂ (8 mL) at 0 °C was treated with triethylamine (0.520 mL, 3.76 mmol). After 6 hours, the mixture was diluted with ethyl acetate (25 mL) and washed sequentially with 1M HCl (10 mL), saturated aqueous NaHCO₃ solution (10 mL), water (15 mL), and brine (10 mL). The organic portion was dried (Na₂SO₄), filtered, and concentrated to provide an oily residue which was purified by flash chromatography (elution with 50% ethyl acetate/hexanes) to provide 750 mg of the desired product as a sticky white solid.
MS (APCI+) m/z 249 (M+H)⁺.

### Example 22E

### N-(1-amino-2,2-dimethypropyl)-4-methylbenzamide hydrochloride

A solution of iodobenzene diacetate (1.50 g, 3.48 mmol) in 50% aqueous CH₃CN (6 mL) at ambient temperature was treated with Example 22D (721 mg, 2.90 mmol). After 16 hours, water (50 mL) and concentrated hydrochloric acid (5 mL) were added, and the mixture was extracted with ethyl acetate (2x40 mL). The aqueous layer was lyophilized leaving the hydrochloride salt which was triturated with diethyl ether to provide 422 mg of the desired product as a white solid.
MS (APCI+) m/z 221 (M+H)⁺.

### Example 22F

### N-(2,2-dimethyl-1-{[(3-pyridinylamino)carbothioyl]amino}propyl)-4-methylbenzamide

A solution of Example 22E (152 mg, 0.592 mmol) and 3-pyridyl isothiocyanate (81 mg, 0.58 mmol) in THF (3 mL) at ambient temperature was treated with triethylamine (0.83 mL, 0.59 mmol). The mixture was stirred for 10 hours, diluted with ethyl acetate (20 mL), and washed sequentially with 1M HCl (5 mL), saturated aqueous NaHCO₃ solution (5 mL), water (10 mL), and brine (5 mL). The organic portion was dried (MgSO₄), filtered, and concentrated to provide an oily residue which was purified by flash chromatography (elution with 2% ethanol/ethyl acetate) to provide 126 mg of the desired product as an off-white solid.
mp 168-169 °C;
MS (APCI+) m/z 357 (M+H)⁺;
¹H NMR (DMSO-d₆) δ 10.04 (s, 1H), 8.59 (d, 1H, J=9 Hz), 8.39-8.30 (m, 2H), 8.04 (d, 1H, J=9 Hz), 7.80 (d, 1H, J=9 Hz), 7.73 (d, 2H, J=9 Hz), 7.37 (dd, 1H, J=9, 3 Hz), 7.28 (d, 2H, J=9 Hz), 6.23 (br s, 1H), 2.35 (s, 3H), 1.00 (s, 9H);
Anal. calcd for C₁₉H₂₄N₄OS·0.2 C₄H₈O₂: C, 63.57; H, 6.90; N, 14.98. Found: C, 63.21; H, 6.80; N, 14.86.

### Example 23

### N-((1R)-2,2-dimethyl-1-{[(3-pyridinylamino)carbothioyl]amino}propyl)-4-methylbenzamide

Optically pure (R)-tert-butylglycine was processed as described in Example 22 to provide the desired product.
mp 168-170 °C;
MS (ESI+) m/z 357 (M+H)⁺;
¹H NMR (DMSO-d₆) δ 10.05 (s, 1 H), 8.59 (d, 1H, J=3 Hz), 8.39-8.31 (m, 2H), 8.05 (br d, 1H, J=9 Hz), 7.80 (br d, 1H, J=9 Hz), 7.78 (d, 2H, J=9 Hz), 7.38 (dd, 1H, J=9, 6 Hz), 7.28 (d, 2H, J=9 Hz), 6.25 (br s, 1 H), 2.36 (s, 3H), 1.00 (s, 9H);
Anal. calcd for C₁₉H₂₄N₄OS: C, 64.02; H, 6.79; N, 15.72. Found: C, 64.01; H, 6.91; N, 15.55.

### Example 24

### N-((1 1S)-2,2-dimethyl-1-{[(3-pyridinylamino)carbothioyl]amino}propyl)-4-methylbenzamide

Optically pure (S)-tert-butylglycine was processed as described in Example 22 to provide the desired product.
mp 166-168 °C;
MS (ESI+) m/z 357 (M+H)⁺;
¹H NMR (DMSO-d₆) δ 10.02 (s, 1H), 8.60 (s, 1H), 8.39-8.31 (m, 2H), 8.05 (br d, 1H, J=9 Hz), 7.82 (d, 1H, J=9 Hz), 7.75 (d, 2H, J=9 Hz), 7.38 (dd, 1H, J=9, 6 Hz), 7.28 (d, 2H, J=9 Hz), 6.25 (br s, 1H), 2.36 (s, 3H), 1.00 (s, 9H);
Anal. calcd for C₁₉H₂₄N₄OS: C, 64.02; H, 6.79; N, 15.72. Found: C, 64.09; H, 6.82; N, 15.44.

### Example 25

### N-(2,2-dimethyl-1-{[(3-nitroanilino)carbothioyl]amino}propyl)-4-methylbenzamide

Example 22E and 3-nitrophenyl isothiocyanate were processed as described in Example 22F to provide the desired product.
mp 181-182 °C;
MS (APCI+) m/z 401 (M+H)⁺;
¹H NMR (DMSO-d₆) δ 10.35 (s, 1H); 8.72 (s, 1H), 8.38-8.32 (m, 1H), 7.92 (q, 3H, J=9 Hz), 7.75 (d, 2H, J=9 Hz), 7.62 (t, 1H, J=9 Hz), 7.29 (d, 2H, J=9 Hz), 6.24 (br s, 1H), 2.35 (s, 3H), 1.00 (s, 9H);
Anal. calcd for C₂₀H₂₄N₄O₃S: C, 59.98; H, 6.04; N, 13.99. Found: C, 59.83; H, 6.10; N, 13.97.

### Example 26

### N-(2,2-dimethyl-1-{ [(3-pyridinylamino)carbothioyl]amino}propyl)-2-methylbenzamide

### Example 26A

### 3,3-dimethyl-2-((2-methylbenzoyl)amino)butanoic acid

Racemic tert-butylglycine (502 mg, 3.83 mmol) was dissolved in water (10 mL) containing NaOH (153 mg, 3.83 mmol). This solution was cooled to 5 °C and treated with o-toluoyl chloride (0.501 mL, 3.83 mmol) followed by additional NaOH (153 mg, 3.83 mmol). The mixture was warmed to ambient temperature, stirred for 1.5 hours, recooled to 5 °C, and treated with 1M HCl (pH 3). The thick precipitate that formed was collected by filtration and washed with cold water to provide 550 mg of the desired product as a white solid.
MS (APCI+) m/z 250 (M+H)⁺.

### Example 26B

### N-(1-(aminocarbonyl)-2,2-dimethylpropyl)-2-methylbenzamide

A stirred solution of Example 26A (458 mg, 1.84 mmol) in THF (10 mL) at -15 °C was treated with isobutyl chloroformate (0.240 mL, 1.84 mmol) followed by N-methylmorpholine (0.200 mL, 1.84 mmol). After 15 minutes, the milky white reaction mixture was treated dropwise with ammonium hydroxide (4.2 mL of 30% reagent, 15.0 mmol). The reaction flask was warmed to -15 °C, stirred for 45 minutes, treated with brine (20 mL), and the clear homogeneous mixture was extracted with ethyl acetate (2x40 mL). The organic portions were combined, washed with aqueous KHSO₄ solution (15 mL), aqueous NaHCO₃ solution (15 mL), and brine (15 mL), and dried (MgSO₄). Filtration and removal of solvent provided 232 mg of the desired product a white solid. MS (APCI+) m/z 249 (M+H)⁺.

### Example 26C

### N-(1-amino-2,2-dimethylpropyl)-2-methylbenzamide hydrochloride

Example 26B and iodobenzene diacetate were processed as described in Example 22E to provide the desired product.
MS (APCI+) m/z 221 (M+H)⁺.

### Example 26D

### N-(2,2-dimethyl-1-{[(3-pyridinylamino)carbothioyl]amino}propyl)-2-methylbenzamide

Example 26C and 3-pyridyl isothiocyanate were processed as described in Example 22F to provide the desired product.
mp 186-187°C;
MS (APCI+) m/z 357 (M+H)⁺;
¹H NMR (DMSO-d₆) δ 10.00 (s, 1H), 8.61 (d, 1H, J=3 Hz), 8.39-8.28 (m, 2H), 8.14 (d, 1H, J=9 Hz), 7.77 (d, 1H, J=9 Hz), 7.40-7.18 (m, 5H), 6.07 (br s, 1H), 2.34 (s, 3H), 1.02 (s, 9H);
Anal. calcd for C₁₉H₂₄N₄OS: C, 64.02; H, 6.79; N, 15.72. Found: C, 63.83; H, 6.69; N, 15.64.

### Example 27

### 4-chloro-N-(2,2-dimethyl-1-{[(3-pyridinylamino)carbothioyl]amino}propyl)benzamide

### Example 27A

### 2-((4-chlorobenzoyl)amino)-3,3-dimethylbutanoic acid

Racemic tert-butylglycine and 4-chlorobenzoyl chloride were processed as described in Example 26A to provide the desired product.
MS (APCI+) m/z 270 (M+H)⁺.

### Example 27B

### N-(1-(aminocarbonyl)-2,2-dimethylpropyl)-4-chlorobenzamide

Example 27A, isobutyl chloroformate, and ammonium hydroxide were processed as described in Example 26B to provide the desired product.
MS (APCI+) m/z 269 (M+H)⁺.

### Example 27C

### N-(1-amino-2,2-dimethylpropyl)-4-chlorobenzamide hydrochloride

Example 27B and iodobenzene diacetate were processed as described in Example 22E to provide the desired product.
MS (APCI+) m/z 241 (M+H)⁺.

### Example 27D

### 4-chloro-N-(2,2-dimethyl-1-{[(3-pyridinylamino)carbothioyl]amino}propyl)benzamide

Example 27C and 3-pyridyl isothiocyanate were processed as described in Example 22F to provide the desired product.
mp 164-167 °C;
MS (APCI+) m/z 377 (M+H)⁺;
¹H NMR (DMSO-d₆) δ 10.04 (s, 1H), 8.60 (d, 1H, J=3 Hz), 8.49 (d, 1H, J=9 Hz), 8.31 (dd, 1H, J=9, 3 Hz), 8.07 (br d, 1H, J=9 Hz), 7.88-7.76 (m, 3H), 7.55 (d, 1H, J=9 Hz), 7.36 (dd, 2H, J=9, 6 Hz), 6.18 (br s, 1H), 1.00 (s , 9H);
Anal. calcd for C₁₈H₂₁ClN₄OS: C, 57.36; H, 5.62; N, 14.87. Found: C, 57.19; H, 5.50; N, 14.73.

### Example 28

### N-(2,2-dimethyl-1-{[(3-pyridinylamino)carbothioyl]amino}propyl)benzamide

### Example 28A

### 2-(benzoylamino)-3,3-dimethylbutanoic acid

Racemic tert-butylglycine and benzoyl chloride were processed as described in Example 26A to provide the desired product.
MS (APCI+) m/z 236 (M+H)⁺.

### Example 28B

### N-(1-(aminocarbonyl)-2,2-dimethylpronyl)benzamide

Example 28A, isobutyl chloroformate, and ammonium hydroxide were processed as described in Example 26B to provide the desired product.
MS (APCI+) m/z 236 (M+H)⁺.

### Example 28C

### N-(1-amino-2,2-dimethylpropyl)benzamide hydrochloride

Example 28B and iodobenzene diacetate were processed as described in Example 22E to provide the desired product.
MS (APCI+) m/z 207 (M+H)⁺.

### Example 28D

### N-(2,2-dimethyl-1-{[(3-pyridinylamino)carbothioyl]amino}propyl)benzamide

Example 28C and 3-pyridyl isothiocyanate were processed as described in Example 22E to provide the desired product.
mp 193-194 °C;
MS (APCI+) m/z 343 (M+H)⁺;
¹H NMR (DMSO-d₆) δ 10.05 (s, 1H), 8.60 (d, 1H, J=3 Hz), 8.41 (d, 1H, J=9 Hz), 8.31 (dd, 1H, J=9, 3 Hz), 8.06 (br d, 1H, J=9 Hz), 7.85-7.78 (m, 3H), 7.57-7.42 (m, 3H), 7.36 (dd, 2H, J=9, 6 Hz), 6.22 (br s, 1H), 1.00 (s, 9H);
Anal. calcd for C₁₈H₂₂N₄OS: C, 63.13; H, 6.48; N, 16.36. Found: C, 62.99; H, 6.30; N, 16.24.

### Example 29

### 4-methyl-N-(1-{[(3-nitroanilino)carbothioyl]amino}ethyl)benzamide

### Example 29A

### N-(4-methylbenzoyl)alanine

Racemic alanine and p-toluoyl chloride were processed as described in Example 26A to provide the desired product.
MS (APCI+) m/z 208 (M+H)⁺.

### Example 29B

### benzyl 1-((4-methylbenzoyl)amino)ethylcarbamate

Example 29A (511 mg, 2.46 mmol) was suspended in benzyl alcohol (5 mL) at ambient temperature and treated with diisopropylethylamine (0.470 mL, 2.71 mmol). The homogeneous solution was treated with diphenyl phosphorylazide (0.580 mL, 2.71 mmol), stirred at ambient temperature for 30 minutes, then stirred at 90 °C for 12 hours. The reaction was cooled, and benzyl alcohol was removed in vacuo. The resulting residue was dissolved in ethyl acetate (30 mL) and washed sequentially with 10% aqueous citric acid (10 mL), saturated aqueous NaHCO₃ (10 mL), water (10 mL), and brine (5 mL). Removal of solvent left an oily residue that was recrystallized from hot ethyl acetate/hexanes to provide a white precipitate which was collected by filtration and washed with hexanes to provide 77 mg of the desired product as a white solid.
MS (APCI+) m/z 313 (M+H)⁺.

### Example 29C

### N-(1-aminoethyl)-4-methylbenzamide hydrochloride

A suspension of Example 29B (66 mg, 0.21 mmol) in methanol (9 mL) at ambient temperature was treated with 10% Pd/C (20 mg) and sufficient 1 M HCl to solubilize the substrate (ca. 0.20 mL). The system was equipped with a hydrogen balloon and stirred for 4 hours at ambient temperature. The reaction mixture was purged with nitrogen, filtered through diatomaceous earth (Celite®), rinsed with methanol and water, and the aqueous filtrate was washed with diethyl ether (2×15 mL). The aqueous layer was lyophilized providing 38 mg of the desired product as a white solid.
MS (APCI+) m/z 179 (M+H)⁺.

### Example 29D

### 4-methyl-N-(1-{[(3-nitroanilino)carbothioyl]amino]ethyl)benzamide

Example 29C and 3-nitrophenyl isothiocyanate were processed as described in Example 22E to provide the desired product.
mp 172-175 °C;
MS (APCI+) m/z 359 (M+H)⁺;
¹H NMR (DMSO-d₆) δ 10.32 (s, 1H), 8.75 (s, 1H), 8.31 (br s, 1H), 7.98 (m, 3H), 7.81 (d, 2H, J=9 Hz), 7.62 (t, 1H, J=9 Hz), 7.28 (d, 2H, J=9 Hz), 6.15 (br s, 1H), 2.53 (d, 3H, J=6 Hz), 2.37 (s, 3H);
Anal. calcd for C₁₇H₁₈N₄O₃S·0.5 CH₂Cl₂: C, 52.43; H, 4.78; N, 13.98. Found: C, 52.43; H, 4.60; N, 13.85.

### Example 30

### 4-methyl-N-(1-{[(3-nitroanilino)carbothioyl]amino}-2-phenylethyl)benzamide

### Example 30A

### N-(4-methylbenzoyl)phenylalanine

Racemic phenylalanine and p-toluoyl chloride were processed as described in Example 26A to provide the desired product.
MS (APCI+) m/z 284 (M+H)⁺.

### Example 30B

### N-(2-amino-1-benzyl-2-oxoethyl)-4-methylbenzamide

Example 30A, isobutyl chloroformate, and ammonium hydroxide were processed as described in Example 26B to provide the desired product.
MS (APCI+) m/z 283 (M+H)⁺.

### Example 30C

### N-(1-amino-2-phenylethyl)-4-methylbenzamide hydrochloride

Example 30B and iodobenzene diacetate were processed as described in Example 22E to provide the desired product.
MS (APCI+) m/z 255 (M+H)⁺.

### Example 30D

### 4-methyl-N-(1{[(3-nitroanilino)carbothioyl]amino}-2-phenylethyl)benzamide

Example 30C and 3-nitrophenyl isothiocyanate were processed as described in Example 22E to provide the desired product.
mp 170-171 °C;
MS (APCI+) m/z 435 (M+H)⁺;
¹H NMR (DMSO-d₆) δ 10.16 (s, 1H), 8.75 (s, 1H), 8.40 (br s, 1H), 7.99-7.88 (m, 3H), 7.75 (d, 2H, J=9 Hz), 7.60 (t, 1H, J=9 Hz), 7.42-7.27 (m, 5H), 7.20 (d, 2H, J=9 Hz), 6.20 (br s, 1H), 3.25 (m, 2H), 2.35 (s, 3H);
Anal. calcd for C₂₃H₂₂N₄O₃S: C, 63.58; H, 5.10; N, 12.89. Found: C, 63.32; H, 5.09; N, 12.74.

### Example 31

### N-((1R)2-tert-butoxy)-1-{[(3-nitroanilino)carbothioyl]amino}ethyl)-4-methylbenzamide

### Example 31 A

### benzyl (1 S)-2-amino-1-(tert-butoxymethyl)-2-oxoethylcarbamate

A stirred solution of racemic (2S)-2-(((benzyloxy)carbonyl)amino)-3-tert-butoxypropanoic acid (1.01 g, 3.43 mmol) in THF (10 mL) at -15 °C was treated with isobutyl chloroformate (0.440 mL, 3.43 mmol) followed by N-methylmorpholine (0.380 mL, 3.43 mmol). After 15 minutes, the milky white reaction mixture was treated dropwise with ammonium hydroxide (2.8 mL of 30% reagent, 5.0 mmol). The reaction flask was warmed to -15 °C and stirred for 45 minutes. The clear homogeneous mixture was treated with brine (20 mL) and extracted with ethyl acetate (2x40 mL). The organic portions were combined and washed with saturated aqueous NaHCO₃ solution (15 mL) and brine (15 mL), and dried (MgSO₄). Filtration and removal of solvent provided 928 mg of the desired product a white solid.
MS (APCI+) m/z 295 (M+H)⁺.

### Example 31 B

### (2S)-2-amino-3-(tert-butoxy)propanamide hydrochloride

A suspension of Example 31A (784 mg, 2.67 mmol) in methanol (9 mL) at ambient temperature was treated with 10% Pd/C (75 mg) and sufficient 1 M HCl to solubilize the substrate (ca. 0.50 mL). The system was equipped with a hydrogen balloon and stirred for 4 hours at ambient temperature. The reaction mixture was purged with nitrogen, filtered through diatomaceous earth (Celite®), rinsed with methanol and water, and the aqueous filtrate was washed with diethyl ether (2x40 mL). The aqueous layer was lyophilized providing 467 mg of the desired product as a white solid.
MS (APCI+) m/z 161 (M+H)⁺.

### Example 31 C

### N-((1S)-2-amino-1-(tert-butoxymethyl)-2-oxoethyl)-4-methylbenzamide

A solution of Example 31C (435 mg, 2.21 mmol) and p-toluoyl chloride (0.320 mL, 2.43 mmol) in CH₂Cl₂ (12 mL) at 0 °C was treated with triethylamine (0.310 mL, 2.21 mmol). After 3 hours, the mixture was diluted with ethyl acetate (25 mL) and washed sequentially with 1M HCl (10 mL), saturated aqueous NaHCO₃ solution (10 mL), water (15 mL), and brine (10 mL). The organic portion was dried (Na₂SO₄), filtered, and concentrated to provide 606 mg of the desired product as a white solid.
MS (APCI+) m/z 279 (M+H)⁺.

### Example 31 D

### N-((1S)-1-amino-2-(tert-butoxy ethyl)-4-methylbenzamide hydrochloride

Example 31C and iodobenzene diacetate were processed as described in Example 22E to provide the desired product.
MS (APCI+) m/z 251 (M+H)⁺.

### Example 31 E

### N-((1R)-2-(tert-butoxy)-1-{[(3-nitroanilino carbothioyl]amino}ethyl)-4-methylbenzamide

Example 31D and 3-nitrophenyl isothiocyanate were processed as described in Example 22F to provide the desired product.
mp 169-171 °C;
MS (APCI+) m/z 431 (M+H)⁺;
¹H NMR (DMSO-d₆) δ: 10.32 (s, 1H), 8.75 (s, 1H), 8.15 (br s, 1H), 7.99-7.88 (m, 3H), 7.78 (d, 2H, J=9 Hz), 7.62 (t, 1H, J=9 Hz),), 7.30 (d, 2H, J=9 Hz), 3.70-3.61 (m, 2H), 2.35 (s, 3H), 1.15 (s, 1 H);
Anal. calcd for C₂₁H₂₆N₄O₄S: C, 58.59; H, 6.09; N, 13.01. Found: C, 58.56; H, 5.99; N, 12.94.

### Example 32

### N-(2-fluoro-1-{[(3-nitroanilino)carbothioyl]amino}ethyl)-4-methylbenzamide

### Example 32A

### 3-fluoro-N-(4-methylbenzoyl)alanine

Racemic fluoromethyl glycine and p-toluoyl chloride were processed as described in Example 26A to provide the desired product.
MS (APCI+) m/z 226 (M+H)⁺.

### Example 32B

### N-(2-amino-1-(fluoromethyl)-2-oxoethyl)-4-methylbenzamide

Example 32B, isobutyl chloroformate, and ammonium hydroxide were processed as described in Example 26B to provide the desired product.
MS (APCI+) m/z 225 (M+H)⁺.

### Example 32C

### N-(1-amino-2-fluoroethyl)-4-methylbenzamide hydrochloride

Example 32B and iodobenzene diacetate were processed as described in Example 22E to provide the desired product.
MS (APCI+) m/z 197 (M+H)⁺.

### Example 32D

### N-(2-fluoro-1- {[(3-nitroanilino)carbothioyl]amino}ethyl)-4-methylbenzamide

Example 32C and 3-nitrophenyl isothiocyanate were processed as described in Example 22F to provide the desired product.
mp 148-155 °C;
MS (APCI+) m/z 377 (M+H)⁺;
¹H NMR (DMSO-d₆) δ 10.35 (s, 1H), 9.06 (s, 1H), 8.70 (d, 1H, J=9 Hz), 8.81 (br s, 1H), 7.96 (d, 2H, J=9 Hz), 7.90-7.78 (m, 3H), 7.60 (t, 1H, J=9 Hz),), 7.31 (d, 2H, J=9 Hz), 6.35 (m, 2H), 4.76 (d, 1H, J=7 Hz), 4.60 (d, 1H, J=7 Hz), 2.38 (s, 3H);
Anal. calcd for C₁₇H₁₇FN₄O₃S: C, 54.25; H, 4.55; N, 14.88. Found: C, 54.36; H, 4.59; N, 14.57.

### Example 33

### 4-methyl-N-[{(3-nitroanilino)carbothioyl]amino}(phenyl)methyl]benzamide

### Example 33A

### 2-((4-methylbenzoyllaminol-2-phenylacetic acid

Racemic phenylglycine and p-toluoyl chloride were processed as described in Example 26A to provide the desired product.
MS (APCI+) m/z 270 (M+H)⁺.

### Example 33B

### N-(2-amino-2-oxo-1-phenylethyl)-4-methylbenzamide

Example 33A, isobutyl chloroformate, and ammonium hydroxide were processed as described in Example 26B to provide the desired product.
MS (APCI+) m/z 269 (M+H)⁺.

### Example 33C

### N-(amino(phenyl)methyl)-4-methylbenzamide hydrochloride

Example 33B and iodobenzene diacetate were processed as described in Example 22E to provide the desired product.
MS (APCI+) m/z 241 (M+H)⁺.

### Example 33D

### 4-methyl-N-[{[(3-nitroanilino)carbothioyl]amino}(phenyl)methyl]benzamide

Example 33C and 3-nitrophenyl isothiocyanate were processed as described in Example 22F to provide the desired product.
mp 170-172 °C;
MS (APCI+) m/z 421 (M+H)⁺;
¹H NMR (DMSO-d₆) δ 10.44 (s, 1H), 9.45 (d, 1H, J= 8 Hz), 8.80 (s, 1H), 8.67 (d, 1H, J=8 Hz), 7.96 (dd, J=8, 4, 1H), 7.90 (dd, J=8, 4, 1H), 7.83 (d, 2H, J=8 Hz), 7.62 (t, 1H, J=8 Hz), 7.48-7.36 (m, 4H), 7.35-7.25 (m, 4H), 2.38 (s, 3H);
Anal. calcd for C₂₂H₂₀N₄O₃S·0.4 H₂O: C, 61.78; H, 4.90; N, 13.10. Found: C, 61.49; H, 4.72; N, 13.21.

### Example 34

### 4-methyl-N-(phenyl{[(3-pyridinylamino)carbothioyl]amino}methyl)benzamide

Example 33C and 3-pyridyl isothiocyanate were processed as described in Example 22F to provide the desired product.
mp 156-158 °C;
MS (ESI+) m/z 357 (M+H)⁺;
¹H NMR (DMSO-d₆) δ 8.04 (m, 2H), 7.84-7.73 (m, 3H), 7.50 (br s, 1H), 7.43-7.39 (m, 1H), 7.29-7.21 (m, 3H), 7.22-7.13 (m, 5H), 7.05 (br s, 1H), 4.63 (t, 1H, J=9 Hz), 2.38 (s, 3H);
Anal. calcd for C₂₁H₂₀N₄OS: C, 67.00; H, 5.35; N, 14.88. Found: C, 66.87; H, 5.38; N, 14.81.

### Example 35

### 4-methyl-N-(2-methyl-1{[(3-pyridinylamino)carbothioyl]amino}propyl)benzamide

### Example 35A

### N-(4-methylbenzoyl)valine

Racemic valine and p-toluoyl chloride were processed as described in Example 26A to provide the desired product.
MS (APCI+) m/z 236 (M+H)⁺.

### Example 35B

### N-(1-(aminocarbonyl)-2-methylpropyl)-4-methylbenzamide

Example 35A, isobutyl chloroformate, and ammonium hydroxide were processed as described in Example 26B to provide the desired product.
MS (APCI+) m/z 235 (M+H)⁺.

### Example 35C

### N-(1-amino-2-methylpropyl)-4-methylbenzamide hydrochloride

Example 35B and iodobenzene diacetate were processed as described in Example 22E to provide the desired product.
MS (APCI+) m/z 207 (M+H)⁺.

### Example 35D

### 4-methyl-N-(2-methyl-1-1-{[(3-pyridinylamino)carbothioyl]amino}propyl)benzamide

Example 35C and 3-pyridyl isothiocyanate were processed as described in Example 22F to provide the desired product.
mp 184-186 °C;
MS (ESI+) m/z 343 (M+H)⁺;
¹H NMR (DMSO-d₆) δ 8.05 (m, 2H), 7.86-7.74 (m, 3H), 7.48 (br s, 1H), 7.43-7.36 (m, 1H), 7.30-7.22 (m, 3H), 7.05 (br s, 1H), 4.25 (t, 1H, J=9 Hz), 2.38 (s, 3H), 2.17-2.08 (m, 1H), 1 .04-0.91 (m, 6H);
Anal. calcd for C₁₈H₂₂N₄OS: C, 63.13; H, 6.48; N, 16.36. Found: C, 63.35; H, 6.38; N, 16.47.

### Example 36

### 4-methyl-N-((1R,2S)-2-methyl-{[3-pyridinylamino)carbothioyl]amino}butyl)benzamide

### Example 36A

### (2R,3R)-3-methyl-2-((4-methylbenzoyl)amino)pentanoic acid

(L)-Isoleucine and p-toluoyl chloride were processed as described in Example 26A to provide the desired product.
MS (APCI+) m/z 250 (M+H)⁺.

### Example 36B

### N-((1R,2R)-1-(aminocarbonyl)-2-methylbutyl)-4-methylbenzamide.

Example 36B, isobutyl chloroformate, and ammonium hydroxide were processed as described in Example 26B to provide the desired product.
MS (APCI+) m/z 249 (M+H)⁺.

### Example 36C

### N-((1R, 2R)-1-amino-2-methylbutyl)-4-methylbenzamide hydrochloride

Example 36B and iodobenzene diacetate were processed as described in Example 22E to provide the desired product.
MS (APCI+) m/z 221 (M+H)⁺.

### Example 36D

### 4-methyl-N-((1R,2S)-2-methyl-1-{[(3-pyridinylamino)carbothioyl]amino}butyl)benzamide

Example 36C and 3-pyridyl isothiocyanate were processed as described in Example 22F to provide the desired product.
mp 102-104 °C;
MS (ESI+) m/z 357 (M+H)⁺;
¹H NMR (DMSO-d₆) δ 10.05 (s, 1H), 8.62 (s, 1H), 8.30 (d, 1H, J=3 Hz), 8.11-8.07 (m, 2H), 7.88 (br s, 1H), 7.75 (d, 2H, J=9 Hz), 7.38-7.33 (m, 1H), 7.25 (d, 2H, J=9 Hz), 6.10 (br s, 1H), 2.38 (s, 3H), 2.15-2. 10 (m, 1H), 1.55-1.51 (m, 1H), 1.26-1.18 (m, 1H), 0.96-0.89 (m, 6H);
Anal. calcd for C₁₉H₂₄N₄OS: C, 64.02; H, 6.79; N, 15.72. Found: C, 64.24; H, 6.74; N, 15.41.

### Example 37 (Comparative example)

### 4-methyl-N-{2,2.2-trichloro-1-[3-(3-fluorophenyl)-2-thioxo-1-imidazolidinyl]ethyl} benzamide

### Example 37A

### N-(3-fluorophenyl)-1,2-ethanediamine monohydrochloride

Equimolar quantities (45.7 mmol) of 2-oxazolidinone and 3-fluoroaniline hydrochloride were combined, heated to 165 °C, and stirred for 18 hours. The dark-colored mixture was allowed to cool to ambient temperature and treated with ethanol (125 mL). The suspension was heated to reflux for 1 hour, allowed to cool to ambient temperature, treated with diethyl ether (125 mL), and the suspension was cooled to 0 °C for 2 hours. Filtration provided 5.53 g of the desired product as colorless crystals which were used without further purification.
MS (ESI+) m/z 155 (M+H)⁺.

### Example 37B

### N-(3-fluorophenyl)-2-imidazolinethione

A suspension of Example 37A (3.70 g, 19.5 mmol), in CH₂Cl₂ (50 mL) at ambient temperature was treated with triethylamine (4.5 mL). The reaction was treated with 1,1'-thiocarbonyldiimidazole (3.86 g, 19.5 mmol) and allowed to proceed for 1 hour at ambient temperature. The reaction mixture was poured into 2 N HCl (100mL), and the aqueous phase was extracted with CH₂Cl₂ (2x25 mL). The combined organic extracts were washed with brine (25 mL), dried over anhydrous Na₂SO₄, filtered, and the solvent was removed in vacuo. Trituration with ether provided 3.14 g of the pure desired product as colorless crystals.
MS (ESI+) m/z 197 (M+H)⁺.

### Example 37C

### 4-methyl-N-(2,2,2-trichloro-1-hydroxyethyl)benzamide

A mixture of p-toluamide (11.0 g, 81.3 mmol) and 2,2,2-trichloro-1,1-ethanediol (16.6 g, 100 mmol) in benzene (175 mL) was stirred at reflux in a Soxhlet extraction device charged with molecular sieves (20 g). After 12 hours, the extraction thimble of molecular sieves was replaced with a fresh portion of molecular sieves (20 g), and the reaction was allowed to heat at reflux for an additional 12 hours. Concentration in vacuo provided a heavy syrup which was dissolved in ethyl acetate (15 mL) and diluted with hexanes (175 mL). Refrigeration at 8 °C facilitated precipitation of a white solid that was collected by filtration and washed with hexanes to provide 20.2 g of the desired product. MS (APCI+) m/z 264 (M-H₂O)⁺.

### Example 37D

### 4-methyl-N-(1,2,2,2-tetrachloroethyl)benzamide

A stirred solution of Example 37C (20.0 g, 70.8 mmol) in CH₂Cl₂ (300 mL) at 0 °C was treated with pyridine (10 mL). The reaction mixture was treated dropwise with thionyl chloride (10.4 mL, 141 mmol), and the reaction flask was equipped with a calcium chloride drying tube. The reaction mixture was warmed to ambient temperature and stirred for 4 hours. Concentration of the reaction mixture to a reduced volume and addition of diethyl ether (100 mL) resulted in a precipitate which was filtered off, and the filtrate was concentrated and dried under reduced pressure to provide 19.0 g of the desired product as a white solid.
MS (APCI+) m/z 300 (M+H)⁺.

### Example 37E

### 4-methyt-N-{2,2,2-trichloro-1-[3-(3-fluorophenyl)-2-thioxo-1-imidazolidinyl]ethyl} benzamide

A solution of Example 37B (0.460 g, 2.35 mmol) in DMF (10 mL) was treated with solid potassium bis(trimethylsilyl)amide (0.470 g, 2.35 mmol). The reaction was stirred at ambient temperature for 15 minutes, treated with Example 37D (0.710 g, 2.35 mmol), allowed to proceed for 16 hours, diluted with ethyl acetate (30 mL), and poured into 2 N HCl (15 mL). The aqueous phase was extracted with ethyl acetate (2x5 mL), and the combined organic extracts were washed with brine (25 mL), dried (Na₂SO₄), filtered, and evaporated in vacuo. Purification by flash chromatography (elution with 50% ethyl acetate/hexanes) provided 460 mg of the desired product as a white powder. mp 184-186 °C;
MS (ESI+) m/z 460 (M+H)⁺;
¹H NMR (DMSO-d₆) δ 9.30 (d, 1H, J=10 Hz), 7.95 (d, 1H, J=10 Hz), 7.81 (d, 2H, J=8 Hz), 7.64-7.57 (m, 1H), 7.50-7.40 (m, 3H), 7.35 (d, 2H, J=8 Hz), 7.13-7.06 (m, 1H), 4.28-4.01 (m, 4H), 2.39 (s, 3H);
Anal. calcd for C₁₉H₁₇Cl₃FN₃OS: C, 49.53; H, 3.72; N, 9.12. Found: C, 49.21; H, 3.88; N, 8.97.

### Example 38

### 4-methyl-N-(2,2,2-trichloro-1-{(3-pyridinylamino)carbonyl]amino}ethyl)benzamide

### Example 38A

### N-( 1-amino-2,2,2-trichloroethyl)-4-methylbenzamide

Diethyl ether (250 mL) was placed in a 500 mL three-necked round-bottom flask fitted with a gas dispersion tube, dropping funnel, and stirrer. The solvent was cooled to 0 °C, and dry ammonia was passed into the solution for 10 minutes. While the ammonia was allowed to continue to pass through the solution, it was treated dropwise with Example 37D (9.50 g, 31.6 mmol) in diethyl ether (50 mL) for 30 minutes. The white solid that formed was removed by filtration and washed with diethyl ether. The combined filtrates were concentrated to provide 9.18 g of the desired product as a white solid.
MS (APCI+) m/z 281 (M+H)⁺.

### Example 38B

### 4-methyl-N-(2,2,2-trichloro-1-(((3-pyridinylamino)carbothioyl)amino)ethyl)benzamide

Example 38A (500 mg, 2.79 mmol) and 3-pyridyl isothiocyanate (335 mg, 2.79 mmol) were dissolved in CH₂Cl₂ (15 mL) and heated at 60 °C for 10 hours. The reaction was cooled and concentrated to provide a residue that was purified by flash chromatography (elution with 20% ethyl acetate/hexanes) to provide 396 mg of the desired product as an off-white solid.
MS (APCI+) m/z 417 (M+H)⁺.

### Example 38C

### 4-methyl-N-(2,2,2-trichloro-1-{[(3-pyridinylamino)carbonyl]amino}ethyl)benzamide

A solution of Example 38B (250 mg, 0.200 mmol) in glacial acetic acid (4 mL) at ambient temperature was treated slowly with acetic acid (6 mL) containing 30% hydrogen peroxide (4 mL), and the reaction was stirred for 30 minutes. The precipitate which formed was filtered off, washed with water, and crystallized from ethanol. The salt was dissolved in ethyl acetate and neutralized with saturated aqueous sodium carbonate solution. The organic solution was washed with brine (5 mL), dried (Na₂SO₄), and concentrated to provide a white solid. Recrystallization from ethanol/diethyl ether provided 71 mg of the desired product as a white solid.
mp 162-163 °C;
MS (ESI+) m/z 401 (M)⁺;
¹H NMR (DMSO-d₆) δ 9.60 (s, 1H), 9.20 (d, 1H, J=9 Hz), 8.68 (s, 1H), 8.22 (d, 1H, J=6 Hz), 7.98 (d, 1H, J=9 Hz), 7.78 (d, 2H, J=9 Hz), 7.38 (q, 1H, J=3 Hz), 7.32 (d, 2H, J=9 Hz), 7.12 (d, 1H, J=9 Hz), 6.75 (t, 1H, J=9 Hz), 2.38 (s, 3H);
Anal. calcd for C₁₆H₁₅Cl₃N₄O₂: C, 47.84; H, 3.76; N, 13.95. Found: C, 47.81; H, 3.67; N, 13.62.

### Example 39

### 2-methyl-N-(2,2,2-trichloro-1-{[3-pyridinylamino)carbonyl]amino}ethyl)benzamide

### Example 39A

### 2-methyl-N-(2,2,2-trichloro-1-hydroxyethyl)benzamide

2,2,2-Trichloro-1,1-ethanediol and o-toluamide were processed as described in Example 37C to provide the desired product.
MS (APCI+) m/z 263 (M-H₂O)⁺.

### Example 39B

### 2-methyl-N-(1,2,2,2-tetrachloroethyl)benzamide

Example 39A and thionyl chloride were processed as described in Example 37D to provide the desired product.
MS (APCI+) m/z 300 (M+H)⁺.

### Example 39C

### N-(1-amino-2,2,2-trichloroethyl)-2-methylbenzamide

Example 39B and ammonia were processed as described in Example 38A to provide the desired product.
MS (APCI+) m/z 281 (M+H)⁺.

### Example 39D

### 2-methyl-N-(2,2,2-trichloro-1-(((3-pyridinylamino)carbothioyl)amino)ethyl)benzamide

Example 39C and 3-pyridyl isothiocyanate were processed as described in Example 38B to provide the desired product.
MS (APCI+) m/z 417 (M+H)⁺.

### Example 39E

### 2-methyl-N-(2,2,2-trichloro-1-{ [(3-pyridinylamino)carbonyl]amino} ethyl)benzamide

Example 39D and hydrogen peroxide were processed as described in Example 38C to provide the desired product.
mp 182-184 °C;
MS (APCI+) m/z 402 (M+H)⁺;
¹H NMR (DMSO-d₆) δ 9.38 (s, 1H), 8.95 (s, 1H), 8.55 (s, 1H), 8.20 (d, 1H, J=6 Hz), 7.93-7.88 (m, 1H), 7.68 (d, 2H, J=3 Hz), 7.44-7.28 (m, 4H), 6.72 (t, 1H, J=9 Hz), 2.37 (s, 3H);
Anal. calcd for C₁₆H₁₅Cl₃N₄O₂: C, 47.84; H, 3.76; N, 13.95. Found: C, 47.68; H, 3.52; N, 13.59.

### Example 40

### N-(2,2,2-trichloro-1-{[(3-pyridinylamino)carbonyl]amino}ethyl)benzamide

### Example 40A

### N-(2,2,2-trichloro-1-hydroxyethyl)benzamide

2,2,2-Trichloro-1,1-ethanediol and benzamide were processed as described in Example 37C to provide the desired product.
MS (APCI+) m/z 245 (M-H₂O)⁺.

### Example 40B

### N-(1,2,2,2-tetrachloroethyl)benzamide

Example 40A and thionyl chloride were processed as described in Example 37D to provide the desired product.
MS (APCI+) m/z 286 (M+H)⁺,

### Example 40C

### N-(1-amino-2,2,2-trichloroethyl)benzamide

Example 40B and ammonia were processed as described in Example 38A to provide the desired product.
MS (APCI+) m/z 267 (M+H)⁺.

### Example 40D

### N-2,2,2-trichloro-1-(((3-pyridinylamino)carbothioyl)amino)ethyl)benzamide

Example 40C and 3-pyridyl isothiocyanate were processed as described in Example 38B to provide the desired product.
MS (APCI+) m/z 403 (M+H)⁺.

### Example 40E

### N-(2,2,2-trichloro-1-{[(3-pyridinylamino)carbonyl]amino}ethyl)benzamide

Example 40D and hydrogen peroxide were processed as described in Example 38C to provide the desired product.
mp 214-215 °C;
MS (ESI+) m/z 388 (M+H)⁺;
¹H NMR (DMSO-d₆) δ 9.48 (s, 1H), 9.32 (d, 1H, J=9 Hz), 8.55 (d, 1H, J=3 Hz), 8.20 (dd, 1H, J=6, 3 Hz), 7.95-7.89. (m, 1H), 7.85 (d, 2H, J=9 Hz), 7.64-7.59 (m, 1H), 7.52 (t, 2H, J=9 Hz), 7.32 (q, 1H, J=3 Hz), 7.10 (d, 1H, J=9 Hz), 6.75 (t, 1H, J=9 Hz);
Anal. calcd for C₁₅H₁₃Cl₃N₄O₂: C, 46.48; H, 3.38; N, 14.45. Found: C, 46.24; H, 3.41; N, 14.41.

### Example 41

### 4-chloro-N-(2,2,2-trichloro-1-{[(3-pyridinylamino)carbonyl]amino}ethyl)benzamide

### Example 41 A

### 4-chloro-N-(2,2,2-trichloro-1-hydroxyethyl)benzamide

2,2,2-Trichloro-1,1-ethanediol and 4-chlorobenzamide were processed as described in Example 37C to provide the desired product.
MS (APCI+) m/z 283 (M-H₂O)⁺.

### Example 41 B

### 4-chloro-N-(1,2,2,2-tetrachloroethyl)benzamide

Example 41 A and thionyl chloride were processed as described in Example 37D to provide the desired product.
MS (APCI+) m/z 320 (M+H)⁺.

### Example 41 C

### N-( 1-amino-2,2,2-trichloroethyl)-4-chlorobenzamide

Example 41B and ammonia were processed as described in Example 38A to provide the desired product.
MS (APCI+) m/z 301 (M+H)⁺.

### Example 41 D

### 4-chloro-N-(2,2,2-trichloro-1-(((3-pyridinylamino)carbothioyl)amino)ethyl)benzamide

Example 41C and 3-pyridyl isothiocyanate were processed as described in Example 38B to provide the desired product.
MS (APCI+) m/z 437 (M+H)⁺.

### Example 41 E

### 4-chloro-N-(2,2,2-trichloro-1-{[(3-pyridinylamino)carbonyl]amino} ethyl)benzamide

Example 41D and hydrogen peroxide were processed as described in Example 38C to provide the desired product.
mp 166-168 °C;
MS (ESI+) m/z 423 (M+H)⁺;
¹H NMR (DMSO-d₆) δ 9.45 (s, 1H), 8.62 (s, 1H), 8.20 (d, 1H, J=6 Hz), 7.98-7.80 (m, 4H), 7.65-7.59 (m, 2H), 7.52 (d, 1H, J=9 Hz), 7.30 (q, 1H, J=3 Hz), 7.10 (d, 1H, J=9 Hz), 6.75 (t, 1H, J=9 Hz);
Anal. calcd for C₁₅H₁₂Cl₄N₄O₂: C, 42.68; H, 2.87; N, 13.27. Found: C, 42.56; H, 2.67; N, 13.25.

### Example 42

### N-{1-[(anilinocarbonyl)amino]-2,2,2-trichloroethyl}-4-methylbenzamide

A solution of Example 38A (183 mg, 0.650 mmol) and phenyl isocyanate (77 mg, 0.65 mmol) in THF (4 mL) at ambient temperature was treated with triethylamine (0.93 mL, 0.66 mmol). The mixture was stirred for 10 hours, diluted with ethyl acetate (20 mL), and washed with aqueous NH₄Cl (10 mL), water (10 mL), and brine (5 mL). The organic portion was dried (MgSO₄), filtered, and concentrated to provide an oily residue which was purified by flash chromatography (elution with 5% methanol/CH₂Cl₂) to provide 151 mg of the desired product as an off-white solid.
mp 230-233 °C;
MS (ESI+) m/z 400 (M)⁺;
¹H NMR(DMSO-d₆) δ 9.35 (s, 1H), 9.25 (d, 1H, J=9 Hz), 7.80 (d, 2H, J=9 Hz), 7.45 (d, 2H, J=9 Hz), 7.32 (d, 2H, J=9 Hz), 7.25 (q, 3H, J=7 Hz), 6.95 (t, 1H, J=7 Hz), 6.75 (t, 1 H, J=9 Hz), 2.45 (s, 3H);
Anal. calcd for C₁₇H₁₆Cl₃N₃O₂: C, 50.96; H, 4.02; N, 10.49. Found: C, 50.66; H, 3.99; N, 10.38.

### Example 43

### 4-methyl-N-(2,2,2-trichloro-1-{[(2-fluoroanilino)carbonyl]amino}ethyl)benzamide

### Example 43A

### 4-methyl-N-(2,2,2-trichloro-1-isocyanatoethyl)benzamide

A stirred solution of Example 37D (1.00 g, 3.32.00 mmol) in acetone (20 mL) at ambient temperature was treated with potassium cyanate (1.60 g, 16.0 mmol). The reaction mixture was stirred for 12 hours, concentrated, and the crude residue was purified by flash chromatography (elution with 50% ethyl acetate/hexanes) to provide 620 mg of the desired product as an off-white solid.
MS (APCI+) m/z 307 (M+H)⁺.

### Example 43B

### 4-methyl-N-(2,2,2-trichloro-1-{[(2-fluoroanilino)carbonyl]amino}ethyl)benzamide

A solution of 2-fluoroaniline (132 mg, 1.40 mmol) in THF (8 mL) at ambient temperature was treated with a solution of Example 43A (386 mg, 1.26 mmol) in THF (1.5 mL). The reaction mixture was stirred for 2 hours and concentrated to a nominal volume. The white solids which precipitated from solution were collected by filtration and washed with diethyl ether. Recrystallization from 25% ethyl acetate/hexanes provided the desired product as a white solid.
mp 257-259 °C;
MS (APCI+) m/z 418 (M+H)⁺;
¹H NMR (DMSO-d₆) δ 9.32-9.29 (d, 1H, J=9 Hz), 9.14 (br s, 1H), 8.12-8.06 (t, 1H, J=8 Hz), 7.81 (d, 2H, J=8 Hz), 7.55 (d, 1H, J=10 Hz), 7.30 (d, 2H, J=8 Hz), 7.25-7.18 (m, 1H), 7.11 (t, 1H, J=7 Hz), 7.03-6.96 (m, 1H), 6.78 (t, 1H, J=9 Hz), 2.37 (s, 3H);
Anal. calcd for C₁₇H₁₅Cl₃FN₃O₂: C, 48.77; H, 3.61; N, 10.04. Found: C, 48.76; H, 3.53; N, 9.97.

### Example 44

### 4-methyl-N-(2,2,2-trichloro-1- {[(cyanoimino)(3-pyridinylamino)methyl]amino}ethyl)benzamide

### Example 44A

### N-cyano-N'-(3-pyridinyl)thiourea

A solution of cyanamide (352 mg, 8.40 mmol) in THF (20 mL) at 0 °C was treated with sodium hydride (211 mg of 95% reagent, 8.80 mmol). The slurry was stirred for 30 minutes at 0 °C and treated with a solution of 3-pyridyl isothiocyanate (1.12 g, 8.20 mmol) in THF (8 mL). The cooling bath was removed, and the reaction mixture was stirred for 30 minutes. The reaction was quenched with water (15 mL), poured into ethyl acetate (40 mL), and partitioned. The organic phase was washed with brine (10 mL), dried (Na₂SO₄), filtered, and concentrated. The residue was dissolved in diethyl ether, treated with HCl (2M solution in diethyl ether), and the resulting precipitate was collected providing 1.06 g of the desired product as an off-white solid.
MS (APCI+) m/z 179 (M+H)⁺.

### Example 44B

### 4-methyl-N-(2,2,2-trichloro-1-{[(cyanoimino)(3-pyridinylamino)methyl]amino}ethyl)benzamide

A solution of Example 44A (200 mg, 0.932 mmol) in DMF (7 mL) at 23 °C was treated with EDCI (250 mg, 1.30 mmol) followed by diisopropyethylamine (0.180 mL, 1.03 mmol). The mixture was stirred for 30 minutes, then treated with a solution of Example 38A (262 mg, 0.932 mmol) as a solution in DMF (2 mL) along with additonal diisopropyethylamine (0.360 mL, 2.06 mmol). The solution was stirred for 10 hours, poured into ethyl acetate (25 mL), and washed with water (25 mL). The aqueous layer was extracted with ethyl acetate (20 mL), and the combined organic portions were washed with water (3x20 mL) and brine (20 mL). The organic portion was dried (Na₂SO₄), filtered, and concentrated. Purification of the residue by flash chromatography (elution with 5% ethanol/hexanes) provided 146 mg of the desired product as an off-white solid.
mp 165-166°C;
MS (ESI+) m/z 425 (M)⁺;
¹H NMR (DMSO-d₆) δ 10.15 (s, 1H), 8.70 (d, 1H, J=9 Hz), 8.52 (d; 1H, J=3 Hz), 8.48-8.43 (m, 1H), 7.72 (d, 1H, J=9 Hz), 7.70-7.66 (m, 1H), 7.50 (q, 1H, J=4 Hz), 7.45 (d, 2H, J=9 Hz), 7.35 (d, 1H, J=9 Hz), 6.90 (t, 1H, J=9 Hz), 2.38 (s, 3H);
Anal. calcd for C₁₇H₁₅N₆Cl₃O: C, 47.96; H, 3.55; N, 19.74. Found: C, 47.72; H, 3.82; N, 19.71.

### Example 45

### 4-chloro-N-(2,2,2-trichloro-1-{[(cyanoimino)(3-pyridinylamino)methyl]amino}ethyl)benzamide

### Example 45A

### 4-chloro-N-(2,2,2-trichloro-1-isothiocyanatoethyl)benzamide

A solution of Example 41B (1.91 g, 6.31 mmol) in acetone (30 mL) at ambient temperature was treated with potassium thiocyanate (1.38 g, 14.1 mmol). The mixture was stirred for 6 hours and solids were removed by filtration. The filtrate was concentrated to provide a yellow residue. The residue was treated with diethyl ether, and the suspension was sonicated and filtered. Concentration of the residue and purification by recrystallization (25% ethyl acetate/hexanes) provided 1.85 mg of the desired product as a yellow solid.
MS (APCI+) m/z 343 (M+H)⁺.

### Example 45B

### 4-chloro-N-(2,2,2-trichloro-1-(((3-pyridinylamino)carbothioyl)amino)ethyl)benzamide

A solution of Example 45A (239mg, 0.695 mmol) and 3-aminopyridine (65 mg, 0.69 mmol) in THF (4 mL) at ambient temperature was treated with triethylamine (1.66 mL, 1.18 mmol). The mixture was stirred for 3 hours, diluted with ethyl acetate (20 mL), and washed with water (2x10 mL) and brine (10 mL). The organic portion was dried (MgSO₄), filtered, and concentrated to provide an oily residue which was purified by flash chromatography (elution with 5% ethanol/hexanes) to provide 158 mg of the desired product as an off-white solid.
MS (APCI+) m/z 437 (M+H)⁺.

### Example 45C

### 4-chloro-N-(2,2,2-trichloro-1- {[(cyanoimino)(3-pyridinylamino)methyl]amino}ethyl)benzamide

A stirred solution of Example 45B (500 mg, 1.14 mmol) and EDCI (656 mg, 3.42 mmol) in CH₂Cl₂ (10 ml) was heated at reflux for 10 hours. The mixture was cooled and was diluted with ethyl acetate (20 mL). The solution was washed with aqueous NaHCO₃ solution (10 mL) and brine (10 mL), dried (MgSO₄), filtered, and concentrated. The residue was dissolved in 5% ethanol/ethyl acetate and filtered through a short plug of silica gel. Concentration provided 440 mg of a pale yellow solid that was used without further purification.

The solid prepared above was dissolved in CH₂Cl₂ (10 mL), then treated with 2,6-lutidine (0.120 mL, 1.00 mmol), 3A molecular sieves (200 mg), and cyanamide (208 mg, 4.99 mmol). This stirred suspension was treated with titanium isopropoxide (0.300 mL, 1.00 mmol), and the resulting mixture was heated at reflux for 8 hours. The mixture was cooled, diluted with CH₂Cl₂ (20 mL), and washed with water (10 mL) and brine (10 mL). The solution was dried (Na₂SO₄), filtered, and concentrated. The resulting residue was purified by flash chromatography (elution with 10% methanol/CH₂Cl₂) to provide 229 mg of the desired product as a white solid.
mp 126-128°C;
MS (ESI+) m/z 426 (M+H)⁺;
¹H NMR (DMSO-d₆) δ 10.12 (s, 1H), 8.72 (d, 1H, J=9 Hz), 8.50 (dd, 1H, J=12, 3 Hz), 7.70-7.67 (m, 1H), 7.51 (q, 1H, J=6 Hz), 7.45-7.38 (m, 1H), 7.30 (d, 1H, J=9 Hz), 6.82 (t, 1H, J=9 Hz), 2.38 (s, 3H);
Anal. calcd for C₁₇H₁₅Cl₃N₆O: C, 47.96; H, 3.55; N, 19.74. Found: C, 47.62; H, 3.25; N, 19.84.

### Example 46

### N-(1-{[anilino(cyanoimino)methyl]amino}-2,2,2-trichloroethyl)-4-methylbenzamide

### Example 46A

### 4-methyl-N-(2,2,2-trichloro-1-isothiocyanatoethyl)benzamide

Example 37D and potassium thiocyanate were processed as described in Example 45A to provide the desired product.
MS (APCI+) m/z 323 (M+H)⁺.

### Example 46B

### 4-methyl-N-(2,2,2-trichloro-1-(((3-pyridinylamino)carbothioyl)amino)ethyl)benzamide

Example 46A and 3-aminopyridine were processed as described in Example 45B to provide the desired product.
MS (APCI+) m/z 417 (M+H)⁺.

### Example 46C

### N-(1-{[anilino(cyanoimino)methyl]amino}-2,2,2-trichloroethyl)-4-methylbenzamide

Example 46B, cyanamide, and titanium isopropoxide were processed as described in Example 45C to provide the desired product.
mp 197-199°C;
MS (ESI+) m/z 424 (M)⁺;
¹H NMR (DMSO-d₆) δ 9.86 (s, 1H), 8.77-8.74 (d, 1H, J=8 Hz), 7.72-7.70 (d, 2H, J=8 Hz), 7.49-7.44 (m, 2H), 7.34-7.26 (m, 5H), 6.94-6.88 (t, 1 H, J=9 Hz), 2.36 (s, 3H); Anal. calcd for C₁₈H₁₆Cl₃N₅O: C, 50.90; H, 3.80; N, 16.49. Found: C, 50.87; H, 3.78; N, 16.51.

### Example 47

### 4-methyl-N-(2,2,2-trichloro-1-{[(cyanoimino)(2-fluoroanilino)methyl]amino }ethyl)benzamide

### Example 47A

### 4-methyl-N-(2,2,2-trichloro-1-(((2-fluoroanilino)carbothioyl)amino)ethyl)benzamide

Example 46A and 3-fluoroaniline were processed as described in Example 45B to provide the desired product.
MS (APCI+) m/z 434 (M+H)⁺.

### Example 47B

### 4-methyl-N-(2,2,2-trichloro-1-{[(cyanoimino)(2-fluoroanilino)methyl]amino}ethyl)benzamide

Example 47A, cyanamide, and titanium isopropoxide were processed as described in Example 45C to provide the desired product.
mp 220-222 °C;
MS (APCI+) m/z 442 (M+H)⁺;
¹H NMR (DMSO-d₆) δ 9.85 (s, 1H), 8.83 (d, 1H, J=8 Hz), 7.73 (d, 2H, J=8 Hz), 7.25-7.40 (m, 6H), 7.1 (br d, 1H), 6.88 (t, 1H, J=8 Hz), 2.37 (s, 3H);
Anal. calcd for C₁₈H₁₅Cl₃FN₅O: C, 48.83; H, 3.41; N, 15.81. Found: C, 48.63; H, 3.44; N, 15.77.

### Example 48

### 4-methyl-N-(2,2,2-trichloro-1-{[cyanoimino)(5-pyrimidinylamino)methyl]amino}ethyl)benzamide

### Example 48A

### 4-methyl-N-(2,2,2-trichloro-1-(((5-pyrimidinylamino)carbothioyl)amino)ethyl)benzamide

Example 46A and 5-aminopyrimidine were processed as described in Example 45B to provide the desired product.
MS (APCI+) m/z 418 (M+H)⁺.

### Example 48B

### 4-methyl-N-(2,2,2-trichloro-1-{[(cyanoimino)(5-pyrimidinylamino)methyl]amino}ethyl)benzamide

Example 48A, cyanamide, and titanium isopropoxide were processed as described in Example 45C to provide the desired product.
mp 186-188 °C;
MS (APCI+) m/z 426 (M+H)⁺;
¹H NMR (DMSO-d₆) δ 10.22 (s, 1H), 9.08 (s, 1H), 8.77-8.73 (m, 2H), 7.81-7.75 (m, 3H), 7.73-7.68 (m, 1H), 7.38 (d, 2H, J=6 Hz), 6.90 (t, 1H, J=6 Hz), 2.38 (s, 3H);
Anal. calcd for C₁₆H₁₄Cl₃N₇O: C, 45.03; H, 3.31; N, 22.98. Found: C, 44.66; H, 3.55; N, 22.66.

### Example 49

### N-(2,2,2-trichloro-1-{[(cyanoimino)(3-pyridinylamino)methyl]amino}ethyl)benzamide

### Example 49A

### N-(2,2,2-trichloro-1-isothiocyanatoethyl)benzamide

Example 40B and potassium thiocyanate were processed as described in Example 45A to provide the desired product.
MS (APCI+) m/z 308 (M+H)⁺.

### Example 49B

### N-(2,2,2-trichloro-1-(((3-pyridinylamino)carbothioyl)amino)ethyl)benzamide

Example 49A and 3-aminopyridine were processed as described in Example 45B to provide the desired product.
MS (APCI+) m/z 403 (M+H)⁺.

### Example 49C

### N-(2,2,2-trichloro-1-{[(cyanoimino)(3-pyridinylamino)methyl]amino}ethyl)benzamide

Example 498, cyanamide, and titanium isopropoxide were processed as described in Example 45C to provide the desired product.
mp 133-135 °C;
MS (ESI+) m/z 412 (M+H)⁺;
¹H NMR (DMSO-d₆) δ 10.15 (s, 1H), 8.82 (d, 1H, J=9 Hz), 8.53-8.48 (m, 2H), 7.82 (d, 2H, J=9 Hz), 7.72 (d, 1H, J=6 Hz), 7.62 (d, 1H, J=6 Hz), 7.58-7.51 (m, 3H), 7.45 (d, 1H, J=6 Hz), 6.90 (t, 1 H, J=9 Hz);
Anal. calcd for C₁₆H₁₃Cl₃N₆O: C, 46.68; H, 3.18; N, 20.41. Found: C, 46.88; H, 3.41; N, 20.43.

### Example 50

### 2-methyl-N-(2,2,2-trichloro-1-{[(cyanoimino)(3-pyridinylamino)methyl]amino}ethyl)benzamide

### Example 50A

### 2-methyl-N-(2,2,2-trichloro-1-isothiocyanatoethyl)benzamide

Example 39B and potassium thiocyanate were processed as described in Example 45A to provide the desired product.
MS (APCI+) m/z 323 (M+H)⁺.

### Example 50B

### 2-methyl-N-(2,2,2-trichloro-1-(((3-pyridinylamino)carbothioyl)amino)ethyl)benzamide

Example 50A and 3-aminopyridine were processed as described in Example 45B to provide the desired product.
MS (APCI+) m/z 417 (M+H)⁺.

### Example 50C

### 2-methyl-N-(2,2,2-trichloro-1-1{[(cyanoimino)(3-pyfidinylamino)methyl]amino}ethyl)benzamide

Example 50B, cyanamide, and titanium isopropoxide were processed as described in Example 45C to provide the desired product.
mp 126-128 °C;
MS (ESI+) m/z 426 (M+H)⁺;
¹H NMR (DMSO-d₆) δ 10.12 (s, 1H), 8.72 (d, 1H, J=9 Hz), 8.50 (dd, 2H, J=3;12 Hz), 7.69-7.65 (m, 1H),), 7.51 (q, 1H, J=6 Hz), 7.43-7.38 (m, 3H), 7.30 (d, 2H, J=9 Hz), 6.82 (t, 1 H, J=9 Hz), 2.3 8 (s, 3H);
Anal. calcd for C₁₇H₁₅Cl₃N₆O: C, 47.96; H, 3.55; N, 19.74. Found: C, 47.62; H, 3.25; N, 19.84.

### Example 51 N-(1-{[(cyanoimino)(3-pyridinylamino)methyl]amino}-2,2-dimethylpropyl)-4-methylbenzamide

Example 44A, Example 22E, and EDCI were processed as described in Example 44B to provide the desired product.
mp 187-188 °C;
MS (APCI+) m/z 365 (M+H)⁺;
¹H NMR (DMSO-d₆) δ 9.58 (s, 1H), 8.48 (d, 1H, J=3 Hz), 8.42 (d, 1H, J=3 Hz), 8.25 (d, 1H, J=9 Hz), 7.75 (d, 2H, J=9 Hz), 7.73-7.69 (m, 1H), 7.47-7.43 (m, 1H), 7.30 (d, 2H, J=9 Hz), 6.85 (d, 1H, J=9 Hz), 5.85 (t, 1H, J=9 Hz), 2.37 (s, 3H), 0.97 (s, 9H);
Anal. calcd for C₂₀H₂₄N₆O: C, 65.91; H, 6.64; N, 23.06. Found: C, 65.95; H, 6.59; N, 23.20.

### Example 52

### 4-chloro-N-(1-{[(cyanoimino)(3-pyridinylamino)methyl]amino}-2,2-dimethylpropyl)benzamide

### Example 52A

### N-(1-(aminocarbonyl)-2,2-dimethylpropyl)-4-chlorobenzamide

Example 22C and 4-chlorobenzoyl chloride were processed as described in Example 22D to provide the desired product.
MS (APCI+) m/z 269 (M+H)⁺.

### Example 52B

### N-(1-amino-2,2-dimethylpropyl)-4-chlorobenzamide hydrochloride

Example 52A and iodobenzene diacetate were processed as described in Example 22E to provide the desired product.
MS (APCI+) m/z 241 (M+H)⁺.

### Example 52C

### 4-chloro-N-(1-{[(cyanoimino)(3-pyridinylamino)methyl]amino}-2,2-dimethylpropyl)benzamide

Example 52B, Example 44A, and EDCI were processed as described in Example 44B to provide the desired product.
mp 194-195 °C;
MS (APCI+) m/z 393 (M+H)⁺;
¹H NMR (DMSO-d₆) δ 9.55 (s, 1H), 8.48 (d, 1H, J=3 Hz), 8.47-7.38 (m, 2H), 7.85 (d, 2H, J=9 Hz), 7.71-7.68 (m, 1H), 7.58 (d, 2H, J=9 Hz), 7.42 (dd, 1H, J=9, 3 Hz), 6.85 (d, 1H, J=9 Hz), 5.84 (t, 1H, J=9 Hz), 0.97 (s, 9H);
Anal. calcd for C₁₉H₂₁ClN₆O: C, 59.29; H, 5.50; N, 21.84. Found: C, 59.16; H, 5.53; N, 21.90.

### Example 53

### N-(1- {[(cyanoimino)(3-fluoroanilino)methyl]amino}-2,2-dimethylpropyl)-4-methylbenzamide

### Example 53A

### N-(1-(1 H-1,2,3-benzotriazol-1-yl)-2,2-dimethylpropyl)-4-methylbenzamide

A suspension of p-toluamide (4.11 g, 30.4 mmol), pivaldehyde (2.62 g, 30.4 mmol), and benzotriazole (3.62 g, 30.4 mmol) in toluene (200 mL) were treated with p-toluenesulfonic acid (286 mg, 1.52 mmol). The solution was heated at reflux under Dean-Stark conditions for 10 hours, cooled gradually to ambient temperature, and further cooled at 5 °C. The white precipitate which formed was collected by filtration and was washed with 50% ether/hexanes (100 mL) to provide 6.67 g of the desired product as a white solid.
MS (DCI/NH₃) m/z 323 (M+H)⁺.

### Example 53B

### N-(1-amino-2,2-dimethylpropyl)-4-methylbenzamide hydrochloride

A stirred suspension of Example 53A (13.3 g, 38.8 mmol) in methanol (50 mL) was treated with finely powdered K₂CO₃ (11.8 g, 85.4 mmol) followed by ammonia (200 mL of a 2M solution in methanol). The mixture was stirred at ambient temperature for 3.5 hours, the solid was removed by filtration, and the filtrate was concentrated. The resulting solid was suspended in diethyl ether (200 mL) and stirred for 45 minutes at ambient temperature. The mixture was again filtered, and the filtrate was concentrated to a volume of 75 mL. This solution was treated with 1N HCl (200 mL of a 1N solution in diethyl ether), and the resulting suspension was sonicated to promote salt formation. The solid was isolated by filtration and washed with ethyl acetate (2x75 mL) to provide 8.88 g of the desired product as a white solid.
MS (DCI/NH₃) m/z 220 (M+H)⁺.

### Example 53C

### N-cyano-N'-(3-fluorophenyl)thiourea

Cyanamide and 3-fluoroaniline were processed as described in Example 44A to provide the desired product.
MS (DCI/NH₃) m/z 196 (M+H)⁺.

### Example 53D

### N-(1-{[(cyanoimino)(3-fluoroanilino)methyl]amino}-2,2-dimethylropyl)-4-methylbenzamide

Example 53B, Example 53C, and EDCI were processed as described in Example 44B to provide the desired product.
mp 193-194 °C;
MS (FAB+) m/z 382 (M+H)⁺;
¹H NMR (DMSO-d₆) δ 9.57 (s, 1H); 8.26 (d, 1H, J=9 Hz); 7.72 (d, 2H, J=8 Hz); 7.41 (dd, 1H, J=8, 4 Hz); 7.29 (d, 2H, J=8 Hz); 7.11 (t, 2H, J=7 Hz); 7.01 (t, 1H, J=8 Hz); 6.85 (d, 1H, J=4); 5.82 (t, 1H, J=5 Hz); 2.36 (s, 3H); 0.98 (s, 9H);
Anal. calcd for C₂₁H₂₄FN₅O**•**0.25 H₂O: C, 65.35; H, 6.40; N, 18.15. Found: C, 65.33; H, 6.40; N, 18.20.

### Example 54

### 4-chloro-N-[{[(cyanoimino)(3-pyridinylamino)methyl]amino}(cyclopropyl)methyl]benzamide

### Example 54A

### N-( 1H-1,2,3-benzotriazol-1-yl(cyclopropyl)methyl)-4-chlorobenzamide

Benzotriazole, 4-chlorobenzamide, cyclopropanecarboxaldhehyde, and p-toluenesulfonic acid were processed as described in Example 53A to provide the desired product.
MS (ESI+) m/z 327 (M+H)⁺.

### Example 54B

### methyl N'-cyano-N-(3-pyridinyl)carbamimidothioate

A suspension of 3-aminopyridine (5.16 g, 54.8 mmol) and dimethyl N-cyanodithioiminocarbonate (8.02 g, 54.8 mmol) in acetonitrile (200 mL) was heated at reflux for 6 days. The solution was cooled and concentrated to a volume of 75 mL where upon a white solid precipitated from solution. The solid was collected by filtration and washed with 50% ether/hexanes (500 mL) to provide 7.79 g of the desired product as a pale yellow solid.
MS (ESI+) m/z 193 (M+H)⁺.

### Example 54C

### N"-cyano-N-(3-pyridinyl)guanidine

Example 54B (510 mg, 2.44 mmol) was dissolved in a 2M solution of ammonia in methanol (7 mL) and heated in a sealed tube at 80 °C for 12 hours. The reaction mixture was cooled to ambient temperature and further cooled to -5 °C where upon a white solid precipitated from solution. The solid was filtered and the filter cake washed with cold ethanol to provide 310 mg of the desired product as a white solid.
MS (ESI+) m/z 162 (M+H)⁺.

### Example 54D

### 4-chloro-N-[{[(cyanoimino)(3-pyridinylamino)methyl]aminol}(cycloproyl)methyl]benzamide

A solution of Example 54C (50 mg, 0.27 mmol) and Example 54A (55 mg, 0.18 mmol) in DMF (2 mL) at 23 °C was treated with finely powdered K₂CO₃ (62 g, 0.45 mmol). The reaction mixture was stirred for 4 hours, then partitioned between ethyl acetate (15 mL) and water (10 mL). The aqueous layer was extracted with ethyl acetate (10 mL), and the combined organics were washed with water (2x5 mL) and brine (5 mL). The organic portions were dried (Na₂SO₄), filtered, and concentrated. Purification by flash chromatography (elution with 5% methanol/CH₂Cl₂) provided 21 mg of the desired product as a white solid.
mp 137-139 °C;
MS (ESI+) m/z 369 (M+H)⁺;
¹H NMR (DMSO-d₆) δ 9.57 (s, 1H), 9.16 (d, 1H, J=7 Hz), 8.47 (d, 1H, J=3 Hz), 8.31 (dd, 1H, J=4, 1Hz), 7.92 (d, 2H, J=8 Hz), 7.79-7.73 (m, 1H), 7.69 (d, 1H, J=8 Hz), 7.59 (d, 2H, J=8 Hz), 7.37 (dd, 1H, J=8, 5 Hz), 5.16 (dd, 1H, J=16, 8 Hz), 1.56-1.50 (m, 1H), 0.57-0.50 (m, 2H), 0.44-0.38 (m, 1H);
Anal. calcd for C₁₈H₁₇ClN₆O: C, 58.62; H, 4.65; N, 22.79. Found: C, 58.41; H, 4.88; N, 22.44.

### Example 55

### N-(1-{[[(6-chloro-3-pyridinyl)amino](cyanoimino)methyl]amino}-2,2-dimethylpropyl)-4-methylbenzamide

### Example 55A

### 2-chloro-5-isothiocyanatopyridine

A solution of thiophosgene (3.21 mL, 42.1 mmol) in chloroform (10 mL) was heated at reflux and treated with a solution of 5-amino-2-chloropyridine (3.61 g, 28.1 mmol) in chloroform (25 mL) for 40 minutes. The solution was heated an additional 2 hours, the reaction mixture was cooled, and the solids formed were removed by filtration. Concentration of the filtrate provided 2.73 g of the desired product as a white solid.
MS (DCI/NH₃) m/z 188 (M+NH₄)⁺.

### Example 55B

### methyl N-(6-chloro-3-pyridinyl)-N'-cyanocarbamimidothioate

A solution of cyanamide (705 mg, 16.8 mmol) in **THF** (40 mL) at 0 °C was treated with sodium hydride (422 mg of 95% reagent, 17.6 mmol). The slurry was stirred for 30 minutes at 0 °C, then treated with Example 55A (2.72 g, 16.0 mmol) as a solution in THF (20 mL) for 10 minutes. The cooling bath was removed, the reaction mixture was stirred for 30 minutes, and treated with methyl iodide (2.00 mL, 32.0 mmol). The reaction mixture was stirred for 15 minutes, quenched with water (50 mL), poured into ethyl acetate (150 mL), and partitioned. The organic phase was washed with brine (20 mL), dried (Na₂SO₄), filtered, and concentrated. The resulting precipitate was collected providing 3.24 g of the desired product as an off-white solid.
MS (DCI/NH₃) m/z 227 (M+H)⁺.

### Example 55C

### N-(6-chloro-3-pyridinyl)-N"-cyanoguanidine

Example 55B (2.08 mg, 9.18 mmol) was dissolved in a 2M solution of ammonia in methanol (45 mL) and heated in a sealed Pyrex vessel at 80 °C for 6 hours. The reaction mixture was cooled to ambient temperature and concentrated to provide an off-white solid. Recrystallization of this material from hot ethanol provided 1.51 g of the desired product as a white solid.
MS (DCI/NH₃) m/z 213 (M+NH₄)⁺.

### Example 55D

### N-(1-{[[(6-chloro-3-pyridinyl)amino](cyanoimino)methyl]amino}-2,2-dimethylpropyl)-4-methylbenzamide

Example 55C and Example 53A were processed as described in Example 54D to provide the desired product.
mp 194-196°C;
MS (DCI/NH₃) m/z 399 (M+H)⁺;
¹H NMR (DMSO-d₆) δ 9.63 (s, 1H), 8.33 (d, 1H, J=2 Hz), 8.23 (br d, 1H, J=5 Hz), 7.79-7.61 (m, 2H), 7.75 (d, 1H, J=7Hz), 7.56 (d, 1H, J=8 Hz), 7.30 (d, 1H, J=7 Hz), 6.98 (br d, 1H, J=6 Hz), 5.83 (t, 1H, J=6 Hz), 2.37 (s, 3H), 0.97 (s, 9H);
Anal. calcd for C₂₀H₂₃ClN₆O: C, 60.22; H, 5.81; N, 21.07. Found: C, 60.82; H, 5.95; N, 20.74.

### Example 56

### 4-chloro-N-[{[(cyanoimino)(3-fluoroanilino)methyl]amino}(3-thienyl)methyl]benzamide

### Example 56A

### N-(1H-1,2,3-benzotriazol-1-yl-(3-thienyl)methyl)-4-chlorobenzamide

Benzotriazole, 4-chlorobenzamide, 3-thiophenecarboxaldhehyde, and p-toluenesulfonic acid were processed as described in Example 53A to provide the desired product.
MS (DCI/NH₃) m/z 369 (M+H)⁺.

### Example 56B

### 1-fluoro-3-isothiocyanatobenzene

A solution of thiophosgene and 3-fluoroaniline were processed as described in Example 55A to provide the desired product.
MS (DCI/NH₃) m/z 154 (M+H)⁺;

### Example 56C

### methyl N'-cyano-N-(3 -fluorophenyl)carbamimidothioate

Example 56B, cyanamide, and methyl iodide were processed as described in Example 55B to provide the desired product.
MS (DCI/NH₃) m/z 210 (M+H)⁺.

### Example 56D

### N"-cyano-N-(3-fluorophenyl)guanidine

Example 56C and ammonia were processed as described in Example 54C to provide the desired product.
MS (DCI/NH₃) m/z 196 (M+NH₄)⁺.

### Example 56E

### 4-chloro-N-[{[4[(cyanoimino)(3-fluoroanilino)methyl]amino}(3-thienyl)methyl]benzamide

Example 56A and Example 56D were processed as described in Example 54D to provide the desired product.
mp 166-168 °C;
MS (ESI+) m/z 428 (M+H)⁺;
¹H NMR (DMSO-d₆) δ 9.65 (s, 1H), 9.24 (d, 1H, J=8 Hz), 7.92 (d, 2H, J=7 Hz), 7.82-7.76 (m, 1H), 7.59 (d, 2H, J=7 Hz), 7.59-7.57 (m, 2H), 7.40-7.34 (m, 1H), 7.20-7.18 (m, 1H), 7.15-7.06 (m, 2H), 6.99-6.94 (m, 1H), 6.91 (dd, 1 H, J=8, 7 Hz);
Anal. calcd for C₂₀H₁₅ClFN₅O**·**0.75 H₂O: C, 54.42; H, 3.77; N, 15.87. Found: C, 54.41; H, 3.83; N, 15.86.

### Example 57

### (-)-N-(1-{[(cyanoimino)(3-pyridinylamino)methyl]amino}-2,2-dimethylpropyl)-4-methylbenzamide

Example 51 (161 mg) was chromatographed over a Daicel Chiral Technologies Chiralcel OD chiral column (2.0 cmx25 cm) eluting with 5% ethanol/hexanes (flow rate=10 mL/minutes) to provide 51 mg (retention time=15 minutes) of the desired product as the faster enantiomer.
mp 187-188 °C;
[α]D²³ -38° (c 0.4, DMSO);
MS (APCI+) m/z 365 (M+H)⁺;
¹H NMR (DMSO-d₆) δ 9.58 (s, 1H), 8.48 (d, 1H, J=3 Hz), 8.42 (d, 1H, J=3 Hz), 8.25 (d, 1H, J=9 Hz), 7.75 (d, 2H, J=9 Hz), 7.73-7.69 (m, 1H), 7.47-7.43 (m, 1H), 7.30 (d, 2H, J=9 Hz), 6.85 (d, 1H, J=9 Hz), 5.85 (t, 1H, J=9 Hz), 2.37 (s, 3H), 0.97 (s, 9H);
Anal calcd for C₂₀H₂₄N₆O: C, 65.91; H, 6.64; N, 23.06. Found: C, 66.00; H, 6.63; N, 23.15.

### Example 58

### (+)-N-(1-{[(cyanoimino)(3-pyridinylaminolmethyl]amino}-2,2-dimethylpropyl)-4-methylbenzamide

Example 51 (161 mg) was chromatographed over a Daicel Chiral Technologies Chiralcel OD chiral column (2.0 cmx25 cm) eluting with 5% ethanol/hexanes (flow rate=10 mL/minutes) to provide 36 mg (retention time=25 minutes) of the desired product as the slower enantiomer.
mp 188-189 °C;
[a]_{D}²³ +51° (c 0.3, DMSO);
MS (APCI+) m/z 365 (M+H)⁺;
¹H NMR (DMSO-d₆) 8 9.58 (s, 1H), 8.48 (d, 1H, J=3 Hz), 8.42 (d, 1H, J=3 Hz), 8.25 (d, 1H, J=9 Hz), 7.75 (d, 2H, J=9 Hz), 7.73-7.69 (m, 1H), 7.47-7.43 (m, 1H), 7.30 (d, 2H, J=9 Hz), 6.85 (d, 1H, J=9 Hz), 5.85 (t, 1H, J=9 Hz), 2.37 (s, 3H), 0.97 (s, 9H);
Anal calcd for C₂₀H₂₄N₆O: C, 65.91; H, 6.64; N, 23.06. Found: C, 65.99; H, 6.60; N, 23.20.

### Example 59

### 4-chloro-N-(1-{[(cyanoimino)(3-pyridinylamino)methyl]amino}-2-ethylbutyl)benzamide

### Example 59A

### N-[1-(1H-1,2,3-benzotriazol-1-yl)-2-ethylbutyl]-4-chlorobenzamide

Benzotriazole, 4-chlorobenzamide, 2-ethylbutanal, and p-toluenesulfonic acid were processed as in Example 53A to provide the desired product.
MS (ESI+) m/z 357 (M+H)⁺.

### Example 59B

### 4-chloro-N-(1-{[(cyanoimino)(3-pyridinylamino)methyl]amino}-2-ethylbutyl)benzamide

Example 54C and Example 59A were processed as in Example 54D to provide the desired product.
mp 185-187 °C;
MS (ESI+) m/z 399 (M+H)⁺;
¹H NMR (DMSO-d₆) δ 9.68 (s, 1H), 8.88 (br s, 1H), 8.47 (d, 1H, J=2Hz), 8.33 (dd, 1H, J=5, 1Hz), 7.90 (d, 2H, J=8 Hz), 7.69 (d, 1H, J=8 Hz), 7.45 (br s, 1H), 7.38 (dd, 1H, J=9, 5 Hz), 5.57 (dd, 1H, J=17, 9 Hz), 1.90 (m, 1H), 1.54-1.32 (m, 4H), 0.87 (t, 3H, J=8 Hz), 0.84 (t, 4H, J=8 Hz;
Anal calcd for C₂₀H₂₃ClN₆O·0.15 C₄H₈O₂: C, 60.04; H, 5.92; N, 20.39; Cl, 8.61. Found: C, 59.75; H, 5.77; N, 20.22; Cl, 8.93.

### Example 60

### 4-chloro-N-(1-{[(cyanoimino)(3-pyridinylamino)methyl]amino}-3-methylbutyl)benzamide

### Example 60A

### N-[1-(1H-1,2,3-benzotriazol-1-yl)-3-methylbuty]-4-chlorobenzamide

Benzotriazole, 4-chlorobenzamide, 3-methylbutanal, and p-toluenesulfonic acid were processed as in Example 53A to provide the desired product.
MS (ESI+) m/z 343 (M+H)⁺.

### Example 60B

### 4-chloro-N-(1-{[(cyanoimino)(3-pyridinylamino)methyl]amino}-3-methylbutyl)benzamide

Example 54C and Example 60A were processed as in Example 54D to provide the desired product.
mp 193-194°C;
MS (ESI+) m/z 385 (M+H)⁺;
¹H NMR (DMSO-d₆) δ 9.77 (br s, 1H), 9.00 (d, 1H, J=6 Hz), 8.49 (s, 1H), 8.31 (d, 1H, J=5 Hz), 7.92 (d, 2H, J=8 Hz), 7.71 (d, 1H, J=8 Hz), 7.64 (d, 1H, J=7 Hz), 7.57 (d, 2H, J=8 Hz), 7.36 (dd, 1H, J=8, 5 Hz), 5.69 (m, 1H), 3.32 (m, 2H), 1.68 (m, 1H), 0.92 (t, 6H, J=6 Hz);
Anal calcd for C₁₉H₂₁ClN₆O: C, 59.30; H, 5.50; N, 9.21; Cl, 21.84. Found: C, 59.16; H, 5.50; N, 9.08; Cl, 21.50.

### Example 61

### 4-chloro-N-[{[cyanoimino)(3-pyridinylamino)methyl]amino}(cyclohexyl)methyl]benzamide

### Example 61 A

### N-[ 1H-1,2,3-benzotriazol-1-yl(cyclohexyl)methyl]-4-chlorobenzamide

Benzotriazole, 4-chlorobenzamide, cyclohexanecarboxaldehyde, and p-toluenesulfonic acid were processed as in Example 53A to provide the desired product. MS (ESI+) m/z 369 (M+H)⁺.

### Example 61 B

### 4-chloro-N-[{[(cyanoimino)(3-pyridinylamino)methyl]amino}(cyclohexyl)methyl]benzamide

Example 54C and Example 61A were processed as in Example 54D to provide the desired product.
mp 190-192 °C;
MS (ESI+) m/z 411 (M+H)⁺;
¹H NMR (DMSO-d₆) δ 9.69 (s, 1H), 8.92 (br s, 1H), 8.49 (s, 1H), 8.31 (d, 1H, J=4 Hz), 7.91 (d, 2H, J=8 Hz), 7.71 (d, 1H, J=7 Hz), 7.57 (d, 2H, J=8 Hz), 7.37 (dd, 1H, J=5, 7.62 Hz), 5.43 (m, 1H), 1.97-1.62 (m, 5H), 1.16-0.97 (m, 6H);
Anal calcd for C₂₁H₂₃ClN₆O·0.25 C₄H₆O₂: C, 61.64; H, 5.82; N, 19.41; Cl, 8.19. Found: C, 61.33; H, 6.01; N, 19.17; Cl, 8.14.

### Example 62

### 4-chloro-N-(1-{[(cyanoimino)(3-pyridinylamino)methyl]amino)-3,3-dimethylbutyl)benzamide

### Example 62A

### N-[1-(1H-1,2,3-benzotriazol-1-yl)-3,3-dimethylbutyl]-4-chlorobenzamide

Benzotriazole, 4-chlorobenzamide, 3,3-dimethylbutanal, and p-toluenesulfonic acid were processed as in Example 53A to provide the desired product.
MS (ESI+) m/z 357 (M+H)⁺.

### Example 62B

### 4-chloro-N-(1-{[(cyanoimino)(3-pyridinylamino)methyl]amino}-3,3-dimethylbutyl)benzamide

Example 54C and Example 62A were processed as in Example 54D to provide the desired product.
mp 181-183 °C;
MS (ESI+) m/z 399 (M+H)⁺;
¹H NMR (DMSO-d₆) δ 9.69 (s, 1H), 9.00 (d, 1H, J=5 Hz), 5.01 (d, 1H, J=3 Hz), 8.33 (dd, 1H, J=5,2 Hz), 7.92 (d, 2H, J=9 Hz), 7.70 (d, 1H, J=8 Hz), 7.58 (d, 2H, J=8 Hz), 7.56 (m, 1H), 7.38 (dd, 1H, J=8, 5 Hz), 5.73 (m, 1H), 1.87 (m, 2H), 0.96 (s, 9H);
Anal calcd for C₂₀H₂₃ClN₆O: C, 60.22; H, 5.81; N, 21.07; Cl, 8.89. Found: C, 60.04; H, 6.01; N, 20.75; Cl, 8.74.

### Example 63

### 4-chloro-N-(1-{[(cyanoimino)(3-pyridinylamino)methyl]amino}-2-methylpropyl)benzamide

### Example 63A

### N-[1-(1H-1,2,3-benzotriazol-1-yl)-2-methylpropyl]-4-chlorobenzamide

Benzotriazole, 4-chlorobenzamide, isobutyraldehyde, and p-toluenesulfonic acid were processed as in Example 53A to provide the desired product.
MS (ESI+) m/z 329 (M+H)⁺.

### Example 63B

### 4-chloro-N-( 1-{ [(cyanoimino)(3-pyridinylamino)methyl]amino}-2-methylpropyl)benzamide

Example 54C and Example 63A were processed as in Example 54D to provide the desired product.
mp 182-184 °C;
MS (ESI+) m/z 371 (M+H)⁺;
¹H NMR (DMSO-d₆) δ 9.72 (s, 1H), 8.25 (d, 1H, J=6 Hz), 8.50 (d, 1H, J=2 Hz), 8.33 (d, 1H, J=4 Hz), 7.92 (d, 2H, J=9 Hz), 7.72 (d, 1H, J=9 Hz), 7.60 (d, 2H, J=8 Hz), 7.53 (m, 1H), 7.38 (dd, 1H, J=8, 5Hz), 5.42 (q, 1H, J=8 Hz), 2.22 (m, 1H), 1.00 (d, 3H, J=7 Hz), 0.97 (d, 3H, J=7 Hz);
Anal calcd for C₁₈H₁₉ClN₆O: C, 58.30; H, 5.16; N, 22.66. Found: C, 58.37; H, 5.02; N, 22.76.

### Example 64

### 4-chloro-N-(1-{[(cyanoimino)(3-pyridinylamino)methyl]amino}-2,2,2-trifluoroethyl)benzamide

### Example 64A

### 4-chloro-N-(2,2,2-trifluoro-1-hydroxyethyl)benzamide

Trifluoroacetaldehyde ethyl hemiacetal and 4-chlorobenzamide were processed as in Example 1A to provide the desired product.
MS (ESI+) m/z 235 (M-H₂O)⁺.

### Example 64B

### N-(1-chloro-2,2,2-trifluoroethyl)-4-chlorobenzamide

Example 64A and thionyl chloride were processed as in Example 1B to provide the desired product.
MS (ESI+) m/z 272 (M+H)⁺.

### Example 64C

### 4-chloro-N-(1-{[(cyanoimino)(3pyridinylamino)methyl]amino}-2,2,2-trifluoroethyl)benzamide

Example 54C and Example 64B were processed as in Example 54D to provide the desired product.
mp 136-138 °C;
MS (ESI+) m/z 397 (M+H)⁺;
¹H NMR (DMSO-d₆) δ 9.98 (s, 1H), 9.12 (d, 1H, J=8 Hz), 8.48 (m, 2H), 7.86 (d, 2H, J=8 Hz), 7.65 (m, 1H), 7.63 (d, 2H, J=8 Hz), 7.51 (m, 1H), 7.46 (m, 1H), 6.67 (m, 1H);
Anal calcd for C₁₆H₁₂ClF₃N₆O·0.6 CHCl₃: C, 48.44; H, 3.05; N, 21.18. Found: C, 24.27; H, 3.00; N, 18.08.

### Example 65

### 4-chloro-N-(4-cyano-1-{[(cyanoimino)(3-pyridinylamino)methyl]aminol-2.2-diethylbutyl)benzamide

### Example 65A

### N-[1-(1H-1,2,3-benzotriazol-1-yl)-4-cyano-2,2-diethylbutyl]-4-chlorobenzamide

Benzotriazole, 4-chlorobenzamide, 4-cyano-2,2-diethylbutanal, and p-toluenesulfonic acid were processed as in Example 53A to provide the desired product. MS (ESI+) m/z 410 (M+H)⁺.

### Example 65B

### 4-chloro-N-(4-cyano-1-{[(cyanoimino)(3-pyridinylamino)methyl]amino}-2,2-diethylbutyl)benzamide

Example 54C and Example 65A were processed as in Example 54D to provide the desired product.
mp 187-188 °C;
MS (ESI+) m/z 452 (M+H)⁺;
¹H NMR (DMSO-d₆) δ 9.54 (s, 1H), 8.45 (d, 1H, J=2 Hz), 8.40 (d, 1H, J=5 Hz), 8.33 (d, 1H, J=8 Hz), 7.83 (d, 2H, J=8 Hz), 7.65 (d, 1H, J=8 Hz), 7.60 (d, 2H, J=9 Hz), 7.43 (dd, 1H, J=8, 5 Hz), 6.72 (d, 1H, J=9 Hz), 5.94 (t, 1H, J=9 Hz), 2.54 (m, 2H), 1.72 (t, 2H, J=8 Hz), 1.39 (m, 4H), 0.85 (t, 3H, J=7 Hz), 0.84 (t, 3H, J=7 Hz);
Anal calcd for C₂₃H₂₆ClN₇O·0.3 H₂O: C, 61.12; H, 5.80; N, 21.69; Cl, 7.84. Found: C, 60.41; H, 5.77; N, 21.45; Cl, 7.58.

### Example 66

### 4-chloro-N-[1-{[(cyanoimino)(3-pyridinylamino)methyl]amino}-2-(2,6,6-trimethyl-1-cyclohexen-1-yl)ethyl]benzamide

### Example 66A

### N-[1-(1H-1,2,3-benzotriazol-1-yl)-2-(2,6,6-trimethyl-1-cyclohexen-1-yl)ethyl]-4-chlorobenzamide

Benzotriazole, 4-chlorobenzamide, (2,6,6-trimethyl-1-cyclohexenyl)acetaldehyde, and p-toluenesulfonic acid were processed as in Example 53A to provide the desired product.
MS (ESI+) m/z 423 (M+H)⁺.

### Example 66B

### 4-chloro-N-[1-{[(cyanoimino)(3-pyridinylamino)methyl]amino}-2-(2,6,6-trimethyl-1-cyclohexen-1-yl)ethyl]benzamide

Example 54C and Example 66A were processed as in Example 54D to provide the desired product.
mp 199-201 °C;
MS (ESI+) m/z 465 (M+H)⁺;
¹H NMR (DMSO-d₆) δ 9.65 (br s, 1H), 8.80 (m, 1H), 8.46 (d, 1H, J=2 Hz), 8.37 (d, 1H, J=5 Hz), 7.86 (d, 2H, J=9 Hz), 7.66 (dt, 1H, J=8, 2 Hz), 7.60 (d, 2H, J=8 Hz), 7.41 (dd, 1H, J=8, 5Hz), 7.12 (m, 1H), 5.85 (m, 1H), 2.60 (m, 2H), 1.87 (m, 2H), 1.63 (s, 3H), 1.53 (m, 2H), 1.38 (m, 2H), 1.02 (d, 3H, J=7 Hz);
Anal calcd for C₂₅H₂₉CIN₆O: C, 64.58; H, 6.29; N, 18.07. Found: C, 64.18; H, 6.14; N, 18.04.

### Example 67

### 4-chloro-N-(1-{[(cyanoimino)(3-pyridinylamino)methyl]amino}-2,2-dimethyl-4-pentenyl)benzamide

### Example 67A

### N-[1-(1H-1,2,3-benzotriazol-1-yl)-2,2-dimethyl-4-pentenyl]-4-chlorobenzamide

Benzotriazole, 4-chlorobenzamide, 2,2-dimethyl-4-pentenal, and p-toluenesulfonic acid were processed as in Example 53A to provide Example 67A.
MS (ESI+) m/z 369 (M+H)⁺.

### Example 67B

### 4-chloro-N-(1-{[(cyanoimino)(3-pyridinylamino)methyl]amino}1-2,2-dimethyl-4-pentenyl)benzamide

Example 54C and Example 67A were processed as in Example 54D to provide the desired product.
mp 174-175 °C;
MS (ESI+) m/z 411 (M+H)⁺;
¹H NMR (DMSO-d₆) δ 9.54 (s, 1H), 8.47 (d, 1H, J=3 Hz), 8.39 (m, 2H), 7.84 (d, 2H, J=9 Hz), 7.68 (m, 1H), 7.57 (d, 2H, J=8 Hz), 7.43 (dd, 1H, J=8, 5 Hz), 6.84 (d, 1H, J=9 Hz), 5.91 (m, 1H), 5.84 (t, 1H, J=9 Hz), 5.05 (d, 2H, J=5 Hz), 2.09 (m, 2H), 0.93 (s, 6H); Anal calcd for C₂₁H₂₃ClN₆O·0.25 H₂O: C, 61.38; H, 5.64; N, 20.45. Found: C, 60.49; H, 5.43; N, 20.39.

### Example 68

### 4-chloro-N-(2-ethyl-1-{[2-nitro-1-(3-pyridinylamino)ethenyl]amino}butyl)benzamide

### Example 68A

### N-[1-(methylsulfanyl)-2-nitroethenyl]-3-pyridinamine

3-Aminopyridine and 1,1-bis(methylthio)-2-nitroethylene were processed as in Example 54B to provide the desired product.
MS (ESI+) m/z 212 (M+H)⁺.

### Example 68B

### 2-nitro-N-(3-pyridinyl)-1,1-ethenediamine

Example 68A and ammonia were processed as in Example 54C to provide the desired product.
MS (ESI+) m/z 181 (M+H)⁺.

### Example 68C

### 4-chloro-N-(2-ethyl-1-{[2-nitro-1-(3-pyridinylamino)ethenyl]amino}butyl)benzamide

Example 68B and 59A were processed as in Example 54D to provide the desired product.
mp 192-193 °C;
MS (ESI+) m/z 418 (M+H)⁺;
¹H NMR (DMSO-d₆) δ 9.83 (m, 1H), 9.32 (d, 1H, J=7 Hz), 8.50 (d, 1H, J=5 Hz), 8.47 (d, 1H, J=2 Hz), 7.94 (d, 2H, J=8 Hz), 7.71 (d, 1H, J=8 Hz), 7.62 (d, 2H, J=8 Hz), 7.49 (dd, 1H, J=8, 5 Hz), 6.20 (s, 1H), 5.64 (q, 1H, J=8 Hz), 1.96 (m, 1H), 1.63 (m, 1H), 1.49 (m, 3H), 0.93 (t, 3H, J=7 Hz), 0.90 (t, 3H, J=8 Hz);
Anal calcd for C₂₀H₂₄ClN₅O₃: C, 57.48; H, 5.79; N, 16.76. Found: C, 57.39; H, 5.69; N, 16.82.

### Example 69

### 4-chloro-N-(1-{[(cyanoimino)(2-fluoroanilino)methyl]amino}-2,2-dimethylpropyl)benzamide

### Example 69A

### N-cyano-N'-(2-fluorophenyl)thiourea

Cyanamide and 2-fluorophenyl isothiocyanate were processed as described in Example 44A to provide the desired product which was used without further purification.

### Example 69B

### N-(1-(1H-1,2,3-benzotriazol-1-yl)-2,2-dimethylpropyl)-4-chlorobenzamide

A suspension of 4-chlorobenzamide, pivaldehyde, benzotriazole, and p-toluenesulfonic acid were processed as described in Example 53A to provide the desired product.
MS (DCI/NH₃) m/z 343 (M+H)⁺.

### Example 69C

### N-(1-amino-2,2-dimethylpropyl)-4-chlorobenzamide hydrochloride

A suspension of Example 69B , K₂CO₃, and ammonia were processed as described in Example 53B to provide the desired compound.
MS (DCI/NH₃) m/z 241 (M+H -HCl)⁺.

### Example 69D

### 4-chloro-N-(1-{[(cyanoimino)(2-fluoroanilino)methyl]amino}-2,2-dimethylpropyl)benzamide

Example 69A, Example 69C, and EDCI were processed as described in Example 44B to provide the desired product.
mp 208-209 °C;
MS (DCl/NH₃) m/z 402 (M+H)⁺;
¹H NMR (DMSO-d₆) δ 9.37 (s, 1H), 8.47 (d, 1H, J=8.6 Hz), 7.93 (d, 2H, J=8.6 Hz), 7.68 (d, 2H, J=8.5 Hz), 7.45 (m, 3H), 7.35 (m, 1H), 6.69 (d, 1H, J=8.6 Hz), 5.93 (t, 1H, J=8.5 Hz), 1.05 (s, 9H);
Anal. calcd for C₂₀H₂₁ClFN₅O 0.2C₇H₇FN₄: C, 59.07; H, 5.19; N, 18.67. Found: C, 59.21; H, 4.91; N, 18.58.

### Example 70

### 4-chloro-N-(1-{[(cyanoimino)(3-fluoroanilino)methyl]amino}-2.2-dimethylpropyl)benzamide

Example 56D and Example 69B were processed as described in Example 54D to provide the desired product.
mp 167-170 °C;
MS (ESI+) m/z 402 (M+H)⁺;
¹H NMR (DMSO-d₆) δ 9.53 (br s, 1H), 8.35 (d, 1H, J=8.5 Hz), 7.80 (d, 2H, J=8.6 Hz), 7.53 (d, 2H, J=8.6 Hz), 7.42-7.33 (m, 1H), 7.11-6.93 (m, 3H), 6.84 (br d, 1H, J=9.2 Hz), 5.78 (t, 1H, J =8.8 Hz), 0.95 (s, 9H);
HRMS (FAB) calcd m/z for C₂₀H₂₁ClFN₅O (M⁺): 401.1419. Found 401.1429.

### Example 71

### 4-chloro-N-[1-({(cyanoimino)[3-(trifluoromethyl)anilino]methyl}amino)-2,2-dimethylpropyl]benzamide

### Example 71 A

### N"-cyano-N-[3-(trifluoromethyl)phenyl]guanidine

3-Trifluoromethylaniline (10 g, 62.1 mmol) was dissolved in 6N HCl (10.35 mmol, 62.1 mmol) and 50 ml of water. Sodium dicyanamide (5.53 g, 62.1 mmol) was added and the mixture was stirred for 12 hours at ambient temperature. The mixture was then cooled to 0 °C and stirred for 1 hour resulting in the formation of a precipitate. Filtration provided 12.22 g of the desired product as a white solid. MS (ESI-) m/z 227 (M-H)⁻.

### Example 71 B

### 4-chloro-N-[1-({(cyanoimino)[3-(trifluoromethyl)anilino]methyl}amino)-2,2-dimethylpropyl]benzamide

Example 71 A and Example 69B were processed as described in Example 54D to provide the desired product.
mp 184-186 °C;
MS (ESI+) m/z 452 (M+H)⁺;
¹H NMR (DMSO-d₆) δ 9.65 (s, 1H), 8.39 (d, 1H, J=8.8 Hz), 7.84 (d, 2H, J=8.8 Hz), 7.59 (m, 4H), 7.57 (d, 2H, J=8.8 Hz), 6.96 (d, 1H, J=9.8 Hz), 5.83 (t, 1H, J=8.8 Hz), 0.99 (s, 3H);
Anal. calcd for C₂₁H₂₁ClF₃N₅O: C, 55.82; H, 4.68; N, 15.50. Found: C, 55.85; H, 4.77; N, 15.40.

### Example 72

### 4-chloro-N-(1-{[(cyanoimino)(3,5-difluoroanilino)methyl]amino}-2,2-dimethylpropyl)benzamide

### Example 72A

### N"-cyano-N-(3,5-difluorophenyl)guanidine

3,5-Difluoroaniline and sodium dicyanamide were procesed as described in Example 71 A to provide the desired compound.
MS (ESI-) m/z 195 (M-H)⁻.

### Example 72B

### 4-chloro-N-(1-{[(cyanoimino)(3,5-difluoroanilino)methyl}amino}-2,2-dimethylpropyl)benzamide

Example 72A and Example 69B were processed as described in Example 54D to provide the desired product.
mp 196-198 °C;
MS (ESI+) m/z 420 (M+H)⁺;
¹H NMR (DMSO-d₆) δ 9.67 (s, 1H), 8.41 (d, 1H, J=9.2 Hz), 7.85 (d, 2H, J=8.5 Hz), 7.57 (d, 2H, J=8.5 Hz), 7.12 (d, 1H, J=9.5 Hz), 6.98 (m, 3H),5.78 (t, 1H, J=9.3 Hz), 1.00 (s, 3H);
Anal. calcd for C₂₀H₂₀ClF₂N₅O: C, 57.21; H, 4.80; N, 16.68. Found: C, 56.98; H, 4.78; N, 16.78.

### Example 73

### 4-chloro-N-(1-{[(cyanoimino)(2,5-difluoroanilino)methyl]amino}-2,2-dimethylpropyl)benzamide

### Example 73A

### N"-cyano-N-(2,5-difluorophenyl)guanidine

2,5-Difluoroaniline and sodium dicyanamide were procesed as described in Example 71 A to provide the desired compound.
MS (ESI-) m/z 195 (M-H)⁻.

### Example 73B

### 4-chloro-N-(1-{[(cyanoimino)(2,5-difluoroanilino)methyl]amino}-2,2-dimethylpropyl)benzamide

Example 73A and Example 69B were processed as described in Example 54D to provide the desired product.
mp 196-198 °C;
MS (ESI+) m/z 420 (M+H)⁺;
¹H NMR (DMSO-d₆) δ 9.67 (s, 1H), 8.41 (d, 1H, J=9.2 Hz), 7.85 (d, 2H, J=8.5 Hz), 7.57 (d, 2H, J=8.5 Hz), 7.12 (d, 1H, J=9.5 Hz), 6.98 (m, 3H), 5.78 (t, 1H, J=9.3 Hz), 1.00 (s, 3H);
Anal. calcd for C₂₀H₂₀ClF₂N₅O: C, 57.21; H, 4.80; N, 16.68. Found: C, 56.98; H, 4.78; N, 16.78.

### Example 74

### 4-chloro-N-(1-{[(cyanoiminol(2,6-difluoroanilino)methyl]amino)-2,2-dimethylpropyl)benzamide

### Example 74A

### N"-cyano-N-(2,6-difluorophenyl)guanidine

2,6-Difluoroaniline and sodium dicyanamide were procesed as described in Example 71 A to provide the desired compound.
MS (ESI-) m/z 195 (M-H)⁻.

### Example 74B

### 4-chloro-N-(1-{[(cyanoimino)(2,6-difluoroanilino)methyl]amino}-2,2-dimethylpropyl)benzamide

Example 74A and Example 69B were processed as described in Example 54D to provide the desired product.
mp 211-213 °C;
MS (ESI+) m/z 420 (M+H)⁺;
¹H NMR (DMSO-d₆) δ 9.23 (s, 1H), 8.40 (d, 1H, J=8.5 Hz), 7.84 (d, 2H, J=8.5 Hz), 7.58 (d, 2H, J=8.5 Hz), 7.43 (m, 1H), 7.21 (dd, 2H, J=8.5, 8.0 Hz), 6.89 (m, 1H), 5.80 (t, 1H, J=8.9 Hz), 0.95 (s, 3H);
Anal. calcd for C₂₀H₂₀ClF₂N₅O: C, 57.20; H, 4.80; N, 16.68. Found: C, 57.05; H, 4.68; N, 16.55.

### Example 75

### 4-chloro-N-(1-{[(cyanoimino)3-chloroanilino)methyl)amino}2,2-dimethylpropyl)benzamide

### Example 75A

### N"-cyano-N-(3-chlorophenyl)guanidine

3-Chloroaniline and sodium dicyanamide were processed as described in Example 71 A to provide the desired compound.
MS (ESI-) m/z 193 (M-H)⁻.

### Example 75B

### 4-chloro-N-(1-{[(cyanoimino)(3-chloroanilino)methyl]amino}-2,2-dimethylpropyl)benzamide

Example 75A and Example 69B were processed as described in Example 54D to provide the desired product.
mp 158-160 °C;
MS (ESI+) m/z 418 (M+H)⁺;
¹H NMR (DMSO-d₆) δ 9.52 (s, 1H), 8.87 (d, 1H, J=8.5 Hz), 7.84 (d, 2H, J=8.5 Hz), 7.57 (d, 2H, J=8.5 Hz), 7.41 (t, 1H, J=8.1 Hz), 7.32 (m, 1H), 7.23 (m, 2H), 6.84 (d, 1H, J=9.0 Hz), 5.81 (t, 1H, J=8.8Hz), 0.98 (s, 3H);
Anal. calcd for C₂₀H₂₁Cl₂N₅O 0.9C₃H₈O: C, 56.32; H, 5.68; N, 17.07. Found: C, 56.07; H, 5.79; N, 17.12.

### Example 76

### 4-chloro-N-(1-{[(cyanoimino)(3-methoxyanilino)methy]amino}-2,2-dimethylpropyl)benzamide

### Example 76A

### N"-cyano-N-(3-methoxyphenyl)guanidine

3-Methoxyaniline and sodium dicyanamide were procesed as described in Example 71A to provide the desired compound.
MS (ESI-) m/z 191 (M-H)⁻.

### Example 76B

### 4-chloro-N-(1-{[(cyanoimino)(3-methoxyanilino)methyl]amino}-2,2-dimethylpropyl)benzamide

Example 76A and Example 69B were processed as described in Example 54D to provide the desired product.
mp 173-175 °C;
MS (ESI+) m/z 414 (M+H)⁺;
¹H NMR (DMSO-d₆) δ 9.39 (s, 1H), 8.37 (d, 1H, J=8.8 Hz), 7.81 (d, 2H, J=8.5 Hz), 7.57 (d, 2H, J=8.5 Hz), 7.32 (t, 1H, J=8.1 Hz), 6.82 (m, 3H), 6.63 (d, 1H, J=9.1 Hz), 5.84 (t, 1H, J=8.8 Hz), 3.74 (s, 3H), 0.95 (s, 3H);
Anal. calcd for C₂₁H₂₄ClN₅O₂: C, 60.94; H, 5.84; N, 16.92. Found: C, 60.98; H, 5.77; N, 17.03.

### Example 77

### 4-chloro-N-(1-{[[(2-chlorobenzyl)aminol(cyanoimino)methyl]amino}-2,2-dimethylpronyl)benzamide

### Example 77A

### methyl N-(2-chlorobenzyl)-N'-cyanoimidothiocarbamate

2-Chlorobenzylamine and dimethyl N-cyanodithioiminocarbonate were processed as described in Example 54B to give the desired product.
MS (ESI+) m/z 240 (M+H)⁺.

### Example 77B

### N-(2-chlorobenzyl)-N"-cyanoguanidine

Example 77A and ammonia were processed as described in Example 54C to give the desired product.
MS (ESI+) m/z 209 (M+H)⁺.

### Example 77C

### 4-chloro-N-(1-{[[(2-chlorobenzyl)amino](cyanoimino)methyl]amino}-2,2-dimethylpropyl)benzamide

Example 77B and Example 69B were processed as described in Example 54D to provide the desired product.
mp 180-182 °C;
MS (DCI/NH₃) m/z 432 (M+H)⁺;
¹H NMR (DMSO-d₆) δ 8.49 (br d, 1H, J=5.1 Hz), 7.90-7.82 (m, 3H), 7.83 (d, 1H, J=8.5 Hz), 7.67 (d, 1H, J=8.6 Hz), 7.46-7.41 (m, 1H), 7.33-7.25 (m, 3H), 6.44 (d, 1H, J=8.6 Hz), 5.71 (t, 1H, J=8.1 Hz), 4.46-4.38 (m, 2H), 0.97 (s, 9H);
Anal. calcd for C₂₁H₂₃Cl₂N₅O: C, 58.34; H, 5.36; N, 16.20. Found: C, 58.00; H, 5.20; N, 16.65.

### Example 78

### 4-chloro-N-(1-{[[(3-chlorobenzyl)aminol(cyanoimino)methyl]amino}-2,2-dimethylpropyl)benzamide

### Example 78A

### methyl N-(3-chlorobenzyl-N'-cyanoimidothiocarbamate

3-Chlorobenzylamine and dimethyl N-cyanodithioiminocarbonate were processed as described in Example 54B to give the desired product.
MS (ESI+) m/z 240 (M+H)⁺.

### Example 78B

### N-(3-chlorobenzyl)-N"-cyanoguanidine

Example 78A and ammonia were processed as described in Example 54C to give the desired product.
MS (ESI+) m/z 209 (M+H)⁺.

### Example 78C

### 4-chloro-N-(1-{[[(3-chlorobenzyl)amino](cyanoiminolmethyl]amino-2,2-dimethylpropyl)benzamide

Example 78B and Example 69B were processed as described in Example 54D to provide the desired product.
mp 178-180 °C;
MS (DCI/NH₃) m/z 432 (M+H)⁺;
¹H NMR (DMSO-d₆) δ 8.33 (br d, 1H, J=5.2 Hz), 7.95-7.85 (m, 2H), 7.83 (d, 1H, J=8.5 Hz), 7.79-7.73 (m, 1H), 7.67 (d, 1H, J=8.5 Hz), 7.46-7.41 (m, 1H), 7.33-7.25 (m, 3H), 6.41 (d, 1H, J=8.7 Hz), 5.66 (t, 1H, J=8.1 Hz), 4.46-4.38 (m, 2H), 0.98 (s, 9H);
Anal. calcd for C₂₁H₂₃Cl₂N₅O: C, 58.34; H, 5.36; N, 16.20. Found: C, 57.87; H, 5.22; N, 16.02.

### Example 79

### 4-chloro-N-(1-{[[(4-chlorobenzyl)amino](cyanoimino)methyl]amino}-2,2-dimethylpropyl)benzamide

### Example 79A

### methyl N-(4-chlorobenzyl)-N'-cyanoimidothiocarbamate

4-Chlorobenzylamine and dimethyl N-cyanodithioiminocarbonate were processed as described in Example 54B to give the desired product.
MS (ESI+) m/z 240 (M+H)⁺.

### Example 79B

### N-(4-chlorobenzyl)-N"-cyanoguanidine

Example 79A and ammonia were processed as described in Example 54C to give the desired product.
MS (ESI+) m/z 209 (M+H)⁺.

### Example 79C

### 4-chloro-N-(1-{[[(4-chlorobenzyl)aminol(cyanoimino)methyl]amino} -2,2-dimethypropyl)benzamide

Example 79B and Example 69B were processed as described in Example 54D to provide the desired product.
mp 173-175 °C;
MS (DCI/NH₃) m/z 432 (M+H)⁺;
¹H NMR (DMSO-d₆) δ 8.62 (br d, 1H, J=5.5 Hz), 7.91-7.84 (m, 2H), 7.83 (d, 1H, J=8.6 Hz), 7.83-7.77 (m, 1H), 7.79 (d, 1H, J=8.2 Hz), 7.56-7.50 (m, 1H), 7.39-7.32 (m, 2H), 7.27-7.22 (m, 1H), 6.50 (d, 1H, J=8.4 Hz), 5.70 (t, 1H, J=8.5 Hz), 4.31 (d, 2H, J=6.0 Hz), 0.97 (s, 9H);
Anal. calcd for C₂₁H₂₃Cl₂N₅O: C, 58.34; H, 5.36; N, 16.20. Found: C, 57.12; H, 5.18; N, 16.04.

### Example 80

### 4-chloro-N-[1-({(cyanoimino)[(3-pyridinylmethyl)aminolmethyl}amino)-2,2-dimethylpropyl]benzamide

### Example 80A

### methyl N'-cyano-N-(3-pyridinylmethyl)imidothiocarbamate

3-(Aminomethyl)pyridine and dimethyl N-cyanodithioiminocarbonate were processed as described in Example 54B to give the desired product.
MS (ESI+) m/z 207 (M+H)⁺.

### Example 80B

### N"-cyano-N-(3-pyridinylmethyl)guanidine

Example 80A and ammonia were processed as described in Example 54C to give the desired product.
MS (ESI+) m/z 176 (M+H)⁺.

### Example 80C

### 4-chloro-N-[1-({(cyanoimino)[(3-pyridinylmethyl)amino]methyl}amino)-2,2-dimethylpropyl]benzamide

Example 80B and Example 69B were processed as described in Example 54D to provide the desired product.
mp 188-189°C;
MS (ESI+) m/z 399 (M+H)⁺;
¹H NMR (DMSO-d₆)) δ 8.49 (d, 1H, J=1.8 Hz), 8.45 (dd, 1H, J=5.3, 1.5 Hz), 7.86 (m, 1H), 7.84 (d, 2H, J=8.5 Hz), 7.65 (m, 1H), 7.60 (d, 2H, J=8.5 Hz), 7.33 (dd, 1H, J=10.4, 5.5 Hz), 6.50 (d, 1H, J=9.6 Hz), 6.68 (t, 1H, J=9.2 Hz), 4.41 (m, 2H), 0.90 (s, 3H);
Anal. calcd for C₂₀H₂₃ClN₆O 0.15H₂O: C, 59.81; H, 5.85; N, 20.93. Found: C, 59.71; H, 5.77; N, 20.66.

### Example 81

### 4-chloro-N-[1-({(cyanoimino)[(4-pyridinylmethyl)amino]methyl}amino)-2,2-dimethylpropyl]benzamide

### Example 81 A

### methyl N'-cyano-N-(4-pyridinylmethyl)imidothiocarbamate

4-(Aminomethyl)pyridine and dimethyl N-cyanodithioiminocarbonate were processed as described in Example 54B to provide the desired product.
MS (ESI+) m/z 207 (M+H)⁺.

### Example 81 B

### N"-cyano-N-(3-pyridinylmethyl)guanidine

Example 81 A and ammonia were processed as described in Example 54C to give the desired product.
MS (ESI+) m/z 176 (M+H)⁺.

### Example 81C

### 4-chloro-N-[1-({(cyanoimino)[(4-pyridinylmethyl)amino]methyl}aminol-2,2-dimethylpropyl]benzamide

Example 81B and Example 69B were processed as described in Example 54D to provide the desired product. mp 189-191 °C;
MS (ESI+) m/z 399 (M+H)⁺;
¹H NMR (DMSO-d₆) δ 8.62 (d, 1H, J=8.1 Hz), 8.47 (d, 2H, J=6.5 Hz), 8.08 (t, 1H, J=5.9 Hz), 7.87 (d, 2H, J=8.5 Hz), 7.59 (d, 2H, J=8.5 Hz), 7.24 (d, 2H, J=5.9 Hz), 6.50 (d, 1H, J=9.9 Hz), 5.70 (t, 1H, J=8.8 Hz), 4.44 (dd, 1H, J=16.7, 6.3 Hz), 4.36 (dd, 1H, J=16.6, 5.9 Hz), 0.92 (s, 3H);
Anal. calcd for C₂₀H₂₃ClN₆O: C, 60.22; H, 5.81; N, 21.07. Found: C, 60.03; H, 6.01; N, 21.23.

### Example 82

### 4-chloro-N-[1-({(cyanoimino)[(2-pyridinylmethyl)amino]methyl}amino)-2,2-dimethylpropyl]benzamide

### Example 82A

### methyl N'-cyano-N-(2-pyridinylmethyl)imidothiocarbamate

2-(Aminomethyl)pyridine and dimethyl N-cyanodithioiminocarbonate were processed as described in Example 54B to give the desired product.
MS (ESI+) m/z 207 (M+H)⁺.

### Example 82B

### N"-cyano-N-(2-pyridinylmethyl)guanidine

Example 82A and ammonia were processed as described in Example 54C to give the desired product.
MS (ESI+) m/z 176 (M+H)⁺.

### Example 82C

### 4-chloro-N-[1-({(cyanoimino)[(2-pyridinlmethyl)amino]methyl]amino)-2,2-dimethylpropyl]benzamide

Example 82B and Example 69B were processed as described in Example 54D to provide the desired product.
mp 184-185 °C;
MS (ESI+) m/z 399 (M+H)⁺;
¹H NMR (DMSO-d₆) δ 8.52 (dd, 1H, J=5.3, 2.2 Hz), 8.45 (d, 1H, J=8.1 Hz), 7.93 (t, 1H, J=5.5 Hz), 7.86 (d, 2H, J=8.8 Hz), 7.82 (m, 1H), 7.58 (d, 2H, J=8.8 Hz), 7.35 (m, 2H), 7.05 (m, 1H), 5.72 (t, 1H, J=9.2 Hz), 4.46 (d, 2H, J=5.9 Hz), 0.90 (s, 9H);
Anal. Calcd for C₂₀H₂₃ClN₆O: C, 60.22; H, 5.81; N, 21.07. Found: C, 60.09; H, 5.90; N, 21.29.

### Example 83

### 4-chloro-N-(1-{[(cyanoimino)(3-quinolinylamino)methyl]amino}-2,2-dimethylpropyl)benzamide

### Example 83A

### methyl N'-cyano-N-(2-quinolinylmethyl)imidothiocarbamate

3-Aminoquinoline and dimethyl N-cyanodithioiminocarbonate were processed as described in Example 54B to provide the desired product.
MS (ESI+) m/z 243 (M+H)⁺.

### Example 83B

### N"-cyano-N-(2-quinolinylmethyl)guanidine

Example 83A and ammonia were processed as described in Example 54C to provide the desired product.
MS (ESI+) m/z 212 (M+H)⁺.

### Example 83C

### 4-chloro-N-(1-{[(cyanoimino)(3-quinolinylamino)methyl]amino}-2,2-dimethylpropyl)benzamide

Example 83B and Example 69B were processed as described in Example 54D to provide the desired product.
mp 218-219 °C;
MS (DCI/NH₃) m/z 435 (M+H)⁺;
¹H NMR (DMSO-d₆) δ 9.75 (s, 1H), 8.80 (d, 1H, J=1.9 Hz), 8.35 (d, 1H, J=8.4 Hz), 8.18 (d, 1H, J=3.5 Hz), 8.02 (d, 1H, J=8.6 Hz), 7.94 (dd, 1H, J=8.5, 1.5 Hz), 7.85 (d, 2H, J=8.7 Hz), 7.73 (ddd, 1H, J=8.4, 7.8, 2.3 Hz), 7.62 (ddd, 1H, J=8.4, 7.3, 1.8 Hz), 7.57 (d, 2H, J=8.6 Hz), 6.94 (d, 1H, J=8.8 Hz), 5.88 (t, 1H, J=8.6 Hz), 1.01 (s, 9H);
Anal. calcd for C₂₃H₂₃ClN6O: C, 63.52; H, 5.33; N, 19.32. Found: C, 63.31; H, 5.42; N, 18.98.

### Example 84

### 4-chloro-N-(1-{[ (cyanoimino)(3-pyridinylamino)methyl]amino}propyl)benzamide

### Example 84A

### N-[1-(1H-1,2,3-benzotriazol-1-yl)propyl]-4-chlorobenzamide

A suspension of 4-chlorobenzamide (1.50 g, 9.6 mmol), propionaldehyde diethyl acetal (1.53 g, 11.6 mmol), and benzotriazole (1.15 g, 9.6 mmol) in toluene (35 mL) was treated with p-toluenesulfonic acid (100 mg, 0.53 mmol). The mixture was heated at reflux under Dean-Stark conditions for 16 hours, cooled to ambient temperature, and concentrated to dryness. The crude material was purified by flash chromatography (elution with 40% ethyl acetate/hexanes) to provide 0.860 g of the desired product as a white solid.
MS (DCI/NH₃) m/z 315 (M+H)⁺.

### Example 84B

### 4-chloro-N-(1-{ [(cyanoimino)(3-pyridinylamino)methyl]amino}propyl)benzamide

Example 54C and Example 84A were processed as described in Example 54D to provide the desired product.
mp 181-183°C;
MS (DCI/NH₃) m/z 357 (M+H)⁺;
¹H NMR (DMSO-d₆) δ 9.76 (s, 1H), 8.99 (d, 1H, J=6.9 Hz), 8.50 (d, 1H, J=2.4 Hz), 8.32 (dd, 1H, J=4.5, 2.2 Hz), 7.93 (d, 2H, J=8.6 Hz), 7.37 (dd, 1H, J=7.6, 3.3 Hz), 5.58 (m, 1H), 1.88 (m, 2H), 0.94 (t, 3H, J=7.3 Hz);
Anal. calcd for C₁₇H₁₇ClN₆O: C, 57.22; H, 4.80; N, 23.55. Found: C, 56.92; H, 4.99; N, 23.27.

### Example 85

### 4-chloro-N-({[(cyanoimino)(3-pyridinylaminolmethyl]amino}methyl)benzamide

### Example 85A

### N-(1H-1,2,3-benzotriazol-1ylmethyl)-4-chlorobenzamide

A suspension of 4-chlorobenzamide (2.61 g, 16.8 mmol), paraformaldehyde (0.50 g, 16.8 mmol), benzotriazole (2.00 g, 16.8mmol), and MgSO₄ (2.00 g, 16.6 mmol) in toluene (50 mL) was treated with p-toluenesulfonic acid (168 mg, 0.88 mmol). The mixture was heated at reflux for 4 hours, cooled to ambient temperature, filtered, and concentrated to dryness. The crude material was purified by trituration with diethyl ether to provide 2.11 g of the desired product as a white solid.
MS (DCI/NH₃) m/z 287 (M+H)⁺.

### Example 85B

### 4-chloro-N-({[(cyanoimino)(3-pyridinylamino)methyl]amino}methyl)benzamide

Example 54C and Example 85A were processed as described in Example 54D to provide the desired product.
mp 196-197°C;
MS (DCI/NH₃) m/z 329 (M+H)⁺;
¹H NMR (DMSO-d₆) δ 9.81 (s, 1H), 9.42 (s, 1H), 8.55 (d, 1H, J=1.4 Hz), 8.34 (dd, 1H, J=4.8, 2.2 Hz), 8.09 (s, 1H), 7.95 (d, 2H, J=8.5 Hz), 7.79 (ddd, 1H, J=8.5, 3.1, 2.2 Hz), 7.58 (d, 2H, J=7.6 Hz), 7.38 (dd, 1H, J=7.6, 4.4 Hz), 4.82 (s, 2H);
Anal. calcd for C₁₅H₁₃ClN₆O: C, 54.80; H, 3.99; N, 25.56. Found: C, 54.44; H, 4.04; N, 25.29.

### Example 86

### (-) 4-chloro-N-(4-cyano-1-{[(cyanoimino)(3-pyridinylamino)methyl]amino}-2-2 diethylbutyl)benzamide

Example 65B was chromatographed over a Daicel Chiral Technologies Chiralcel AS chiral column (2.0 cmx25 cm) eluting with 5% ethanol/hexanes (flow rate=10 mL/minutes) to provide the desired product as the levorotatory enantiomer.
[α]_{D}23 = -23° (c 0.5, DMSO);
mp 150-152 °C;
MS (ESI+) m/z 452 (M+H)⁺;
¹H NMR (DMSO-d₆) δ 9.56 (s, 1H), 8.45 (d, 1H, J=2.2 Hz), 8.41 (d, 1H, J=4.4 Hz), 8.34 (d, 1H, J=8.8 Hz), 7.83 (d, 2H, J=8.8 Hz), 7.64 (m, 1H), 7.60 (d, 2H, J=8.5 Hz), 7.43 (dd, 1H, J=8.1, 4.8 Hz), 6.75 (m, 1H), 5.95 (dd, 1H, J=8.8, 8.0 Hz), 2.54 (m, 2H), 1.71 (t, 2H, J=8.1 Hz), 1.39 (m, 4H), 0.85 (m, 6H); Anal. calcd for C₂₃H₂₆ClN₇O: C, 60.52; H, 5.85; N, 21.48. Found: C, 60.64; H, 5.86; N, 21.57.

### Example 87

### (+) 4-chloro-N-(4-cyano-1-{[(cyanoimino)(3-pyridinylamino)methyl]amino}-2,2-diethylbutyl)benzamide

Example 65B was chromatographed over a Daicel Chiral Technologies Chiralcel AS chiral column (2.0 cmx25 cm) eluting with 5% ethanol/hexanes (flow rate=10 mL/minutes) to provide the desired product as the dextrorotatory enantiomer.
[α]_{D}²³ = +21° (c 0.48, DMSO);
mp 146-148 °C;
MS (ESI+) m/z 452 (M+H)⁺;
¹H NMR (DMSO-d₆) δ 9.55 (s, 1H), 8.45 (d, 1H, J=2.2 Hz), 8.41 (d, 1H, J=4.4 Hz), 8.34 (d, 1H, J=8.8 Hz), 7.83 (d, 2H, J=8.5 Hz), 7.64 (m, 1H), 7.60 (d, 2H, J=8.5 Hz), 7.43 (dd, 1H, J=4.8, 8.1 Hz), 6.75 (m, 1H), 5.94 (dd, 1H, J=8.8, 7.8 Hz), 2.54 (m, 2H), 1.72 (t, 2H, J=8.1 Hz), 1.39 (m, 4H), 0.85 (m, 6H);
Anal. calcd for C₂₃H₂₆ClN₇O: C, 60.52; H, 5.85; N, 21.48. Found: C, 60.64; H, 5.84; N, 21.29.

### Example 88

### (+)4-chloro-N-[1-{[(cyanoimino)(3-pyridinylamino)methyl]amino}-2-(2,6,6-trimethyl-1-cyclohexen-1-yl)ethyl]benzamide

Example 66B was chromatographed over a Daicel Chiral Technologies Chiralcel OJ chiral column (2.0 cmx25 cm) eluting with 5% ethanol/hexanes (flow rate=10 mL/minutes) to provide the desired product as the dextrorotatory enantiomer.
[α]_{D}²³ = +12.2° (c 0.24, DMSO);
mp 95-96 °C;
MS (ESI+) m/z 465 (M+H)⁺;
¹H NMR (DMSO-d₆) δ 9.65 (br s, 1H), 8.80 (m, 1H), 8.46 (d, 1H, J=2.4 Hz), 8.37 (d, 1H, J=4.8 Hz), 7.86 (d, 2H, J=8.5 Hz), 7.66 (dt, 1H, J=7.8, 1.5 Hz), 7.60 (d, 2H, J=8.5 Hz), 7.41 (dd, 1H, J=8.5, 4.8 Hz), 7.12 (m, 1H), 5.85 (m, 1H), 2.60 (m, 2H), 1.87 (m, 2H), 1.63 (s, 3H), 1.53 (m, 2H), 1.38 (m, 2H), 1.02 (d, 3H, J=7.5 Hz);
Anal. calcd for C₂₅H₂₉ClN₆O 0.2CH₂Cl₂ 0.1C₆H₁₄: C, 63.16; H, 6.33; N, 17.13. Found: C, 63.30; H, 6.39; N, 16.92.

### Example 89

### (-) 4-chloro-N-[1-{[(cyanoimino)(3-pyridinylamino)methyl]amino}2-(2,6,6-trimethyl-1-cyclohexen-1-yl)ethyl]benzamide

Example 66B was chromatographed over a Daicel Chiral Technologies Chiralcel OJ chiral column (2.0 cmx25 cm) eluting with 5% ethanol/hexanes (flow rate=10 mL/minutes) to provide the desired product as the levorotatory enantiomer.
[α]D²³ = -13.5° (c 0.25, DMSO);
mp 94-96 °C;
MS (ESI+) m/z 465 (M+H)⁺;
¹H NMR (DMSO-d₆) δ 9.65 (brs, 1H), 8.80 (m, 1H), 8.46 (d, 1H, J=2.4 Hz), 8.37 (d, 1H, J=4.8 Hz), 7.86 (d, 2H, J=8.5 Hz), 7.66 (dt, 1H, J=7.8, 1.5 Hz), 7.60 (d, 2H, J=8.5 Hz), 7.41 (dd, 1H, J=8.5, 4.8 Hz), 7.12 (m, 1H), 5.85 (m, 1H), 2.60 (m, 2H), 1.87 (m, 2H), 1.63 (s, 3H), 1.53 (m, 2H), 1.38 (m, 2H), 1.02 (d, 3H, J=7.5 Hz);
Anal. calcd for C₂₅H₂₉ClN₆O 0.15CH₂Cl₂ 0.07C₆H₁₄: C, 63.48; H, 6.31; N, 17.37. Found: C, 63.74; H, 6.33; N, 17.13.

### Example 90

### (-) 4-chloro-N-(1-{[(cyanoimino)(3-pyridinylaminolmethyl]amino}-2,2-dimethyl-4-pentenyl)benzamide

Example 67B was chromatographed over a Daicel Chiral Technologies Chiralcel AS chiral column (2.0 cmx25 cm) eluting with 5% ethanol/hexanes (flow rate=10 mL/minutes) to provide the desired product as the levorotatory enantiomer.
[α]_{D}²³ = -18.3° (c 0.23, DMSO);
mp 97-98 °C;
MS (ESI+) m/z 411 (M+H)⁺;
¹H NMR (DMSO-d₆) δ 9.54 (br s, 1H), 8.47 (d, 1H, J=2.5 Hz), 8.39 (m, 2H), 7.84 (d, 2H, J=8.9 Hz), 7.68 (m, 1H), 7.57 (d, 2H, J=8.5 Hz), 7.43 (dd, 1H, J=8.1, 4.7 Hz), 6.84 (m, 1H), 5.88 (m, 2H), 5.09(s, 1H), 5.05 (m, 1H) 2.09 (m, 2H), 0.94 (s, 3H), 0.92 (s, 3H); Anal. calcd for C₂₁H₂₃ClN₆O 0.35H₂O: C, 60.46; H, 5.73; N, 20.14. Found: C, 60.20; H, 5.65; N, 20.21.

### Example 91

### (+)4-chloro-N-(1-{([(cyanoimino)(3-pyridinylamino)methyl]amino}-2,2-dimethyl-4-pentenyl)benzamide

Example 67B was chromatographed over a Daicel Chiral Technologies Chiralcel AS chiral column (2.0 cmx25 cm) eluting with 5% ethanol/hexanes (flow rate=10 mL/minutes) to provide the desired product as the dextrorotatory enantiomer.
[α]D²³ = +17.5° (c 0.24, DMSO);
mp 98-99 °C;
MS (ESI+) m/z 411 (M+H)⁺;
¹H NMR (DMSO-d₆) δ 9.54 (br s, 1H), 8.47 (d, 1H, J=2.5 Hz), 8.39 (m, 2H), 7.84 (d, 2H, J=8.9 Hz), 7.68 (m, 1H), 7.57 (d, 2H, J=8.5 Hz), 7.43 (dd, 1H, J=8.1, 4.7 Hz), 6.84 (m, 1H), 5.88 (m, 2H), 5.09 (s, 1H), 5.05 (m, 1H) 2.09 (m, 2H), 0.94 (s, 3H), 0.92 (s, 3H); Anal. calcd for C₂₁H₂₃ClN₆O 0.3H₂O: C, 60.59; H, 5.71; N, 20.19. Found: C, 60.53; H, 5.71; N, 19.97.

### Example 92

### 4-chloro-N-(1-{[(cyanoimino)(3-pyridinylamino)methlyl]amino}-3,3-dimethyl-4-pentenyl)benzamide

### Example 92A

### 3,3-dimethyl-4-pentenal

Prepared according to the method of Buchi, et. al. J.Org.Chem. (1983) 48, 5406-5408.
MS (DCl/NH₃) 113 (M+H)⁺.

### Example 92B

### N-[1-(1H-1,2,3-benzotriazol-1-yl)-3,3-dimethyl-4-pentenyl]-4-chlorobenzamide

A suspension of 4-chlorobenzamide (1.32 g, 8.5 mmol), Example 92A (0.96 g, 8.5 mmol), benzotriazole (1.01 g, 8.5 mmol), and MgSO₄ (2.00 g, 16.6 mmol) in toluene (50 mL) was treated with p-toluenesulfonic acid (100 mg, 0.53 mmol). The solution was heated at reflux for 48 hours, cooled to ambient temperature, filtered and concentrated to dryness. The crude material was purified by recrystallization from EtOAc/hexanes to provide 1.301 g of the desired product as a white solid.
MS (DCI/NH₃) 369 (M+H)⁺;
¹H NMR (DMSO-d₆) δ 9.80 (d, 1H, J=8.5 Hz), 8.03 (dd, 2H, J=8.6, 2.7 Hz), 7.88 (d, 2H, J= 8.6 Hz), 7.61-7.54 (m, 3H), 7.43-7.38 (m, 1H), 6.84 (q, 1H, J= 7.4 Hz), 5.75 (dd, 1H, J= 17.3, 10.7 Hz), 4.84-4.75 (m, 2H), 2.65-2.43 (m, 2H), 1.05 (s, 3H), 0.93 (s, 3H)

### Example 92C

### 4-chloro-N-(1-{[(cyanoimino)(3-pyridinylamino)methyl]amino)-3,3-dimethyl-4-pentenyl)benzamide

Example 54C and Example 92B were processed as described in Example 54D to provide the desired product.
mp 171-172°C;
MS (DCI/NH₃) m/z 411 (M+H)⁺;
¹H NMR (DMSO-d₆) δ 9.73 (s, 1H), 9.00 (s, 1H), 8.49 (d, 1H, J=2.4 Hz), 8.33 (dd, 1H, J=5.3, 1.9 Hz), 7.90 (m, 3H), 7.70 (m, 1H), 7.58 (d, 2H, J=8.6 Hz), 7.40-7.36 (m, 1H), 5.89 (dd, 1H, J=17.2, 11.5 Hz), 5.60 (pentet, 1H, J=7.4 Hz), 4.93-4.86 (m, 2H), 2.00 (d, 2H, J=6.3 Hz), 1.06 (s, 3H), 1.05 (s, 3H);
Anal. calcd for C₂₁H₂₃ClN₆O: C, 61.38; H, 5.64; N, 20.45. Found: C, 61.24; H, 5.76; N, 20.33.

### Example 93

### 4-chloro-N-(1-{[(cyanoimino)(3-pyridinylamino)methyl]amino}-2-cyclohexyl-2-methylpropyl)benzamide

### Example 93A

### N-[1-(1H-1,2,3-benzotriazol-1-yl)-2-cyclohexyl-2-methylpropyl]-4-chlorobenzamide

Oxalyl chloride (7.8 g, 61.42 mmol) in methylene chloride (60 mL) at -78 °C was treated with dimethylsufoxide (8.4 g, 107.5 mmol). After stirring at -78 °C for 10 minutes, the mixture was treated with 2-cyclohexyl-2-methylpropanol (4.8 g, 36.86 mmol) in 15 ml of methylene chloride. The mixture was allowed to stir at -78 °C for 30 minutes and then treated with triethylamine (15.54 g, 153.6 mmol). The reaction mixture was stirred for 10 minutes and then stirred an additional 5 minutes at 0 °C. The mixture was quenched with saturated aqueous ammonium (10 mL) chloride and extracted with diethyl ether (2 x 50 mL). The organic layer was washed with brine (15 mL), dried over anhydrous sodium sulfate and concentrated under vacuo. The crude product was redissolved in diethyl ether and the resulting precipitate was filtered through a pad of Celite. The solution was concentrated under reduced pressure to provide 4.5 g of 2-cyclohexyl-2-methylpropanal as an oil.

A suspension of p-chlorobenzamide (5.31 g, 35.10 mmol), 2-cyclohexyl-2-methylpropanal (4.5 g, 35.10 mmol), and benzotriazole (4.18 g, 35.10 mmol) in toluene (100 mL) was treated with p-toluenesulfonic acid (334 mg, 1.76 mmol). The solution was heated at reflux under Dean-Stark conditions for 10 hours, then cooled gradually to ambient temperature. The solvent was removed under reduced pressure and the residue was purified by flash chromatography (elution with 10% EtOAc/hexane) to provide 5.3 g of the desired product as a white solid.
MS (ESI+) m/z 383 (M+H)⁺.

### Example 93B

### 4-chloro-N-(1-{[(cyanoimino)(3-pyridinylamino)methyl]amino}-2-cyclohexyl-2-methylpropyl)benzamide

Example 54C and Example 93A were processed as described in Example 54D to provide the desired compound.
mp 181-183 °C;
MS (ESI+) m/z 453 (M+H)⁺;
¹H NMR (DMSO-d₆) δ 9.54 (s, 1H), 8.45 (d, 1H, J=2.2 Hz), 8.39 (d, 1H, J=5.8 Hz), 8.29 (d, 1H, J=8.8 Hz), 7.83 (d, 2H, J=8.8 Hz), 7.62 (m, 1H), 7.58 (d, 2H, J=8.8 Hz), 7.43 (dd, 1H, J=8.5, 4.8 Hz), 6.78 (d, 1H, J=9.1 Hz), 6.02 (t, 1H, J=8.8 Hz), 1.78 (m, 4H), 1.62 (m, 1 H), 1.28-0.96 (m, 6H), 0.87 (s, 3H), 0.83 (s, 3H); Anal. calcd for C₂₄H₂₉ClN₆O: C, 66.63; H, 6.45; N, 18.55. Found: C, 63.74; H, 6.39; N, 18.57.

### Example 94

### 4-chloro-N-(1-{[(cyanoimino)(3-pyridinylamino)methyl]amino}-2,2-dimethylhexyl)benzamide

### Example 94A

### N-[1-(1H-1,2,3-benzotriazol-1-yl)-2,2-dimethyl]hexyl]-4-chlorobenzamide

2,2-Dimethylhexanol was processed as described in Example 93A to provide the desired product.
MS (ESI+) m/z 383 (M+H)⁺.

### Example 94B

### 4-chloro-N-(1-{[(cyanoimino)(3-pyridinylamino)methyl]amino}-2,2-dimethylhexyl)benzamide

Example 54C and Example 94A were processed as described in Example 54D to provide the desired product.
mp 168-170 °C;
MS (ESI+) m/z 427 (M+H)⁺;
¹H NMR (DMSO-d₆) δ 9.53 (s, 1H), 8.46 (d, 1H, J=2.4 Hz), 8.38 (d, 1H, J=4.7 Hz), 8.34 (m, 1H), 7.83 (d, 2H, J=8.5 Hz), 7.65 (m, 1H), 7.57 (d, 2H, J=8.5 Hz), 7.43 (dd, 1H, J=4.4, 8.1 Hz), 6.79 (m, 1H), 5.86 (dd, 1H, J=8.9 Hz), 1.28 (m, 6H), 0.94 (s, 3H), 0.92 (s, 3H), 0.87 (t, 3H, J=13 Hz);
Anal. calcd for C₂₂H₂₇ClN₆O: C, 61.89; H, 6.37; N, 19.68. Found: C, 62.22; H, 6.37; N, 19.62.

### Example 95

### N-(2-(1-adamantyl)-1-{ [(cyanoimino)(3-pyridinylamino)methyl]amino}ethyl)-4-chlorobenzamide

### Example 95A

### N-[2-(1-adamantyl)-1-(1H-1,2,3-benzotriazol-1-yl)ethyl]-4-chlorobenzamide

2-(1-Adamantyl)ethanol was processed as described in Example 93A to provide the desired product.
MS (ESI+) m/z 435 (M+H)⁺.

### Example 95B

### N-(2-(1-adamantyl)-1-{[(cyanoimino)(3-pyridinylamino)methy]amino}ethyl)-4-chlorobenzamide

Example 54C and Example 95A were processed as described in Example 54D to provide the desired product.
mp 202-203 °C;
MS (ESI+) m/z 477 (M+H)⁺;
¹H NMR (DMSO-d₆) δ 9.70 (s, 1H), 9.00 (m, 1H), 8.46 (d, 1H, J=2.2 Hz), 8.33 (dd, 1H, J=4.8, 1.5 Hz), 7.91 (d, 2H, J=8.5 Hz), 7.66 (m, 2H), 7.58 (d, 2H, J=8.5 Hz), 7.38 (dd, 1H, J=8.1, 4.8 Hz), 5.76 (m, 1H), 4.11 (dd, 1H, J=6.6, 5.2 Hz), 3.17 (d, 1H, J=5.2 Hz), 1.91 (m, 3H), 1.82-1.51 (m, 12H);
Anal. calcd for C₂₆H₂₉ClN₆O: C, 65.47; H, 6.13; N, 17.62. Found: C, 65.30; H, 6.13; N, 17.69.

### Example 96

### N-(2,2-bis[(allyloxy)methyl]-1-{[(cyanoimino)(3-pyridinylamino)methyl]amino}butyl)-4-chlorobenzamide

### Example 96A

### N-[2,2-bis[(allyloxy)methyl]-1-(1H-1,2,3-benzotriazol-1-yl)butyl]-4-chlorobenzamide

2,2-Bis(allyloxymethyl)-1-butanol was processed as described in Example 93A to provide the desired product.
MS (ESI+) m/z 469 (M+H)⁺.

### Example 96B

### N-(2,2-bis[(allyloxy)methyl]-1-{[(cyanoimino)(3-pyridinylamino)methyl]amino}butyl)-4-chlorobenzamide

Example 54C and Example 96A were processed as described in Example 54D to provide the desired product.
MS (ESI+) m/z 511 (M+H)⁺;
¹H NMR (DMSO-d₆) δ 9.63 (s, 1H), 8.88 (m, 1H), 8.54 (d, 1H, J=2.7 Hz), 8.39 (dd, 1H, J=4.5, 1.4 Hz), 7.84 (d, 2H, J=8.5 Hz), 7.80 (m, 1H), 7.60 (d, 2H, J=8.5 Hz), 7.42 (dd, 1H, J=8.1, 4.8 Hz), 5.93-5.74 (m, 3H), 5.27-5.08 (m, 4H), 4.03-3.08 (m, 4H), 3.60 (d, 1H, J=9.5 Hz), 3.47-3.32 (m, 3H), 1.43 (m, 2H), 0.86 (t, 3H, J=7.4 Hz);
Anal. calcd for C₂₆H₃₁ClN₆O₃ 0.35 C₃H₇NO: C, 60.55; H, 6.28; N, 16.58. Found: C, 60.84; H, 6.08; N, 16.96.

### Example 97

### 4-chloro-N-[1-{[(cyanoimino)(3-pyridinylamino)methyl]amino}-3-(dimethylamino)-2,2-dimethylpropyl]benzamide

### Example 97A

### N-[1-(1H-1,2,3-benzotriazol-1-yl)3-(dimethylamino)-2,2-dimethylpropyl]-4-chlorobenzamide

3-(Dimethylamino)-2,2-dimethyl-1-propanol was processed as described in Example 93A to provide the desired product.
MS (ESI+) m/z 386 (M+H)⁺.

### Example 97B

### 4-chloro-N-(1--1[(cyanoimino)(3-pyridinylamino)methyl]amino}-3-(dimethylamino)-2,2-dimethylpropyl]benzamide

Example 54C and Example 97A were processed as described in Example 54D to provide the desired product.
mp 101-103 °C;
MS (ESI+) m/z 428 (M+H)⁺;
¹H NMR (DMSO-d₆) δ 9.52 (s, 1H), 8.81 (m, 1H), 8.56 (m, 1H), 8.38(m, 1H), 7.81 (d, 2H, J=8.5 Hz), 7.80 (m, 1H), 7.59 (d, 2H, J=8.5 Hz), 7.43 (dd, 1H, J=8.1, 4.1 Hz), 5.57 (t, 1H, J=8.4 Hz), 2.09 (m, 8H), 1.12 (s, 3H), 0.83 (s, 3H);
Anal. calcd for C₂₁H₂₆ClN₇O: C, 58.94; H, 6.12; N, 22.91. Found: C, 58.72; H, 5.97; N, 22.76.

### Example 98

### tert-butyl (2R)-2-((R)-[(4-chlorobenzoyl)amino]{[(cyanoimino)(3-pyridinylamino)methyl]amino}methyl)-1-pyrrolidinecarboxylate

### Example 98A

### tert-butyl (2R)-2- {1H-1,2,3-benzotriazol-1-yl[4-chlorobenzoyl)amino]methyl}-1-pyrrolidinecarboxylate

4-Chlorobenzamide, tert-butyl (2R)-formyl-1-pyrrolidinecarboxylate, benzotriazole, and p-toluenesulfonic acid were processed as described in Example 53A to provide the desired product.
MS (ESI+) m/z 456 (M+H)⁺.

### Example 98B

### tert-butyl (2R)-2-((R)-[(4-chlorobenzoyl)amino]{[(cyanoimino)(3 - pyridinylamino)methyl]amino}methyl)-1-pyrrolidinecarboxylate

Example 54C and Example 98A were processed as described in Example 54D to provide the desired product.
mp 184-185 °C;
MS (ESI+) m/z 498 (M+H)⁺;
¹H NMR (DMSO-d₆) δ 9.61 (s, 1H), 8.78 (m, 1H), 8.45 (d, 1H, J=2.2 Hz), 8.36 (m, 1H), 7.83 (d, 2H, J=8.5 Hz), 7.65 (m, 1H), 7.60 (d, 2H, J=8.5 Hz), 7.38 (m, 1H), 5.65 (m, 1H), 4.21 (m, 1H), 1.90 (m, 6H), 1.31 (s, 9H);
Anal. calcd for C₂₄H₂₈ClN₇O₃: C, 57.89; H, 5.67; N, 19.69. Found: C, 57.94; H, 5.59; N, 19.72.

### Example 99

### 4-chloro-N-[1-{[(cyanoimino)(3-pyridinylamino)methyl]amino}-3-(methylsulfanyl)propyl]benzamide

### Example 99A

### N-[1-(1H-1,2,3-benzotriazol-1-yl)-3-(methylsulfanyl)propyl]-4-chlorobenzamide

4-Chlorobenzamide, 3-methylthio-1-propanal, benzotriazole, and p-toluenesulfonic acid were processed as described in Example 53A to provide the desired product.
MS (ESI+) m/z 361 (M+H)⁺.

### Example 99B

### 4-chloro-N-[1-{[(cyanoimino)(3-pyridinylaminolmethyl]amino}-3-(methylsulfanyl)propyl]benzamide

Example 54C and Example 99A were processed as described in Example 54D to provide the desired product.
mp 104-105 °C;
(ESI+) m/z 403 (M+H)⁺;
¹H NMR (DMSO-d₆) δ 9.82 (br s, 1H), 9.09 (m, 1H), 8.49 (d, 1H, J=2.6 Hz), 8.32 (d, 1H, J=4.9 Hz), 7.94 (d, 2H, J=8.5 Hz), 7.85 (m, 1H), 7.70 (m, 1H), 7.59 (d, 2H, J=8.5 Hz), 7.38 (dd, 1H, J=8.3, 4.4 Hz), 5.73 (m, 1H), 2.58 (m, 2H), 2.16 (m, 2H), 2.08 (s, 3H); Anal. calcd for C₁₈H₁₉ClN₆OS 0.1CH₂Cl₂: C, 52.84; H, 4.70; N, 20.43. Found: C, 53.04; H, 4.89; N, 20.08.

### Example 100

### N-(1-adamantyl {[(cyanoimino)(3-pyridinylamino)methyl]amino}methyl)-4-chlorobenzamide

### Example 100A

### N-[1-adamantyl(1H-1,2,3-benzotriazol-1-yl)methyl]-4-chlorobenzamide

1-Adamantylmethanol was processed as described in Example 93A to provide the desired product.
MS (ESI+) m/z 421 (M+H)⁺.

### Example 100B

### N-(1-adamantyl{[(cyanoimino)(3-pyridinylamino)methyl]amino}methyl)-4-chlorobenzamide

Example 54C and Example 100A were processed as described in Example 54D to provide the desired product.
mp 209-210 °C;
MS (ESI+) m/z 463 (M+H)⁺;
¹H NMR (DMSO-d₆) δ 9.52 (s, 1H), 8.49 (d, 1H, J=2.6 Hz), 8.38 (m, 2H), 7.84 (d, 2H, J=8.5 Hz), 7.69 (m, 1H), 7.59 (d, 2H, J=8.5 Hz), 7.42 (dd, 1H, J=8.0, 4.8 Hz), 6.82 (m, 1H), 5.69 (t, 1H, J=8.8, 8.8 Hz), 1.72-1.51 (m, 15H);
Anal. calcd for C₂₅H₂₇CIN₆O 0.2 CH₂Cl₂ 0.4 C₄H₈O₂: C, 62.48; H, 5.99; N, 16.31. Found: C, 62.68; H, 6.26; N, 16.37.

### Example 101

### 4-chloro-N-[{[(cyanoimino)(3-pyridinylamino)methyl]amino}(5-ethyl-1,3-dioxan-5-yl)methyl]benzamide

### Example 101A

### N-[1H-1,2,3-benzotriazol-1-yl(5-ethyl-1,3-dioxan-5-yl)methyl]-4-chlorobenzamide

5-Ethyl-1,3-dioxan-5-ol was processed as described in Example 93A to provide the desired product.
MS (ESI+) m/z 399 (M+H)⁺.

### Example 101B

### 4-chloro-N-[{[(cyanoimino)(3-pyridinylamino)methyl]amino}(5-ethyl-1,3-dioxan-5-yl)methyl]benzamide

Example 54C and Example 101A were processed as described in Example 54D to provide the desired product.
mp 194-195 °C;
MS (ESI+) m/z 443 (M+H)⁺;
¹H NMR (DMSO-d₆) δ 9.55 (s, 1H), 8.74 (m, 1H), 8.52 (d, 1H, J=2.2 Hz), 8.40 (d, 1H, J=4.1 Hz), 7.88 (d, 2H, J=8.8 Hz), 7.75 (m, 1H), 7.58 (d, 2H, J=8.8 Hz), 7.42 (dd, 1H, J=8.3, 4.4 Hz), 7.32 (m, 1H), 6.07 (t, 1H, J=8.5 Hz), 4.78 (d, 1H, J=5.9 Hz), 4.70 (d, 1H, J=5.9 Hz), 4.15 (m, 1H), 4.03 (m, 1H), 3.68 (d, 1H, J=12.1 Hz), 3.64 (d, 1H, J=12.1 Hz), 1.35 (m, 2H), 0.85 (t, 3H, J=7.4 Hz);
Anal. calcd for C₂₁H₂₃ClN₆O₃: C, 56.95; H, 5.23; N, 18.98. Found: C, 56.69; H, 5.20; N, 19.02.

### Example 102

### 4-chloro-N-(1-{[(cyanoimino)(3-pyridinylamino)methyl]amino}-2,2-dimethyl-3-phenylpropyl)benzamide

### Example 102A

### N-[1-(1H-1,2,3-benzotriazol-1-yl)-2,2-dimethyl-3-phenylpropyl]-4-chlorobenzamide

2,2-Dimethyl-3-phenyl-1-propanol was processed as described in Example 93A to provide the desired product.
MS (ESI+) m/z 419 (M+H)⁺.

### Example 102B

### 4-chloro-N-(1-{[(cyanoimino)(3-pyridinylamino)methyl]amino}-2,2-dimethyl-3-phenylpropyl)benzamide

Example 54C and Example 102A were processed as described in Example 54D to provide the desired product.
mp 182-183 °C;
MS (ESI+) m/z 461 (M+H)⁺;
¹H NMR (DMSO-d₆) δ 9.59 (s, 1H), 8.50 (d, 1H, J=2.2 Hz), 8.47 (m, 1H), 8.39 (d, 1H, J=4.4 Hz), 7.86 (d, 2H, J=8.5 Hz), 7.70 (m, 1H), 7.59 (d, 2H, J=8.5 Hz), 7.43 (dd, 1H, J=7.7, 4.8 Hz), 7.29 (m, 2H), 7.21 (m, 3H), 6.98 (m, 1H), 5.91 (t, 1H, J=8.8 Hz), 2.72 (d, 1H, J=12.9 Hz), 2.60 (d, 1H, J=12.9 Hz), 0.87 (s, 3H), 0.86 (s, 3H);
Anal. calcd for C₂₅H₂₅ClN₆O: C, 65.14; H, 5.47; N, 18.23. Found: C, 65.07; H, 5.48; N, 18.36.

### Example 103

### N-(1-{[(cyanoimino)(3-pyridinylamino)methyl]amino}-2,2-dimethylpropyl)-4-iodobenzamide

### Example 103A

### 4-iodobenzamide

A suspension of 4-iodobenzoic acid (20.0 g, 80.6 mL) in CH₂Cl₂ (350 mL) at 0 °C was treated with oxalyl chloride (42.3 mL of a 2.0 M solution in CH₂Cl₂, 84.7 mmol). Dimethylformamide was added (0.5 mL) until a homogeneous solution was achieved and then the cooling bath was removed. The reaction mixture was stirred at ambient temperature for 3 hours and then concentrated to provide crude 4-iodobenzoyl chloride as a waxy oil that was used without further purification.

Crude 4-iodobenzoyl chloride (11.4 g, 42.7 mmol) in THF (75 mL), prepared above, was treated with ammonium hydroxide (5 mL) followed by water (40 mL) at ambient temperature. The reaction mixture was stirred for 3 hours and then partitioned between EtOAc (50 mL) and brine (50 mL). The phases were separated and the organic phase was washed with 10% aqueous NaHCO₃ solution (30 mL) and brine (30 mL), dried over Na₂SO₄, and filtered. The filtrate volume was reduced until a white solid precipitated from solution. The white solid was collected by filtration and washed with diethyl ether to provide the desired product (10.2 g, 41.4 mmol, 97%).
MS (DCl/NH₃) m/z 265 (M+NH₄)⁺.

### Example 103B

### N-[1-(1H-1,2,3-benzotriazol-1-yl)-2,2-dimethylpropyl]-4-iodobenzamide

Example 103A, pivaldehyde, benzotriazole, and p-toluenesulfonic acid were processed as described in Example 53A to provide the desired product.
MS (ESI+) m/z 435 (M+H)⁺.

### Example 103C

### N-(1-{[(cyanoimino)(3-pyridinylamino)methyl]amino}-2,2-dimethylpropyl)-4-iodobenzamide

Example 54C and Example 103B were processed as described in Example 54D to provide the desired product.
mp 180-183 °C;
MS (ESI+) m/z 477 (M+H)⁺;
¹H NMR (DMSO-d₆) δ 9.52 (br s, 1H), 8.48 (d, 1H, J=2.4 Hz), 8.41-8.35 (m, 2H), 7.49 (d, 2H, J=8.5 Hz), 7.68 (br d, 1H, J=8.3 Hz), 7.60 (d, 2H, J=8.5 Hz), 7.42 (dd, 1H, J=8.1, 2.7 Hz), 6.83 (br d, 1H, J=9.2 Hz), 5.83. (t, 1H, J =8.8 Hz), 0.98 (s, 9H);
Anal. calcd for C₁₉H₂₁IN₆O: C, 47.91; H, 4.44; N, 17.64. Found: C, 47.80; H, 4.71; N, 17.30.

### Example 104

### N-(1- {([(cyanoimino)(3-pyridinylamino)methyl]amino}-2,2-dimethylpropyl)-4-(2-furyl)benzamide

Example 103C (0.230 g, 0.48 mmol), triphenylarsenine (0.04 g, 0.12 mmol), and 2-(tributylstannyl)furan (0.28 g, 0.77 mmol) in N-methylpyrrolidinone (3.5 mL) were treated with tris(dibenzylidineacetone)dipalladium(0) (0.02 g, 0.02 mmol) and stirred at ambient temperature for 6 hours. The reaction mixture was diluted with 100 mL EtOAc and filtered through a pad of celite and the solvent removed under reduced pressure. The crude material was purified by flash chromatography (elution with 80% ethyl acetate/hexanes) to provide 0.088 g of the desired product as a white solid.
mp 181-182°C;
MS (ESI+) m/z 417 (M+H)⁺;
¹H NMR (DMSO-d₆) δ 9.57 (s, 1H), 8.50 (d, 1H, J=8.4 Hz), 8.40 (dd, 1H, J=5.3, 1.2 Hz), 8.36 (d, 1H, J=8.5 Hz), 7.89 (d, 2H, J=8.6 Hz), 7.83 (s, 1H), 7.82 (d, 2H, J=8.6 Hz), 7.71 (d, 1H, J=8.7 Hz), 7.44 (dd, 1H, J=8.7, 5.9 Hz), 7.12 (d, 1H, J=3.6 Hz), 6.87 (d, 1H, J=11.2 Hz), 6.65 (dd, 1H, J=3.6, 1.7 Hz), 5.87 (t, 1H, J=9 Hz), 1.00 (s, 9H);
Anal. calcd for C₂₃H₂₄N₆O₂·0.2 H₂O: C, 65.76; H, 5.85; N, 20.01. Found: C, 65.73; H, 5.76; N, 20.15.

### Example 105

### 4-bromo-N-(1-{[(cyanoimino)(3-pyridinylamino)methyl]amino} -2,2-dimethypropyl)benzamide

### Example 105A

### N-[1-(1H-1,2,3-benzotriazol-1-yl)-2,2-dimethylpropyl]-4-bromobenzamide

4-Bromobenzamide, pivaldehyde, benzotriazole, and p-toluenesulfonic acid were processed as described in Example 53A to provide the desired product.
MS (DCI/NH₃) m/z 387 (M+H)⁺.

### Example 105B

### 4-bromo-N-(1-{[(cyanoimino)(3-pyridinylamino)methyl]amino} -2,2-dimethylpropyl)benzamide

Example 54C and Example 104A were processed as described in Example 54D to provide the desired product.
mp 180-181 °C;
MS (ESI+) m/z 429 (M+H)⁺;
¹H NMR (DMSO-d₆) δ 9.50 (br s, 1H), 8.48 (d, 1H, J=2.4 Hz), 8.42-8.36 (m, 2H), 7.83-7.65 (m, 5H), 7.44 (dd, 1H, J=8.1, 2.8 Hz); 6.84 (br d, 1H, J =9.1 Hz), 5.83 (t, 1H, J =8.8 Hz), 0.98 (s, 9H);
HRMS (FAB) calcd m/z for C₁₉H₂₁N₆O (M⁺): (428.0960). Found: 428.0966.

### Example 106

### 4-chloro-N-(1-{[(cyanoimino)(3-pyridinylamino)methyl]amino}-2,2-dimethylpropyl)-2-fluorobenzamide

### Example 106A

### N-[1-(1H-1,2,3-benzotriazol-1-yl)-2,2-dimethylpropyl]-4-chloro-2-fluorobenzamide

4-Chloro-2-fluorobenzamide, pivaldehyde, benzotriazole and p-toluenesulfonic acid were processed as described in Example 53A to provide the desired product.
MS (DCI/NH₃) m/z 361 (M+H)⁺.

### Example 106B

### 4-chloro-N-(1-{[(cyanoimino)(3-pyridinylamino)methyl]amino}-2,2-dimethylpropyl)-2-fluorobenzamide

Example 54C and Example 106A were processed as described in Example 54D to provide the desired product.
mp 183-184°C;
MS (DCI/NH₃) m/z 403 (M+H)⁺;
¹H NMR (DMSO-d₆) δ 9.48 (s, 1H), 8.47 (d, 1H, J=2.4 Hz), 8.40 (d, 1H, J=8.4 Hz), 8.37 (dd, 1H, J=5.8, 1.6 Hz), 7.71-7.65 (m, 2H), 7.58 (dd, 1H, J=11.3, 1.8 Hz), 7.43-7.40 (m, 2H), 6.95 (d, 1H, J=8.7 Hz), 5.80 (t, 1H, J=8.6 Hz), 0.98 (s, 9H);
Anal. calcd for C₁₉H₂₀ClFN₆O: C, 56.65; H, 5.00; N, 20.86. Found: C, 56.58; H, 5.18; N, 20.86.

### Example 107

### N-(1-{[(cyanoiminol(3-pyridinylamino)methyl]amino]-2,2-dimethylpropyl)-4-fluorobenzamide

### Example 107A

### N-[1-(1H-1,2,3-benzotriazol-1-yl-2,2-dimethylpropyl]-4-fluorobenzamide

4-Fluorobenzamide, pivaldehyde, benzotriazole and p-toluenesulfonic acid were processed as described in Example 53A to provide the desired product.
MS (ESI+) m/z 327 (M+H)⁺.

### Example 107B

### N-(1-{[(cyanoimino)(3-pyridinylamino)methyl]amino}-2,2-dimethylpropyl)-4-fluorobenzamide

Example 54C and Example 107A were processed as described in Example 54D to provide the desired product.
mp 195-196°C;
MS (ESI+) m/z 369 (M+H)⁺;
¹H NMR (DMSO-d₆) δ 9.53 (s, 1H), 8.48 (d, 1H, J=2 Hz), 8.39 (dd, 1H, J=5,1 Hz), 8.34 (d, 1H, J=8 Hz), 7.90 (m, 2H), 7.69 (ddd, 1H, J= 8, 3, 1 Hz), 7.42 (dd, 1H, J=8, 5 Hz), 7.33 (m, 2H), 6.82 (d, 1H, J=9 Hz), 5.83 (t, 1H, J=9 Hz), 0.98 (s, 9H);
Anal. calcd for C₁₉H₂₁FN₆O: C, 61.94; H, 5.75; N, 22.81. Found: C, 61.75; H, 5.80; N, 22.78.

### Example 108

### N-(1-{[(cyanoimino)(3-pyridinylamino)methyl]amino}-2,2-dimethylpropyl)-3-methylbenzamide

### Example 108A

### N-[1-(1H-1,2,3-benzotriazol-1-yl)-2,2-dimethylpropyl]-3-methylbenzamide

meta-Toluamide, pivaldehyde, benzotriazole and p-toluenesulfonic acid were processed as described in Example 53A to provide the desired product.
MS (ESI+) m/z 323 (M+H)⁺.

### Example 108B

### N-(1-{[(cyanoimino)(3-pyridinylamino)methyl]amino-2,2-dimethylpropyl)-3-methylbenzamide

Example 54C and Example 108A were processed as described in Example 54D to provide the desired product.
MS (ESI+) m/z 365 (M+H)⁺;
¹H NMR (DMSO-d₆) δ 9.67 (s, 1H), 8.85 (br s, 1H), 8.58 (m, 1H), 8.44 (m, 1H), 7.83 (m, 1H), 7.68-7.61(m, 2H), 7.55 (m, 1H), 7.38 (m, 2H), 7.00 (d, 1H), 5.84 (t, 1H), 2.38 (s, 3H), 1.01 (s, 9H).

### Example 109

### N-(1-{[(cyanoimino)(3-pyridinylamino)methyl]amino}-2,2-dimethylpropyl)-2-methylbenzamide

### Example 109A

### N-[1 -(1 H-1,2,3-benzotriazol-1-yl)-2,2-dimethylpropyl]-2-methylbenzamide

ortho-Toluamide, pivaldehyde, benzotriazole and p-toluenesulfonic acid were processed as described in Example 53A to provide the desired product.
MS (ESI+) m/z 323 (M+H)⁺.

### Example 109B

### N-(1-{[(cyanoimino)(3-pyridinylamino)methyl]amino}-2,2-dimethylpropyl)-2-methylbenzamide

Example 54C and Example 109A were processed as described in Example 54D to provide the desired product.
MS (ESI+) m/z 365 (M+H)⁺;
¹H NMR (DMSO-d₆) δ 9.68 (s, 1H), 9.02 (br s, 1H), 8.57 (m, 1H), 8.41 (m, 1H), 7.82 (m, 1H), 7.53(m, 1H), 7.39-7.33 (m, 2H), 7.26 (m, 2H), 7.00 (d, 1H), 5.72 (t, 1H), 2.32 (s, 3H), 1.01 (s, 9H).

### Example 110

### N-(1-{[(cyanoimino)(3-pyridinylamino)methyl]amino}-2,2-dimethylpropyl)-3,5-difluorobenzamide

### Example 110A

### N-[1-(1H-1,2,3-benzotriazol-1-yl)-2,2-dimethylpropyl]-3,5-difluorobenzamide

3,5-Difluorobenzamide, pivaldehyde, benzotriazole and p-toluenesulfonic acid were processed as described in Example 53A to provide the desired product.
MS (ESI+) m/z 345 (M+H)⁺.

### Example 110B

### N-(1-{[(cyanoimino)(3-pyridinylamino)methyl]amino}-2,2-dimethylpropyl)-3,5-difluorobenzamide

Example 54C (161 mg, 1.00 mmol), Example 110A (344 mg, 1.00 mmol) and cesium carbonate (757 mg, 2.33 mmol) in anhydrous DMF (5 mL) were stirred at ambient temperature for 12 hours. The reaction mixture was acidified with 10% HCl and extracted with methylene chloride (3 x 20 mL). The organic extracts were combined, dried (sodium sulfate), filtered, and concentrated. The residue was purified by flash column chromatography (elution with 5% MeOH/methylene chloride) to provide the desired product (86 mg, 43%) as a white solid.
MS (ESI+) m/z 387 (M+H)⁺;
¹H NMR (DMSO-d₆) δ 9.50 (s, 1H), 8.45 (m, 3H), 7.54 (m, 5H), 6.82 (d, 1H), 5.82 (t, 1H), 0.98 (s, 9H);
Anal. calcd for C₁₉H₂₀F₂N₆O: C, 59.06; H, 5.22; N, 21.75. Found: C, 58.73; H, 5.40; N, 21.98.

### Example 111

### 4-chloro-N-{1-[[(cyanoimino)(3-pyridinylamino)methyl](methyl)amino1-2,2-dimethylpropyl}benzamide

### Example 111A

### N"-cyano-N-methyl-N'-(3-pyridinyl)guanidine

Example 54B and methylamine were processed as described as in Example 54C to give the desired product.
MS (ESI+) m/z 176 (M+H)⁺.

### Example 111B

### 4-chloro-N-{ 1-[[(cyanoimino)(3-pyridinylamino)methyl](methyl)amino]-2,2-dimethylpropyl}benzamide

Example 111A and Example 69B were processed as described in Example 54D to provide the desired product.
mp 127-129 °C;
MS (ESI+) m/z 399 (M+H)⁺;
¹H NMR (DMSO-d₆) δ 9.36 (s, 1H), 8.78 (m, 1H), 8.35 (d, 1H, J=2.0 Hz), 8.28 (d, 1H, J=3.7 Hz), 7.92 (d, 2H, J=8.1 Hz), 7.60 (d, 2H, J=8.1 Hz), 7.49 (m, 1H), 7.37 (dd, 1H, J=8.1, 4.4 Hz), 6.04 (m, 1H), 2.97 (s, 3H), 1.08 (s, 3H);
Anal. calcd for C₂₀H₂₃ClN₆O: C, 60.22; H, 5.81; N, 21.07. Found: C, 59.88; H, 6.09; N, 21.06.

### Example 112

### (-) 4-chloro-N-(2,2,2-trichloro-1-{[(cyanoimino)(3-pyridinylamino)methyl]amino}ethyl)benzamide

Example 45C was chromatographed over a Daicel Chiral Technologies Chiralcel AS chiral column (2.0 cmx25 cm) eluting with 5% ethanol/hexanes (flow rate=10 mL/minutes) to provide the desired product as the levorotatory enantiomer.
[α]_{D}²³ = -19.2° (c 0.04, DMSO);
mp 126-128 °C;
MS (ESI+) m/z 426 (M+H)⁺;
¹H NMR (DMSO-d₆) δ 10.12 (s, 1H), 8.72 (d, 1H, J=9 Hz), 8.50 (dd, 1H, J=12, 3 Hz), 7.70-7.67 (m, 1H), 7.51 (q, 1H, J=6 Hz), 7.45-7.38 (m, 1H), 7.30 (d, 1H, J=9 Hz), 6.82 (t, 1H, J=9 Hz), 2.38 (s, 3H);
Anal. calcd for C₁₇H₁₅Cl₃N₆O: C, 47.96; H, 3.55; N, 19.74. Found: C, 47.62; H, 3.25; N, 19.84.

### Example 113

### (+) 4-chloro-N-(2,2,2-trichloro-1-{[(cyanoimino)(3-pyridinylamino)methyl]amino}ethyl)benzamide

Example 45C was chromatographed over a Daicel Chiral Technologies Chiralcel AS chiral column (2.0 cmx25 cm) eluting with 5% ethanol/hexanes (flow rate=10 mL/minutes) to provide the desired product as the dextrorotatory enantiomer.
[α]_{D}²³ = +14.8° (c 0.03, DMSO);
mp 126-128 °C;
MS (ESI+) m/z 426 (M+H)⁺;
¹H NMR (DMSO-d₆) δ 10.12 (s, 1H), 8.72 (d, 1H, J=9 Hz), 8.50 (dd, 1H, J=12, 3 Hz), 7.70-7.67 (m, 1H), 7.51 (q, 1H, J=6 Hz), 7.45-7.38 (m, 1H), 7.30 (d, 1H, J=9 Hz), 6.82 (t, 1H, J=9 Hz), 2.38 (s, 3H);
Anal. calcd for C₁₇H₁₅Cl₃N₆O: C, 47.96; H, 3.55; N, 19.74. Found: C, 47.73; H, 3.31; N, 19.59.

### Example 114

### 4-iodo-N-(2,2,2-trichloro-1-{[(cyanoimino)(3-pyridinylamino)methyl]amino}ethyl)benzamide

### Example 114A

### 4-iodo-N-(2,2,2-trichloro-1-hydroxymethyl)benzamide

Example 103A was processed as described in Example 37C to provide the desired compound.
MS (ESI+) m/z 394 (M+H)⁺.

### Example 114B

### 4-iodo-N-(1,2,2,2-tetrachloroethyl)benzamide

Example 114A was processed as described in Example 37D to provide the desired compound.
MS (ESI+) m/z 412 (M+H)⁺.

### Example 114C

### 4-iodo-N-(2,2,2-trichloro-1-isothiocyanatoethyl)benzamide

Example 114B was processed as described in Example 45A to provide the desired compound.
MS (ESI+) m/z 435 (M+H)⁺.

### Example 114D

### 4-iodo-N-(2,2,2-trichloro-1-{[(3-pyridinylamino)carbothioyl]amino}ethyl)benzamide

Example 114C and 3-aminopyridine were processed as described in Example 45B to provide the desired compound.
MS (ESI+) m/z 545 (M+H)⁺.

### Example 114E

### 4-iodo-N-(2,2,2-trichloro-1-{[(cyanoimino)(3-pyridinylamino)methyl]amino}ethyl)benzamide

Example 114D was processed as described in Example 45C to provide the desired compound.
mp 208-210°C;
MS (DCI/NH₃) m/z 553 (M+NH₄)⁺;
¹H NMR (DMSO-d₆) δ 10.33 (br s, 1H), 9.25 (d, 1H, J=8.20 Hz), 8.62 (d, 1H, J=2.5 Hz), 8.38 (dd, 1H, J=5.4, 1.6 Hz), 8.18 (d, 1H, J=9.5 Hz), 8.04 (dt, 1H, J=8.65, 1.6 Hz), 7.92 (d, 2H, J=8.6 Hz), 7.62 (d, 2H, J=8.6 Hz), 7.48 (t, 1H, J=8.3 Hz), 7.44 (dd, 1H, J=8.8, 5.6 Hz);
Anal. calcd for C₁₆H₁₂Cl₃IN₆O: C, 35.75; H, 2.40; N, 15.47. Found: C, 35.55; H, 2.40; N, 15.47.

### Example 115

### 4-chloro-N-(2,2-dichloro-1-{[(cyanoimino)(3-pyridinylamino)methyl]amino}pentyl)benzamide

### Example 115A

### N-[1-(1H-1,2,3-benzotriazol-1-yl)-2,2-dichloropentyl]-4-chlorobenzamide

4-Chlorobenzamide, 2,2-dichloropentanal, benzotriazole, and p-toluenesulfonic acid were processed as described in Example 53A to provide the desired product.
MS (ESI-) m/z 409 (M-H)⁻.

### Example 115B

### 4-chloro- N-(2,2-dichloro-1- -{[(cyanoimino)(3-pyridinylamino)methyl]amino}pentyl)benzamide

Example 54C and Example 115A were processed as described in Example 54D to provide the desired product.
mp 192-193 °C;
MS (ESI+) m/z 453 (M+H)⁺;
¹H NMR (DMSO-d₆) δ 9.92 (s, 1H), 8.70 (d, 1H, J=8.1 Hz), 8.52 (d, 1H, J=2.0 Hz), 8.46 (d, 1H, J=4.1 Hz), 7.85 (d, 2H, J=8.5 Hz), 7.69 (m, 1H), 7.60 (d, 2H, J=8.5 Hz), 7.48 (dd, 1H, J=8.0, 4.4 Hz), 7.15 (d, 1H, J=8.8 Hz), 6.58 (t, 1H, J=8.6 Hz), 2.12 (m, 2H), 1.72 (m, 2H), 0.96 (t, 3H, J=7.1 Hz);
Anal. calcd for C₁₉H₁₉Cl₃N₆O: C, 50.29; H, 4.22; N, 18.52. Found: C, 50.54; H, 4.34; N, 18.70.

### Example 116

### 4-chloro-N-(2.2-dichloro-1-{[(cyanoimino)(3-pyridinylamino)methyl]amino}propyl)benzamide

### Example 116A

### 2,2-dichloropropionaldehyde

Chlorine gas was bubbled through dimethylformamide (14.7 g, 0.202 mmol) for 5 minutes. The solution was heated to 45-55 °C and propionaldehyde (11.7, 0.202 mmol) in dimethylformamide (29.5 g, 0.404 mmol) was added slowly, maintaining the reaction temperature at 45-55 °C (a cooling bath was necessary to control the temperature). During the addition, Cl₂ was bubbled through the reaction mixture to maintain a yellow color. After complete addition, the reaction mixture was heated at 45-55 °C for 30 minutes. The solution was cooled to 0 °C and diethyl ether (100 mL) was added followed by cold water (100 mL). The phases were separated and the organic phase was washed with aqueous sodium bicarbonate (20 mL), brine (20 mL), dried (sodium sulfate), and concentrated under reduced pressure to provide 21.1 g of crude 2,2-dichloropropionaldehyde as an oil.

### Example 116B

### N-[1-( 1H-1,2,3-benzotriazol-1-yl)-2,2-dichloropropyl]-4-chlorobenzamide

4-Chlorobenzamide, Example 116A, benzotriazole, and p-toluenesulfonic acid were processed as described in Example 53A to provide the desired product.
MS (ESI-) m/z 381 (M-H)⁻.

### Example 116C

### 4-chloro-N-(2,2-dichloro-1- {[(cyanoimino)(3-pyridinylamino)methyl]amino}propyl)benzamide

Example 54C and Example 116B were processed as described in Example 110B to provide the desired product.
mp 185-187 °C;
MS (ESI+) m/z 425 (M+H)⁺;
¹H NMR (DMSO-d₆) δ 9.95 (s, 1H), 8.75 (d, 1H, J=8.8 Hz), 8.52 (d, 1H, J=2.4 Hz), 8.46 (d, 1H, J=4.8 Hz), 7.85 (d, 2H, J=8.8 Hz), 7.69 (m, 1H), 7.60 (d, 2H, J=8.8 Hz), 7.47 (dd, 1H, J=8.3, 4.8 Hz), 7.16 (d, 1H, J=8:5 Hz), 6.55 (t, 1H, J=8.5 Hz), 2.17 (s, 3H);
Anal. calcd for C₁₇H₁₅Cl₃N₆O: C, 47.96; H, 3.55; N, 19.74. Found: C, 48.21; H, 3.75; N, 19.81.

### Example 117

### (-) 4-chloro-N-(2,2-dichIoro-1-{[(cyanoimino)(3-pyridinylamino)methyl]amino} propyl)benzamide

Example 116C was chromatographed over a Daicel Chiral Technologies Chiralcel AS chiral column (2.0 cmx25 cm) eluting with 5% ethanol/hexanes (flow rate=10 mL/minutes) to provide the desired product as the levorotatory enantiomer.
[α]_{D}²³ = -22° (c 0.19, DMSO);
mp 188-190°C;
MS (ESI+) m/z 425 (M+H)⁺;
¹H NMR (DMSO-d₆) δ 9.93 (s, 1H), 8.75 (d, 1H, J=8.1 Hz), 8.52 (d, 1H, J=2.0 Hz), 8.46 (d, 1H, J=4.4 Hz), 7.85 (d, 2H, J=8.5 Hz), 7.69 (m, 1H), 7.60 (d, 2H, J=8.5 Hz), 7.47 (dd, 1H, J=8.0, 4.4 Hz), 7.16 (d, 1H, J=8.5 Hz), 6.55 (t, 1H, J=8.5 Hz), 2.17 (s, 3H); Anal. calcd for C₁₇H₁₅C₁₃N₆O: C, 47.96; H, 3.55; N, 19.74. Found: C, 48.14; H, 3.64; N, 19.47.

### Example 118

### (+)4-chloro-N-(2,2-dichloro-1-{[(cyanoiminol(3-pyridinylamino)methyl]amino}propyl)benzamide

Example 116C was chromatographed over a Daicel Chiral Technologies Chiralcel AS chiral column (2.0 cmx25 cm) eluting with 5% ethanol/hexanes (flow rate=10 mL/minutes) to provide the desired product as the dextrorotatory enantiomer.
[α]_{D}²³ =+21° (c 0.19, DMSO);
mp 187-189 °C;
MS (ESI+) m/z 425 (M+H)⁺;
¹H NMR (DMSO-d₆) δ 9.93 (s, 1H), 8.75 (d, 1H, J=8.1 Hz), 8.52 (d, 1H, J=2.0 Hz), 8.46 (d, I H, J=4.4 Hz), 7.85 (d, 2H, J=8.5 Hz), 7.69 (m, 1H), 7.60 (d, 2H, J=8.5 Hz), 7.47 (dd, 1H, J=8.0, 4.4 Hz), 7.16 (d, 1H, J=8.5 Hz), 6.55 (t, 1H, J=8.5 Hz), 2.17 (s, 3H);
Anal. calcd for C₁₇H₁₅Cl₃N₆O: C, 47.96; H, 3.55; N, 19.74. Found: C, 48.12; H, 3.73; N, 19.34.

### Example 119

### 3-chloro-N-(2,2-dichloro-1-{[(cyanoimino)(3-pyridinylamino)methyl]amino}propyl)benzamide

### Example 119A

### N-[1-(1H-1,2,3-benzotriazol-1-yl)-2,2-dichloropropyl]-3-chlorobenzamide

3-Chlorobenzamide, Example 116A, benzotriazole, and p-toluenesulfonic acid were processed as described in Example 53A to provide the desired product.
MS (ESI-) m/z 381 (M-H)⁻.

### Example 119B

### 3-chloro-N-(2,2-dichloro-1-{[(cyanoimino)(3-pyridinylamino)methyl]amino}propyl)benzamide

Example 54C and Example 119A were processed as described in Example 110B to provide the desired product.
mp 142-143 °C;
MS (ESI+) m/z 425 (M+H)⁺;
¹H NMR (DMSO-d₆) δ 9.93 (s, 1H), 8.81 (d, 1H, J=8.5 Hz), 8.52 (d, 1H, J=2.4 Hz), 8.46 (d, 1H, J=4.4 Hz), 7.85 (m, 1H), 7.70 (m, 2H), 7.56 (t, 1H, J=8.0 Hz), 7.49 9(dd, I H, J=8.2, 4.8 Hz), 7.14 (d, 1H, J=8.5 Hz), 6.55 (t, 1H, J=8.8 Hz), 2.18 (s, 3H);
Anal. calcd for C₁₇H₁₅Cl₃N₆O: C, 47.96; H, 3.55; N, 19.74. Found: C, 47.82; H, 3.62; N, 19.76.

### Example 120

### N-(2,2-dichloro-1-{[(cyanoimino)(3pyridinylamino)methyl]amino}propyl)-3,5-difluorobenzamide

### Example 120A

### N-[1-(1H-1,2,3-benzotriazol-1-yl)-2,2-dichloropropyl]-3-chlorobenzamide

3,5-Difluorobenzamide, Example 116A, benzotriazole, and p-toluenesulfonic acid were processed as described in Example 53A to provide the desired product.
MS (ESI+) m/z 385 (M+H)⁺.

### Example 120B

### N-(2,2-dichloro-1-{[(cyanoimino)(3-pyridinylamino)methyl]amino}propyl)-3,5-difluorobenzamide

Example 54C and Example 120A were processed as described in Example 110B to provide the desired product.
mp 191-193 °C;
MS (ESI+) m/z 427 (M+H)⁺;
¹H NMR (DMSO-d₆) δ 9.93 (s, 1H), 8.87 (d, 1H, J=8.1 Hz), 8.52 (d, 1H, J=2.0 Hz), 8.46 (dd, 1H, J=4.8, 1.0 Hz), 7.70 (m, 1H), 7.52 (m, 3H), 7.48 (dd, 1H, J=8.1, 4.7 Hz), 7.12 (d, 1H, J=8.5 Hz), 6.53 (t, 1H, J=8.6 Hz), 2.18 (s, 3H);
Anal. calcd for C₁₇H₁₄Cl₂F₂N₆O: C, 47.79; H, 3.30; N, 19.67. Found: C, 47.85; H, 3.38; N, 19.55.

### Example 121

### 4-chloro-N-(1-{[cyanoimino)(3-pyridinylamino)methyl]amino}-2,2,3,3,3-pentafluoropropyl)benzamide

### Example 121A

### N-[1-(1H-1,2,3-benzotriazol-1-yl-2,2,3,3,3-pentafluoropropyl]-4-chlorobenzamide

4-Chlorobenzamide, pentafluoropropanal, benzotriazole, and p-toluenesulfonic acid were processed as described in Example 53A to provide the desired product.
MS (ESI-) m/z 403 (M-H)⁻.

### Example 121B

### 4-chloro-N-(1-{[(cyanoimino)(3-pyridinylamino)methyl]amino)-2,2,3,3,3-pentafluoropropyl)benzamide

Example 54C and Example 121 A were processed as described in Example 110B to provide the desired product.
mp 177-178°C;
MS (ESI+) m/z 447 (M+H)⁺;
¹H NMR (DMSO-d₆) δ 10.05 (s, 1H), 9.04 (d, 1H, J=8.8 Hz), 8.46 (m, 2H), 7.83 (d, 2H, J=8.5 Hz), 7.66 (m, 1H), 7.64 (d, 2H, J=8.5 Hz), 7.48 (dd, 1H, J=8.0, 4.6 Hz), 7.41 (m, 1H), 6.86 (m, 1H);
Anal. calcd for C₁₇H₁₂ClF₅N₆O: C, 45.70; H, 2.71; N, 18.81. Found: C, 45.79; H, 2.50; N, 19.05.

### Example 122

### 3-chloro-N-(1-{[(cyanoimino)(3-pyridinylamino)methyl]amino}-2,2,3,3,3-pentafluoropropyl)benzamide

### Example 122A

### N-[1-(1H-1,2,3-benzotriazol-1-yl)-2,2,3,3,3-pentafluoropropyl]-3-chlorobenzamide

3-Chlorobenzamide, pentafluoropropanal, benzotriazole, and p-toluenesulfonic acid were processed as described in Example 53A to provide the desired product.
MS (ESI-) m/z 403 (M-H)⁻.

### Example 122B

### 3-chloro-N-(1-( {[(cyanoimino)(3-pyridinylamino)methyl]amino} -2,2,3,3,3-pentafluoropropyl)benzamide

Example 54C and Example 122A were processed as described in Example 110B to provide the desired product.
mp 183-185 °C;
MS (ESI+) m/z 447 (M+H)⁺;
¹H NMR (DMSO-d₆) δ 10.05 (br s, 1H), 9.13 (d, 1H, J=8.8 Hz), 8.47 (m, 2H), 7.84 (m, 1H), 7.78 (m, 1H), 7.70 (m, 1H), 7.65 (m, 1H), 7.58 (t, 1H, J=8.1 Hz), 7.48 (m, 2H), 6.86 (m, 1H);
Anal. calcd for C₁₇H₁₂ClF₅N₆O: C, 45.70; H, 2.71; N, 18.81. Found: C, 45.77; H, 2.83; N, 18.90.

### Example 123

### 4-chloro-N-(1-{[(nitroimino)(3-pyridinylamino)methyl]amino}-2,2-dimethylpropyl)benzamide

### Example 123A

### N-(3-pyridinyl)guanidine dihydrochloride

3-Aminopyridine (38.96 g, 413.96 mmol) and cyanamide (21.40 g, 509.2 mmol) in 12N hydrochloric acid (271 mL) were stirred at 140-150 °C for 4 hours. Concentration under reduced pressure provided a heavy syrup which was purified by recrystalization from 1:1 isopropanol:diethyl ether (500 mL) to provide 60.2 g of the desired product as a white solid.
MS (ESI+) m/z 137 (M+H)⁺.

### Example 123B

### N"-nitro-N-(3-pyridinyl)guanidinium nitrate

Example 123A (20.0 g, 146.0 mmol) was added in small portions to concentrated sulfuric acid (122.8 ml, 2.19 mol) at -10°C. Concentrated nitric acid was added dropwise at 0 °C for a period of 10 minutes. The mixture was stirred at 0 °C for 1 hour and then dripped slowly into 700 g of crushed ice. The resulting precipitate was collected by filtration providing 17.5 g of the desired product as a white solid.
MS (ESI+) m/z 182 (M+H)⁺.

### Example 123C

### 4-chloro-N-(1-{[(nitroimino)(3-pyridinylamino)methyl]amino}-2,2-dimethylpropyl)benzamide

Example 123B and Example 69B were processed as described in Example 110B to provide the desired product.
mp 203-204°C;
MS (ESI+) m/z 405 (M+H)⁺;
¹H NMR (DMSO-d₆) δ 9.81 (s, 9H), 9.50-9.10 (br s, 1H), 8.90 (d, 1H, J=8 Hz), 8.52 (d, 1H, J= 2 Hz), 8.40 (dd, 1H, J=5, 2 Hz), 7.91-7.88 (m, 2H), 7.75 (ddd, 1H J=8, 3, 2 Hz), 7.59 (d, 2H, J=9 Hz), 7.52 (d, 1H, J=8 Hz), 7.43 (dd, 1H, J= 8, 5 Hz), 5.90 (t, 1H, J=9 Hz), 1.08 (s, 9H);
Anal. calcd for C₁₈H₂₁ClN₆O₃: C, 53:40; H, 5.23; N, 20.76. Found: C, 53.41; H, 5.30; N, 20.76.

### Example 124

### 4-chloro-N-(1-{[(nitroimino)(3-pyridinylamino)methyl]amino}-3,3-dimethylbutyl)benzamide

Example 62A and Example 123B were processed as described in Example 110B to provide the desired product.
mp 195-197 °C;
MS (ESI+) m/z 419 (M+H)⁺;
¹H NMR (DMSO-d₆) δ 9.30 (br s, 1H), 8.51 (d, 1H), 8.39 (d, 1H), 7.94 (d, 2H), 7.73 (d, 2H), 7.62 (d, 2H), 7.42 (dd, 1H), 5.83-5.78 (m, 1H), 2.01 (dd, 1H), 1.87 (dd, 1H), 0.98 (s, 9H);
Anal. calcd for C₁₉H₂₃ClN₆O₃: C, 54.48; H, 5.53; N, 20.06. Found: C, 54.42; H, 5.54; N, 20.27.

### Example 125

### (+) 4-chloro-N-(1-{[(nitroimino)(3-pyridinylamino)methyl]amino}-3,3-dimethylbutyl)benzamide

Example 124 was chromatographed over a Daicel Chiral Technologies Chiralcel OJ chiral column (2.0 cmx25 cm) eluting with 5% ethanol/hexanes (flow rate=10 mL/minutes) to provide the desired product as the dextrorotatory enantiomer.
[α]D²³ = +64.2° (c 0.308, DMSO);
mp 184-185 °C;
MS (ESI+) m/z 419 (M+H)⁺;
¹H NMR (DMSO-d₆) δ 9.30 (br s, 1H), 8.52 (d, 1H, J=2.0 Hz), 8.38 (d, 1H, J=4.8 Hz), 7.93 (d, 2H, J=8.5 Hz), 7.75 (m, 1H), 7.62 (d, 2H, J=8.5 Hz), 7.40 (dd, 1H, J=8.0, 5.1 Hz), 5.80 (m, 1H), 2.03 (dd, 1H, J=14.1, 7.1 Hz), 1.86 (dd, 1H, J=14.1, 5.4 Hz), 0.98 (s, 3H);
Anal. calcd for C₁₉H₂₃ClN₆O₃: C, 54.48; H, 5.53; N, 20.06. Found: C, 54.69; H, 5.41; N, 20.05.

### Example 126

### (-) 4-chloro-N-(1-{[(nitroimino)(3-pyridinylamino)methyl]amino}-3,3-dimethylbutyl)benzamide

Example 124 was chromatographed over a Daicel Chiral Technologies Chiralcel OJ chiral column (2.0 cmx25 cm) eluting with 5% ethanol/hexanes (flow rate=10 mL/minutes) to provide the desired product as the levorotatory enantiomer.
[α]_{D}²³ = -58.9° (c, 0.292, DMSO);
mp 185-186 °C;
MS (ESI+) m/z 419 (M+H)⁺;
¹H NMR (DMSO-d₆) δ 9.30 (br s, 1H), 8.52 (d, 1H, J=2.0 Hz), 8.38 (d, 1H, J=4.8 Hz), 7.93 (d, 2H, J=8.5 Hz), 7.75 (m, 1H), 7.62 (d, 2H, J=8.5 Hz), 7.40 (dd, 1H, J=8.0, 5.1 Hz), 5.80 (m, 1H), 2.03 (dd, 1H, J=14.1, 7.1 Hz), 1.86 (dd, 1H, J=14.1, 5.4 Hz), 0.98 (s, 3H);
Anal. calcd for C₁₉H₂₃ClN₆O₃: C, 54.48; H, 5.53; N, 20.06. Found: C, 54.63; H, 5.53; N, 20.18.

### Example 127

### 4-chloro-N-(1-{[(nitroimino)(3-pyridinylamino)methyl]amino}-2,2-dimethyl-4-pentenyl)benzamide

Example 123B and Example 67A were processed as described in Example 110B to provide the desired product.
mp 200-203 °C;
MS (ESI+) m/z 431 (M+H)⁺;
¹H NMR (DMSO-d₆) δ 9.88 (br s, 1H), 8.95 (br s, 1H), 8.51 (d, 1H, J=2.5 Hz), 8.40 (dd, 1H, J=8.6, 4.7 Hz), 7.90 (d, 2H, J=8.6 Hz), 7.74 (d, 1H, J=4.7 Hz), 7.60 (d, 2H, J=8.6 Hz), 7.43 (dd, 1H, J=4.8, 2.8 Hz), 5.96-5.82 (m, 2H), 5.10 (s, 1H), 5.11-5.08 (dd, J=8.7, 7.6 Hz, 1H), 2.25-2.10 (m, 2H), 1.06 (s, 3H), 1.04 (s, 3H);
Anal. calcd for C₂₀H₂₃ClN₆O₃: C, 55.75; H, 5.38; N, 19.50. Found: C, 55.84; H, 5.37; N, 19.48.

### Example 128

### 4-chloro-N-(1-{[(nitroimino)(3-pyridinylamino)methyl]amino}-2,2-dimethyl-3-phenylpropyl)benzamide

Example 123B and Example 102A were processed as described in Example 110B to provide the desired product.
mp 205-207 °C;
MS (ESI+) m/z 481 (M+H)⁺;
¹H NMR (DMSO-d₆) δ 9.89 (s, 1H); 8.99 (br s, 1H), 8.520 (s, 1H), 8.41 (d, 1H), 7.92 (d, 2H), 7.75 (d, 1H), 7.60 (d, 2H), 7.44 (dd, 2H), 7.30-7.19 (m, 5H), 5.90 (t, 1H), 2.78 (d, 1H), 2.70 (d, 1H), 1.00 (s, 3H), 0.97 (s, 3H);
Anal. calcd for C₂₄H₂₅ClN₆O3: C, 59.94; H, 5.24; N, 17.47. Found: C, 60.19; H, 5.24; N, 17.58.

### Example 129

### 4-chloro-N-[1-{[(nitroimino)(3-pyridinylamino)methyl]amino}-2-(2,6,6-trimethyl-1-cyclohexen-1-yl)ethyl]benzamide

Example 123B and Example 66A were processed as described in Example 110B to provide the desired product.
mp 217-218 °C;
MS (ESI+) m/z **485** (M+H)⁺;
¹H NMR (DMSO-d₆) δ 8.50 (d, 1H), 8.41 (d, 1H), 7.91 (d, 2H), 7.73 (d, 1H), 7.63 (d, 2H), 7.44 (dd, 1H), 1.96-1.88 (m, 2H), 1.69 (s, 3H), 1.58-1.48 (m, 2H), 1.57-1.47 (m, 2H), 1.04 (s, 3H), 1.02 (s, 3H);
Anal. calcd for C₂₄H₂₉ClN₆O₃: C, 59.44; H, 6.03; N, 17.33. Found: C, 59.55; H, 6.07; N, 17.48.

### Example 130

### 4-chloro-N-(1-{ [(nitroimino)(3-pyridinylamino)methyl]amino }-2-cyclohexyl-2-methylpropyl)benzamide

Example 123B and Example 93A were processed as described in Example 110B to provide the desired product.
mp 201-203 °C;
MS (ESI+) m/z 473 (M+H)⁺;
¹H NMR (DMSO-d₆) δ 9.85 (br s, 1H), 8.89 (br s, 1H), 8.50 (d, 1H), 8.40 (dd, 1H), 7.88 (d, 2H), 7.73 (d, 1H), 7.59 (d, 2H), 7.43 (dd, 1H), 5.93 (t, 1H), 1.83-1.03 (m, 11H), 0.99 (s, 3H), 0.98 (s, 3H);
Anal. calcd for C₂₃H₂₉ClN₆O₃: C, 58.41; H, 6.18; N, 17.77. Found: C, 58.19; H, 6.07; N, 17.71.

### Example 131

### N-(2,2-bis[(allyloxy)methyl]-1-{[(nitroimino)(3-pyridinylamino)methyl]amino}butyl)-4-chlorobenzamide

Example 123B and Example 96A were processed as described in Example 110B to provide the desired product.
mp 175-180°C;
MS (ESI+) m/z 531 (M+H)⁺;
¹H NMR (DMSO-d₆) δ 9.87 (br s, 1H), 9.31 (br s, 1H), 8.56 (d, 1H), 8.40 (d, 1H), 7.89 (d, 2H), 7.80 (d, 1H), 7.61 (d, 2H), 7.44 (dd, 2H), 5.94-5.78 (m, 3H), 5.26 (dd, 1H), 5.20 (dd, 1H), 5.15 (dd, 1H), 5.12 (dd, 1H), 4.04-3.86 (m, 4H), 3.70 (d, 1H), 3.59 (d, 1H), 3.48 (d, 1H), 3.39 (d, 1H), 1.49 (q, 2H), 0.88 (t, 3H);
Anal. calcd for C₂₅H₃₁ClN₆O₅: C, 56.55; H, 5.88; N, 15.83. Found: C, 56.42; H, 5.94; N, 15.55.

### Example 132

### 4-chloro-N-(4-cyano-1-{[(nitroimino)(3-pyridinylamino)methyl]amino}-2,2-diethylbutyl)benzamide

Example 123B and Example 65A were processed as described in Example 110B to provide the desired product.
mp 100-110 °C;
MS (ESI+) m/z 472 (M+H)⁺;
¹H NMR (DMSO-d₆) δ 9.81 (br s, 1H), 8.86 (br s, 1H), 8.49 (d, 1H), 7.87 (d, 2H), 7.71 (d, 1H), 7.60 (d, 2H), 7.44 (dd, 1H), 5.92 (t, 1H), 2.61-2.54 (m, 2H), 1.82 (t, 2H), 1.51 (q, 4H), 0.89 (t, 3H);
Anal. calcd for C₂₂H₂₆ClN₇O₃: C, 55.89; H, 5.80; N, 19.33. Found: C, 55.50; H, 5.61; N, 19.55.

### Example 133

### 4-chloro-N-( 1-{[(nitroimino)(3-pyridinylamino)methyl]amino} -3,3-dimethyl-4-pentenyl)benzamide

Example 123B and Example 92B were processed as described in Example 110B to provide the desired product.
mp 186-186°C;
MS (DCl/NH₃) m/z 431 (M+H)⁺;
¹H NMR (DMSO-d₆) δ 10.12 (s, 1H), 9.30 (s, 1H), 8.51 (d, 1H, J=3.3 Hz), 8.39 (dd, 1 H, J=5.6, 1.5 Hz), 7.93 (d, 2H, J=8.2 Hz), 7.73 (d, 1H, J=8.3 Hz), 7.61 (d, 2H, J=8.3 Hz), 7.42 (dd, 1H, J=18.1,11.7 Hz), 5.68 (m, 1H), 4.91 (d, 1H, J=18.2 Hz), 4.87 (dd, 1H, J=11.6, 1.5 Hz), 2.07 (AB of ABX, 2H, J=46.4 Hz, 14.0, 7.0 Hz), 1.07 (s, 9H);
Anal. calcd for C₂₀H₂₃CIN₆O₃: C, 55.75; H, 5.38; N, 19.50. Found: C, 55.71; H, 5.42; N, 19.70.

### Example 134

### N-(2-(1-adamantyl)-1-{[(nitroimino)(3-pyridinylamino)methyl]amino}ethyl)-4-chlorobenzamide

Example 123B and Example 95A were processed as described in Example 110B to provide the desired product.
mp 219-220 °C;
MS (ESI+) m/z 497 (M+H)⁺;
¹H NMR (DMSO-d₆) δ 10.09 (br s, 1H), 9.83 (br s, 1H), 8.39 (d, 1H), 8.52 (d, 1H), 7.93 (d, 2H), 7.74 (d, 1H), 7.62 (d, 2H), 7.42 (dd, 1H), 5.82 (t, 1H), 1.92-1.58 (m, 17H);
Anal. calcd for C₂₅H₂₉ClN₆O₃: C, 60.42; H, 5.88; N, 16.91. Found: C, 60.44; H, 5.80; N, 16.72.

### Example 135

### N-(1-{[(nitroimino)(3-pridinylamino)methyl]amino}-2 2-dimethylpropyl)-4-phenylbenzamide

### Example 135A

### N-[1-(1 H-1,2,3-benzotriazol-1-yl)-2,2-dimethylpropyl]-4-phenylbenzamide

4-Phenylbenzamide, pivaldehyde, benzotriazole, and p-toluenesulfonic acid were processed as described in Example 53A to provide the desired product.
MS (ESI+) m/z 385 (M+H)⁺.

### Example 135B

### N-(1-{[(nitroimino)(3-pyridinylamino)methyl]amino}-2,2-dimethypropyl)-4-phenylbenzamide

Example 123B and Example 135A were processed as described in Example 110B to provide the desired product.
MS (ESI+) m/z 447 (M+H)⁺;
¹H NMR (DMSO-d₆) δ 9.89 (br, s, 1H), 8.92 (br s, 1H), 8.58 (d, 1H), 8.43 (m, 1H), 7.99 (d, 2H), 7.83(m, 3H), 7.73 (d, 2H), 7.51 (m, 4H), 6.98 (m, 1H), 5.92 (t, 1H), 1.06 (s, 9H).

### Example 136

### 4-chloro-N-(2,2-dichloro-1- {[(nitroimino)(3-pyridinylamino)methyl]amino}pentyl)benzamide

Example 123B and Example 115A were processed as described in Example 110B to provide the desired product.
mp 198-200 °C;
MS (ESI+) m/z 473 (M+H)⁺;
¹H NMR (DMSO-d₆) δ 9.16 (br s, 1H), 8.52 (d, 1H, J=1.4 Hz), 8.47 (d, 1H, J=3.4 Hz), 7.87 (d, 2H, J=8.5 Hz), 7.73 (m, 1H), 7.60 (d, 2H, J=8.5 Hz), 7.48 (dd, 1H, J=8.0, 3.7 Hz), 6.69 (t, 1H, J=8.8 Hz), 2.24 (m, 2H), 1.76 (m, 2H), 0.96 (t, 3H, J=7.1 Hz);
Anal. calcd for C₁₈H₁₉Cl₃N₆O₃: C, 45.64; H, 4.04; N, 17.74. Found: C, 45.85; H, 4.07; N, 17.91.

### Example 137

### 4-chloro-N-(2,2-dichloro-1- {[(nitroimino)(3-pyridinylamino)methyl]amino} propyl)benzamide

Example 123B and Example 116B were processed as described in Example 110B to provide the desired product.
mp 170-172 °C;
MS (ESI+) m/z 445 (M+H)⁺;
¹H NMR (DMSO-d₆) δ 10.30 (m,1H), 9.10 (br s, 1H), 8.53 (d, 1H, J=2.0 Hz), 8.48 (m, 1H), 7.89 (d, 2H, J=8.8 Hz), 7.74 (m, 1H), 7.61 (d, 2H, J=8.8 Hz), 7.47 (m, 1H), 6.68 (t, 1H, J=8.5 Hz), 2.21 (s, 3H);
Anal. calcd for C₁₆H₁₅Cl₃N₆O₃ 0.2C₄H₈O₂: C, 43.55; H, 3.61; N, 18.14. Found: C, 43.98; H, 3.50; N, 18.53.

### Example 138

### 3-chloro-N-(2,2-dichloro-1-{[(nitroimino)(3-pyridinylamino)methyl]amino}propyl)benzamide

Example 123B and Example 119A were processed as described in Example 110B to provide the desired product.
mp 160-161 °C;
MS (ESI+) m/z 445 (M+H)⁺;
¹H NMR (DMSO-d₆) δ 9.18 (m, 1H), 8.53 (d, 1H, J=1.0 Hz), 8.48 (d, 1H, J=4.1 Hz), 7.89 (s, 1H), 7.83 (d, 1H, J=7.5 Hz), 7.68 (m, 2H), 7.57 (dd, 1H, J=8.1, 7.8 Hz), 7.48 (dd, 1H, J=8.1, 4.8 Hz), 6.68 (t, 1H, J=8.8 Hz), 2.23 (s, 3H);
Anal. calcd for C₁₆H₁₅ClN₆O₃: C, 43.12; H, 3.39; N, 18.86. Found: C, 43.34; H, 3.38; N, 18.99.

### Example 139

### 4-chloro-N-(2,2-dimethyl-1-{[[(phenylsulfonyl)imino](3-pyridinylamino)methyl]amino}propyl)benzamide

### Example 139A

### N"-phenylsulfonyl-N-(3-pyridinyl)guanidine

Phenylsulfonamide (1.49 g, 10 mmol) in anhydrous DMF (20 mL) at ambient temperature was treated with sodium hydride (400 mg of 60% reagent, 10.0 mmol). After 30 minutes, the reaction mixture was treated with 3-pyridyl isothiocyanate (1.36 g, 10.0 mmol), stirred for an additional 30 minutes, then treated with methyl iodide (1.42 g, 10.0 mmol). The resulting suspension was stirred for 1.5 hours and then water was added until a clear solution was formed. The solution was extracted with methylene chloride (3 x 50 mL) and the extracts were combined, dried (sodium sulfate), filtered, and concentrated to provide a yellow oil that was used without further purification.

The crude material obtained above was dissolved in methanol (50 mL) and treated with ammonia (150 mL of a 2.0 M solution in methanol). The reaction mixture was then heated at 80 °C in a sealed high-pressure flask for 24 hours. The solvent was removed and the residue was purified by flash chromatography (elution with 5% MeOH/methylene chloride) to provide the desired product (2.10 g) as a white solid.
MS (ESI+) m/z 277 (M+H)⁺.

### Example 139B

### 4-chloro-N-(2,2-dimethyl-1-{[[(phenylsulfonyl)imino](3-pyridinylamino)methyl]amino}propyl)benzamide

Example 139A and Example 69B were processed as described in Example 54D to provide the desired product.
mp 196-197 °C;
MS (DCI/NH₃) m/z 500 (M⁺);
¹H NMR (DMSO-d₆) δ 9.44 (br s, 1H), 9.03 (br s, 1H), 8.49 (d, 1H, J=2.5 Hz), 8.34 (dd, 1H, J=4.8, 1.1 Hz), 7.92 (s, 1H), 7.90 (d, 2H, J = 8.4Hz), 7.80 (d, 2H, J=7.7Hz), 7.75-7.70 (m, 1H), 7.61-7.49 (m, 5H), 7.39 (dd, 1H, J=8.4, 4.8Hz), 5.67 (t, 1H, J = 8.8Hz), 1.02 (s, 9H);
Anal. calcd for C₂₄H₂₆CIN₅O₃S: C, 57.65, H, 5.24, N, 14.01. Found: C, 57.34, H, 5.38, N, 14.22.

### Example 140

### 4-chloro-N-(3,3-dimethyl-1-1-{[[(phenylsulfonyl)imino](3-pyridinylamino)methyl]amino}butyl)benzamide

Example 139A and Example 62A were processed as described in Example 54D to provide the desired product.
mp 199-200°C;
MS (ESI+) m/z 514 (M+H)⁺;
¹H NMR (DMSO-d₆) δ 9.68-9.51 (br s, 1H), 9.49-9.20 (br s, 1H), 8.49 (s, 1H), 8.33 (dd, 1H, J=5, 1Hz), 7.92 (d, 2H, J=8 Hz), 7.81 (d, 2H, J=8 Hz), 7.80 (s, 1H overlapped), 7.71 (d, 1H, J=8 Hz), 7.63-7.43 (m, 5H), 7.37 (ddd, 1H, J=8, 5, 1Hz), 5.72 (m, 1H), 1.99 (s, 1H), 1.70 (dd, 1H, J=14, 5 Hz), 0.92 (s, 9H);
Anal. calcd for C₂₅H₂₈ClN₅O₃S: C, 58.41; H, 5.49; N, 13.62. Found: C, 58.31; H, 5.56; N, 13.65.

### Example 141

### 4-chloro-N-{2,2-dimethyl-1-[((3-pyridinylamino){[(trifluoromethyl)sulfonyl]imino}methyl)amino]propyl}benzamide

### Example 141 A

### N-(3-pyridinyl)-N"-[(trifluoromethyl)sulfonyl]guanidine

Trifluoromethanesulfonamide was processed as described in Example 139A to provide the desired product.
MS (APCI) m/z 269 (M+H)⁺.

### Example 141B

### 4-chloro-N-{2,2-dimethyl-1-1[(3-pyridinylamino){[(trifluoromethyl)sulfonyl]imino}methyl)amino]propyl}benzamide

Example 141 A and Example 69B were processed as described in Example 110B to provide the desired product.
MS (ESI+) m/z 492 (M+H)⁺;
¹H NMR (DMSO-d₆) δ 10.23 (br s, 1H), 8.90 (br s, 1H), 8.54 (m, 2H), 7.87 (m, 4H), 7.60 (m, 3H), 5.88 (t, 1H), 1.06 (s, 9H);
Anal. calcd for C₁₉H₂₁ClF₃N₅O₃S: C, 46.39; H, 4.30; N, 14.24. Found: C, 46.29; H, 4.56; N, 14.27.

### Example 142

### 4-chloro-N-{3,3-dimethyl-1-[((3-pyridinylamino){[(trifluoromethyl)sulfonyl]imino}methyl)amino]butyl}benzamide

Example 141A and Example 62A were processed as described in Example 110B to provide the desired product.
MS (ESI+) m/z 506 (M+H)⁺;
¹H NMR (DMSO-d₆) δ 10.19 (br s, 1H), 9.21 (br s, 1H), 8.49 (m, 2H), 7.92 (m, 3H), 7.71 (d, 1H), 7.61 (d, 2H), 7.44 (m, 1H), 5.83 (m, 1H), 1.88 (m, 2H), 0.97 (s, 9H);
Anal. calcd for C₂₀H₂₃ClF₃N₅O₃S: C, 47.48; H, 4.58; N, 13.84. Found: C, 47.44; H, 4.80; N, 13.56.

### Example 143

### N-(1-{[[(aminosulfonyl)imino](3-pyridinylamino)methyl]amino}-2,2-dimethylpropyl)-4-chlorobenzamide

### Example 143A

### N"-(aminosulfonyl)-N-(3-pyridinyl)guanidine

Sulfamide was processed as described in Example 139A to provide the desired product.
MS (APCI+) m/z 216 (M+H)⁺.

### Example 143B

### N-(1-{[[(aminosulfonyl)imino](3-pyridinylamino)methyl]amino}-2,2-dimethylpropyl)-4-chlorobenzamide

Example 143A and Example 69B were processed as described in Example 110B to provide the desired product.
MS (ESI+) m/z 439 (M+H)⁺.
¹H NMR (DMSO-d₆) δ 9.25 (s, 1H), 8.99 (s, 1H), 8.67 (s, 1H), 8.29 (d, 1H), 7.90 (d, 3H), 7.77 (br s, 1H), 7.59 (d, 1H), 7.35 (m, 1H), 6.57 (s, 2H), 5.67 (t, 1H), 1.09 (s, 9H).

### Example 144

### N-(1-{[[(aminosulfonyl)imino](3-pyridinylamino)methyl]amino}-3,3-dimethylbutyl)-4-chlorobenzamide

Example 143A and Example 62A were processed as described in Example 110B to provide the desired product.
MS (ESI+) m/z 453 (M+H)⁺;
¹H NMR (DMSO-d₆) δ 9.48 (br s, 2H), 8.68 (s, 1H), 8.28 (m, 1H), 7.94 (m, 3H), 7.64 (d, 3H), 7.36 (m, 1H), 6.57 (s, 2H), 5.70 (m 1H), 2.03 (m, 1H), 1.81 (m, 1H), 0.98 (s, 9H).

### Example 145

### 4-chloro-N-(1-{[{[(dimethylamino)sulfonyl]imino}(3-pyridinylamino)methyl]amino}-2,2-dimethylpropyl)benzamide

### Example 145A

### N,N-dimethylsulfamide

Dimethylsulfamoyl chloride (3.70 g) was treated with ammonia (200 mL of a 2 M in solution in methanol) in a sealed high-pressure flask and heated at 60 °C for 12 h. Solvent was removed by rotary evaporation to furnish a white solid. This material was washed with methylene chloride and dried at 50°C under reduced pressure to provide the desired product (3.10 g) as a white solid.
MS (APCI+) m/z 124 (M+H)⁺.

### Example 145B

### N"-(dimethylaminosulfonyl)-N-(3-pyridinyl)guanidine

Example 145A was processed as described in Example 139A to provide the desired product.
MS (ESI+) m/z 244 (M+H)⁺.

### Example 145C

### 4-chloro-N-(1-{[{[(dimethylamino)sulfonyl]imino}(3-pyridinylamino)methyl]amino}-2,2-dimethylpropyl)benzamide

Example 145B and Example 69B were processed as described in Example 110B to provide the desired product.
MS (ESI+) m/z 467 (M+H)⁺;
¹H NMR (DMSO-d₆) δ 9.50 (br s, 1H), 9.05 (br s, 1H), 8.75 (d, 1H), 8.41 (dd, 1H), 8.02 (dd, 1H), 7.91 (d, 2H), 7.73 (br s, 1H), 7.59 (m 3H), 5.69 (t, 1H), 2.55 (s, 6H), 1.08 (s, 9H).

### Example 146

### 4-chloro-N-(1-{[{[(dimethylamino)sulfonyl]imino}(3-pyridinylamino)methyl]amino}-3,3-dimethylbutyl)benzamide

Example 145A and Example 62A were processed as described in Example 110B to provide the desired product.
MS (ESI+) m/z 481 (M+H)⁺;
¹HNMR (DMSO-d₆) δ 9.69 (br s, 1H), 9.39 (br s, 1H), 8.73 (s, 1H), 8.41 (d, 1H), 8.02 (d, 1H), 7.93 (d, 2H), 7,56 (m, 4H), 5.78 (m, 1H), 2.59 (s, 6H), 1.99 (dd, 1H), 1.79 (dd, 1H), 0.98 (s, 9H).

### Example 147

### 4-chloro-N-(1-{[(2-fluoroanilino)carbonyl]amino}-2,2-dimethylpropyl)benzamide

3-Pyridyl isocyanate and Example 69C were processed as described in Example 42 to provide the desired product.
mp 229-230 °C;
MS (ESI+) m/z 378 (M+H)⁺;
¹H NMR (DMSO-d₆) δ 8.63 (d, 1H, J=2.0 Hz), 8.52 (d, 1H, J=8.5 Hz), 8.12 (dt, 1H, J=8.1, 1.7 Hz), 7.87 (d, 2H, J=8.8 Hz), 7.53 (d, 2H, J=8.8 Hz), 7.17 (m, 1H), 7.06 (m, 2H), 6.91 (m, 1H), 5.62 (t, 1H, J=8.5 Hz), 0.96 (s, 9H);
Anal. Calcd for C₁₉H₂₁ClFN₃O₂: C, 60.40; H, 5.60; N, 11.12. Found: C, 60.36; H, 5.62; N, 11.08.

### Example 148

### 4-iodo-N-(2,2,2-trichloro-1-{[(3-pyridinylamino)carbothioyl]amino}ethyl)benzamide

Example 114C and 3-aminopyridine were processed as described in Example 45B to provide the desired product.
mp 197-199 °C;
MS (DCI/NH₃) m/z 469 (M+H)⁺;
¹H NMR (DMSO-d₆) δ 10.57 (s, 1H), 9.23 (d, 1H, J=8.0 Hz), 8.60 (d, 1H, J=2.5 Hz), 8.35 (dd, 1H, J=5.3, 1.7 Hz), 8.27 (d, 1H, J=9.4 Hz), 8.03 (dt, 1H, J=8.5, 1.8 Hz), 7.91 (d, 2H, J=8.5 Hz), 7.63 (d, 2H, J=8.5 Hz), 7.48 (t, 1H, J=8.3 Hz), 7.39 (dd, 1H, J =8.6, 5.1 Hz);
Anal. calcd for C₁₅H₁₂Cl₃IN₅OS: C, 34.02; H, 2.24; N, 10.81. Found: C, 33.91; H, 2.24; N, 10.81.

### Example 149

### 3-phenyl-N-(2,2,2-trichloro-1-{[(3-nitroanilino)carbothioyl]amino}ethyl)propanamide

### Example 149A

### 3-phenyl-N-(2,2,2-trichloro-1-hydroxyethyl)propanamide

3-Phenylpropionamide was processed as described in Example 37C to provide the desired compound.
MS (ESI+) m/z 296 (M+H)⁺.

### Example 149B

### 3-phenyl-N-(1,2,2,2-tetrachloroethyl)propanamide

Example 149A was processed as described in Example 37D to provide the desired compound.
MS (ESI+) m/z 314 (M+H)⁺.

### Example 149C

### 3-phenyl-N-(2,2,2-trichloro-1-isothiocyanatoethyl)propanamide

Example 149B was processed as described in Example 45A to provide the desired compound.
MS (ESI+) m/z 337 (M+H)⁺.

### Example 149D

### 3-phenyl-N-(2,2,2-trichloro-1-1{[(3-nitroanilino)carbothioyl]amino}ethyl)propanamide

Example 149C and 3-nitroaniline were processed as described in Example 45B to provide the desired compound.
mp 171-173 °C;
MS (ESI+) m/z 477 (M+H)⁺;
¹H NMR (DMSO-d₆) δ 10.71 (s, 1H), 8.82 (d, 1H, J=8.2 Hz), 8.72 (t, 1H, J=1.1 Hz), 8.37 (d, 1H, J=8.6 Hz), 7.96 (dd, 1H, J=8.2, 1.2 Hz), 7.87 (dd, 1H, J=8.5, 1.2 Hz), 7.61 (t, 1H, J=8.1 Hz), 7.30-7.09 (m, 8H), 2.81 (t, 2H, J=8.0 Hz);
Anal. calcd for C₁₉H₁₇Cl₃N₅O: C, 52.13; H, 3.91; N, 16.00. Found: C, 52.42; H, 4.10; N, 15.82.

### Example 150

### 4-chloro-N-(2,2-dimethyl-1-{[2-nitro-1-(3-pyridinylamino)ethenyl]amino}propyl)benzamide

Example 68B and Example 69B were processed as described in Example 54D to provide the desired product.
mp 195-196 °C;
MS (ESI+) m/z 404 (M+H)⁺;
¹H NMR (DMSO-d₆) δ 9.61 (br s, 1H), 9.07 (d, 1H, J=8.8 Hz), 8.49 (dd, 1H, J=4.7, 0.7 Hz), 8.45 (d, 1H, J=2.0 Hz), 7.90 (d, 2H, J=8.8 Hz), 7.67 (m, 1H), 7.59 (d, 2H, J=8.8 Hz), 7.54 (m, 1H), 7.47 (dd, 1H, J=8.3, 4.8 Hz), 6.15 (s, 1H), 5.79 (dd, 1H, J=9.2, 8.8 Hz), 1.1 (s, 3H);
Anal. calcd for C₁₉H₂₂ClN₅O₃: C, 56.51; H, 5.49; N, 17.34. Found: C, 56.47; H, 5.55; N, 17.53.

### Example 151

### 4-chloro-N-(2,2-dichloro-1-{[2-nitro-1-(3-pyridinylamino)ethenyl]amino}pentyl)benzamide

Example 68B and Example 115A were processed as described in Example 54D to provide the desired product.
mp 185-186 °C;
MS (ESI+) m/z 472 (M+H)⁺;
¹H NMR (DMSO-d₆) δ 9.75 (br s, 1H), 9.58 (m, 1H), 8.48 (m, 1H), 8.44 (m, 1H), 7.92 (d, 2H, J=8.5 Hz), 7.67 (m, 1H), 7.60 (d, 2H, J=8.5 Hz), 7.47 (m, 1H), 6.59 (br s, 1H), 6.19 (m, 1H), 2.27 (m, 2H), 1.24 (m, 1H), 0.97 (t, 3H, J=6.8 Hz);
Anal. calcd for C₁₉H₂₀Cl₃N₅O₃: C, 48.27; H, 4.26; N, 14.81. Found: C, 48.32; H, 4.16; N, 14.76.

### Example 152

### 4-chloro-N-(1-{[2,2-dicyano-1-(3-pyridinylamino)vinyl]amino}-2,2-dimethylpropyl)benzamide

### Example 152A

### 2-[(methylsulfanyl)(3-pyridinylamino)methylene]malononitrile

3-Aminopyridine (2.76 g, 29.4 mmol) and 2-[bis(methylsulfanyl)methylene]malononitrile (5.00 g, 29.4 mmol) in isopropanol (50 mL) were heated at reflux for 12 hours. The solution was concentrated to a volume of 15 ml and cooled to 0 °C for 2 hours. The solid was colleted by filtration to provide the desired product (4.40 g) as a yellow solid.
MS (ESI-) m/z 215 (M-H)⁻.

### Example 152B

### 2-[amino(3-pyridinylamino)methylene]malononitrile

Example 152A (4.40 g, 20.3 mmol) was dissolved in a 2M solution of ammonia in isopropanol (60 mL) and heated in a sealed tube at 60 °C for 8 hours. The reaction mixture was cooled to ambient temperature and then further cooled to 0 °C, whereupon a solid precipitated from solution. The solid was filtered and the filter cake washed with cold isopropanol to provide the desired product (1.80 g) as a light brown solid.
MS (ESI-) m/z 184 (M-H)⁻.

### Example 152C

### 4-chloro-N-(1-{[2,2-dicyano-1-(3-pyridinylamino)vinyl]amino}-2,2-dimethylpropyl)benzamide

Example 152B and Example 69B were processed as described in Example 110B to provide the desired product.
mp 213-214 °C;
MS (ESI+) m/z 409 (M+H)⁺;
¹H NMR (DMSO-d₆) δ 9.84 (s, 1H), 8.69 (d, 1H, J=8.1 Hz), 8.35 (d, 1H, J=2.4 Hz), 8.33 (dd, 1H, J=4.8,1.4 Hz), 7.85 (d, 2H, J=8.8 Hz), 7.82 (m, 1H), 7.60 (d, 2H, J=8.8 Hz), 7.51 (m, 1H), 7.40 (dd, 1H, J=8.2, 4.8 Hz), 5.56 (dd, 1H, J=8.6, 8.2 Hz), 1.04 (s , 9H);
Anal. calcd for C₂₁H₂₁ClN₆O: C, 61.68; H, 5.17; N, 20.55. Found: C, 61.47; H, 5.33; N, 20.32.

### Determination of Potassium Channel Opening Activity Membrane Hyperpolarization Assays

Compounds were evaluated for potassium channel opening activity using primary cultured guinea-pig urinary bladder (GPB) cells.

For the preparation of urinary bladder smooth muscle cells, urinary bladders were removed from male guinea-pigs (Hartley, Charles River, Wilmington, MA) weighing 300-400 g and placed in ice-cold Ca²⁺-free Krebs solution (Composition, mM: KCI, 2.7; KH₂PO₄, 1.5; NaCl, 75; Na₂HPO₄, 9.6; Na₂HPO₄·7H₂O, 8; MgSO₄, 2; glucose, 5; HEPES, 10; pH 7.4). Cells were isolated by enzymatic dissociation as previously described with minor modifications (Klockner, U. and Isenberg, G., Pflugers Arch. (1985), 405, 329-339). The bladder was cut into small sections and incubated in 5 mL of the Kreb's solution containing 1 mg/mL collagenase (Sigma, St. Louis, MO) and 0.2 mg/mL pronase (Calbiochem, La Jolla, CA) with continuous stirring in a cell incubator for 30 minutes. The mixture was then centrifuged at 1300 x g for 5 minutes, and the pellet resuspended in Dulbecco's PBS (GIBCO, Gaithersburg, MD) and recentrifuged to remove residual enzyme. The cell pellet was resuspended in 5 mL growth media (composition: Dulbecco's modified Eagle's medium supplemented with 10% fetal bovine serum, 100 units/mL penicillin, 100 units/mL streptomycin and 0.25 mg/mL amphotericin B) and further dissociated by pipetting the suspension through a flame-polished Pasteur pipette and passing it through a polypropylene mesh membrane (Spectrum, Houston, TX). The cell density was adjusted to 100,000 cells/mL by resuspension in growth media. Cells were plated in clear-bottomed black 96-well plates (Packard) for membrane potential studies at a density of 20,000 cells/well and maintained in a cell incubator with 90% air: 10% CO₂ until confluent. Cells were confirmed to be of smooth muscle type by cytoskeletal staining using a monoclonal mouse anti human-α-smooth muscle actin (Biomeda, Foster City, CA).

Functional activity at potassium channels was measured by evaluating changes in membrane potential using the bis-oxonol dye DiBAC(4)₃ (Molecular Probes) in a 96-well cell-based kinetic assay system, Fluorescent Imaging Plate Reader (FLIPR) (K.S. Schroeder et al., J. Biomed. Screen., v. 1 pp. 75-81 (1996)). DiBAC(4)₃ is an anionic potentiometric probe which partitions between cells and extracellular solution in a membrane potential-dependent manner. With increasing membrane potential (for example, K⁺ depolarization), the probe further partitions into the cell; this is measured as an increase in fluorescence due to dye interaction with intracellular lipids and proteins. Conversely, decreasing membrane potential (hyperpolarization by potassium channel openers) evokes a decrease in fluorescence.

Confluent guinea-pig urinary bladder cells cultured in black clear-bottomed 96-well plates were rinsed twice with 200 mL assay buffer (composition, mM: HEPES, 20; NaCl, 120; KCI, 2; CaCl₂, 2; MgCl₂, 1; glucose, 5; pH 7.4 at 25 °C) containing 5 µM DiBAC(4)₃ and incubated with 180 mL of the buffer in a cell incubator for 30 minutes at 37 °C to ensure dye distribution across the membrane. After recording the baseline fluorescence for 5 minutes, the reference or test compounds, prepared at 10 times the concentration in the assay buffer, were added directly to the wells. Changes in fluorescence were monitored for an additional 25 minutes. Hyperpolarization responses were corrected for any background noise and were normalized to the response observed with 10 µM of the reference compound P1075 (assigned as 100%), a potent opener of smooth muscle K_{ATP} channels (Quast et al., Mol. Pharmacol., v. 43 pp. 474-481 (1993)).

Routinely, five concentrations of P1075 or test compounds (log or half-log dilutions) were evaluated and the maximal steady-state hyperpolarization values (expressed as % relative to P1075) plotted as a function of concentration. The EC₅₀ (concentration that elicites 50% of the maximal response for the test sample) values were calculated by non-linear regression analysis using a four parameter sigmoidal equation. The maximal response of each compound (expressed as % relative to P1075) is reported. Stock solutions of compounds were prepared in 100% DMSO and further dilutions were carried out in the assay buffer and added to a 96-well plate.

**Table 1**

| Membrane Hyperpolarization (MHP) in Guinea-Pig Bladder (GPB) Cells | | |
|---|---|---|
| Example # | Maximal Response (% P1075) | MHP GPB EC₅₀ (µM) |
| 1 | 116 | 0.07 |
| 3 | 79 | 0.89 |
| 4 | 133 | 0.30 |
| 5 | 106 | 1.08 |
| 6 | 107 | 1.55 |
| 7 | 101 | 0.45 |
| 8 | 10 | 10 |
| 9 | 104 | 0.08 |
| 10 | 65 | 0.70 |
| 11 | 109 | 0.51 |
| 12 | 4 | 10 |
| 13 | 91 | 0.35 |
| 14 | 110 | 0.30 |
| 15 | 70 | 0.55 |
| 16 | 65 | 0.370 |
| 17 | 109 | 0.09 |
| 18 | 123 | 0.09 |
| 19 | 117 | 0.06 |
| 20 | 85 | 0.09 |
| 21 | 96 | 0.09 |
| 22 | 100 | 0.22 |
| 23 | 66 | 3.96 |
| 24 | 93 | 0.08 |
| 25 | 103 | 0.07 |
| 26 | 80 | 0.99 |
| 27 | 80 | 0.10 |
| 28 | 77 | 0.50 |
| 29 | 28 | 1.0 |
| 30 | 22 | 3.2 |
| 32 | 15 | 1.0 |
| 33 | 102 | 0.22 |
| 34 | 80 | 0.50 |
| 35 | 40 | 13.1 |
| 36 | 50 | 25.5 |
| 37 | 85 | 0.78 |
| 38 | 100 | 0.10 |
| 39 | 101 | 0.46 |
| 40 | 100 | 0.12 |
| 41 | 98 | 0.14 |
| 42 | 66 | 2.14 |
| 43 | 80 | 1.21 |
| 44 | 103 | 0.04 |
| 45 | 92 | 0.04 |
| 46 | 105 | 0.04 |
| 47 | 98 | 0.06 |
| 48 | 96 | 0.20 |
| 49 | 92 | 0.04 |
| 50 | 92 | 0.10 |
| 51 | 88 | 0.57 |
| 52 | 72 | 0.27 |
| 57 | 80 | 4.6 |
| 58 | 87 | 0.65 |
| 59 | 73 | 0.31 |
| 60 | 62 | 1.0 |
| 61 | 36 | 0.33 |
| 62 | 81 | 0.33 |
| 63 | 73 | 4.4 |
| 64 | 89 | 0.40 |
| 65 | 89 | 0.130 |
| 66 | 79 | 0.082 |
| 68 | 99 | 0.43 |
| 70 | 74 | 0.32 |
| 84 | 69 | 6 |
| 85 | <20 | >10 |
| 86 | 83 | 0.086 |
| 87 | 85 | 0.52 |
| 88 | 87 | 0.22 |
| 89 | 88 | 0.1 |
| 90 | 55 | 0.17 |
| 91 | 68 | 2.9 |
| 92 | 71 | 0.25 |
| 93 | 75 | 0.14 |
| 94 | 82 | 0.16 |
| 95 | 97 | 0.84 |
| 96 | 93 | 0.24 |
| 97 | 74 | 1.6 |
| 98 | 62 | 1.1 |
| 99 | 84 | 3.5 |
| 100 | 87 | 0.18 |
| 101 | 59 | 6.1 |
| 102 | <20 | >10 |
| 103 | 103 | 0.05 |
| 105 | 106 | 0.053 |
| 106 | 85 | 0.63 |
| 123 | 90 | 0.85 |
| 124 | 98 | 0.11 |
| 127 | 65 | 1.2 |
| 128 | 116 | 0.47 |
| 129 | 132 | 0.12 |
| 130 | 147 | 0.53 |
| 131 | 53 | 2 |
| 132 | 124 | 0.13 |
| 133 | 106 | 0.039 |
| 134 | 86 | 2 |
| 148 | 61 | 0.096 |
| 149 | 129 | 0.084 |

### In Vitro Functional Models

Compounds were evaluated for functional potassium channel opening activity using tissue strips obtained from Landrace pig bladders.

Landrace pig bladders were obtained from female Landrace pigs of 9-30 kg. Landrace pigs were euthanized with an intraperitoneal injection of pentobarbital solution, Somlethal^{®} , J.A. Webster Inc., Sterling MA. The entire bladder was removed and immediately placed into Krebs Ringer bicarbonate solution (composition, mM: NaCl, 120; NaHCO₃, 20; dextrose, 11; KCI, 4.7; CaCl₂, 2.5; MgSO₄, 1.5; KH₂PO₄, 1.2; K₂EDTA, 0.01, equilibrated with 5% CO₂/95% O₂ pH 7.4 at 37°C). Propranolol (0.004 mM) was included in all of the assays to block β-adrenoceptors. The trigonal and dome portions were discarded. Strips 3-5 mm wide and 20 mm long were prepared from the remaining tissue cut in a circular fashion. The mucosal layer was removed. One end was fixed to a stationary glass rod and the other to a Grass FT03 transducer at a basal preload of 1.0 gram. Two parallel platinum electrodes were included in the stationary glass rod to provide field stimulation of 0.05 Hz, 0.5 milli-seconds at 20 volts. This low frequency stimulation produced a stable twitch response of 100-500 centigrams. Tissues were allowed to equilibrate for at least 60 minutes and primed with 80 mM KCI. A control concentration response curve (cumulative) was generated for each tissue using the potassium channel opener P1075 as the control agonist. P1075 completely eliminated the stimulated twitch in a dose dependent fashion over a concentration range of 10⁻⁹ to 10⁻⁵ M dissolved in DMSO using 1/2 log increments. After a 60 minute rinsing period, a concentration response curve (cumulative) was generated for the test agonist in the same fashion as that used for the control agonist P1075. The maximal efficacy of each compound (expressed as % relative to P1075) is reported. The amount of agent necessary to cause 50% of the agent's maximal response (ED₅₀) was calculated using "ALLFIT" (DeLean et al., Am. J. Physiol., 235, E97 (1980)). Agonist potencies were also expressed as an index relative to P1075. The index was calculated by dividing the ED₅₀ for P1075 by the ED₅₀ for the test agonist in a given tissue. Each tissue was used for only one test agonist, and the indices obtained from each tissue were averaged to provide an average index of potency. These data are shown in Table 2.

**Table 2**

| Functional Potassium Channel Opening Activity in Isolated Bladder Strips | | | |
|---|---|---|---|
| | Landrace Pig Bladder | | |
| Example # | Efficacy (%P1075) | ED₅₀ (µM) | Index |
| 1 | 100 | 9 | 0.009 |
| 3 | 97 | 9 | 0.012 |
| 4 | 89 | 18 | 0.015 |
| 9 | 91 | 17 | 0.011 |
| 11 | 100 | 19 | 0.011 |
| 13 | 95 | 10 | 0.022 |
| 14 | 96 | 14 | 0.027 |
| 15 | 97 | 28 | 0.005 |
| 17 | 100 | 12 | 0.006 |
| 18 | 97 | 8 | 0.020 |
| 20 | 84 | 28 | 0.011 |
| 21 | 86 | 21 | 0.014 |
| 22 | 100 | 5 | 0.017 |
| 23 | 100 | 16 | 0.009 |
| 24 | 100 | 17 | 0.007 |
| 25 | 100 | 1 | 0.078 |
| 27 | 94 | 25 | 0.009 |
| 28 | 100 | 42 | 0.004 |
| 33 | 80 | 12 | 0.027 |
| 34 | 99 | 23 | 0.003 |
| 38 | 98 | 7 | 0.015 |
| 40 | 100 | 15 | 0.007 |
| 44 | 95 | 2 | 0.040 |
| 46 | 84 | 5 | 0.015 |
| 47 | 67 | 36 | 0.019 |
| 49 | 100 | 3 | 0.031 |
| 50 | 90 | 17 | 0.007 |
| 51 | 100 | 17 | 0.006 |
| 52 | 72 | 19 | 0.012 |
| 57 | 85 | 14 | 0.007 |
| 58 | 59 | 12 | 0.008 |
| 86 | 19 | 30 | 0.01 |
| 87 | 67 | 7.6 | 0.02 |
| 88 | 36 | 10 | 0.074 |
| 89 | 41 | 3.4 | 0.120 |
| 90 | 44 | 0.42 | 0.420 |
| 91 | 46 | 0.83 | 0.360 |
| 92 | 76 | 1.8 | 0.047 |
| 98 | 30 | 33 | 0.004 |
| 103 | 83 | 2.4 | 0.450 |
| 106 | 69 | 12 | 0.010 |
| 116 | 94 | 3.3 | 0.028 |
| 121 | 89 | 4.8 | 0.044 |
| 123 | 63 | 10 | 0.028 |
| 124 | 47 | 9.3 | 0.012 |
| 125 | 92 | 9.2 | 0.030 |
| 126 | 94 | 3.8 | 0.096 |
| 133 | 58 | 8.4 | 0.038 |
| 148 | 78 | 1.1 | 0.082 |
| 149 | 95 | 2.4 | 0.076 |

As shown by the data in Tables 1 and 2, the compounds of this invention reduce stimulated contractions of the bladder by opening potassium channels and therefore have utility in the treatment of diseases prevented by or ameliorated with potassium channel openers.

### In Vivo Data

The utility of compounds of the present invention for the treatment of urinary incontinence, bladder overactivity, and bladder instability is illustrated by the ability of compounds of the present invention to inhibit bladder contractions in vivo. The following method is illustrative of the in vivo bladder efficacy of compounds of the present invention.

In Vivo Bladder Efficacy Protocol (Isovolumetric Contractions Model)

Male CD rats (400-450 g) were anesthetized with urethane (0.6 g/kg ip + 0.6 g/kg sc). The left femoral vein was cannulated with polyethylene (PE-50) tubing for the administration of test compound. A third polyethylene catheter (PE-60) was inserted 3-4 mm into the apex of the bladder dome and secured using a 5-0 silk purse string suture. The bladder was emptied via this catheter and additionally by applying slight manual pressure on the lower abdomen. The urinary catheter was connected using a Y-tube connector to both a pressure transducer and a syringe pump. The urethra was then ligated using 4-0 silk suture and the bladder was slowly filled using a constant infusion of room temperature saline at the rate of 0.1 mL/min until spontaneous rhythmic contractions were evident (1.0-1.3 mL). After the contractions stabilized to a consistent pattern, bladder pressure was monitored for 20 minutes before and after a dose of the vehicle (equal parts of β-cyclodextrin stock solution (100 g β-cyclodextrin dissolved in 200 mL) and sterile water) alone. Then three doses of a test compound were administered cumulatively intravenous (iv) at 20 minute intervals. Each dosing solution (1 mL/kg) was warmed to body temperature before dosing and was infused over 3 minutes to minimize dosing artifacts on the bladder pressure trace. Data were averaged over the last 10 minutes of each period and presented as percent change from control. Area under the curve of the bladder contractions was determined from the respective waveforms using a Modular Instruments, Inc. computerized data acquisition system and averaged over the last ten minutes of each twenty minute period. The doses required to reduce the area under the curve of the bladder contractions by 30% (AUC ED30%) relative to control were estimated using a customized Excel spreadsheet. Data for representative compounds of the present invention are shown in Table 3.

**Table 3**

| Bladder Pressure Effects in the Rat Isovolumetric Contractions Model | |
|---|---|
| Example # | AUC EC₃₀ (µmol/kg) |
| 46 | 0.70 |
| 51 | 0.28 |
| 52 | 0.78 |

The data in Table 3 illustrates the ability of compounds of the present invention to inhibit bladder contractions in vivo.

The term "pharmaceutically acceptable carrier," as used herein, means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The present invention provides pharmaceutical compositions which comprise compounds of the present invention formulated together with one or more non-toxic pharmaceutically acceptable carriers wherein formula I is as defined in Claim 83. The pharmaceutical compositions can be formulated for oral administration in solid or liquid form, for parenteral injection or for rectal administration.

Further included within the scope of the present invention are pharmaceutical compositions comprising one or more of the compounds of formula I, VI or VII prepared and formulated in combination with one or more non-toxic pharmaceutically acceptable compositions wherein formula I is as defined in Claim 83. The pharmaceutical compositions can be formulated for oral administration in solid or liquid form, for parenteral injection or for rectal administration.

The pharmaceutical compositions of this invention can be administered to humans and other mammals orally, rectally, parenterally, intracisternally, intravaginally, intraperitoneally, topically (as by powders, ointments or drops), bucally or as an oral or nasal spray. The term "parenterally," as used herein, refers to modes of administration which include intravenous, intramuscular, intraperitoneal, intrasternal, subcutaneous, intraarticular injection and infusion.

Pharmaceutical compositions of this invention for parenteral injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity may be maintained, for example, by the use of a coating such as lecithin, by the maintenance of the required particle size in the case of dispersions, and by the use of surfactants.

These compositions may also contain adjuvants such as preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars and sodium chloride. Prolonged absorption of the injectable pharmaceutical form may be brought about by the use of agents delaying absorption, for example, aluminum monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Suspensions, in addition to the active compounds, may contain suspending agents, as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, the compounds of the present invention can be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

The active compounds can also be in micro-encapsulated form, if appropriate, with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound can be admixed with at least one inert diluent such as sucrose, lactose, or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of such composition that they release the active ingredient(s) only, or preferentially, in a certain part of the intestinal tract in a delayed manner. Examples of embedding compositions which can be used include polymeric substances and waxes.

Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic, parenterally acceptable diluent or solvent such as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid; b) binders such as carboxymethylcellulose, alginates, gelatin, polyvinylpyrrolidinone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin); f) absorption accelerators such as quaternary ammonium compounds; g) wetting agents such as cetyl alcohol and glycerol monostearate;) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract in a delayed manner. Examples of embedding compositions which can be used include polymeric substances and waxes.

Compositions for rectal or vaginal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and therefore melt in the rectum or vaginal cavity and release the active compound.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose; talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes may be used. The present compositions in liposome form may contain, in addition to the compounds of the present invention, stabilizers, preservatives, excipients, and the like. The preferred lipids are the natural and synthetic phospholipids and phosphatidylcholines (lecithins) used separately or together.

Methods to form liposomes are known in the art. See, for example, Prescott, Ed., Methods in Cell Biology, Volume XIV, Academic Press, New York, N. Y., (1976), p 33 et seq.

The term "pharmaceutically acceptable salts," as used herein, refers to carboxylate salts, amino acid addition salts and zwitterions of compounds of formula I, VI and VII which are, within the scope of sound medical judgement, suitable for use in contact with the tissues of humans and lower animals without undue toxicity, irritation, and allergic response, are commensurate with a reasonable benefit/risk ratio, and are effective for their intended use.

The term "pharmaceutically acceptable salt," as used herein, refers to salts that are well known in the art. For example, S. M Berge et al. describe pharmaceutically acceptable salts in detail in J. Pharmaceutical Sciences, 66:1-19 (1977). Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid, or malonic acid or by using other methods used in the art such as ion exchange. Other pharmaceutically acceptable salts include nitrate, bisulfate, borate, formate, butyrate, valerate, 3-phenylpropionate, camphorate, adipate, benzoate, oleate, palmitate, stearate, laurate, lactate, fumarate, ascorbate, aspartate, nicotinate, p-toluenesulfonate, camphorsulfonate, methanesulfonate, 2-hydroxyethanesulfonate, gluconate, glucoheptonate, lactobionate, glycerophosphate, pectinate, and lauryl sulfate, metal salts such as sodium, potassium, magnesium or calcium salts or amino salts such as ammonium, triethylamine salts, all of which may be prepared according to conventional methods.

Dosage forms for topical administration of a compound of this invention include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also contemplated as being within the scope of this invention.

Actual dosage levels of active ingredients in the pharmaceutical compositions of this invention can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved.

The compounds of the invention possess potassium channel opening activity in mammals (especially humans). As potassium channel openers, the compounds of the present invention may be useful for the treatment and prevention of diseases such as asthma, epilepsy, male sexual dysfunction, female sexual dysfunction, pain, bladder overactivity, stroke, diseases associated with decreased skeletal blood flow such as Raynaud's phenomenon and intermittent claudication, eating disorders, functional bowel disorders, neurodegeneration, benign prostatic hyperplasia (BPH), dysmenorrhea, premature labor, alopecia, cardioprotection, coronary artery disease, angina and ischemia.

The ability of the compounds of the present invention to treat bladder overactivity, sensations of incontinence urgency, urinary incontinence, pollakiuria, bladder instability, nocturia, bladder hyerreflexia, and enuresis may be demonstrated by (Resnick, The Lancet (1995) 346, 94-99; Hampel, Urology (1997) 50 (Suppl 6A), 4-14; Bosch, BJU International (1999) 83 (Suppl 2), 7-9; Andersson, Urology (1997) 50 (Suppl 6A), 74-84; Lawson, Pharmacol. Ther., (1996) 70, 39-63; Nurse., Br. J. Urol., (1991) 68, 27-31; Howe, J. Pharmacol. Exp. Ther., (1995) 274, 884-890; Gopalakrishnan, Drug Development Research, (1993) 28, 95-127).

The ability of the compounds of the present invention to treat male sexual dysfunction such as male erectile dysfunction, impotence and premature ejaculation may be demonstrated by (Andersson, Pharmacological Reviews (1993) 45, 253; Lee, Int. J. Impot. Res. (1999) 11(4), 179-188; Andersson, Pharmacological Reviews (1993) 45, 253; Lawson, Pharmacol. Ther., (1996) 70, 39-63, Vick, J. Urol. (2000) 163: 202).

The ability of the compounds of the present invention to treat female sexual dysfunction such as clitoral erectile insufficiency, vaginismus and vaginal engorgement may be demonstrated by (J.J. Kim, J.W. Yu, J.G. Lee, D.G. Moon, "Effects of topical K-ATP channel opener solution on clitoral blood flow", J. Urol. (2000) 163 (4): 240; I. Goldstein and J.R. Berman., "Vasculogenic female sexual dysfunction: vaginal engorgement and clitoral erectile insufficiency syndromes"., Int. J. Impotence Res. (1998) 10:S84-S90).

The ability of the compounds of the present invention to treat benign prostatic hyperplasia (BPH) may be demonstrated by (Pandita, The J. of Urology (1999) 162, 943; Andersson; Prostate (1997) 30: 202-215).

The ability of the compounds of the present invention to treat premature labor and dysmenorrhoea may be demonstrated by (Sanborn, Semin. Perinatol. (1995) 19, 31-40; Morrison, Am. J. Obstet. Gynecol. (1993) 169(5), 1277-85; Kostrzewska, Acta Obstet. Gynecol. Scand. (1996) 75(10), 886-91; Lawson, Pharmacol. Ther., (1996) 70, 39-63).

The ability of the compounds of the present invention to treat functional bowel disorders such as irritable bowel syndrome may be demonstrated by (Lawson, Pharmacol. Ther., (1996) 70, 39-63).

The ability of the compounds of the present invention to treat asthma and airways hyperreactivity may be demonstrated by (Lawson, Pharmacol. Ther., (1996) 70, 39-63; Buchheit, Pulmonary Pharmacology & Therapeutics (1999) 12, 103; Gopalakrishnan, Drug Development Research, (1993) 28, 95-127).

The ability of the compounds of the present invention to treat various pain states including but not limited to migraine and dyspareunia may be demonstrated by (Rodrigues, Br. J. Pharmacol. (2000) 129(1), 110-4; Vergoni, Life Sci. (1992) 50(16), PL135-8; Asano, Anesth. Analg. (2000) 90(5), 1146-51; Lawson, Pharmacol. Ther., (1996) 70, 39-63; Gopalakrishnan, Drug Development Research, (1993) 28, 95-127; Gehlert, Prog. Neuro-Psychopharmacol. & Biol. Psychiat., (1994) 18, 1093-1102).

The ability of the compounds of the present invention to treat epilepsy may be demonstrated by (Lawson, Pharmacol. Ther., (1996) 70, 39-63; Gopalakrishnan, Drug Development Research, (1993) 28, 95-127; Gehlert, Prog. Neuro-Psychopharmacol & Biol. Psychiat., (1994) 18, 1093-1102).

The ability of the compounds of the present invention to treat neurodegenerative conditions and diseases such as cerebral ischemia, stroke, Alzheimer's disease and Parkinson's disease may be demonstrated by (Lawson, Pharmacol. Ther., (1996) 70, 39-63; Gopalakrishnan, Drug Development Research, (1993) 28, 95-127; Gehlert, Prog. Neuro-Psychopharmacol. & Biol. Psychiat., (1994) 18, 1093-1102; Freedman, The Neuroscientist (1996) 2, 145).

The ability of the compounds of the present invention to treat diseases or conditions associated with decreased skeletal muscle blood flow such as Raynaud's syndrome and intermittent claudication may be demonstrated by (Lawson, Pharmacol. Ther., (1996) 70, 39-63; Gopalakrishnan, Drug Development Research, (1993) 28, 95-127; Dompeling Vasa. Supplementum (1992) 3434; WO9932495).

The ability of the compounds of the present invention to treat eating disorders such as obesity may be demonstrated by (Spanswick, Nature, (1997) 390, 521-25; Freedman, The Neuroscientist (1996) 2, 145).

The ability of the compounds of the present invention to treat alopecia may be demonstrated by (Lawson, Pharmacol. Ther., (1996) 70, 39-63; Gopalakrishnan, Drug Development Research, (1993) 28, 95-127).

The ability of the compounds of the present invention to treat myocardial injury during ischemia and reperfusion may be demonstrated by (Garlid, Circ Res (1997) 81(6), 1072-82; Lawson, Pharmacol. Ther., (1996) 70, 39-63; Grover, J. Mol. Cell Cardiol. (2000) 32, 677).

The ability of the compounds of the present invention to treat coronary artery disease may be demonstrated by (Lawson, Pharmacol. Ther., (1996) 70, 39-63, Gopalakrishnan, Drug Development Research, (1993) 28, 95-127).

Aqueous liquid compositions of the present invention are particularly useful for the treatment and prevention of asthma, epilepsy, hypertension, Raynaud's syndrome, male sexual dysfunction, female sexual dysfunction, migraine, pain, eating disorders, urinary incontinence, functional bowel disorders, neurodegeneration and stroke.

When used in the above or other treatments, a therapeutically effective amount of one of the compounds of the present invention can be employed in pure form or, where such forms exist, in pharmaceutically acceptable salt, ester, amide or prodrug form. Alternatively, the compound can be administered as a pharmaceutical composition containing the compound of interest in combination with one or more pharmaceutically acceptable excipients. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or lower animal may range from about 0.003 to about 10 mg/kg/day. For purposes of oral administration, more preferable doses can be in the range of from about 0.01 to about 5 mg/kg/day. If desired, the effective daily dose can be divided into multiple doses for purposes of administration; consequently, single dose compositions may contain such amounts or submultiples thereof to make up the daily dose.

## Claims

1. A compound having formula I: 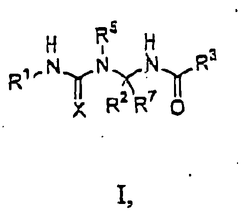 or a pharmaceutically acceptable salt thereof wherein,
X is selected from the group consisting of O, S, CHCN, C(CN)₂, CHNO₂, and NR⁸;
R⁸ is selected from the group consisting of hydrogen, alkoxy, alkyl, alkylsulfonyl, arylalkoxy, aryloxy, arylsulfonyl, cyano, haloalkylsulfonyl, heterocyclealkoxy, heterocycleoxy, hydroxy, nitro, and sulfamyl;
R¹ is selected from the group consisting of aryl, arylalkyl, heterocycle, and heterocyclealkyl;
R² is selected from hydrogen, alkenyl, alkenyloxyalkyl, alkenyloxy (alkenyloxy)alkyl, alkoxyakyl, alkoxycarbonyl, alkoxycarbonylalkyl, alkoxycarbonyl(halo)alkyl, alkoxy(halo)alkyl, alkyl, alkylcarbonyl, alkylcarbonylalkyl, alkylcarbonyl(halo)alkyl, alkylcarbonyloxyalkyl, alkylsulfinylalkyl, alkylsulfonylalkyl, alkylthioalkyl, alkynyl, amido, amidoalkyl, aryl, arylalkoxyalkyl, arylalkoxycarbonyl, arylakoxycarbonylakyl, arylalkyl, arylcarbonyl, arylcarbonylalkyl, arylcarbonyloxyalkyl, aryl(halo)alkyl, aryloxyalkyl, aryloxycarbonyl, aryloxycarbonylalkyl, arylalkylthioalkyl, arylsulfonylalkyl, carboxy, carboxyalkyl, carboxy(halo)alkyl, cyanoalkyl, cyano(halo)alkyl, cycloalkenyl, cycloalkenylakyl, cycloalkyl, cycloalkylalkoxyalkyl, cycloalkylalkyl, cycloalkylcarbonyl, cycloalkyloxyalkyl, cycloalkylalkythioalkyl, formyl, haloalkenyl, haloalkyl, haloalkylcarbonyl, haloalkynyl, heterocycle, heterocyclealkoxyalkyl, heterocyclealkyl, heterocyclecarbonyl, heterocycleoxyalkyl, heterocyclealkylthioalkyl, hydroxyalkyl, mercaptoalkyl, sulfamylalkyl, sulfamyl(halo)alkyl, and (NR⁹R¹⁰)alkyl wherein R⁹ and R¹⁰ are independently selected front hydrogen, alkyl, alkylcarbonyl, aryl, arylalkyl, arylcarbonyl, formyl, and S(O)₂R¹¹, wherein R¹¹ is selected from alkyl, aryl, and arylalkyl;
R³ is selected from the group consisting of alkyl, aryl, arylalkyl, heterocycle, and heterocyclealkyl;
R⁵ is selected from the group consisting of hydrogen, alkyl and OR¹²; R¹² is selected from the group consisting of hydrogen, alkyl and arylalkyl; and
R⁷ is selected from the group consisting of hydrogen, haloalkyl, and C₁-C₆ alkyl; or
R⁷ and R² taken together with the carbon atom to which they are attached, together form a 5 or 6 membered carbocyclic ring wherein the 5 or 6 membered carbocyclic ring is optionally substituted with 1 or 2 substituents independently selected from the group consisting of alkenyl, alkoxy, alkyl, alkynyl, halogen, haloalkoxy, and haloalkyl;
wherein unless otherwise specified alkyl by itself or as part of another group contains from one to ten carbon atoms;
alkenyl by itself or as part of another group contains from two to ten carbon atoms;
alkynyl by itself or as part of another group contains from two to ten carbon atoms;
aryl by itself or as part of another group is a monocyclic carbocyclic ring system or a bicyclic carbocyclic fused ring system having one or more aromatic rings,
aryl may optionally have 1, 2, 3, 4 or 5 substituents independently selected from the group consisting of alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, phenyl, furyl, -NR^{A}R^{B} and (NR^{A}R^{B})alkyl wherein R^{A} and R^{B} are independently selected from hydrogen, alkyl, alkylcarbonyl and formyl;
cycloalkyl is a monocyclic, bicyclic or tricyclic ring system;
cycloalkyl may optionally have 1, 2, 3, 4 or 5 substituents independently selected from the group consisting of alkenyl, alkoxy, alkoxycarbonyl, alkoxycarbonylalkyl, alkyl, alkylcarbonylalkyl, alkylcarbonyloxy, alkylcarbonyloxyalkyl, alkylsulfonylalkyl, alkynyl, amido, amidoalkyl, cyanoalkyl, halo, haloalkoxy, haloalkyl, hydroxy, hydroxyalkyl, sulfamylalkyl, -NR^{A}R^{B} and (NR^{A}R^{B})alkyl wherein R^{A} and R^{B} are independently selected from hydrogen, alkyl, alkylcarbonyl and formyl;
heterocycle by itself or as part of another group is a monocyclic or bicyclic ring system wherein (i) the monocyclic ring system is a 5- or 6-membered ring containing 1, 2, 3 or 4 heteroatoms independently selected from oxygen, nitrogen and sulfur wherein the 5-membered ring has from 0-2 double bonds and the 6-membered ring has from 0-3 double bonds and (ii) the bicyclic ring system is any of the above monocyclic ring systems fused to a monocyclic aryl group as defined above, a monocyclic cycloalkyl group as defined above or a monocyclic heterocyclic ring system as defined above;
heterocycle may optionally have 1, 2 or 3 substituents independently selected from the group consisting of alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, oxo, sulfamyl, sulfamylalkyl, -NR^{A}R^{B}, (NR^{A}R^{B})alkyl wherein R^{A} and R^{B} are independently selected from hydrogen, alkyl, alkylcarbonyl and formyl, and phenyl optionally having 1 or 2 substituents independently selected from alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, -NR^{A}R^{B}, (NR^{A}R^{B})alkyl wherein R^{A} and R^{B} are independently selected from hydrogen, alkyl, alkylcarbonyl and formyl;
amido by itself or as part of another group is -C(O)NR⁹R¹⁰ wherein R⁹ and R¹⁰ are independently selected from the group consisting of hydrogen, alkyl, alkylcarbonyl, phenyl, phenylalkyl, phenylcarbonyl, formyl and S(O)₂R¹¹ wherein R¹¹ is selected from alkyl, phenyl and phenylalkyl;
cycloalkenyl by itself or as part of another group is a cyclic hydrocarbon containing from three to eight carbons and containing at least one carbon-carbon double bond;
cycloalkenyl may optionally have 1,2,3,4 or 5 substituents independently selected from alkenyl, alkoxy, alkoxycarbonyl, alkoxycarbonylalkyl, alkyl, alkylcarbonyloxy, alkylcarbonyloxyalkyl, alkynyl, amido, amidoalkyl, halo, haloalkoxy, haloalkyl, hydroxy, hydroxyalkyl, sulfamylalkyl, -NR^{A}R^{B} and (NR^{A}R^{B})alkyl wherein R^{A} and R^{B} are independently selected from hydrogen alkyl, alkylcarbonyl and formyl;
sulfamyl is a -SO₂NR⁹⁴R⁹⁵ group wherein R⁹⁴ and R⁹⁵ are independently selected from hydrogen, alkyl, aryl and arylalkyl;
provided that when X is O; R² is -CCl₃; R³ is alkyl or phenyl; and R⁵ and R⁷ are hydrogen; then R¹ is other than phenyl;
further provided that
N-[[[[(2-methylphenyl)amino]carbonyl]amino]phenylmethyl]-benzamide;
N-[[[[(4-chlorophenyl)amino]carbonyl]amino]phenylmethyl]-benzamide;
N-[2,2-dichloro-1-[[phenylamino)thioxomethyl]amino]ethyl]-benzamide;
N-[2,2-dichloro-1-[[phenylamino)thioxomethyl]amino]ethyl]-4-methyl-benzamide;
N-[2,2-dichloro-1-[[[4-methylphenyl)amino)thioxomethyl]amino]ethyl]-benzamide;
N-[2,2-dichloro-1-[[[4-methylphenyl)amino)thioxomethyl]amino]ethyl]-4-methyl-benzamide;
N-[2,2-dichloro-1-[[[4-chlorophenyl)amino)thioxomethyl]amino]ethyl]-benzamide;
N-[2,2-dichloro-1-[[[4-chlorophenyl)amino)thioxomethyl]amino]ethyl]-4-methyl-benzamide;
N-[[[(phenylamino)carbonyl]amino]methyl]-3-pyridinecarboxamide;
N-[[[[(2-methylphenyl)amino)carbonyl]amino]methyl]-3-pyridinecarboxamide;
N-[[[[(4-methylphenyl)amino)carbonyl]amino]methyl]-3-pyridinecarboxamide;
-N-[[[[(3-ethoxyphenyl)amino)carbonyl]amino]methyl]-3-pyridinecarboxamide;
N-[[[[(4-nitrophenyl)amino)carbonyl]amino]methyl]-3-pyridinecarbyxamide;
N-[[([(3-methylphenyl)amino)carbonyl]amino]methyl]-3-pyridinecarboxamide;
N-[[[(phenylamino)carbonyl]amino]methyl]-benzamide;
N-[[[(phenylamino)thioxomethyl]amino]methyl]-benzamide;
N-[[[(phenylamino)carbonyl]amino]methyl]-4-chlorv-benzamide;
N-[[[(phenylamino)thioxomethyl]amino]methyl]-4-chloro-benzamide; 1-(1-acetamido-2,2,2-trichloroethyl)-3-phenyl-2-thio-urea;
1-phenyl-2-thio-3-(2,2,2-trichloro-1-propionamidoethyl)-urea; and
1-(1-butyramido-2,2,2-trichloroethyl)-3 -phenyl-2-thio-urea are excluded.

2. A compound according to claim 1 wherein
R⁸ is selected from the group consisting of alkoxy, alkylsulfonyl, arylalkoxy, arylsulfonyl, cyano, haloalkylsulfonyl, hydroxy, and nitro;
R² is selected from the group consisting of hydrogen, alkenyl, alkenyloxyalkyl; alkenyloxy(alkenyloxy)alkyl, alkoxyalkyl, alkoxycarbonylalkyl, alkyl, alkylcarbonyl, alkylsulfonylalkyl, alkylthioalkyl, aryl, arylalkyl, arylsulfonylalkyl, cyanoalkyl, cycloalkenyl, cycloalkenylalkyl, cycloalkyl, cycloalkylalkyl, haloalkyl, haloalkylcarbonyl, heterocycle, heterocyclealkyl, hydroxyalkyl, sulfamylalkyl, and (NR⁹R¹⁰)alkyl;
R³ is selected from the group consisting of aryl, arylalkyl, and heterocycle;
R⁵ is selected from the group consisting of hydrogen and alkyl; and
R⁷ is selected from the group consisting of hydrogen, haloalkyl, and C₁-C₆ alkyl;
or
R⁷ and R² taken together with the carbon atom to which they are attached, together form a 5 or 6 membered carbocyclic ring wherein the 5 or 6 membered carbocyclic ring is optionally substituted with 1 or 2 substituents independently selected from the group consisting of alkyl, halo, haloalkoxy, and haloalkyl.

3. A compound according to claim 2 wherein
R⁸ is selected from the group consisting of alkoxy, alkylsulfonyl, haloalkylsulfonyl, cyano, hydroxy, nitro, arylalkoxy wherein the aryl portion of arylalkoxy is phenyl, and arylsulfonyl wherein the aryl portion of arylsulfonyl is phenyl;
R¹ is pyridine, pyrimidine or quinoline optionally substituted with 1, 2, or 3 substituents independently selected from the group consisting of alkoxy, alkyl, halo, haloalkyl, nitro, phenylsulfonyl and sulfamyl and aryl wherein aryl is phenyl optionally substituted with 1,2, or 3 substituents independently selected from the group consisting of alkoxy, alkyl, halo, haloalkyl, nitro, phenylsulfonyl, and sulfamyl;
R² is selected from the group consisting of hydrogen, alkenyl, alkenyloxyalkyl, alkenyloxy(alkenyloxy)alkyl, alkoxyalkyl, alkoxycarbonylalkyl, alkyl, alkylcarbonyl, alkylsulfonylalkyl, aryl wherein aryl is phenyl, arylalkyl wherein the aryl portion of arylalkyl is phenyl, arylsulfonylalkyl wherein the aryl portion of arylsulfonylalkyl is phenyl, cyanoalkyl, cycloalkenylalkyl, cycloalkyl, haloalkyl, haloalkylcarbonyl, hydroxyalkyl, sulfamylalkyl, (NR⁹R¹⁰)alkyl and heterocycle wherein heterocycle is selected from the group consisting of 1,3-dioxane, pyrrolidine and thiophene; and
R³ is selected from the group consisting of aryl wherein aryl is phenyl and arylalkyl wherein the aryl portion of arylalkyl is phenyl.

4. A compound according to claim 1 wherein,
X is selected from the group consisting of O, S, CHNO₂, C(CN)₂, and NR⁸;
R⁸ is selected from the group consisting of arylsulfonyl, cyano, haloalkylsulfonyl, nitro and sulfamyl;
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl;
R³ is selected from the group consisting of aryl and arylalkyl; R⁵ is selected from the group consisting of hydrogen and alkyl; and
R⁷ is hydrogen.

5. A compound according to claim 1 wherein,
X is selected from the group consisting of O, S, CHNO₂, C(CN)₂, and NR⁸;
R⁸ is selected from the group consisting of arylsulfonyl wherein the aryl portion of arylsulfonyl is phenyl, cyano, haloalkylsulfonyl, nitro and sulfamyl;
R¹ is selected from the group consisting of aryl wherein aryl is phenyl, arylalkyl wherein the aryl portion of arylalkyl is phenyl, heterocycle wherein heterocycle is selected from the group consisting of quinoline, pyridine and pyrimidine, and heterocyclalkyl wherein the heterocycle portion of heterocyclealkyl is pyridine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is selected from the group consisting of aryl wherein aryl is phenyl and arylalkyl wherein the aryl portion of arylalkyl is phenyl;
R⁵ is selected from the group consisting of hydrogen and alkyl; and
R⁷ is hydrogen.

6. A compound according to claim 1 wherein,
X is NR⁸ ;
R⁸ is cyano;
R¹ is selected from the group consisting of heterocycle and heterocyclealkyl;
R³ is selected from the group consisting of heterocycle and heterocyclealkyl; and
R⁵ is hydrogen.

7. A compound according to claim 1 wherein,
X is NR⁸;
R⁸ is cyano;
R¹ is selected from the group consisting of heterocycle and heterocyclealkyl;
R³ is selected from the group consisting of aryl and arylalkyl; and
R⁵ is hydrogen.

8. A compound according to claim 7 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; and
R⁷ is hydrogen.

9. A compound according to claim 1 wherein,
X is NR⁸;
R⁸ is cyano;
R¹ is heterocyclealkyl wherein the heterocycle portion of heterocyclealkyl is pyridine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl; and
R⁵ is hydrogen.

10. A compound according to claim 9 selected from the group consisting of
4-chloro-N-[1-({(cyanoimino)[(3-pyridinylmethyl)amino]methyl} amino)-2,2-dimethylpropyl]benzamide;
4-chloro-N-[1-({(cyanoimino)[(4-pyridinylmethyl)amino]methyl} amino)-2,2-dimethylpropyl]benzamide; and
4-chloro-N-[1-({(cyanoimino)[(2-pyridinylmethyl)amino]methyl} amino)-2,2-dimethylpropyl]benzamide.

11. A compound according to claim 1 wherein,
X is NR⁸;
R⁸ is cyano;
R¹ is heterocycle wherein heterocycle is selected from the group consisting of quinoline and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

12. A compound according to claim 11 that is selected from the group consisting of
4-methyl-N-(2,2,2-trichloro-1- {[(cyanoimino)(5-pyrimidinylamino) methyl]amino}ethyl)benzamide and
4-chloro-N-(1- {[(cyanoimino)(3-quinolinylamino)methyl]amino}-2,2-dimethylpropyl)benzamide.

13. A compound according to claim 1 wherein,
X is NR⁸;
R⁸ is cyano;
R¹ is heterocycle wherein heterocycle is pyridine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

14. A compound according to claim 13 selected from the group consisting of
N-(1-{[(cyanoimino)(3-pyridinylamino)methyl]amino}-2,2-dimethylpropyl)-4-methylbenzamide;
4-chloro-N- (1-{([(cyanoimino)(3-pyridinylamino)methyl]amino} -2,2-dimethylpropyl)benzamide;
4-chloro-N-[{[(cyanoimino)(3-pyridinylamino)methyl]amino} (cyclopropyl)methyl]benzamide;
N-(1-{[[(6-chloro-3-pyridinyl)amino](cyanoimino)methyl]amino}-2,2-dimethylpropyl)-4-methylbenzamide;
(-)-N-(1-{[(cyanoimino)(3-pyridinylamino)methyl]amino}-2,2-dimethylpropyl)-4-methylbenzamide;
(+)-N-(1-{[(cyanoimino)(3-pyridinylamino)methyl]amino}-2,2-dimethylpropyl)-4-methylbenzamide;
4-chloro-N-(1-{[(cyanoimino)(3-pyridinylamino)methyl]amino}-2-ethylbutyl)benzamide;
4-chloro-N-(1-{[(cyanoimino)(3-pyridinylamino)methyl]amino}-3-methylbutyl)benzamide;
4-chloro-N-[{[(cyanoimino)(3-pyridinylamino)methyl]amino} (cyclohexyl)methyl]benzamide;
4-chloro-N-(1-{[(cyanoimino)(3-pyridinylamino)methyl]amino}-3,3-dimethylbutyl)benzamide;
4-chloro-N-(1-{ [(cyanoimino)(3-pyridinylamino)methyl]amino}-2-methylpropyl)benzamide;
4-chloro-N-(4-cyano-1-{[(cyanoimino)(3-pyridinylamino)methyl] amino}-2,2-diethylbutyl)benzamide;
4-chloro-N-[1-{[(cyanoimino)(3-pyridinylamino)methyl]amino}-2-(2,6,6-trimethyl-1-cyclohexen-1-yl)ethyl]benzamide;
4-chloro-N-(1-{[(cyanoimino)(3-pyridinylamino)methyl]amino}-2,2-dimethyl-4-pentenyl)benzamide;
4-chloro-N-(1-{[(cyanoimino)(3-pyridinylamino)methyl]amino} propyl)benzamide;
4-chloro-N-({[(cyanoimino)(3-pyridinylamino)methyl]amino}methyl) benzamide;
(-) 4-chloro-N-(4-cyano-1-{[(cyanoimino)(3-pyridinylamino)methyl] amino} -2,2-diethylbutyl)benzamide;
(+) 4-chloro-N-(4-cyano-1-{[(cyanoimino)(3-pyridinylamino)methyl] amino}-2,2-diethylbutyl)benzamide;
(+) 4-chloro-N-[1-{[(cyanoimino)(3-pyridinylamino)methyl]amino}-2-(2,6,6-trimethyl-1-cyclohexen-1-yl)ethyl]benzamide;
(-) 4-chloro-N-[1-{[(cyanoimino)(3-pyridinylamino)methyl]amino}-2-(2,6,6-trimethyl-1-cyclohexen-1-yl)ethyl]benzamide;
(-) 4-chloro-N-(1-{[(cyanoimino)(3-pyridinylamino)methyl]amino}-2,2-dimethyl-4-pentenyl)benzamide;
(+) 4-chloro-N-(1- {[(cyanoimino)(3-pyridinylamino)methyl]amino}-2,2-dimethyl-4-pentenyl)benzamide;
4-chloro-N-(1-{ [(cyanoimino)(3-pyridinylamino)methyl]amino}-3,3-dimethyl-4-pentenyl)benzamide;
4-chloro-N-(1-{[(cyanoimino)(3-pyridinylamino)methyl]amino}-2-cyclohexyl-2-methylpropyl)benzamide;
4-chloro-N-(1-{[(cyanoimino)(3-pyridinylamino)methyl]amino}-2,2-dimethylhexyl)benzamide;
N-(2-(1-adamantyl)-1-{[(cyanoimino)(3-pyridinylamino)methyl] amino }ethyl)-4-chlorobenzamide;
N-(2,2-bis[(allyloxy)methyl]-1-{[(cyanoimino)(3-pyridinylamino) methyl] amino }butyl)-4-chlorobenzamide;
4-chloro-N-[1-{[(cyanoimino)(3-pyridinylamino)methyl]amino}-3-(dimethylamino)-2,2-dimethylpropyl]benzamide;
tert-butyl (2R)-2-((R)-[(4-chlorobenzoyl)amino]{[(cyanoimino)(3-pyridinylamino)methyl]amino}methyl)-1-pyrrolidinecarboxylate;
4-chloro-N-[1-{[(cyanoimino)(3-pyridinylamino)methyl]amino}-3-(methylsulfanyl)propyl]benzamide;
N-(1-adamantyl { [(cyanoimino)(3-pyridinylamino)methyl]amino} methyl)-4-chlorobenzamide;
4-chloro-N-[{[(cyanoimino)(3-pyridinylamino)methyl]amino}(5-ethyl-1,3-dioxan-5-yl)methyl]benzamide;
4-chloro-N-(1-{[(cyanoimino)(3-pyridinylamino)methyl]amino}-2,2-dimethyl-3-phenylpropyl)benzamide;
N-(1-{[(cyanoimino)(3-pyridinylamino)methyl]amino}-2,2-dimethylpropyl)-4-iodobenzamide;
N-(1-{[(cyanoimino)(3-pyridinylamino)methyl]amino}-2,2-dimethylpropyl)-4-(2-furyl)benzamide;
4-bromo-N-(1-{[(cyanoimino)(3-pyridinylamino)methyl]amino}-2,2-dimethylpropyl)benzamide;
4-chloro-N-(1-{[(cyanoimino)(3-pyridinylamino)methyl]amino} -2,2-dimethylpropyl)-2-fluorobenzamide;
N-(1-{([(cyanoimino)(3-pyridinylamino)methyl]amino)-2,2-dimethylpropyl)-4-fluorobenzamide;
N-(1-{[(cyanoimino)(3-pyridinylamino)methyl]amino}-2,2-dimethylpropyl)-3-methylbenzamide;
N-(1-{[(cyanoimino)(3-pyridinylamino)methyl]amino}-2,2-dimethylpropyl)-2-methylbenzamide; and
N-(1-{[(cyanoimino)(3-pyridinylamino)methyl]amino}-2,2-dimethylpropyl)-3,5-difluorobenzamide.

15. A compound according to claim 1 wherein,
X is NR⁸;
R⁸ is cyano;
R¹ is heterocycle;
R² is haloalkyl;
R³ is aryl;
R⁵ is hydrogen; and
R⁷ is hydrogen.

16. A compound according to claim 1 wherein,
X is NR⁸;
R⁸ is cyano;
R¹ is heterocycle wherein heterocycle is pyridine;
R² is haloalkyl;
R³ is aryl wherein aryl is phenyl;
R⁵ is hydrogen; and
R⁷ is hydrogen.

17. A compound according to claim 16 selected from the group consisting of
4-methyl-N-(2,2,2-trichloro-1- {[(cyanoimino)(3-pyridinylamino) methyl] amino} ethyl)benzamide;
4-chloro-N-(2,2,2-trichloro-1- {[(cyanoimino)(3-pyridinylamino) methyl] amino }ethyl)benzamide;
N-(2,2,2-trichloro-1- [(cyanoimino)(3-pyridinylamino)methyl] amino} ethyl)benzamide;
2-methyl-N-(2,2,2-trichloro-1- {[(cyanoimino)(3-pyridinylamino) methyl]amino} ethyl)benzamide;
4-chloro-N-(1- {[(cyanoimino)(3-pyridinylamino)methyl]amino}-2,2,2-trifluoroethyl)benzamide;
(-) 4-chloro-N-(2,2,2-trichloro-1-{[(cyanoimino)(3-pyridinylamino) methyl]amino}ethyl)benzamide;
(+) 4-chloro-N-(2,2,2-trichloro-1-{[(cyanoimino)(3-pyridinylamino) methyl]amino}ethyl)benzamide;
4-iodo-N-(2,2,2-trichloro-1-{[(cyanoimino)(3-pyridinylamino) methyl]amino}ethyl)benzamide;
4-chloro-N-(2,2-dichloro-1-{[(cyanoimino)(3-pyridinylamino) methyl]amino}pentyl)benzamide;
4-chloro-N-(2,2-dichloro-1-{[(cyanoimino)(3-pyridinylamino) methyl]amino}propyl)benzamide;
(-) 4-chloro-N-(2,2-dichloro-1-{[(cyanoimino)(3-pyridinylamino) methyl]amino}propyl)benzamide;
(+) 4-chloro-N-(2,2-dichloro-1-{[(cyanoimino)(3-pyridinylamino) methyl]amino}propyl)benzamide;
3-chloro-N-(2,2-dichloro-1-{[(cyanoimino)(3-pyridinylamino) methyl]amino }propyl)benzamide;
N-(2,2-dichloro-1-{[(cyanoimino)(3-pyridinylamino)methyl]amino} propyl)-3,5-difluorobenzamide;
4-chloro-N-(1-{[(cyanoimino)(3-pyridinylamino)methyl]amino}-2,2,3,3,3-pentafluoropropyl)benzamide; and
3-chloro-N-(1- {[(cyanoimino)(3-pyridinylamino)methyl]amino}-2,2,3,3,3-pentafluoropropyl)benzamide.

18. A compound according to claim 1 wherein,
X is NR⁸;
R⁸ is cyano;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is alkyl; and
R⁷ is hydrogen.

19. A compound according to claim 18 that is 4-chloro-N-{1-[[(cyanoimino)(3-pyridinylamino)methyl](methyl)amino]-2,2-dimethylpropyl} benzamide.

20. A compound according to claim 1 wherein,
X is NR⁸;
R⁸ is cyano;
R¹ is selected from the group consisting of heterocycle and heterocyclealkyl;
R³ is alkyl; and
R⁵ is hydrogen.

21. A compound according to claim 1 wherein,
X is NR⁸;
R⁸ is cyano;
R¹ is selected from the group consisting of aryl and arylalkyl;
R³ is selected from the group consisting of heterocycle and heterocyclealkyl; and
R⁵ is hydrogen.

22. A compound according to claim 1 wherein,
X is NR⁸;
R⁸ is cyano;
R¹ is selected from the group consisting of aryl and arylalkyl;
R³ is selected from the group consisting of aryl and arylalkyl; and
R⁵ is hydrogen.

23. A compound according to claim 22 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl;
and
R⁷ is hydrogen.

24. A compound according to claim 1 wherein,
X is NR⁸;
R⁸ is cyano;
R¹ is arylalkyl wherein the aryl portion of arylalkyl is phenyl;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

25. A compound according to claim 24 selected from the group consisting of
4-chloro-N-(1 - {[[(2-chlorobenzyl)amino](cyanoimino)methyl] amino}-2,2-dimethylpropyl)benzamide;
4-chloro-N-(1-{[[(3-chlorobenzyl)amino](cyanoimino)methyl] amino}-2,2-dimethylpropyl)benzamide; and
4-chloro-N-(1-{[[(4-chlorobenzyl)amino](cyanoimino)methyl] amino}-2,2-dimethylpropyl)benzamide.

26. A compound according to claim 1 wherein,
X is NR⁸;
R⁸ is cyano;
R¹ is aryl wherein aryl is phenyl;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

27. A compound according to claim 26 that is selected from the group consisting of
N-(1-{[anilino(cyanoimino)methyl]amino}-2,2,2-trichloroethyl)-4-methylbenzamide;
4-methyl-N-(2,2,2-trichloro-1-{ [(cyanoimino)(2-fluoroanilino) methyl]amino} ethyl)benzamide;
N-(1-{ [(cyanoimino)(3-fluoroanilino)methyl]amino}-2,2-dimethylpropyl)-4-methylbenzamide;
4-chloro-N-[{[(cyanoimino)(3 -fluoroanilino)methyl] amino } (3-thienyl)methyl]benzamide;
4-chloro-N-(1-{[(cyanoimino)(2-fluoroanilino)methyl]amino -2,2-dimethylpropyl)benzamide;
4-chloro-N-(1-{ [(cyanoimino)(3-fluoroanilino)methyl]amino}-2,2-dimethylpropyl)benzamide;
4-chloro-N-[ 1-({(cyanoimino)[3-(trifluoromethyl)anilino]methyl} amino)-2,2-dimethylpropyl]benzamide;
4-chloro-N-(1-{[(cyanoimino)(3,5-difluoroanilino)methyl]amino}-2,2-dimethylpropyl)benzamide;
4-chloro-N-(1-{[(cyanoimino)(2,5-difluoroanilino)methyl]amino}-2,2-dimethylpropyl)benzamide;
4-chloro-N-( 1- {[(cyanoimino)(2,6-difluoroanilino)methyl]amino}-2,2-dimethylpropyl)benzamide;
4-chloro-N-(1-([(cyanoimino)(3-chloroanilino)methyl]amino)-2,2-dimethylpropyl)benzamide; and
4-chloro-N-(1-{ [(cyanoimino)(3-methoxyanilino)methyl]amino}-2,2-dimethylpropyl)benzamide.

28. A compound according to claim 1 wherein,
X is NR⁸;
R⁸ is cyano;
R¹ is selected from the group consisting of aryl and arylalkyl;
R³ is alkyl; and
R⁵ is hydrogen.

29. A compound according to claim 1 wherein,
X is NR⁸;
R⁸ is selected from the group consisting of hydrogen, alkoxy, alkyl, alkylsulfonyl, arylalkoxy, aryloxy, arylsulfonyl, haloalkylsulfonyl, heterocyclealkoxy, heterocycleoxy, hydroxy, nitro, and sulfamyl; and
R⁵ is hydrogen.

30. A compound according to claim 1 wherein,
X is NR⁸;
R⁸ is nitro; and
R⁵ is hydrogen.

31. A compound according to claim 1 wherein,
X is NR⁸;
R⁸ is nitro;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl; and
R⁵ is hydrogen.

32. A compound according to claim 31 selected from the group consisting of
4-chloro-N-(1-{[(nitroimino)(3-pyridinylamino)methyl]amino}-2,2-dimethylpropyl)benzamide;
4-chloro-N-(1-{[(nitroimino)(3-pyridinylamino)methyl]amino }-3,3-dimethylbutyl)benzamide;
(+) 4-chloro-N-(1-{ [(nitroimino)(3-pyridinylamino)methyl]amino}-3,3-dimethylbutyl)benzamide;
(-) 4-chloro-N-(1-{[(nitroimino)(3-pyridinylamino)methyl]amino}-3,3-dimethylbutyl)benzamide;
4-chloro-N-(1-{ [(nitroimino)(3-pyridinylamino)methyl]amino -2,2-dimethyl-4-pentenyl)benzamide;
4-chloro-N-(1- {[(nitroimino)(3-pyridinylamino)methyl]amino}-2,2-dimethyl-3-phenylpropyl)benzamide;
4-chloro-N-[1-{ [(nitroimino)(3-pyridinylamino)methyl]amino }-2-(2,6,6-trimethyl-1-cyclohexen-1-yl)ethyl]benzamide;
4-chloro-N-(1-{[(nitroimino)(3-pyridinylamino)methyl]amino}-2-cyclohexyl-2-methylpropyl)benzamide;
N-(2,2-bis[(allyloxy)methyl]-1-{[(nitroimino)(3-pyridinylamino) methyl] amino } butyl)-4-chlorobenzamide;
4-chloro-N-(4-cyano-1-{[(nitroimino)(3-pyridinylamino)methyl] amino}-2,2-diethylbutyl)benzamide;
4-chloro-N-(1-{[(nitroimino)(3-pyridinylamino)methyl]amino}-3,3-dimethyl-4-pentenyl)benzamide;
N-(2-(1-adamantyl)-1-{[(nitroimino)(3-pyridinylamino)methyl] amino} ethyl)-4-chlorobenzamide;
N-(1-{ [(nitroimino)(3-pyridinylamino)methyl]amino}-2,2-dimethylpropyl)-4-phenylbenzamide;
4-chloro-N-(2,2-dichloro-1-{[(nitroimino)(3-pyridinylamino)methyl] amino }pentyl)benzamide;
4-chloro-N-(2,2-dichloro-1-{ [(nitroimino)(3-pyridinylamino)methyl] amino }propyl)benzamide; and
3-chloro-N-(2,2-dichloro-1-{[(nitroimino)(3-pyridinylamino)methyl] amino} propyl)benzamide.

33. A compound according to claim 1 wherein,
X is NR⁸;
R⁸ is selected from the group consisting of arylsulfonyl, haloalkylsulfonyl and sulfamyl; and
R⁵ is hydrogen.

34. A compound according to claim 1 wherein,
X is NR⁸;
R⁸ is selected from the group consisting of arylsulfonyl wherein the aryl portion of arylsulfonyl is phenyl, haloalkylsulfonyl and sulfamyl;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl; and
R⁵ is hydrogen.

35. A compound according to claim 34 selected from the group consisting of
4-chloro-N-(2,2-dimethyl-1-{[[(phenylsulfonyl)imino](3-pyridinylamino)methyl]amino}propyl)benzamide;
4-chloro-N-(3,3-dimethyl-1-{[[(phenylsulfonyl)imino](3-pyridinylamino)methyl]amino}butyl)benzamide;
4-chloro-N-{2,2-dimethyl-1-[((3-pyridinylamino) {[(trifluoromethyl) sulfonyl]imino}methyl)amino]propyl}benzamide;
4-chloro-N- 3,3-dimethyl-1-[((3-pyridinylamino) {[(trifluoromethyl) sulfonyl]imino}methyl)amino]butyl}benzamide;
N-(1-{ [[(aminosulfonyl)imino](3-pyridinylamino)methyl]amino}-2,2-dimethylpropyl)-4-chlorobenzamide;
N-(1-{[[(aminosulfonyl)imino](3-pyridinylamino)methyl]amino}-3,3-dimethylbutyl)-4-chlorobenzamide;
4-chloro-N-(1-{[{[(dimethylamino)sulfonyl]imino}(3-pyridinylamino)methyl]amino}-2,2-dimethylpropyl)benzamide; and
4-chloro-N-(1-{[{[(dimethylamino)sulfonyl]imino) (3-pyridinylamino)methyl]amino}-3,3-dimethylbutyl)benzamide.

36. A compound according to claim 1 wherein,
X is S; and
R⁵ is hydrogen.

37. A compound according to claim 1 wherein,
X is S;
R¹ is selected from the group consisting of heterocycle and heterocyclealkyl;
R³ is selected from the group consisting of aryl and arylalkyl; and
R⁵ is hydrogen.

38. A compound according to claim 37 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl;
and
R⁷ is hydrogen.

39. A compound according to claim 1 wherein,
X is S;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

40. A compound according to claim 39 that is selected from the group consisting of
N-(2,2-dimethyl-1-{[(3-pyridinylamino)carbothioyl]amino}propyl)-4-methylbenzamide;
N-((1R)-2,2-dimethyl-1-{[(3-pyridinylamino)carbothioyl]amino} propyl)-4-methylbenzamide;
N-((1S)-2,2-dimethyl-1-{ [(3-pyridinylamino)carbothioyl]amino} propyl)-4-methylbenzamide;
N-(2,2-dimethyl-1-{[(3-pyridinylamino)carbothioyl]amino}propyl)-2-methylbenzamide;
4-chloro-N-(2,2-dimethyl-1-{[(3-pyridinylamino)carbothioyl]amino} propyl)benzamide;
N-(2,2-dimethyl-1-{[(3-pyridinylamino)carbothioyl]amino}propyl) benzamide;
4-methyl-N-(phenyl {[(3-pyridinylamino)carbothioyl]amino} methyl) benzamide;
4-methyl-N-(2-methyl-1-{[(3-pyridinylamino)carbothioyl]amino} propyl)benzamide; and
4-methyl-N-((1R,2S)-2-methyl-1-{[(3-pyridinylamino)carbothioyl] amino}butyl)benzamide.

41. A compound according to claim 1 wherein,
X is S;
**R**¹ is heterocycle;
R² is haloalkyl;
**R**³ is aryl;
**R**¹ is hydrogen; and
**R**⁷ is hydrogen.

42. A compound according to claim 1 wherein,
X is S;
R¹ is heterocycle wherein heterocycle is pyridine;
R² is haloalkyl;
R³ is aryl wherein aryl is phenyl;
R⁵ is hydrogen; and
R⁷ is hydrogen.

43. A compound according to claim 42 that is selected from the group consisting of
4-methyl-N-(2,2,2-trifluoro-1-{[(3-pyridinylamino)carbothioyl] amino}ethyl)benzamide;
N-[1-({[(6-chloro-3-pyridinyl)amino]carbothioyl}amino)-2,2,2-trifluoroethyl]-4-methylbenzamide;
4-chloro-N-(2,2,2-trifluoro-1-{[(3-pyridinylamino)carbothioyl] amino}ethyl)benzamide; and
4-iodo-N-(2,2,2-trichloro-1-{[(3-pyridinylamino)carbothioyl]amino} ethyl)benzamide.

44. A compound according to claim 1 wherein,
X is S;
R¹ is selected from the group consisting of aryl and arylalkyl;
R³ is selected from the group consisting of aryl and arylalkyl; and
R⁵ is hydrogen.

45. A compound according to claim 44 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl;
and
R⁷ is hydrogen.

46. A compound according to claim 1 wherein,
X is S;
R¹ is aryl wherein aryl is phenyl;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

47. A compound according to claim 46 that is selected from the group consisting of
4-methyl-N- {2,2,2-trifluoro-1-[(2-toluidinocarbothioyl)amino]ethyl} benzamide;
4-methyl-N-(2,2,2-trifluoro-1-{[(4-fluoroanilino)carbothioyl]amino} ethyl)benzamide;
4-methyl-N-(2,2,2-trifluoro-1-{ [(3-nitroanilino)carbothioyl]amino} ethyl)benzamide;
4-methyl-N-[2,2,2-trifluoro-1-({[2-fluoro-3-(trifluoromethyl)anilino] carbothioyl}amino)ethyl]benzamide;
4-methyl-N-(2,2,2-trifluoro-1- {[(4-methoxyanilino)carbothioyl] amino} ethyl)benzamide;
4-methyl-N-(2,2,2-trifluoro-1-{ [(2-methoxyanilino)carbothioyl] amino} ethyl)benzamide;
N- {1-[(anilinocarbothioyl)amino] -2,2,2-trifluoroethyl} -4-methylbenzamide;
4-methyl-N-{2,2,2-trifluoro-1-[(4-toluidinocarbothioyl)amino]ethyl} benzamide;
4-methyl-N-(2,2,2-trifluoro-1-{ ([(2-fluoroanilino)carbothioyl]amino)} ethyl)benzamide;
4-methyl-N-(2,2,2-trifluoro-1-{ [(3-methoxyanilino)carbothioyl] amino }ethyl)benzamide;
4-methyl-N-(2,2,2-trifluoro-1-{ ([(3-fluoroanilino)carbothioyl]amino} ethyl)benzamide;
N-(1-{([(2,5-difluoroanilino)carbothioyl] amino} -2,2,2-trifluoroethyl)-4-methylbenzamide;
N-(1-{[(2,4-difluoroanilino)carbothioyl]amino }-2,2,2-trifluoroethyl)-4-methylbenzamide;
4-methyl-N-{2,2,2-trifluoro-1-[(3-toluidinocarbothioyl)amino]ethyl} benzamide;
N-(1-{[(2,6-difluoroanilino)carbothioyl]amino}-2,2,2-trifluoroethyl)-4-methylbenzamide;
N-(1-{[(2,3-difluoroanilino)carbothioyl]amino}-2,2,2-trifluoroethyl)-4-methylbenzamide;
N-{1-[(anilinocarbothioyl)amino]-2,2,2-trifluoroethyl}-4-chlorobenzamide;
4-chloro-N-(2,2,2-trifluoro-1-{[(2-fluoroanilino)carbothioyl]amino} ethyl)benzamide;
N-(2,2-dimethyl-1-{ [(3-nitroanilino)carbothioyl]amino}propyl)-4-methylbenzamide;
4-methyl-N-(1- {[(3-nitroanilino)carbothioyl]amino}ethyl)benzamide;
4-methyl-N-(1- {[(3-nitroanilino)carbothioyl]amino} -2-phenylethyl) benzamide;
N-((1R)-2-(tert-butoxy)-1-{[(3-nitroanilino)carbothioyl]amino} ethyl)-4-methylbenzamide;
N-(2-fluoro-1-{([(3-nitroanilino)carbothioyl]amino) ethyl)-4-methylbenzamide;
and
4-methyl-N-[{[(3-nitroanilino)carbothioyl]amino} (phenyl)methyl] benzamide.

48. A compound according to claim 1 wherein,
X is S;
R¹ is aryl wherein aryl is phenyl;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle;
R³ is aryl wherein aryl is phenyl;
R⁵ is hydrogen; and
R⁷ is hydrogen.

49. A compound according to claim 48 that is 3-phenyl-N-(2,2,2-trichloro-1-{([(3-nitroanilino)carbothioyl]amino}ethyl)propanamide.

50. A compound according to claim 1 wherein,
X is O;
R¹ is selected from the group consisting of heterocycle and heterocyclealkyl;
R³ is selected from the group consisting of aryl and arylalkyl; and
R⁵ is hydrogen.

51. A compound according to claim 50 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl;
and
R⁷ is hydrogen.

52. A compound according to claim 1 wherein,
X is O;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

53. A compound according to claim 1 wherein,
X is O;
R¹ is heterocycle;
R² is haloalkyl;
R³ is aryl;
R⁵ is hydrogen; and
R⁷ is hydrogen.

54. A compound according to claim 1 wherein,
X is O;
R¹ is heterocycle wherein heterocycle is pyridine;
R² is haloalkyl;
R³ is aryl wherein aryl is phenyl;
R⁵ is hydrogen; and
R⁷ is hydrogen.

55. A compound according to claim 54 that is selected from the group consisting of
4-methyl-N-(2,2,2-trichloro-1-{[(3-pyridinylamino)carbonyl]amino} ethyl)benzamide;
2-methyl-N-(2,2,2-trichloro-1-{([(3-pyridinylamino)carbonyl]amino} ethyl)benzamide;
N-(2,2,2-trichloro-1-{[(3-pyridinylamino)carbonyl]amino}ethyl) benzamide;and
4-chloro-N-(2,2,2-trichloro-1-{[(3-pyridinylamino)carbonyl]amino} ethyl)benzamide.

56. A compound according to claim 1 wherein,
X is O;
R¹ is selected from the group consisting of heterocycle and heterocyclealkyl;
R³ is selected from the group consisting of heterocycle, and heterocyclealkyl; and
R⁵ is hydrogen.

57. A compound according to claim 1 wherein,
X is O;
R¹ is selected from the group consisting of aryl and arylalkyl;
R³ is selected from the group consisting of aryl and arylalkyl; and
R⁵ is hydrogen.

58. A compound according to claim 57 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl; and
R⁷ is hydrogen.

59. A compound according to claim 1 wherein,
X is O;
R¹ is aryl wherein aryl is phenyl;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
R³ is aryl wherein aryl is phenyl; and
R⁵ is hydrogen.

60. A compound according to claim 59 that is 4-chloro-N-(1-{[(2-fluoroanilino)carbonyl] amino} -2,2-dimethylpropyl)benzamide.

61. A compound according to claim 1 wherein,
X is selected from the group consisting of CHCN and CHNO₂;
R¹ is selected from the group consisting of heterocycle and heterocyclealkyl;
R³ is selected from the group consisting of heterocycle and heterocyclealkyl; and
R⁵ is hydrogen.

62. A compound according to claim 1 wherein,
X is selected from the group consisting of CHCN and CHNO₂;
R¹ is selected from the group consisting of heterocycle and heterocyclealkyl ;
R³ is selected from the group consisting of aryl and arylalkyl; and
R⁵ is hydrogen.

63. A compound according to claim 62 wherein,
R² is selected from the group consisting of hydrogen, alkenyl, alkenyloxyalkyl, alkenyloxy(alkenyloxy)alkyl, alkoxyalkyl, alkyl, alkylthioalkyl, aryl, arylalkyl, cyanoalkyl, cycloalkenylalkyl, cycloalkyl, cycloalkylalkyl, haloalkyl, heterocycle and (NR⁹R¹⁰)alkyl;
and
R⁷ is hydrogen.

64. A compound according to claim 1 wherein,
X is selected from the group consisting of CHCN and CHNO₂;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, (NR⁹R¹⁰)alkyl and heterocycle wherein heterocycle is selected from the group consisting of 1,3-dioxane, pyrrolidine and thiophene;
R³ is aryl wherein aryl is phenyl;
R⁵ is hydrogen; and
R⁷ is hydrogen.

65. A compound according to claim 64 selected from the group consisting of
4-chloro-N-(2-ethyl-1- {[2-nitro-1-(3-pyridinylamino)ethenyl]amino} butyl)benzamide; and
4-chloro-N-(2,2-dimethyl-1-{[2-nitro-1-(3-pyridinylamino)ethenyl] amino}propyl)benzamide.

66. A compound according to claim 1 wherein,
X is selected from the group consisting of CHCN and CHNO₂;
R¹ is heterocycle;
R² is haloalkyl;
R³ is aryl;
R⁵ is hydrogen; and
R⁷ is hydrogen.

67. A compound according to claim 1 wherein,
X is selected from the group consisting of CHCN and CHNO₂;
R¹ is heterocycle wherein heterocycle is pyridine;
R² is haloalkyl;
R³ is aryl wherein aryl is phenyl;
R⁵ is hydrogen; and
R⁷ is hydrogen.

68. A compound according to claim 67 that is 4-chloro-N-(2,2-dichloro-1-{[2-nitro-1-(3-pyridinylamino)ethenyl]amino }pentyl)benzamide.

69. A compound according to claim 1 wherein,
X is selected from the group consisting of CHCN and CHNO₂;
R¹ is selected from the group consisting of heterocycle and heterocyclealkyl;
R³ is alkyl; and
R⁵ is hydrogen.

70. A compound according to claim 1 wherein,
X is selected from the group consisting of CHCN and CHNO₂;
R¹ is selected from the group consisting of aryl and arylalkyl;
R³ is selected from the group consisting of heterocycle and heterocyclealkyl; and
R⁵ is hydrogen.

71. A compound according to claim 1 wherein,
X is selected from the group consisting of CHCN and CHNO₂;
R¹ is selected from the group consisting of aryl and arylalkyl;
R³ is selected from the group consisting of aryl and arylalkyl; and
R⁵ is hydrogen.

72. A compound according to claim 1 wherein,
X is selected from the group consisting of CHCN and CHNO₂;
R¹ is selected from the group consisting of aryl and arylalkyl;
R³ is alkyl; and
R⁵ is hydrogen.

73. A compound according to claim 1 wherein,
X is C(CN)₂;
R¹ is selected from the group consisting of heterocycle and heterocyclealkyl;
R³ is selected from the group consisting of aryl and arylalkyl; and
R⁵ is hydrogen.

74. A compound according to claim 1 wherein,
X is C(CN)₂;
R¹ is heterocycle wherein heterocycle is selected from the group consisting of quinoline, pyridine and pyrimidine;
R² is selected from the group consisting of hydrogen, alkenyl, alkenyloxyalkyl, alkenyloxy(alkenyloxy)alkyl, alkoxyalkyl, alkyl, alkylthioalkyl, aryl wherein aryl is phenyl, arylalkyl wherein the aryl portion of arylalkyl is phenyl, cyanoalkyl, cycloalkenylalkyl, cycloalkyl, cycloalkylalkyl, haloalkyl, (NR⁹R¹⁰)alkyl and heterocycle wherein heterocycle is selected from the group consisting of 1,3-dioxane, pyrrolidine and thiophene;
**R**³ is aryl wherein aryl is phenyl; and
**R**⁵ is hydrogen.

75. A compound according to claim 74 that is 4-chloro-N-(1-{[2,2-dicyano-1-(3-pyridinylamino)vinyl]amino}-2,2-dimethylpropyl)benzamide.

76. A compound according to claim 1 of formula VI: 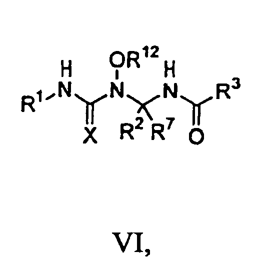 or a pharmaceutically acceptable salt thereof.

77. A compound according to claim 76 wherein,
X is NR⁸; and
R⁸ is cyano.

78. A compound according to claim 76 wherein,
X is NR⁸; and
R⁸ is selected from the group consisting of hydrogen, alkoxy, alkyl, alkylsulfonyl, arylalkoxy, aryloxy, arylsulfonyl, haloalkylsulfonyl, heterocyclealkoxy, hydroxy, nitro, and sulfamyl.

79. A compound according to claim 76 wherein X is S.

80. A compound according to claim 76 wherein X is O.

81. A compound according to claim 76 wherein X is selected from the group consisting of CHCN and CHNO₂.

82. A compound selected from the group consisting of
4-chloro-N-(1-{ [(hydroxyimino)(3-pyridinylamino)methyl]amino}-2,2-dimethylpropyl)benzamide;
4-chloro-N-(1-{[(methoxyimino)(3-pyridinylamino)methyl]amino}-2,2-dimethylpropyl)benzamide;
4-chloro-N-(1-{[{[(4-fluorobenzyl)oxy]imino}(3-pyridinylamino) methyl]amino}-2,2-dimethylpropyl)benzamide;
4-chloro-N-(2,2-dimethyl-1-{[[(methylsulfonyl)imino](3-pyridinylamino)methyl]amino}propyl)benzamide;
3-(4-chlorophenyl)-N-(1-{[(cyanoimino)(3 -pyridinylamino)methyl] amino}-2,2-dimethylpropyl)propanamide;
N-(1-{[(cyanoimino)(3-pyridinylamino)methyl]amino}-2,2-dimethylpropyl)-3-phenylpropanamide;
N-(1-{[(cyanoimino)(3-pyridinylamino)methyl]amino}-2,2-dimethylpropyl)-2-phenylacetamide;
4-(aminosulfonyl)-N-(1-{[(cyanoimino)(3-pyridinylamino)methyl] amino}-2,2-dimethylpropyl)-2-fluorobenzamide;
4-chloro-N-[1-({(cyanoimino)[(4-ethyl-3-pyridinyl)amino]methyl} amino)-2,2-dimethylpropyl]benzamide;
N-(1-{[(cyanoimino)(3-pyridinylamino)methyl]amino}-2,2-dimethylpropyl)-4-(trifluoromethoxy)benzamide;
4-chloro-N-[1-({(cyanoimino)[(4-ethyl-3-pyridinyl)amino]methyl} amino)-2,2-dimethylpropyl]-2-fluorobenzamide;
4-chloro-N-(1-{ [(cyanoimino)(5-pyrimidinylamino)methyl]amino }-2,2-dimethylpropyl)benzamide;
4-chloro-N-(1-{ [(cyanoimino)(5-pyrimidinylamino)methyl]amino}-2,2-dimethylpropyl)-2-fluorobenzamide;
N-(1-{[[(4-bromo-3-pyridinyl)amino](cyanoimino)methyl]amino}-2,2-dimethylpropyl)-4-chlorobenzamide;
4-chloro-2-fluoro-N-[2,2,2-trichloro-1-({(cyanoimino)[(4-ethyl-3-pyridinyl)amino]methyl}amino)ethyl]benzamide;
4-chloro-N-(2,2,2-trichloro-1-{[(cyanoimino)(5-pyrimidinylamino) methyl]amino}ethyl)benzamide;
4-chloro-2-fluoro-N-(2,2,2-trichloro-1- {[(cyanoimino)(5-pyrimidinylamino)methyl]amino}ethyl)benzamide;
N-(1-{[[(4-bromo-3-pyridinyl)amino](cyanoimino)methyl]amino}-2,2,2-trichloroethyl)-4-chlorobenzamide;
N-(1-{ [[(2-bromo-3-pyridinyl)amino](cyanoimino)methyl]amino}-2,2-dimethylpropyl)-4-chlorobenzamide;
4-chloro-N-[1-({(cyanoimino)[(2-ethyl-3-pyridinyl)amino]methyl} amino)-2,2-dimethylpropyl]benzamide;
N-(1-{[[(5-bromo-4-ethyl-3-pyridinyl)amino](cyanoimino)methyl] amino}-2,2-dimethylpropyl)-4-chlorobenzamide;
4-chloro-N-[1-({(cyanoimino)[(4,5-dibromo-3-pyridinyl)amino] methyl}amino)-2,2-dimethylpropyl]benzamide;
4-chloro-N-(1-{[[(5-chloro-3-pyridinyl)amino](cyanoimino)methyl] amino} -2,2-dimethylpropyl)benzamide;
N-(1-{[[(5-bromo-6-chloro-3-pyridinyl)amino](cyanoimino)methyl] amino}-2,2-dimethylpropyl)-4-chlorobenzamide;
N-(1-{[[(5-bromo-3-pyridinyl)amino](cyanoimino)methyl]amino}-2,2-dimethylpropyl)-4-chlorobenzamide;
N-(1-{[[(6-bromo-3-pyridinyl)amino](cyanoimino)methyl]amino}-2,2-dimethylpropyl)-4-chlorobenzamide;
4-chloro-N-(1-{[(cyanoimino)({ {S-[(4-fluorophenyl)sulfonyl]-3-pyridinyl}amino)methyl]amino}-2,2-dimethylpropyI)benzamide;
N-(1-{[({5-[(aminoperoxy)sulfanyl]-3-pyridinyl}amino)(cyanoimino) methyl]amino}-2,2-dimethylpropyl)-4-chlorobenzamide;
N-(1-{[[(6-bromo-4-fluoro-3-pyridinyl)amino](cyanoimino)methyl] amino}-2,2-dimethylpropyl)-4-chlorobenzamide;
4-chloro-N-[1-{[(cyanoimino)(3-pyridinylamino)methyl]amino}-2,2,2-trifluoro-1-(trifluoromethyl)ethyl]benzamide;
4-chloro-N-(1-{[(cyanoimino)(3-pyridinylamino)methyl]amino} cyclopentyl)benzamide;
4-chloro-N-(1-{[(cyanoimino)(3-pyridinylamino)methyl]amino} cyclohexyl)benzamide;
4-chloro-N-[{[(cyanoimino)(3-pyridinylamino)methyl]amino}(2,6-dimethylphenyl)methyl]benzamide;
4-chloro-N-[{[(cyanoimino)(3-pyridinylamino)methyl]amino}(3-pyridinyl)methyl]benzamide;
4-chloro-N-[{[(cyanoimino)(3-pyridinylamino)methyl]amino}(2-pyridinyl)methyl]benzamide;
4-chloro-N-(1-{[(cyanoimino)(3-pyridinylamino)methyl]amino}-2-methyl-2-phenylpropyl)benzamide;
4-chloro-N-(1-{[(cyanoimino)(3-pyridinylamino)methyl]amino}-3,3-dimethyl-2-oxobutyl)benzamide;
4-chloro-N-(1- {[(cyanoimino)(3-pyridinylamino)methyl]amino}-3,3,3-trifluoro-2-oxopropyl)benzamide;
4-chloro-N-[1-{[(cyanoimino)(3-pyridinylamino)methyl]amino}-3,3,3-trifluoro-2-methyl-2-(trifluoromethyl)propyl]benzamide;
methyl 4-[(4-chlorobenzoyl)amino]-4-{ [(cyanoimino)(3-pyridinylamino)methyl]amino} -3 ,3-dimethylbutanoate;
4-chloro-N-[1- {[(cyanoimino)(3-pyridinylamino)methyl]amino }-4-(dimethylamino)-2,2-dimethylbutyl]benzamide;
4-chloro-N-(4-cyano-1-{[(cyanoimino)(3-pyridinylamino)methyl] amino}-2,2-dimethylbutyl)benzamide;
4-chloro-N-(1-{[(cyanoimino)(3-pyridinylamino)methyl]amino}-4-methoxy-2,2-dimethylbutyl)benzamide;
4-chloro-N-(1-{[(cyanoimino)(3-pyridinylamino)methyl]amino}-4-hydroxy-2,2-dimethylbutyl)benzamide;
N-(4-(aminosulfonyl)-1-{[(cyanoimino)(3-pyridinylamino)methyl] amino}-2,2-dimethylbutyl)-4-chlorobenzamide;
4-chloro-N-[1-{[(cyanoimino)(3-pyridinylamino)methyl]amino-2,2-dimethyl-4-(phenylsulfonyl)butyl]benzamide;
4-chloro-N-(1-{[(cyanoimino)(3-pyridinylamino)methyl]amino} -3-hydroxy-2,2-dimethylpropyl)benzamide;
N-(4-(aminosulfonyl)-2,2-dichloro-1-{[(cyanoimino)(3-pyridinylamino)methyl]amino}butyl)-4-chlorobenzamide;
4-chloro-N-[4-cyano-1-{[(cyanoimino)(3-pyridinylamino)methyl] amino}-2,2-bis(trifluoromethyl)butyl]benzamide;
4-chloro-N-(1-{[(cyanoimino)(3-pyridinylamino)methyl]amino }-2,2-difluoro-4-oxopentyl)benzamide;
4-chloro-N-(1- {[2-cyano-1-(3-pyridinylamino)ethenyl]amino -2,2-dimethylpropyl)benzamide;
4-chloro-N-{1-[[(cyanoimino)(3-pyridinylamino)methyl](hydroxy) amino]-2,2-dimethylpropyl}benzamide;
4-chloro-N-(2,2,2-trichloro-1-{([2-nitro-1-(3-pyridinylamino)ethenyl] amino} ethyl)benzamide; and
4-chloro-N-(2,2,2-trichloro-1-{[2-cyano-1-(3-pyridinylamino) ethenyl]amino}ethyl)benzamide.

83. A pharmaceutical composition comprising a therapeutically effective amount of a compound having formula I: 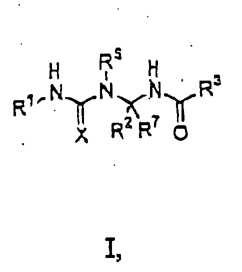 or a pharmaceutically acceptable salt thereof in combination with a pharmaceutically acceptable carrier, wherein
X is selected from the group consisting of O, S, CHCN, C(CN)₂, CHNO₂, and NR⁸;
R⁸ is selected from the group consisting of hydrogen, alkoxy, alkyl, alkylsulfonyl, arylalkoxy, aryloxy, arylsulfonyl, cyano, haloalkylsulfonyl, heterocyclealkoxy, heterocycleoxy, hydroxy, nitro, and sulfamyl;
R¹ is selected from the group consisting of aryl, arylalkyl, heterocycle, and heterocyclealkyl;
R² is selected from hydrogen, alkenyl, alkenyloxyalkyl, alkenyloxy (alkenyloxy)alkyl, alkoxyalkyl, alkoxycarbonyl, alkoxycarbonylalkyl, alkoxycarbonyl(halo)alkyl, alkoxy(halo)alkyl, alkyl, alkylcarbonyl, alkylcarbonylalkyl, alkylcarbonyl(halo)alkyl, alkylcarbonyloxyalkyl, alkylsulfinylalkyl, alkylsulfonylalkyl, alkylthioalkyl, alkynyl, amido, amidoalkyl, aryl, arylalkoxyakyl, arylalkoxycarbonyl, arylalkoxycarbonylalkyl, arylalkyl, arylcarbonyl, arylcarbanylalkyl, arylcarbonyloxyalkyl, aryl(halo)alkyl, aryloxyalkyl, aryloxycarbonyl, aryloxycarbonylalkyl, arylalkylthioalkyl, arylsulfonylalkyl, carboxy, carboxyalkyl, carboxy(halo)alkyl, cyanoalkyl, cyano(halo)alkyl, cycloalkenyl, cycloalkenylalkyl, cycloalkyl, cycloalkylalkoxyalkyl, cycloalkylalkyl, cycloalkylcarbonyl, cycloalkyloxyalkyl, cyclvalkylalltylthioalkyl, formyl, haloalkenyl, haloalkyl, haloalkylcarbonyl, haloalkynyl, heterocycle, heterocyclealkoxyalkyl, heterocyclealkyl, heterocyclecarbonyl, heterocycleoxyalkyl, heterocyclealkylthioalkyl, hydroxyalkyl, mercaptoalkyl, sulfamylalkyl, sulfamyl(halo)alkyl, and (NR⁹R¹⁰)alkyl wherein R⁹ and R¹⁰ are independently selected from hydrogen, alkyl, alkylcarbonyl, aryl, arylalkyl, arylcarbonyl, formyl, and S(O)₂R¹¹, wherein R¹¹ is selected from alkyl, aryl, and arylalkyl;
R³ is selected from the group consisting of alkyl, aryl, arylalkyl, heterocycle, and heterocyclealkyl;
R⁵ is selected from the group consisting of hydrogen, alkyl and OR¹²; R¹² is selected from the group consisting of hydrogen, alkyl and arylalkyl; and
R⁷ is selected from the group consisting of hydrogen, haloalkyl, and C₁-C₆ alkyl; or
R⁷ and R² taken together with the carbon atom to which they are attached, together form a 5 or 6 membered carbocyclic ring wherein the 5 or 6 membered carbocyclic ring is optionally substituted with 1 or 2 substituents independently selected from the group consisting of alkenyl, alkoxy, alkyl, alkynyl, halogen, haloalkoxy, and haloalkyl;
wherein unless otherwise specified alkyl by itself or as part of another group contains from one to ten carbon atoms;
alkenyl by itself or as part of another group contains from two to ten carbon atoms;
alkynyl by itself or as part of another group contains from two to ten carbon atoms;
aryl by itself or as part of another group is a monocyclic carbocyclic ring system or a bicyclic carbocyclic fused ring system having one or more aromatic rings,
aryl may optionally have 1, 2, 3, 4 or 5 substituents independently selected from the group consisting of alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, phenyl, furyl, -NR^{A}R^{B} and (NR^{A}R^{B})alkyl wherein R^{A} and R^{B} are independently selected from hydrogen, alkyl, alkylcarbonyl and formyl;
cycloalkyl is a monocyclic, bicyclic or tricyclic ring system;
cycloalkyl may optionally have 1, 2, 3, 4 or 5 substituents independently selected from the group consisting of alkenyl, alkoxy, alkoxycarbonyl, alkoxycarbonylalkyl, alkyl, alkylcarbonylalkyl, alkylcarbonyloxy, alkylcarbonyloxyalkyl, alkylsulfonylalkyl, alkynyl, amido, amidoalkyl, cyanoalkyl, halo, haloalkoxy, haloalkyl, hydroxy, hydroxyalkyl, sulfamylalkyl, -NR^{A}R^{B} and (NR^{A}R^{B})alkyl wherein R^{A} and R^{B} are independently selected from hydrogen, alkyl, alkylcarbonyl and formyl;
heterocycle by itself or as part of another group is a monocyclic or bicyclic ring system wherein (i) the monocyclic ring system is a 5- or 6-membered ring containing 1, 2, 3 or 4 heteroatoms independently selected from oxygen, nitrogen and sulfur wherein the 5-membered ring has from 0-2 double bonds and the 6-membered ring has from 0-3 double bonds and (ii) the bicyclic ring system is any of the above monocyclic ring systems fused to a monocyclic aryl group as defined above, a monocyclic cycloalkyl group as defined above or a monocyclic heterocyclic ring system as defined above;
heterocycle may optionally have 1, 2 or 3 substituents independently selected from the group consisting of alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, oxo, sulfamyl, sulfamylalkyl, -NR^{A}R^{B}, (NR^{A}R^{B})alkyl wherein R^{A} and R^{B} are independently selected from hydrogen, alkyl, alkylcarbonyl and formyl, and phenyl optionally having 1 or 2 substituents independently selected from alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkohycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, -NR^{A}R^{B}, (NR^{A}R^{B})alkyl wherein R^{A} and R^{B} are independently selected from hydrogen, alkyl, alkylcarbonyl and formyl;
amido by itself or as part of another group is -C(O)NR⁹R¹⁰ wherein R⁹ and R¹⁰ are independently selected from the group consisting of hydrogen, alkyl, alkylcarbonyl, phenyl, phenylalkyl, phenylcarbonyl, formyl and S(O)₂R¹¹ wherein R¹¹ is selected from alkyl, phenyl and phenylalkyl;
cycloalkenyl by itself or as part of another group is a cyclic hydrocarbon containing from three to eight carbons and containing at least one carbon-carbon double bond;
cycloalkenyl may optionally have 1, 2, 3, 4 or 5 substituents independently selected from alkenyl, alkoxy, alkoxycarbonyl, alkoxycarbonylalkyl, alkyl, alkylcarbonyloxy, alkylcarbonyloxyalkyl, alkynyl, amido, amidoalkyl, halo, haloalkoxy, haloalkyl, hydroxy, hydroxyalkyl, sulfamylalkyl, -NR^{A}R^{B} and (NR^{A}R^{B})alkyl wherein R^{A} and R^{B} are independently selected from hydrogen, alkyl, alkylcarbonyl and formyl;
sulfamyl is a -SO₂NR⁹⁴R⁹⁵ group wherein R⁹⁴ and R⁹⁵ are independently selected from hydrogen, alkyl, aryl and arylalkyl;
provided that when X is O; R² is -CCl₃; R³ is alkyl or phenyl; and R⁵ and R⁷ are hydrogen; then R¹ is other than phenyl;
further provided that
N-[[[(phenylamino)carbonyl]amino]methyl]-3-pyridinecarboxamide;
N-[[[[(2-methylphenyl)amino)carbonyl]amino]methyl]-3-pyridinecarboxamide;
N-[[[[(4-methylphenyl)amino)carbonyl]amino]methyl]-3-pyridinecarboxamide;
N-[[[[(3-ethoxyphenyl)amino)carbonyl]amino]methyl]-3-pyridinecarbohamide;
N-[[[[(4-nitrophenyl)amino)carbonyl]amino]methyl]-3-pyridiner-arboxamide; and
N-[[[[(3-methylphenyl)amino)carbonyl]amino]methyl]-3-pyridinecarboxamide
are excluded.

84. Use of a compound having formula I: 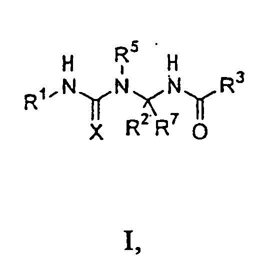 or a pharmaceutically acceptable salt thereof for the manufacture of a medicament for treating or preventing a disease which is prevented or ameliorated with potassium channel openers in a host mammal in need of such treatment by administration of a therapeutically effective amount,
wherein,
X is selected from the group consisting of O, S, CHCN, C(CN)₂, CHNO₂, and NR⁸;
R⁸ is selected from the group consisting of hydrogen, alkoxy, alkyl, alkylsulfonyl, arylalkoxy, aryloxy, arylsulfonyl, cyano, haloalkylsulfonyl, heterocyclealkoxy, heterocycleoxy, hydroxy, nitro, and sulfamyl;
R¹ is selected from the group consisting of aryl, arylalkyl, heterocycle, and heterocyclealkyl;
R² is selected from hydrogen, alkenyl, alkenyloxyalkyl, alkenyloxy (alkenyloxy)alkyl, alkoxyalkyl, alkoxycarbonyl, alkoxycarbonylalkyl, alkoxycarbonyl(halo)alkyl, alkoxy(halo)alkyl, alkyl, alkylcarbonyl, alkylcarbonylalkyl, alkylcarbonyl(halo)alkyl, alkylcarbonyloxyalkyl, alkylsulfinylalkyl, alkylsulfonylalkyl, alkylthioalkyl, alkynyl, amido, amidoalkyl, aryl, arylalkoxyalkyl, arylalkoxycarbonyl, arylalkoxycarbonylalkyl, arylalkyl, arylcarbonyl, arylcarbonylalkyl, arylcarbonyloxyalkyl, aryl(halo)alkyl, aryloxyalkyl, aryloxycarbonyl, aryloxycarbonylalkyl, arylalkylthioalkyl, arylsulfonylalkyl, carboxy, carboxyalkyl, carboxy(halo)alkyl, cyanoalkyl, cyano(halo)alkyl, cycloalkenyl, cycloalkenylalkyl, cycloalkyl, cycloalkylalkoxyalkyl, cycloalkylalkyl, cycloalkylcarbonyl, cycloalkyloxyalkyl, cycloalkylalkylthioalkyl, formyl, haloalkenyl, haloalkyl, haloalkylcarbonyl, haloalkynyl, heterocycle, heterocyclealkoxyalkyl, heterocyclealkyl, hoterocyclecarbonyl, heterocycleoxyalkyl, heterocyclealkylthioalkyl, hydroxyalkyl, mercaptoalkyl, sulfamylalkyl, sulfamyl(halo)allcyl, and (NR⁹R¹⁰)alkyl wherein R⁹ and R¹⁰ are independently selected from hydrogen, alkyl, alkylcarbonyl, aryl, arylalkyl, arylcarbonyl, formyl, and S(O)₂R¹¹, wherein R¹¹ is selected from alkyl, aryl, and arylalkyl;
R³ is selected from the group consisting of alkyl, aryl, arylalkyl, heterocycle, and heterocyclealkyl;
R⁵ is selected from the group consisting of hydrogen, alkyl and OR¹²; R¹² is selected from the group consisting of hydrogen, alkyl and arylalkyl; and
R⁷ is selected from the group consisting of hydrogen, haloalkyl, and C₁-C₆ alkyl; or
R⁷ and R² taken together with the carbon atom to which they are attached, together form a 5 or 6 membered carbocyclic ring wherein the 5 or 6 membered carbocyclic ring is optionally substituted with 1 or 2 substituents independently selected from the group consisting of alkenyl, alkoxy, alkyl, alkynyl, halogen, haloalkoxy, and haloalkyl;
wherein unless otherwise specified alkyl by itself or as part of another group contains from one to ten carbon atoms;
alkenyl by itself or as part of another group contains from two to ten carbon atoms;
alkynyl by itself or as part of another group contains from two to ten carbon atoms;
aryl by itself or as part of another group is a monocyclic carbocyclic ring system or a bicyclic carbocyclic fused ring system having one or more aromatic rings,
aryl may optionally have 1, 2, 3, 4 or 5 substituents independently selected from the group consisting of alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, sulfamyl, sulfamylalkyl, phenyl, furyl, -NR^{A}R^{B} and (NR^{A}R^{B})alkyl wherein R^{A} and R^{B} are independently selected from hydrogen, alkyl, alkylcarbonyl and formyl;
cycloalkyl is a monocyclic, bicyclic or tricyclic ring system;
cycloalkyl may optionally have 1, 2, 3, 4 or 5 substituents independently selected from the group consisting of alkenyl, alkoxy, alkoxycarbonyl, alkoxycarbonylalkyl, alkyl, alkylcarbonylalkyl, alkylcarbonyloxy, alkylcarbonyloxyalkyl, alkylsulfonylalkyl, alkynyl, amido, amidoalkyl, cyanoalkyl, halo, haloalkoxy, haloalkyl, hydroxy, hydroxyalkyl, sulfamylalkyl, -NR^{A}R^{B} and (NR^{A}R^{B})alkyl wherein R^{A} and R^{B} are independently selected from hydrogen, alkyl, alkylcarbonyl and formyl;
heterocycle by itself or as part of another group is a monocyclic or bicyclic ring system wherein (i) the monocyclic ring system is a 5- or 6-membered ring containing 1, 2, 3 or 4 heteroatoms independently selected from oxygen, nitrogen and sulfur wherein the 5-membered ring has from 0-2 double bonds and the 6-membered ring has from 0-3 double bonds and (ii) the bicyclic ring system is any of the above monocyclic ring systems fused to a monocyclic aryl group as defined above, a monocyclic cycloalkyl group as defined above or a monocyclic heterocyclic ring system as defined above;
heterocycle may optionally have 1, 2 or 3 substituents independently selected from the group consisting of alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycarbonylalkyl, phenylsulfonyl, cyano, halo, haloalkyl, haloalkoxy, nitro, oxo, sulfamyl, sulfamylalkyl, -NR^{A}R^{B}, (NR^{A}R^{B})alkyl wherein R^{A} and R^{B} are independently selected from hydrogen, alkyl, alkylcarbonyl and formyl, and phenyl optionally having 1 or 2 substituents independently selected from alkenyl, alkoxy, alkoxycarbonyl, alkoxycarbonylalkyl, alkyl, alkylcarbonyl, alkylcarbonyloxy, alkylcarbonyloxyalkyl, alkylsulfinyl, alkylsulfonyl, alkynyl, amido, amidoalkyl, phenylalkoxycarbonyl, phenylalkoxycarbonylalkyl, phenylcarbonyloxy, phenylcarbonyloxyalkyl, phenyloxycarbonyl, phenyloxycorbonylalkyl, phenylsulfonyl, cyano, halo, haloalkyl, haloalkoxy, nitro, sulfamyl, sulfamylalkyl, -NR^{A}R^{B}, (NR^{A}R^{B})alkyl wherein R^{A} and R^{B} are independently selected from hydrogen, alkyl, alkylcarbonyl and formyl;
amido by itself or as part of another group is -C(O)NR⁹R¹⁰ wherein R⁹ and R¹⁰ are independently selected from the group consisting of hydrogen, alkyl, alkylcarbonyl, phenyl, phenylalkyl, phenylcarbonyl, formyl and S(O) ₂R¹¹ wherein R¹¹ is selected from alkyl, phenyl and phenylalkyl;
cycloalkenyl by itself or as part of another group is a cyclic hydrocarbon containing from three to eight carbons and containing at least one carbon-carbon double bond;
cycloalkenyl may optionally have 1, 2, 3, 4 or 5 substituents independently selected from alkenyl, alkoxy, alkoxycarbonyl, alkoxycarbonylalkyl, alkyl, alkylcarbonyloxy, alkylcarbonyloxyalkyl, alkynyl, amido, amidoalkyl, halo, haloalkoxy, haloalkyl, hydroxy, hydroxyalkyl, sulfamylalkyl, -NR^{A}R^{B} and (NR^{A}R^{B})alkyl wherein R^{A} and R^{B} are independently selected from hydrogen, alkyl, alkylcarbonyl and formyl;
sulfamyl is a -SO₂NR⁹⁴R⁹⁵ group wherein R⁹⁴ and R⁹⁵ are independently selected from hydrogen, alkyl, aryl and arylalkyl;
provided that when X is O; R² is -CCl₃; R³ is alkyl or phenyl; and R⁵ and R⁷ are hydrogen; then R¹ is other than phenyl;
further provided that the use of
N-[[[(phenylamino)carbonyl]amino]methyl]-3-pyridinecarboxamide;
N-[[[[(2-methylphenyl)amino)carbonyl]amino]methyl]-3-pyridinecarboxamide;
N-[[[[(4-methylphenyl)amino)carbonyl]amino]methyl]-3 - pyridinecarboxamide;
N-[[[[(3-ethoxyphenyl)amino)carbonyl]amino]methyl]-3-pyridinecarboxamide;
N-[[[[(4-nitrophenyl)amino)carbonyl]amino]methyl]-3-pyridinecarboxamide; or
N-[[[[(3-methylphenyl)amino)carbonyl)amino]methyl]-3-pyridinecarboxamide
for the manufacture of a medicament for the treatment of Parkinson's disease is excluded.

85. The use of claim 84 wherein the disorder is selected from the group consisting of asthma, epilepsy, Raynaud's syndrome, intermittent claudication, migraine, pain, pollakiuria, bladder instability, nocturia, bladder hyperreflexia, enuresis, alopecia, cardioprotection, ischemia, eating disorders, functional bowel disorders and neurodegeneration.

86. The use of claim 84 wherein the disorder is bladder overactivity.

87. The use of claim 84 wherein the disorder is benign prostatic hyperplasia.

88. The use of claim 84 wherein the disorder is dysmenorrhea.

89. The use of claim 84 wherein the disorder is premature labor.

90. The use of claim 84 wherein the disorder is urinary incontinence.

91. The use of claim 84 wherein the disorder is selected from the group consisting of male erectile dysfunction and premature ejaculation.

92. The use of claim 84 wherein the disorder is female sexual dysfunction.

93. Use of a compound of formula VII: 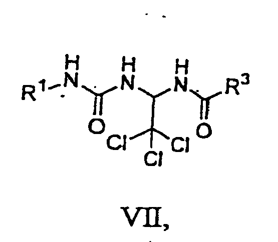 or a pharmaceutically acceptable salt thereof wherein,
R¹ is phenyl;
R³ is selected from the group consisting of alkyl and phenyl,
for the manufacture of a medicament for treating or preventing disease which is prevented or ameliorated with potassium channel openers in a host mammal in need of such treatment by administration of a therapeutically effective amount.

94. A compound according to claim 93 selected from the group consisting of
N-{1-[(anilinocarbonyl)amino]-2,2,2-trichloroethyl}-4-methylbenzamide and
4-methyl-N-(2,2,2-trichloro-1-{[(2-fluoroanilino)carbonyl]amino} ethyl)benzamide.

95. The use of claim 93 wherein the disorder is selected from the group consisting of asthma, epilepsy, Raynaud's syndrome, intermittent claudication, migraine, pain, pollakiuria, bladder instability, nocturia, bladder hyperreflexia, enuresis, alopecia, cardioprotection, ischemia, eating disorders, functional bowel disorders, and neurodegeneration.

96. The use of claim 93 wherein the disorder is bladder overactivity.

97. The use of claim 93 wherein the disorder is benign prostatic hyperplasia.

98. The use of claim 93 wherein the disorder is dysmenorrhea.

99. The use of claim 93 wherein the disorder is premature labor.

100. The use of claim 93 wherein the disorder is urinary incontinence.

101. The use of claim 93 wherein the disorder is selected from the group consisting of male erectile dysfunction and premature ejaculation.

102. The use of claim 93 wherein the disorder is female sexual dysfunction.

103. A compound of any one of claims 1-82 and 94 for use as a therapeutic agent, provided that
N-[[[[(2-methylphenyl)amino]carbonyl]amino]phenylmethyl]-benzamide;
N-[[[[(4-chlorophenyl)amino]carbonyl]amino]phenylmethyl]-benzamide;
N-[2,2-dichloro-1-[[phenylamino)thioxomethyl]amino]ethyl]-benzamide;
N-[2,2-dichloro-1-[[phenylamino)thioxomethyl]amino]ethyl]-4-methyl-benzamide;
N-[2,2-dichloro-1-[[[4-methylphenyl)amino)thioxomethyl]amino]ethyl]-benzamide;
N-[2,2-dichloro-1-[[[4-methylphenyl)amino)thioxomethyl]ammo]ethyl]-4-methyl-benzamide;
N-[2,2-dichloro-1-[[[4-chlorophenyl)amino)thioxomethyl] amino] ethyl]-benzamide;
N-[2,2-dichloro-1-[[[4-chlorophenyl)amino)thioxomethyl]amino]ethyl]-4-methyl-benzamide;
N-[[[(phenylamino)carbonyl]amino]methyl]-benzamide;
N-[[[(phenylamino)thioxomethyl]amino]methyl]-benzamide;
N-[[[(phenylamino)carbonyl]amino]methyl]-4-chloro-benzamide;
N-[[[(phenylamino)thioxomethyl]amino]methyl]-4-chloro-benzamide; 1-(1-acetamido-2,2,2-trichloroethyl)-3-phenyl-2-thio-urea;
1-phenyl-2-thio-3-(2,2,2-trichloro-1-propionamidoethyl)-urea; and 1-(1-butyramido-2,2,2-trichloroethyl)-3-phenyl-2-thio-urea are not excluded.

## Patentansprüche

1. Eine Verbindung mit der Formel I: 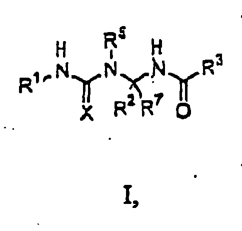 oder ein pharmazeutisch verträgliches Salz davon, worin
X gewählt ist aus der Gruppe bestehend aus O, S, CHCN, C(CN)₂, CHNO₂ und NR⁸;
R⁸ ist gewählt aus der Gruppe bestehend aus Wasserstoff, Alkoxy, Alkyl, Alkylsulfonyl, Arylalkoxy, Aryloxy, Arylsulfonyl, Cyano, Haloalkylsulfonyl, Heterocyclusalkoxy, Heterocyclusoxy, Hydroxy, Nitro und Sulfamyl;
R¹ ist gewählt aus der Gruppe bestehend aus Aryl, Arylalkyl, Heterocyclus und Heterocyclusalkyl;
R² ist gewählt aus Wasserstoff, Alkenyl, Alkenyloxyalkyl, Alkenyloxy (Alkenyloxy)alkyl, Alkoxyalkyl, Alkoxycarbonyl, Alkoxycarbonylalkyl, Alkoxycarbonyl(halo)alkyl, Alkoxy(halo)alkyl, Alkyl, Alkylcarbonyl, Alkylcarbonylalkyl, Alkylcarbonyl(halo)alkyl, Alkylcarbonyloxyalkyl, Alkylsulfinylalkyl, Alkylsulfonylalkyl, Alkylthioalkyl, Alkinyl, Amido, Amidoalkyl, Aryl, Arylalkoxyalkyl, Arylalkoxycarbonyl, Arylalkoxycarbonylalkyl, Arylalkyl, Arylcarbonyl, Arylcarbonylalkyl, Arylcarbonyloxyalkyl, Aryl(halo)alkyl, Aryloxyalkyl, Aryloxycarbonyl, Aryloxycarbonylalkyl, Arylalkylthioalkyl, Arylsulfonylalkyl, Carboxy, Carboxyalkyl, Carboxy(halo)alkyl, Cyanoalkyl, Cyano(halo)alkyl, Cycloalkenyl, Cycloalkenylalkyl, Cycloalkyl, Cycloalkylalkoxyalkyl, Cycloalkylalkyl, Cycloalkylcarbonyl, Cycloalkyloxyalkyl, Cycloalkylalkylthioalkyl, Formyl, Haloalkenyl, Haloalkyl, Haloalkylcarbonyl, Haloalkinyl, Heterocyclus, Heterocyclusalkoxyalkyl, Heterocyclusalkyl, Heterocycluscarbonyl, Heterocyclusoxyalkyl, Heterocyclusalkylthioalkyl, Hydroxyalkyl, Mercaptoalkyl, Sulfamylalkyl, Sulfamyl (halo) alkyl und (NR⁹R¹⁰) Alkyl, worin R⁹ und R¹⁰ unabhängig gewählt sind aus Wasserstoff, Alkyl, Alkylcarbonyl, Aryl, Arylalkyl, Arylcarbonyl, Formyl und S(O)₂R¹¹, worin R¹¹ gewählt ist aus Alkyl, Aryl und Arylalkyl;
R³ ist gewählt aus der Gruppe bestehend aus Alkyl, Aryl, Arylalkyl, Heterocyclus und Heterocyclusalkyl;
R⁵ ist gewählt aus der Gruppe bestehend aus Wasserstoff, Alkyl und OR¹², R¹² ist gewählt aus der Gruppe bestehend aus Wasserstoff, Alkyl und Arylalkyl; und
R⁷ ist gewählt aus der Gruppe bestehend aus Wasserstoff, Haloalkyl und C₁-C₆ Alkyl; oder
R⁷ und R² zusammengenommen mit dem Kohlenstoffatom, an welches sie gebunden sind, bilden zusammen einen 5- oder 6-gliedrigen carbocyclischen Ring, worin der 5- oder 6-gliedrige carbocyclische Ring wahlweise substituiert ist mit 1 oder 2 Substituenten unabhängig gewählt aus der Gruppe bestehend aus Alkenyl, Alkoxy, Alkyl, Alkinyl, Halogen, Haloalkoxy und Haloalkyl;
worin, wenn nichts anderes angegeben ist, Alkyl alleine oder als Teil einer anderen Gruppe, ein bis zehn Kohlenstoffatome enthält;
Alkenyl alleine oder als Teil einer anderen Gruppe, enthält von zwei bis zehn Kohlenstoffatome;
Alkinyl alleine oder als Teil einer anderen Gruppe, enthält von zwei bis zehn Kohlenstoffatome;
Aryl alleine oder als Teil einer anderen Gruppe ist ein monocyclisches carbocyclisches Ringsystem oder ein bicyclisches carbocyclisches ankondensiertes Ringsystem, mit ein oder mehreren aromatischen Ringen,
Aryl kann wahlweise 1, 2, 3, 4 oder 5 Substituenten haben, unabhängig gewählt aus der Gruppe bestehend aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Sulfamyl, Sulfamylalkyl, Phenyl, Furyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Cycloalkyl ist ein monocyclisches, bicyclisches oder tricyclisches Ringsystem;
Cycloalkyl kann wahlweise 1, 2, 3, 4 oder 5 Substituenten haben, unabhängig gewählt aus der Gruppe bestehend aus Alkenyl, Alkoxy, . Alkoxycarbonyl, Alkoxycarbonylalkyl, Alkyl, Alkylcarbonylalkyl, Alkylcarbonyloxy, Alkylcarbonyloxyalkyl, Alkylsulfonylalkyl, Alkinyl, Amido, Amidoalkyl, Cyanoalkyl, Halo, Haloalkoxy, Haloalkyl, Hydroxy, Hydroxyalkyl, Sulfamylalkyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Heterocyclus alleine oder als Teil einer anderen Gruppe ist ein monocyclisches oder bicyclisches Ringsystem, worin (i) das monocyclische Ringsystem ein 5- oder 6-gliedriger Ring ist, enthaltend 1, 2, 3 oder 4 Heteroatome, unabhängig gewählt aus Sauerstoff, Stickstoff und Schwefel, worin der 5-gliedrige Ring von 0-2 Doppelbindungen hat, und der 6-gliedrige Ring von 0-3 Doppelbindungen hat, und (ii) das bicyclische Ringsystem ist irgendeines der obigen monocyclischen Ringsysteme, ankondensiert an eine monocyclische Arylgruppe, wie oben definiert, eine monocyclische Cycloalkylgruppe, wie oben definiert oder ein monocyclisches heterocyclisches Ringsytem, wie oben definiert;
Heterocyclus kann wahlweise 1, 2 oder 3 Substituenten haben, unabhängig gewählt aus der Gruppe bestehend aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Oxo, Sulfamyl, Sulfamylalkyl, -NR^{A}R^{B}, (NR^{A}R^{B})Alkyl, worin R^{A} und R^{B} unabhängig gewählt sind aus Wasserstoff, Alkyl, Alkylcarbonyl und Formyl, und Phenyl hat wahlweise 1 oder 2 Substituenten unabhängig gewählt aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Sulfamyl, Sulfamylalkyl, -NR^{A}R^{B}, (NR^{A}R^{B})Alkyl, worin R^{A} und R^{B} unabhängig gewählt sind aus Wasserstoff, Alkyl, Alkylcarbonyl und Formyl;
Amido alleine oder als Teil einer anderen Gruppe ist -C (O) NR⁹R¹⁰, worin R⁹ und R¹⁰ unabhängig gewählt sind aus der Gruppe bestehend aus Wasserstoff, Alkyl, Alkylcarbonyl, Phenyl, Phenylalkyl, Phenylcarbonyl, Formyl und S(O)₂R¹¹, worin R¹¹ gewählt ist aus Alkyl, Phenyl und Phenylalkyl;
Cycloalkenyl alleine oder als Teil einer anderen Gruppe ist ein cyclischer Kohlenwasserstoff, enthaltend von drei bis acht Kohlenstoffe und enthaltend mindestens eine Kohlenstoff-Kohlenstoff-Doppelbindung;
Cycloalkenyl kann wahlweise 1, 2, 3, 4 oder 5 Substituenten haben, unabhängig gewählt aus Alkenyl, Alkoxy, Alkoxycarbonyl, Alkoxycarbonylalkyl, Alkyl, Alkylcarbonyloxy, Alkylcarbonyloxyalkyl, Alkinyl, Amido, Amidoalkyl, Halo, Haloalkoxy, Haloalkyl, Hydroxy, Hydroxyalkyl, Sulfamylalkyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Sulfamyl ist eine -SO₂NR⁹⁴R⁹⁵ Gruppe, worin R⁹⁴ und R⁹⁵ unabhängig gewählt sind aus Wasserstoff, Alkyl, Aryl und Arylalkyl;
vorausgesetzt, daß wenn X O ist; R² -CCl₃ ist; R³ Alkyl oder Phenyl ist; und R⁵ und R⁷ Wasserstoff sind; dann ist R¹ nicht Phenyl;
weiter vorausgesetzt, daß
N-[[[[(2-Methylphenyl)amino]carbonyl]amino]phenylmethyl]-benzamid;
N-[[[[(4-Chlorphenyl)amino]carbonyl]amino]phenylmethyl]-benzamid;
N-[2,2-Dichlor-1-[[phenylamino)thioxomethyl]amino]ethyl]-benzamid;
N-[2,2-Dichlor-1-[[phenylamino)thioxomethyl]amino]ethyl]-4-methyl-benzamid;
N-[2,2-Dichlor-1-[[[4-methylphenyl)amino)thioxomethyl]amino]ethyl]-benzamid;
N-[2,2-Dichlor-1-[[[4-methylphenyl)amino)thioxomethyl]amino]ethyl]-4-methyl-benzamid;
N-[2,2-Dichlor-1-[[[4-methylphenyl)amino)thioxomethyl]amino]ethyl]-benzamid;
N-[2,2-Dichlor-1-[[[4-methylphenyl)amino)thioxomethyl]amino]ethyl]-4-methyl-benzamid;
N-[[[(Phenylamino)carbonyl]amino]methyl]-3-pyridincarboxamid;
N-[[[[(2-Methylphenyl)amino)carbonyl]amino]methyl]-3-pryridincarboxamid;
N-[[[[(4-Methylphenyl)amino)carbonyl]amino]methyl]-3-pyridincarboxamid;
N-[[[[(3-Ethoxyphenyl)amino)carbonyl]amino]methyl]-3-pyridincarboxamid;
N-[[[[(4-Nitrophenyl)amino)carbonyl]amino]methyl]-3-pyridincarboxamid;
N-[[[[(3-Methylphenyl)amino)carbonyl]amino]methyl]-3-pyridincarboxamid;
N-[[[(Phenylamino)carbonyl]amino]methyl]-benzamid;
N-[[[(Phenylamino)thioxomethyl]amino]methyl]-benzamid;
N-[[[(Phenylamino)carbonyl]amino]methyl]-4-chlor-benzamid;
N-[[[(Phenylamino)thioxomethyl]amino]methyl]-4-chlor-benzamid;
1-(1-Acetamido-2,2,2-trichlorethyl)-3-phenyl-2-thio-harnstoff;
1-Phenyl-2-thio-3-(2,2,2-trichlor-1-propionamidoethyl)-harnstoff; und
1-(1-Butyramido-2,2,2-trichlorethyl)-3-phenyl-2-thio-harnstoff ausgeschlossen sind.

2. Eine Verbindung gemäß Anspruch 1, worin
R⁸ gewählt ist aus der Gruppe bestehend aus Alkoxy, Alkylsulfonyl, Arylalkoxy, Arylsulfonyl, Cyano, Haloalkylsulfonyl, Hydroxy und Nitro;
R² ist gewählt aus der Gruppe bestehend aus Wasserstoff, Alkenyl, Alkenyloxyalkyl, Alkenyloxy(alkenyloxy)alkyl, Alkoxyalkyl, Alkoxycarbonylalkyl, Alkyl, Alkylcarbonyl, Alkylsulfonylalkyl, Alkylthioalkyl, Aryl, Arylalkyl, Arylsulfonylalkyl, Cyanoalkyl, Cycloalkenyl, Cycloalkenylalkyl, Cycloalkyl, Cycloalkylalkyl, Haloalkyl, Haloalkylcarbonyl, Heterocyclus, Heterocyclusalkyl, Hydroxyalkyl, Sulfamylalkyl und (NR⁹R¹⁰)Alkyl;
R³ ist gewählt aus der Gruppe bestehend aus Aryl, Arylalkyl und Heterocyclus;
R⁵ ist gewählt aus der Gruppe bestehend aus Wasserstoff und Alkyl; und
R⁷ ist gewählt aus der Gruppe bestehend aus Wasserstoff, Haloalkyl und C₁-C₆ Alkyl; oder
R⁷ und R² bilden zusammengenommen mit dem Kohlenstoffatom, an welches sie gebunden sind, einen 5- oder 6-gliedrigen carbocyclischen Ring, worin der 5- oder 6-gliedrige carbocyclische Ring wahlweise substituiert ist mit 1 oder 2 Substituenten, unabhängig gewählt aus der Gruppe bestehend aus Alkyl, Halo, Haloalkoxy und Haloalkyl.

3. Eine Verbindung gemäß Anspruch 2, worin
R⁸ gewählt ist aus der Gruppe bestehend aus Alkoxy, Alkylsulfonyl, Haloalkylsulfonyl, Cyano, Hydroxy, Nitro, Arylalkoxy, worin der Arylanteil von Arylalkoxy Phenyl ist, und Arylsulfonyl, worin der Arylanteil von Arylsulfonyl Phenyl ist;
R¹ ist Pyridin, Pyrimidin oder Chinolin, wahlweise substituiert mit 1, 2 oder 3 Substituenten unabhängig gewählt aus der Gruppe bestehend aus Alkoxy, Alkyl, Halo, Haloalkyl, Nitro, Phenylsulfonyl und Sulfamyl und Aryl, worin Aryl Phenyl ist, wahlweise substituiert mit 1, 2 oder 3 Substituenten unabhängig gewählt aus der Gruppe bestehend aus Alkoxy, Alkyl, Halo, Haloalkyl, Nitro, Phenylsulfonyl und Sulfamyl;
R² ist gewählt aus der Gruppe bestehend aus Wasserstoff, Alkenyl, Alkenyloxyalkyl, Alkenyloxy(alkenyloxy)alkyl, Alkoxyalkyl, Alkoxycarbonylalkyl, Alkyl, Alkylcarbonyl, Alkylsulfonylalkyl, Aryl, worin Aryl Phenyl ist, Arylalkyl, worin der Arylanteil von Arylalkyl Phenyl ist, Arylsulfonylalkyl, worin der Arylanteil von Arylsulfonylalkyl Phenyl ist, Cyanoalkyl, Cycloalkenylalkyl, Cycloalkyl, Haloalkyl, Haloalkylcarbonyl, Hydroxyalkyl, Sulfamylalkyl, (NR⁹R¹⁰)Alkyl und Heterocyclus, worin der Heterocyclus gewählt ist aus der Gruppe bestehend aus 1,3-Dioxan, Pyrrolidin und Thiophen; und
R³ ist gewählt aus der Gruppe bestehend aus Aryl, worin Aryl Phenyl ist, und Arylalkyl, worin der Arylanteil von Arylalkyl Phenyl ist.

4. Eine Verbindung gemäß Anspruch 1, worin
X gewählt ist aus der Gruppe bestehend aus O, S, CHNO₂, C(CN)₂ und NR⁸;
R⁸ ist gewählt aus der Gruppe bestehend aus Arylsulfonyl, Cyano, Haloalkylsulfonyl, Nitro und Sulfamyl;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰) Alkyl;
R³ ist gewählt aus der Gruppe bestehend aus Aryl und Arylalkyl;
R⁵ ist gewählt aus der Gruppe bestehend aus Wasserstoff und Alkyl; und
R⁷ ist Wasserstoff.

5. Eine Verbindung gemäß Anspruch 1, worin
X gewählt ist aus der Gruppe bestehend aus O, S, CHNO₂, C(CN)₂ und NR⁸;
R⁸ ist gewählt aus der Gruppe bestehend aus Arylsulfonyl, worin der Arylanteil von Arylsulfonyl Phenyl ist, Cyano, Haloalkylsulfonyl, Nitro und Sulfamyl;
R¹ ist gewählt aus der Gruppe bestehend aus Aryl, worin Aryl Phenyl ist, Arylalkyl, worin der Arylanteil von Arylalkyl Phenyl ist, Heterocyclus, worin der Heterocyclus gewählt ist aus der Gruppe bestehend aus Chinolin, Pyridin und Pyrimidin und Heterocyclusalkyl, worin der Heterocyclusanteil von Heterocyclusalkyl Pyridin ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰) Alkyl und Heterocyclus, worin der Heterocyclus gewählt ist aus der Gruppe bestehend aus 1,3-Dioxan, Pyrrolidin und Thiophen;
R³ ist gewählt aus der Gruppe bestehend aus Aryl, worin Aryl Phenyl ist, und Arylalkyl, worin der Arylanteil von Arylalkyl Phenyl ist;
R⁵ ist gewählt aus der Gruppe bestehend aus Wasserstoff und Alkyl; und
R⁷ ist Wasserstoff.

6. Eine Verbindung gemäß Anspruch 1, worin
X NR⁸ ist;
R⁸ ist Cyano;
R¹ ist gewählt aus der Gruppe bestehend aus Heterocyclus und Heterocyclusalkyl;
R³ ist gewählt aus der Gruppe bestehend aus Heterocyclus und Heterocyclusalkyl; und
R⁵ ist Wasserstoff.

7. Eine Verbindung gemäß Anspruch 1, worin
X NR⁸ ist;
R⁸ ist Cyano;
R¹ ist gewählt aus der Gruppe bestehend aus Heterocyclus und Heterocyclusalkyl;
R³ ist gewählt aus der Gruppe bestehend aus Aryl und Arylalkyl; und
R⁵ ist Wasserstoff.

8. Eine Verbindung gemäß Anspruch 7, worin
R² gewählt ist aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰)Alkyl; und
R⁷ ist Wasserstoff.

9. Eine Verbindung gemäß Anspruch 1, worin
X NR⁸ ist;
R⁸ ist Cyano;
R¹ ist Heterocyclusalkyl, worin der Heterocyclusanteil von Heterocyclusalkyl Pyridin ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist; und
R⁵ ist Wasserstoff.

10. Eine Verbindung gemäß Anspruch 9, gewählt aus der Gruppe bestehend aus
4-Chlor-N-[1-({(cyanoimino)[(3-pyridinylmethyl)amino]methyl}amino)-2,2-dimethylpropyl]benzamid;
4-Chlor-N-[1-({(cyanoimino)[(4-pyridinylmethyl)amino]methyl}amino)-2,2-dimethylpropyl]benzamid; und
4-Chlor-N-[1-({(cyanoimino)[(2-pyridinylmethyl)amino]methyl}amino)-2,2-dimethylpropyl]benzamid.

11. Eine Verbindung gemäß Anspruch 1, worin
X NR⁸ ist;
R⁸ ist Cyano;
R¹ ist Heterocyclus, worin der Heterocyclus gewählt ist aus der Gruppe bestehend aus Chinolin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

12. Eine Verbindung gemäß Anspruch 11, die gewählt ist aus der Gruppe bestehend aus
4-Methyl-N-(2,2,2-trichlor-1-{[(cyanoimino)(5-pyrimidinylamino)methyl]amino}ethyl)benzamid und
4-Chlor-N-(1-{[(cyanoimino)(3-chinolinylamino)methyl]amino}-2,2-dimethylpropyl)benzamid.

13. Eine Verbindung gemäß Anspruch 1, worin
X NR⁸ ist;
R⁸ ist Cyano;
R¹ ist Heterocyclus, worin Heterocyclus Pyridin ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

14. Eine Verbindung gemäß Anspruch 13, gewählt aus der Gruppe bestehend aus
N-(1-{[(Cyanoimino)(3-pyridinylamino)methyl]amino}-2,2-dimethylpropyl)-4-methylbenzamid;
4-Chlor-N-(1-{[(cyanoimino)(3-pyridinylamino)methyl]amino}-2,2-dimethylpropyl)benzamid;
4-Chlor-N-[{[(cyanoimino)(3-pyridinylamino)methyl]amino}(cyclopropyl)methyl]benzamid;
N-(1-{[[(6-Chlor-3-pyridinyl)amino](cyanoimino)methyl]amino}-2,2-dimethylpropyl)-4-methylbenzamid;
(-)N-(1-{[(Cyanoimino)(3-pyridinylamino)methyl]amino}-2,2-dimethylpropyl)-4-methylbenzamid;
(+)N-(1-{[(Cyanoimino)(3-pyridinylamino)methyl]amino}-2,2-dimethylpropyl)-4-methylbenzamid;
4-Chlor-N-(1-{[(cyanoimino)(3-pyridinylamino)methyl]amino}-2-ethylbutyl)benzamid;
4-Chlor-N-(1-{[(cyanoimino)(3-pyridinylamino)methyl]amino}-3-methylbutyl)benzamid;
4-Chlor-N-[{[(cyanoimino)(3-pyridinylamino)methyl]amino}(cyclohexyl)methyl]benzamid;
4-Chlor-N-(1-{[(cyanoimino)(3-pyridinylamino)methyl]amino}-3,3-dimethylbutyl)benzamid;
4-Chlor-N-(1-{[(cyanoimino)(3-pyridinylamino)methyl]amino}-2-methylpropyl)benzamid;
4-Chlor-N-(4-cyano-1-{[(cyanoimino)(3-pyridinylamino)methyl]amino}-2,2-diethylbutyl)benzamid;
4-Chlor-N-[1-{[(cyanoimino)(3-pyridinylamino)methyl]amino}-2-(2,6,6-trimethyl-1-cyclohexen-1-yl)ethyl]benzamid;
4-Chlor-N-[1-{[(cyanoimino)(3-pyridinylamino)methyl]amino}-2,2-dimethyl-4-pentenyl)benzamid;
4-Chlor-N-[1-{[(cyanoimino)(3-pyridinylamino)methyl]amino}propyl)benzamid;
4-Chlor-N-({[(cyanoimino)(3-pyridinylamino)methyl]amino}methyl)benzamid;
(-)4-Chlor-N-(4-cyano-1-{[(cyanoimino)(3-pyridinylamino)methyl]amino}-2,2-diethylbutyl)benzamid;
(+) 4-Chlor-N-(4-cyano-1-{[(cyanoimino)(3-pyridinylamino)methyl]amino}-2,2-diethylbutyl)benzamid;
(+) 4-Chlor-N-[1-{[(cyanoimino)(3-pyridinylamino)methyl]amino}-2-(2,6,6-trimethyl-1-cyclohexen-1-yl)ethyl]benzamid;
(-) 4-Chlor-N-[1-{[(cyanoimino)(3-pyridinylamino)methyl]amino}-2-(2,6,6-trimethyl-1-cyclohexen-1-yl)ethyl]benzamid;
(-)4-Chlor-N-[1-{[(cyanoimino)(3-pyridinylamino)methyl]amino}-2,2-dimethyl-4-pentenyl)benzamid;
(+)4-Chlor-N-[1-{[(cyanoimino)(3-pyridinylamino)methyl]amino}-2,2-dimethyl-4-pentenyl)benzamid;
4-Chlor-N-[1-{[(cyanoimino)(3-pyridinylamino)methyl]amino}-3,3-dimethyl-4-pentenyl)benzamid;
4-Chlor-N-[1-{[(cyanoimino)(3-pyridinylamino)methyl]amino}-2-cyclohexyl-2-methylpropyl)benzamid;
4-Chlor-N-[1-{[(cyanoimino)(3-pyridinylamino)methyl]amino}-2,2-dimethylhexyl)benzamid;
N-(2-(1-Adamantyl)-1-{[(cyanoimino)(3-pyridinylamino)methyl]amino}ethyl)-4-chlorbenzamid;
N-(2,2-bis[(Allyloxy)methyl]-1-{[(cyanoimino)(3-pyridinylamino)methyl]amino}butyl)-4-chlorbenzamid;
4-Chlor-N-[1-{[(cyanoimino)(3-pyridinylamino)methyl]amino}-3-(dimethylamino)-2,2-dimethylpropyl]benzamid;
tert-Butyl (2R) -2- ((R) - [(4-Chlorbenzoyl)amino]{[(cyanoimino)(3-pyridinylamino)methyl]amino}methyl)-1-pyrrolidincarboxylat;
4-Chlor-N-[1-{[(cyanoimino)(3-pyridinylamino)methyl]amino}-3-(methylsulfanyl)propyl]benzamid;
N-(1-Adamantyl{[(cyanoimino)(3-pyridinylamino)methyl]amino}methyl)-4-chlorbenzamid;
4-Chlor-N-{[(cyanoimino)(3-pyridinylamino)methyl]amino}(5-ethyl-1,3-dioxan-5-yl)methyl]benzamid;
4-Chlor-N-(1-{[(cyanoimino)(3-pyridinylamino)methyl]amino}-2,2-dimethyl-3-phenylpropyl)benzamid;
N-(1-{[(Cyanoimino)(3-pyridinylamino)methyl]amino}-2,2-dimethylpropyl)-4-jodbenzamid;
N-(1-{[(Cyanoimino)(3-pyridinylamino)methyl]amino}-2,2-dimethylpropyl)-4-(2-furyl)benzamid;
4-Brom-N-(1-{[(cyanoimino)(3-pyridinylamino)methyl]amino}-2,2-dimethylpropyl)benzamid;
4-Chlor-N-(1-{[(cyanoimino)(3-pyridinylamino)methyl]amino}-2,2-dimethylpropyl)-2-fluorbenzamid;
N-(1-{[(Cyanoimino)(3-pyridinylamino)methyl]amino}-2,2-dimethylpropyl)-4-fluorbenzamid;
N-(1-{[(Cyanoimino)(3-pyridinylamino)methyl]amino}-2,2-dimethylpropyl)-3-methylbenzamid;
N-(1-{[(Cyanoimino)(3-pyridinylamino)methyl]amino}-2,2-dimethylpropyl)-2-methylbenzamid; und
N-(1-{[(Cyanoimino)(3-pyridinylamino)methyl]amino}-2,2-dimethylpropyl)-3,5-difluorbenzamid.

15. Eine Verbindung gemäß Anspruch 1, worin
X NR⁸ ist;
R⁸ ist Cyano;
R¹ ist Heterocyclus;
R² ist Haloalkyl;
R³ ist Aryl;
R⁵ ist Wasserstoff; und
R⁷ ist Wasserstoff.

16. Eine Verbindung gemäß Anspruch 1, worin
X NR⁸ ist;
R⁸ ist Cyano;
R¹ ist Heterocyclus, worin der Heterocyclus Pyridin ist;
R² ist Haloalkyl;
R³ ist Aryl, worin Aryl Phenyl ist;
R⁵ ist Wasserstoff; und
R⁷ ist Wasserstoff.

17. Eine Verbindung gemäß Anspruch 16, gewählt aus der Gruppe bestehend aus
4-Methyl-N-(2,2,2-trichlor-1-{[(cyanoimino)(3-pyridinylamino)methyl]amino}ethyl)benzamid;
4-Chlor-N-(2,2,2-trichlor-1-{[(cyanoimino)(3-pyridinylamino)methyl]amino}ethyl)benzamid;
N-(2,2,2-Trichlor-1-{[(cyanoimino)(3-pyridinylamino)methyl]amino}ethyl)benzamid;
2-Methyl-N-(2,2,2-trichlor-1-{[(cyanoimino)(3-pyridinylamino)methyl]amino}ethyl)benzamid;
4-Chlor-N-(1-{[(cyanoimino)(3-pyridinylamino)methyl]amino}-2,2,2-trifluorethyl)benzamid;
(-)4-Chlor-N-(2,2,2-trichlor-1-{[(cyanoimino)(3-pyridinylamino)methyl]amino}ethyl)benzamid;
(+)4-Chlor-N-(2,2,2-trichlor-1-{[(cyanoimino)(3-pyridinylamino)methyl]amino}ethyl)benzamid;
4-Jod-N-(2,2,2-trichlor-1-{[(cyanoimino)(3-pyridinylamino)methyl]amino}ethyl)benzamid;
4-Chlor-N-(2,2-dichlor-1-{[(cyanoimino)(3-pyridinylamino)methyl]amino}pentyl)benzamid;
4-Chlor-N-(2,2-dichlor-1-{[(cyanoimino)(3-pyridinylamino)methyl]amino}propyl)benzamid;
(-)4-Chlor-N-(2,2-dichlor-1-{[(cyanoimino)(3-pyridinylamino)methyl]amino}propyl)benzamid;
(+)4-Chlor-N-(2,2-dichlor-1-{[(cyanoimino)(3-pyridinylamino)methyl]amino}propyl)benzamid;
3-Chlor-N-(2,2-dichlor-1-{[(cyanoimino)(3-pyridinylamino)methyl]amino}propyl)benzamid;
N-(2,2-Dichlor-1-{[(cyanoimino)(3-pyridinylamino)methyl]amino}propyl)-3,5-difluorbenzamid;
4-Chlor-N-(1-{[(cyanoimino)(3-pyridinylamino)methyl]amino}-2,2,3,3,3-pentafluorpropyl)benzamid; und
3-Chlor-N-(1-{[(cyanoimino)(3-pyridinylamino)methyl]amino} 2,2,3,3,3-pentafluorpropyl)benzamid.

18. Eine Verbindung gemäß Anspruch 1, worin
X NR⁸ ist;
R⁸ ist Cyano;
R¹ ist Heterocyclus, worin der Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Alkyl; und
R⁷ ist Wasserstoff.

19. Eine Verbindung gemäß Anspruch 18, die 4-Chlor-N-{1-[[(cyanoimino)(3-pyridinylamino)methyl](methyl)amino]-2,2-dimethylpropyl}benzamid ist.

20. Eine Verbindung gemäß Anspruch 1, worin
X NR⁸ ist;
R⁸ ist Cyano;
R¹ ist gewählt aus der Gruppe bestehend aus Heterocyclus und Heterocyclusalkyl;
R³ ist Alkyl; und
R⁵ ist Wasserstoff.

21. Eine Verbindung gemäß Anspruch 1, worin
X NR⁸ ist;
R⁸ ist Cyano;
R¹ ist gewählt aus der Gruppe bestehend aus Aryl und Arylalkyl;
R³ ist gewählt aus der Gruppe bestehend aus Heterocyclus und Heterocyclusalkyl; und
R⁵ ist Wasserstoff.

22. Eine Verbindung gemäß Anspruch 1, worin
X NR⁸ ist;
R⁸ ist Cyano;
R¹ ist gewählt aus der Gruppe bestehend aus Aryl und Arylalkyl;
R³ ist gewählt aus der Gruppe bestehend aus Aryl und Arylalkyl; und
R⁵ ist Wasserstoff.

23. Eine Verbindung gemäß Anspruch 22, worin
R² gewählt ist aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰)Alkyl;
und
R⁷ ist Wasserstoff.

24. Eine Verbindung gemäß Anspruch 1, worin
X NR⁸ ist;
R⁸ ist Cyano;
R¹ ist Arylalkyl, worin der Arylanteil von Arylalkyl Phenyl ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰) Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

25. Eine Verbindung gemäß Anspruch 24, gewählt aus der Gruppe 25. Eine Verbindung gemäß Anspruch 24, gewählt aus der Gruppe bestehend aus
4-Chlor-N-(1-{[[(2-chlorbenzyl)amino](cyanoimino)methyl]amino}-2,2-dimethylpropyl)benzamid;
4-Chlor-N-(1-{[[(3-chlorbenzyl)amino](cyanoimino)methyl]amino}-2,2-dimethylpropyl)benzamid; und
4-Chlor-N-(1-{[[(4-chlorbenzyl)amino](cyanoimino)methyl]amino}-2,2-dimethylpropyl)benzamid.

26. Eine Verbindung gemäß Anspruch 1, worin
X NR⁸ ist;
R⁸ ist Cyano;
R¹ ist Aryl, worin Aryl Phenyl ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

27. Eine Verbindung gemäß Anspruch 26, die gewählt ist aus der Gruppe bestehend aus
N-(1-{[Anilino(cyanoimino)methyl]amino}-2,2,2-trichlorethyl)-4-methylbenzamid;
4-Methyl-N-(2,2,2-trichlor-1-{[(cyanoimino)(2-fluoranilino)methyl]amino}ethyl)benzamid;
N-(1-{[(Cyanoimino)(3-fluoranilino)methyl]amino}-2,2-dimethylpropyl)-4-methylbenzamid;
4-Chlor-N-[{[(cyanoimino)(3-fluoranilino)methyl]amino}(3-thienyl)methyl]benzamid;
4-Chlor-N-(1-{[(cyanoimino)(2-fluoranilino)methyl]amino}-2,2-dimethylpropyl)benzamid;
4-Chlor-N-(1-{[(cyanoimino)(3-fluoranilino)methyl]amino}-2,2-dimethylpropyl)benzamid;
4-Chlor-N-(1-({(cyanoimino)[3-(trifluormethyl)anilino]methyl}amino)-2,2-dimethylpropyl]benzamid;
4-Chlor-N-(1-1[(cyanoimino)(3,5-difluoranilino)methyl]amino}-2,2-dimethylpropyl]benzamid;
4-Chlor-N-(1-{[(cyanoimino)(2,5-difluoranilino)methyl]amino}-2,2-dimethylpropyl]benzamid;
4-Chlor-N-(1-{[(cyanoimino)(2,6-difluoranilino)methyl]amino}-2,2-dimethylpropyl]benzamid;
4-Chlor-N-(1-{[(cyanoimino)(3-chloranilino)methyl]amino}-2,2-dimethylpropyl]benzamid; und
4-Chlor-N-(1-{[(cyanoimino)(3-methoxyanilino)methyl]amino}-2,2-dimethylpropyl]benzamid.

28. Eine Verbindung gemäß Anspruch 1, worin
X NR⁸ ist;
R⁸ ist Cyano;
R¹ ist gewählt aus der Gruppe bestehend aus Aryl und Arylalkyl;
R³ ist Alkyl; und
R⁵ ist Wasserstoff.

29. Eine Verbindung gemäß Anspruch 1, worin
X NR⁸ ist;
R⁸ ist gewählt aus der Gruppe bestehend aus Wasserstoff, Alkoxy, Alkyl, Alkylsulfonyl, Arylalkoxy, Aryloxy, Arylsulfonyl, Haloalkylsulfonyl, Heterocyclusalkoxy, Heterocyclusoxy, Hydroxy, Nitro und Sulfamyl; und
R⁵ ist Wasserstoff.

30. Eine Verbindung gemäß Anspruch 1, worin
X NR⁸ ist;
R⁸ ist Nitro; und
R⁵ ist Wasserstoff.

31. Eine Verbindung gemäß Anspruch 1, worin
X NR⁸ ist;
R⁸ ist Nitro;
R¹ ist Heterocyclus, worin der Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰) Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist; und
R⁵ ist Wasserstoff.

32. Eine Verbindung gemäß Anspruch 31, gewählt aus der Gruppe bestehend aus
4-Chlor-N-(1-{[(nitroimino)(3-pyridinylamino)methyl]amino}-2,2-dimethylpropyl)benzamid;
4-Chlor-N-(1-{[(nitroimino)(3-pyridinylamino)methyl]amino}-3,3-dimethylbutyl)benzamid;
(+)4-Chlor-N-(1-{[(nitroimino)(3-pyridinylamino)methyl]amino}-3,3-dimethylbutyl)benzamid;
(-)4-Chlor-N-(1-{[(nitroimino)(3-pyridinylamino)methyl]amino}-3,3-dimethylbutyl)benzamid;
4-Chlor-N-(1-{[(nitroimino)(3-pyridinylamino)methyl]amino}-2,2-dimethyl-4-pentenyl)benzamid;
4-Chlor-N-(1-{[(nitroimino)(3-pyridinylamino)methyl]amino}-2,2-dimethyl-3-phenylpropyl)benzamid;
4-Chlor-N-(1-{[(nitroimino)(3-pyridinylamino)methyl]amino}-2-(2,6,6-trimethyl-1-cyclohexen-1-yl)ethyl]benzamid;
4-Chlor-N-(1-{[(nitroimino)(3-pyridinylamino)methyl]amino}-2-cyclohexyl-2-methylpropyl)benzamid;
N-(2,2-bis[[(Allyloxy)methyl]-1-{[(nitroimino) (3-pyridinylamino)methyl]amino}butyl)-4-chlorbenzamid;
4-Chlor-N-(4-cyano-1-{[(nitroimino)(3-pyridinylamino)methyl]amino}-2,2-diethylbutyl)benzamid;
4-Chlor-N-(1-{[(nitroimino)(3-pyridinylamino)methyl]amino}-3,3-dimethyl-4-pentenyl)benzamid;
N-(2-(1-Adamantyl)-1-{[(nitroimino)(3-pyridinylamino)methyl]amino}ethyl)-4-chlorbenzamid;
N-(1-{[(Nitroimino)(3-pyridinylamino)methyl]amino}-2,2-dimethylpropyl)-4-phenylbenzamid;
4-Chlor-N-(2,2-dichlor-1-{[(nitroimino)(3-pyridinylamino)methyl]amino}pentyl)benzamid;
4-Chlor-N-(2,2-dichlor-1-([(nitroimino)(3-pyridinylamino)methyl]amino}propyl)benzamid; und
3-Chlor-N-(2,2-dichlor-1-{[(nitroimino)(3-pyridinylamino)methyl]amino}propyl)benzamid.

33. Eine Verbindung gemäß Anspruch 1, worin
X NR⁸ ist;
R⁸ ist gewählt aus der Gruppe bestehend aus Arylsulfonyl, Haloalkylsulfonyl und Sulfamyl; und
R⁵ ist Wasserstoff.

34. Eine Verbindung gemäß Anspruch 1, worin
X NR⁸ ist;
R⁸ ist gewählt aus der Gruppe bestehend aus Arylsulfonyl, worin der Arylanteil von Arylsulfonyl Phenyl, Haloalkylsulfonyl und Sulfamyl ist;
R¹ ist Heterocyclus, worin der Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist; und
R⁵ ist Wasserstoff.

35. Eine Verbindung gemäß Anspruch 34, gewählt aus der Gruppe bestehend aus
4-Chlor-N-(2,2-dimethyl-1-{[[(phenylsulfonyl)imino](3-pyridinylamino)methyl]amino}propyl)benzamid;
4-Chlor-N-(3,3-dimethyl-1-{[[(phenylsulfonyl)imino](3-pyridinylamino)methyl]amino}butyl)benzamid;
4-Chlor-N-{2,2-dimethyl-1-[((3-pyridinylamino){[(trifluormethyl)sulfonyl]imino}methyl)amino] propyl}benzamid;
4-Chlor-N-{3,3-dimethyl-1-[((3-pyridinylamino){[(trifluormethyl)sulfonyl]imino}methyl)amino] butyl}benzamid;
N-(1-{[[(Aminosulfonyl)imino](3-pyridinylamino)methyl]amino}-2,2-dimethylpropyl)-4-chlorbenzamid;
N-(1-{[[(Aminosulfonyl)imino](3-pyridinylamino)methyl]amino}-3,3-dimethylbutyl)-4-chlorbenzamid;
4-Chlor-N-(1-{[{[(dimethylamino)sulfonyl]imino}(3-pyridinylamino)methyl]amino}-2,2-dimethylpropyl)benzamid; und
4-Chlor-N-(1-{[{[(dimethylamino)sulfonyl]imino}(3-pyridinylamino)methyl]amino}-3,3-dimethylbutyl)benzamid.

36. Eine Verbindung gemäß Anspruch 1, worin
X S ist, und
R⁵ ist Wasserstoff.

37. Eine Verbindung gemäß Anspruch 1, worin
X S ist;
R¹ ist gewählt aus der Gruppe bestehend aus Heterocyclus und Heterocyclusalkyl;
R³ ist gewählt aus der Gruppe bestehend aus Aryl und Arylalkyl, und
R⁵ ist Wasserstoff.

38. Eine Verbindung gemäß Anspruch 37, worin
R² gewählt ist aus der Gruppe bestehend aus Wasserstoff, Alkenyl, Alkenyloxyalkyl, Alkenyloxy(alkenyloxy)alkyl, Alkoxyalkyl-, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰)Alkyl;
und
R⁷ ist Wasserstoff.

39. Eine Verbindung gemäß Anspruch 1, worin
X S ist;
R¹ ist Heterocyclus, worin der Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

40. Eine Verbindung gemäß Anspruch 39, die gewählt ist aus der Gruppe bestehend aus
N-(2,2-Dimethyl-1-{[(3-pyridinylamino)carbothioyl]amino}propyl)-4-methylbenzamid;
N-((1R)-2,2-Dimethyl-1-{[(3-pyridinylamino)carbothioyl]amino}propyl)-4-methylbenzamid;
N-((1S)-2,2-Dimethyl-1-{[(3-pyridinylamino)carbothioyl]amino}propyl)-4-methylbenzamid;
N-(2,2-Dimethyl-1-{[(3-pyridinylamino)carbothioyl]amino}propyl)-2-methylbenzamid;
4-Chlor-N-(2,2-dimethyl-1-{[(3-pyridinylamino)carbothioyl]amino}propyl)benzamid;
N-(2,2-Dimethyl-1-{[(3-pyridinylamino)carbothioyl]amino}propyl)benzamid;
4-Methyl-N-(phenyl{[(3-pyridinylamino)carbothioyl]amino}methyl)benzamid;
4-Methyl-N-(2-methyl-1-{[(3-pyridinylamino)carbothioyl]amino}propyl)benzamid; und
4-Methyl-N-((1R,2S)-2-methyl-1-{[(3-pyridinylamino)carbothioyl]amino}butyl)benzamid.

41. Eine Verbindung gemäß Anspruch 1, worin
X S ist;
R¹ ist Heterocyclus;
R² ist Haloalkyl;
R³ ist Aryl;
R⁵ ist Wasserstoff; und
R⁷ ist Wasserstoff.

42. Eine Verbindung gemäß Anspruch 1, worin
X S ist;
R¹ ist Heterocyclus, worin der Heterocyclus Pyridin ist;
R² ist Haloalkyl;
R³ ist Aryl, worin Aryl Phenyl ist;
R⁵ ist Wasserstoff; und
R⁷ ist Wasserstoff.

43. Eine Verbindung gemäß Anspruch 42, die gewählt ist aus der Gruppe bestehend aus
4-Methyl-N-(2,2,2-trifluor-1-{[(3-pyridinylamino)carbothioyl]amino}ethyl)benzamid;
N-[1-({[(6-Chlor-3-pyridinyl)amino]carbothioyl}amino)-2,2,2-trifluorethyl]-4-methylbenzamid;
4-Chlor-N-(2,2,2-trifluor-1-{[(3-pyridinylamino)carbothioyl]amino}ethyl)benzamid; und
4-Jod-N-(2,2,2-trichlor-1-{[(3-pyridinylamino)carbothioyl]amino}ethyl)benzamid.

44. Eine Verbindung gemäß Anspruch 1, worin
X S ist;
R¹ ist gewählt aus der Gruppe bestehend aus Aryl und Arylalkyl;
R³ ist gewählt aus der Gruppe bestehend aus Aryl und Arylalkyl; und
R⁵ ist Wasserstoff.

45. Eine Verbindung gemäß Anspruch 44, worin
R² gewählt ist aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰)Alkyl;
und
R⁷ ist Wasserstoff.

46. Eine Verbindung gemäß Anspruch 1, worin
X S ist;
R¹ ist Aryl, worin Aryl Phenyl ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist,
R⁵ ist Wasserstoff; und
R⁷ ist Wasserstoff.

47. Eine Verbindung gemäß Anspruch 46, die gewählt ist aus der Gruppe bestehend aus
4-Methyl-N-{2,2,2-trifluor-1-[(2-toluidincarbothioyl)amino]ethyl}benzamid;
4-Methyl-N-{2,2,2-trifluor-1-{[(4-fluoranilino) carbothioyl] amino}ethyl) benzamid;
4-Methyl-N-{2,2,2-trifluor-1-{[(3-nitroanilino)carbothioyl]amino}ethyl)benzamid;
4-Methyl-N-[2,2,2-trifluor-1-({[2-fluor-3-(trifluormethyl)anilino]carbothioyl}amino)ethyl)benzamid;
4-Methyl-N-(2,2,2-trifluor-1-{[(4-methoxyanilino)carbothioyl]amino}ethyl)benzamid;
4-Methyl-N-(2,2,2-trifluor-1-{[(2-methoxyanilino)carbothioyl]amino}ethyl)benzamid;
N-{1-[(Anilinocarbothioyl)amino]-2,2,2-trifluorethyl}-4-methylbenzamid;
4-Methyl-N-{2,2,2-trifluor-1-[(4-toluidinocarbothioyl)amino]ethyl}benzamid;
4-Methyl-N-(2,2,2-trifluor-1-{[(2-fluoranilino)carbothioyl]amino}ethyl)benzamid;
4-Methyl-N-(2,2,2-trifluor-1-{[(3-methoxyanilino)carbothioyl]amino}ethyl)benzamid;
4-Methyl-N-(2,2,2-trifluor-1-{[(3-fluoranilino)carbothioyl]amino}ethyl)benzamid;
N-(1-{[(2,5-Difluoranilino)carbothioyl]amino}-2,2,2-trifluorethyl)-4-methylbenzamid;
N-(1-{[(2,4-Difluoranilino)carbothioyl]amino}-2,2,2-trifluorethyl)-4-methylbenzamid;
4-Methyl-N-{2,2,2-trifluor-1-[(3-toluidinocarbothioyl)amino]ethyl}benzamid;
N-(1-{[(2,6-Difluoranilino)carbothioyl]amino}-2,2,2-trifluorethyl)-4-methylbenzamid;
N-(1-{[(2,3-Difluoranilino)carbothioyl]amino}-2,2,2-trifluorethyl)-4-methylbenzamid;
N-{1-[(Anilinocarbothioyl)amino]-2,2,2-trifluorethyl}-4-chlorbenzamid;
4-Chlor-N-(2,2,2-trifluor-1-{[(2-fluoranilino)carbothioyl]amino}ethyl)benzamid;
N-(2,2-Dimethyl-1-{[(3-nitroanilino)carbothioyl]amino)propyl)-4-methylbenzamid;
4-Methyl-N-(1-{[(3-nitroanilino)carbothioyl]amino}ethyl)benzamid;
4-Methyl-N-(1-{[(3-nitroanilino)carbothioyl]amino}-2-phenylethyl)benzamid;
N-((1R)-2-(tert-Butoxy)-1-{[(3-nitroanilino)carbothioyl]amino}ethyl)-4-methylbenzamid;
N-(2-Fluor-1-{[(3-nitroanilino)carbothioyl]amino}ethyl)-4-methylbenzamid;
und
4-Methyl-N-[{[(3-nitroanilino)carbothioyl]amino}(phenyl)methyl]benzamid.

48. Eine Verbindung gemäß Anspruch 1, worin
X S ist;
R¹ ist Aryl, worin Aryl Phenyl ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰) Alkyl und Heterocyclus;
R³ ist Aryl, worin Aryl Phenyl ist,
R⁵ ist Wasserstoff; und
R⁷ ist Wasserstoff.

49. Eine Verbindung gemäß Anspruch 48, die 3-Phenyl-N-(2,2,2-trichlor-1-{[(3-nitroanilino)carbothioyl]amino}ethyl)propanamid ist.

50. Eine Verbindung gemäß Anspruch 1, worin
X O ist;
R¹ ist gewählt aus der Gruppe bestehend aus Heterocyclus und Heterocyclusalkyl;
R³ ist gewählt aus der Gruppe bestehend aus Aryl und Arylalkyl; und
R⁵ ist Wasserstoff.

51. Eine Verbindung gemäß Anspruch 50, worin
R² gewählt ist aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰)Alkyl;
und
R⁷ ist Wasserstoff.

52. Eine Verbindung gemäß Anspruch 1, worin
X O ist;
R¹ ist Heterocyclus, worin der Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

53. Eine Verbindung gemäß Anspruch 1, worin
X O ist;
R¹ ist Heterocyclus;
R² ist Haloalkyl;
R³ ist Aryl;
R⁵ ist Wasserstoff; und
R⁷ ist Wasserstoff.

54. Eine Verbindung gemäß Anspruch 1, worin
X O ist;
R¹ ist Heterocyclus, worin der Heterocyclus Pyridin ist;
R² ist Haloalkyl;
R³ ist Aryl, worin Aryl Phenyl ist;
R⁵ ist Wasserstoff; und
R⁷ ist Wasserstoff.

55. Eine Verbindung gemäß Anspruch 54, die gewählt ist aus der Gruppe bestehend aus
4-Methyl-N-(2,2,2-trichlor-1-{[(3-pyridinylamino)carbonyl]amino}ethyl)benzamid;
2-Methyl-N-(2,2,2-trichlor-1-{[(3-pyridinylamino)carbonyl]amino}ethyl)benzamid;
N-(2,2,2-Trichlor-1-{[(3-pyridinylamino)carbonyl]amino}ethyl)benzamid; und
4-Chlor-N-(2,2,2-trichlor-1-([(3-pyridinylamino)carbonyl]amino}ethyl)benzamid.

56. Eine Verbindung gemäß Anspruch 1, worin
X O ist;
R¹ ist gewählt aus der Gruppe bestehend aus Heterocyclus und Heterocyclusalkyl;
R³ ist gewählt aus der Gruppe bestehend aus Heterocyclus und Heterocyclusalkyl, und
R⁵ ist Wasserstoff.

57. Eine Verbindung gemäß Anspruch 1, worin
X O ist;
R¹ ist gewählt aus der Gruppe bestehend aus Aryl und Arylalkyl;
R³ ist gewählt aus der Gruppe bestehend aus Aryl und Arylalkyl; und
R⁵ ist Wasserstoff.

58. Eine Verbindung gemäß Anspruch 57, worin
R² gewählt ist aus der Gruppe bestehend asu Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰) Alkyl ; und
R⁷ ist Wasserstoff.

59. Eine Verbindung gemäß Anspruch 1, worin
X O ist;
R¹ ist Aryl, worin Aryl Phenyl ist;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist; und
R⁵ ist Wasserstoff.

60. Eine Verbindung gemäß Anspruch 59, die 4-Chlor-N-(1-{[(2-fluoranilino)carbonyl]amino}-2,2-dimethylpropyl)benzamid ist.

61. Eine Verbindung gemäß Anspruch 1, worin
X gewählt ist aus der Gruppe bestehend aus CHCN und CHNO₂;
R¹ ist gewählt aus der Gruppe bestehend aus Heterocyclus und Heterocyclusalkyl;
R³ ist gewählt aus der Gruppe bestehend aus Heterocyclus und Heterocyclusalkyl; und
R⁵ ist Wasserstoff.

62. Eine Verbindung gemäß Anspruch 1, worin
X gewählt ist aus der Gruppe bestehend aus CHCN und CHNO₂;
R¹ ist gewählt aus der Gruppe bestehend aus Heterocyclus und Heterocyclusalkyl;
R³ ist gewählt aus der Gruppe bestehend aus Aryl und Arylalkyl; und
R⁵ ist Wasserstoff.

63. Eine Verbindung gemäß Anspruch 62, worin
R² gewählt ist aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, Arylalkyl, Cyanoalkyl, Cycloalkenylalkyl, Cycloalkyl, Cycloalkylalkyl, Haloalkyl, Heterocyclus und (NR⁹R¹⁰)Alkyl;
und
R⁷ ist Wasserstoff.

64. Eine Verbindung gemäß Anspruch 1, worin
X gewählt ist aus der Gruppe bestehend aus CHCN und CHNO₂;
R¹ ist Heterocyclus, worin Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, (NR⁹R¹⁰)Alkyl und Heterocyclus, worin der Heterocyclus gewählt ist aus der Gruppe bestehend aus 1,3-Dioxan, Pyrrolidin und Thiophen;
R³ ist Aryl, worin Aryl Phenyl ist;
R⁵ ist Wasserstoff; und
R⁷ ist Wasserstoff.

65. Eine Verbindung gemäß Anspruch 64, gewählt aus der Gruppe bestehend aus
4-Chlor-N-(2-ethyl-1-{[2-nitro-1-(3-pyridinylamino)ethenyl]amino}butyl)benzamid; und
4-Chlor-N-(2,2-dimethyl-1-{[2-nitro-1-(3-pyridinylamino)ethenyl]amino}propyl)benzamid.

66. Eine Verbindung gemäß Anspruch 1, worin
X gewählt ist aus der Gruppe bestehend aus CHCN und CHNO₂;
R¹ ist Heterocyclus;
R² ist Haloalkyl;
R³ ist Aryl;
R⁵ ist Wasserstoff; und
R⁷ ist Wasserstoff.

67. Eine Verbindung gemäß Anspruch 1, worin
X gewählt ist aus der Gruppe bestehend aus CHCN und CHNO₂;
R¹ ist Heterocyclus, worin Heterocyclus Pyridin ist;
R² ist Haloalkyl;
R³ ist Aryl, worin Aryl Phenyl ist;
R⁵ ist Wasserstoff; und
R⁷ ist Wasserstoff.

68. Eine Verbindung gemäß Anspruch 67, die 4-Chlor-N-(2,2-dichlor-1-{[2-nitro-1-(3-pyridinylamino)ethenyl]amino}pentyl)benzamid ist.

69. Eine Verbindung gemäß Anspruch 1, worin
X ist gewählt aus der Gruppe bestehend aus CHCN und CHNO₂;
R¹ ist gewählt aus der Gruppe bestehend aus Heterocyclus und Heterocyclusalkyl;
R³ ist Alkyl; und
R⁵ ist Wasserstoff.

70. Eine Verbindung gemäß Anspruch 1, worin
X gewählt ist aus der Gruppe bestehend aus CHCN und CHNO₂;
R¹ ist gewählt aus der Gruppe bestehend aus Aryl und Arylalkyl;
R³ ist gewählt aus der Gruppe bestehend aus Heterocyclus und Heterocyclusalkyl; und
R⁵ ist Wasserstoff.

71. Eine Verbindung gemäß Anspruch 1, worin
X gewählt ist aus der Gruppe bestehend aus CHCN und CHNO₂;
R¹ ist gewählt aus der Gruppe bestehend aus Aryl und Arylalkyl;
R³ ist gewählt aus der Gruppe bestehend aus Aryl und Arylalkyl; und
R⁵ ist Wasserstoff.

72. Eine Verbindung gemäß Anspruch 1, worin
X gewählt ist aus der Gruppe bestehend aus CHCN und CHNO₂;
R¹ ist gewählt aus der Gruppe bestehend aus Aryl und Arylalkyl;
R³ ist Alkyl; und
R⁵ ist Wasserstoff.

73. Eine Verbindung gemäß Anspruch 1, worin
X C (CN) ₂ ist ;
R¹ ist gewählt aus der Gruppe bestehend aus Heterocyclus und Heterocyclusalkyl;
R³ ist gewählt aus der Gruppe bestehend aus Aryl und Arylalkyl; und
R⁵ ist Wasserstoff.

74. Eine Verbindung gemäß Anspruch 1, worin
X C (CN) ₂ ist;
R¹ ist Heterocyclus, worin Heterocyclus gewählt ist aus der Gruppe bestehend aus Chinolin, Pyridin und Pyrimidin;
R² ist gewählt aus der Gruppe bestehend aus Wasserstoff, Alkenyl, Alkenyloxyalkyl, Alkenyloxy(alkenyloxy)alkyl, Alkoxyalkyl, Alkyl, Alkylthioalkyl, Aryl, worin Aryl Phenyl ist, Arylalkyl, worin der Arylanteil von Arylalkyl Phenyl ist, Cyanoalkyl, Cycloalkenylalkyl, Cycloalkyl, Cycloalkylalkyl, Haloalkyl, (NR⁹R¹⁰) Alkyl und Heterocyclus, worin Heterocyclus gewählt ist aus der Gruppe bestehend aus 1,3-Dioxan, Pyrrolidin und Thiophen;
R³ ist Aryl, worin Aryl Phenyl ist; und
R⁵ ist Wasserstoff.

75. Eine Verbindung gemäß Anspruch 74, die 4-Chlor-N-(1-{[2,2-dicyano-1-(3-pyridinylamino)vinyl]amino}-2,2-dimethylpropyl)benzamid ist.

76. Eine Verbindung gemäß Anspruch 1 von Formel VI: 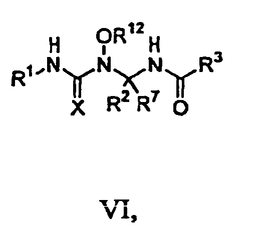 oder ein pharmazeutisch verträgliches Salz davon.

77. Eine Verbindung gemäß Anspruch 76, worin
X NR⁸ ist; und
R⁸ ist Cyano.

78. Eine Verbindung gemäß Anspruch 76, worin
X NR⁸ ist; und
R⁸ ist gewählt aus der Gruppe bestehend aus Wasserstoff, Alkoxy, Alkyl, Alkylsulfonyl, Arylalkoxy, Aryloxy, Arylsulfonyl, Haloalkylsulfonyl, Heterocyclusalkoxy, Hydroxy, Nitro und Sulfamyl.

79. Eine Verbindung gemäß Anspruch 76, worin X S ist.

80. Eine Verbindung gemäß Anspruch 76, worin X O ist.

81. Eine Verbindung gemäß Anspruch 76, worin X gewählt ist aus der Gruppe bestehend aus CHCN und CHNO₂.

82. Eine Verbindung, gewählt aus der Gruppe bestehend aus
4-Chlor-N-(1-{[(hydroxyimino)(3-pyridinylamino)methyl]amino}-2,2-dimethylpropyl)benzamid;
4-Chlor-N-(1-{[(methoxyimino)(3-pyridinylamino)methyl]amino}-2,2-dimethylpropyl)benzamid;
4-Chlor-N-(1-{[{[(4-fluorbenzyl)oxy]imino}(3-pyridinylamino)methyl]amino}-2,2-dimethylpropyl)benzamid;
4-Chlor-N-(2,2-dimethyl-1-{[[(methylsulfonyl)imino](3-pyridinylamino)methyl]amino}propyl)benzamid;
3-(4-Chlorphenyl)-N-(1-{[(cyanoimino)(3-pyridinylamino)methyl]amino}-2,2-dimethylpropyl)propanamid;
N-(1-{[(Cyanoimino)(3-pyridinylamino)methyl]amino}-2,2-dimethylpropyl)-3-phenylpropanamid;
N-(1-{[(Cyanoimino)(3-pyridinylamino)methyl]amino}-2,2-dimethylpropyl)-2-phenylacetamid;
4-(Aminosulfonyl)-N-(1-{[(cyanoimino) (3-pyridinylamino)methyl]amino}-2,2-dimethylpropyl)-2-fluorbenzamid;
4-Chlor-N-[1-({(cyanoimino)[4-ethyl-3-pyridinyl)amino]methyl}amino)-2,2-dimethylpropyl]benzamid;
N-(1-{[(Cyanoimino)(3-pyridinylamino)methyl]amino}-2,2-dimethylpropyl)-4-(trifluormethoxy)benzamid;
4-Chlor-N-[1-({(cyanoimino)[(4-ethyl-3-pyridinyl)amino]methyl}amino)-2,2-dimethylpropyl]-2-fluorbenzamid;
4-Chlor-N-(1-{[(cyanoimino)(5-pyrimidinylamino)methyl]amino}-2,2-dimethylpropyl]benzamid;
4-Chlor-N-(1-{[(cyanoimino)(5-pyrimidinylamino)methyl]amino}-2,2-dimethylpropyl]-2-fluorbenzamid;
N-(1-{[[(4-Brom-3-pyridinyl)amino](cyanoimino)methyl]amino}-2,2-dimethylpropyl)-4-chlorbenzamid;
4-Chlor-2-fluor-N-[2,2,2-trichlor-1-({(cyanoimino)[(4-ethyl-3-pyridinyl)amino]methyl}amino)ethyl]benzamid;
4-Chlor-N-(2,2,2-trichlor-1-{[(cyanoimino)(5-pyrimidinylamino)methyl]amino}ethyl)benzamid;
4-Chlor-2-fluor-N-(2,2,2-trichlor-1-{[(cyanoimino)(5-pyrimidinylamino)methyl]amino}ethyl)benzamid;
N-(1-{[[(4-Brom-3-pyridinyl)amino](cyanoimino)methyl]amino}-2,2,2-trichlorethyl)-4-chlorbenzamid;
N-(1-{[[(2-Brom-3-pyridinyl)amino](cyanoimino)methyl]amino}-2,2-dimethylpropyl)-4-chlorbenzamid;
4-Chlor-N-[1-({(cyanoimino)[(2-ethyl-3-pyridinyl)amino]methyl}amino)2,2-dimethylpropyl]benzamid;
N-(1-{[[(5-Brom-4-ethyl-3-pyridinyl)amino](cyanoimino)methyl]amino}-2,2-dimethylpropyl)-4-chlorbenzamid;
4-Chlor-N-[1-({(cyanoimino)[(4,5-dibrom-3-pyridinyl)amino]methyl}amino)-2,2-dimethylpropyl]benzamid;
4-Chlor-N-(1-{[[(5-chlor-3-pyridinyl)amino](cyanoimino)methyl]amino}-2,2-dimethylpropyl]benzamid;
N-(1-{[[(5-Brom-6-chlor-3-pyridinyl)amino](cyanoimino)methyl]amino}-2,2-dimethylpropyl)-4-chlorbenzamid;
N-(1-{[[(5-Brom-3-pyridinyl)amino](cyanoimino)methyl]amino}-2,2-dimethylpropyl)-4-chlorbenzamid;
N-(1-{[[(6-Brom-3-pyridinyl)amino](cyanoimino)methyl]amino}-2,2-dimethylpropyl)-4-chlorbenzamid;
4-Chlor-N-(1-{[(cyanoimino)({5-[(4-fluorphenyl)sulfonyl]-3-pyridinyl}amino)methyl]amino}-2,2-dimethylpropyl)benzamid;
N-(1-{[({5-[(Aminoperoxy)sulfanyl]-3-pyridinyl}amino)(cyanoimino)methyl]amino}-2,2-dimethylpropyl)-4-chlorbenzamid;
N-(1-{[[(6-Brom-4-fluor-3-pyridinyl)amino](cyanoimino)methyl]amino}-2,2-dimethylpropyl)-4-chlorbenzamid;
4-Chlor-N-[1-{[(cyanoimino)(3-pyridinylamino)methyl]amino}-2,2,2-trifluor-1-(trifluormethyl)ethyl]benzamid;
4-Chlor-N-[1-{[(cyanoimino)(3-pyridinylamino)methyl]amino}cyclopentyl)benzamid;
4-Chlor-N-[1-{[(cyanoimino)(3-pyridinylamino)methyl]amino}cyclohexyl)benzamid;
4-Chlor-N-[{[(cyanoimino)(3-pyridinylamino)methyl]amino}(2,6-dimethylphenyl)methyl]benzamid;
4-Chlor-N-[{[(cyanoimino)(3-pyridinylamino)methyl]amino}(3-pyridinyl)methyl]benzamid;
4-Chlor-N-[{[(cyanoimino)(3-pyridinylamino)methyl]amino}(2-pyridinyl)methyl]benzamid;
4-Chlor-N-(1-{[(cyanoimino)(3-pyridinylamino)methyl]amino}-2-methyl-2-phenylpropyl)benzamid;
4-Chlor-N-(1-{[(cyanoimino)(3-pyridinylamino)methyl]amino}-3,3-dimethyl-2-oxobutyl)benzamid;
4-Chlor-N-(1-{[(cyanoimino)(3-pyridinylamino)methyl]amino)-3,3,3-trifluor-2-oxopropyl)benzamid;
4-Chlor-N-[1-{[(cyanoimino)(3-pyridinylamino)methyl]amino}-3,3,3-trifluor-2-methyl-2-(trifluormethyl)propyl]benzamid;
Methyl 4-[(4-Chlorbenzoyl)aminö]-4-{[(cyanoimino)(3-pyridinylamino)methyl]amino}-3,3-dimethylbutanoat;
4-Chlor-N-[1-{[(cyanoimino)(3-pyridinylamino)methyl]amino}-4-(dimethylamino)-2,2-dimethylbutyl]benzamid;
4-Chlor-N-(4-cyano-1-{[(cyanoimino)(3-pyridinylamino)methyl]amino}-2,2-dimethylbutyl]benzamid;
4-Chlor-N-(1-{[(cyanoimino)(3-pyridinylamino)methyl]amino}-4-methoxy-2,2-dimethylbutyl)benzamid;
4-Chlor-N-(1-{[(cyanoimino)(3-pyridinylamino)methyl]amino}-4-hydroxy-2,2-dimethylbutyl)benzamid;
N-(4-(Aminosulfonyl)-1-{[(cyanoimino)(3-pyridinylamino)methyl]amino}-2,2-dimethylbutyl)-4-chlorbenzamid;
4-Chlor-N-[1-{[(cyanoimino)(3-pyridinylamino)methyl]amino}-2,2-dimethyl-4-(phenylsulfonyl)butyl]benzamid;
4-Chlor-N-[1-{[(cyanoimino)(3-pyridinylamino)methyl]amino}-3-hydroxy-2,2-dimethylpropyl)benzamid;
N-(4-(Aminosulfonyl)-2,2-dichlor-1-{[(cyanoimino)(3-pyridinylamino)methyl]amino}butyl)-4-chlorbenzamid;
4-Chlor-N-[4-cyano-1-{[(cyanoimino)(3-pyridinylamino)methyl]amino}-2,2-bis(trifluormethyl)butyl]benzamid;
4-Chlor-N-(1-{[(cyanoimino)(3-pyridinylamino)methyl]amino}-2,2-difluor-4-oxopentyl)benzamid;
4-Chlor-N-(1-{[2-cyano-1-(3-pyridinylamino)ethenyl]amino}-2,2-dimethylpropylbenzamid;
4-Chlor-N-{1-[[(cyanoimino)(3-pyridinylamino)methyl](hydroxy)amino]-2,2-dimethylpropyl}benzamid;
4-Chlor-N-(2,2,2-trichlor-1-{[2-nitro-1-(3-pyridinylamino)ethenyl]amino}ethyl)benzamid; und
4-Chlor-N-(2,2,2-trichlor-1-{[2-cyano-1-(3-pyridinylamino)ethenyl]amino}ethyl)benzamid.

83. Eine pharmazeutische Zusammensetzung, umfassend eine therapeutisch wirksame Menge einer Verbindung mit der Formel I 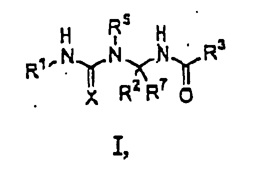 oder eines pharmazeutisch verträglichen Salzes davon, in Kombination mit einem pharmazeutisch verträglichen Träger, worin
X gewählt ist aus der Gruppe bestehend aus O, S, CHCN, C(CN)₂, CHNO₂ und NR⁸;
R⁸ ist gewählt aus der Gruppe bestehend aus Wasserstoff, Alkoxy, Alkyl, Alkylsulfonyl, Arylalkoxy, Aryloxy, Arylsulfonyl, Cyano, Haloalkylsulfonyl, Heterocyclusalkoxy, Heterocyclusoxy, Hydroxy, Nitro und Sulfamyl;
R¹ ist gewählt aus der Gruppe bestehend aus Aryl, Arylalkyl, Heterocyclus und Heterocyclusalkyl;
R² ist gewählt aus Wasserstoff, Alkenyl, Alkenyloxyalkyl, Alkenyloxy(alkenyloxy)alkyl, Alkoxyalkyl, Alkoxycarbonyl, Alkoxycarbonylalkyl, Alkoxycarbonyl(halo)alkyl, Alkoxy(halo)alkyl, Alkyl, Alkylcarbonyl, Alkylcarbonylalkyl, Alkylcarbonyl(halo)alkyl, Alkylcarbonyloxyalkyl, Alkylsulfinylalkyl, Alkylsulfonylalkyl, Alkylthioalkyl, Alkinyl, Amido, Amidoalkyl, Aryl, Arylalkoxyalkyl, Arylalkoxycarbonyl, Arylalkoxycarbonylalkyl, Arylalkyl, Arylcarbonyl, Arylcarbonylalkyl, Arylcarbonyloxyalkyl, Aryl(halo)alkyl, Aryloxyalkyl, Aryloxycarbonyl, Aryloxycarbonylalkyl, Arylalkylthioalkyl, Arylsulfonylalkyl, Carboxy, Carboxyalkyl, Carboxy(halo)alkyl, Cyanoalkyl, Cyano(halo)alkyl, Cycloalkenyl, Cycloalkenylalkyl, Cycloalkyl, Cycloalkylalkoxyalkyl, Cycloalkylalkyl, Cycloalkylcarbonyl, Cycloalkyloxyalkyl, Cycloalkylalkylthioalkyl, Formyl, Haloalkenyl, Haloalkyl, haloalkylcarbonyl, Haloalkinyl, Heterocyclus, Heterocyclusalkoxyalkyl, Heterocyclusalkyl, Heterocycluscarbonyl, Heterocyclusoxyalkyl, Heterocyclusalkylthioalkyl, Hydroxyalkyl, Mercaptoalkyl, Sulfamylalkyl, Sulfamyl (halo) alkyl und (NR⁹R¹⁰)Alkyl, worin R⁹ und R¹⁰ unabhängig gewählt sind aus Wasserstoff, Alkyl, Alkylcarbonyl, Aryl, Arylalkyl, Arylcarbonyl, Formyl und S(O)₂R¹¹, worin R¹¹ gewählt ist aus Alkyl, Aryl und Arylalkyl;
R³ ist gewählt aus der Gruppe bestehend aus Alkyl, Aryl, Arylalkyl, Heterocyclus und Heterocyclusalkyl;
R⁵ ist gewählt aus der Gruppe bestehend aus Wasserstoff, Alkyl und OR¹²; R¹² ist gewählt aus der Gruppe bestehend aus Wasserstoff, Alkyl und Arylalkyl; und
R⁷ ist gewählt aus der Gruppe bestehend aus Wasserstoff, Haloalkyl und C₁-C₆ Alkyl; oder
R⁷ und R² zusammengenommen mit dem Kohlenstoffatom, an welches sie gebunden sind, bilden einen 5- oder 6-gliedrigen carbocyclischen Ring, worin der 5- oder 6-gliedrige carbocyclische Ring wahlweise substituiert ist mit 1 oder 2 Substituenten unabhängig gewählt aus der Gruppe bestehend aus Alkenyl, Alkoxy, Alkyl, Alkinyl, Halogen, Haloalkoxy und Haloalkyl;
worin, wenn nichts anderes angegeben ist, Alkyl alleine oder als Teil einer anderen Gruppe von ein bis zehn Kohlenstoffatome enthält;
Alkenyl alleine oder als Teil einer anderen Gruppe, enthält von zwei bis zehn Kohlenstoffatome;
Alkinyl alleine oder als Teil einer anderen Gruppe, enthält von zwei bis zehn Kohlenstoffatome;
Aryl alleine oder als Teil einer anderen Gruppe ist ein monocyclisches carbocyclisches Ringsystem oder ein bicyclisches carbocyclisches ankondensiertes Ringsystem, mit einem oder mehreren aromatischen Ringen,
Aryl kann wahlweise 1, 2, 3, 4 oder 5 Substituenten haben, unabhängig gewählt aus der Gruppe bestehend aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Sulfamyl, Sulfamylalkyl, Phenyl, Furyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Cycloalkyl ist ein monocyclisches, bicyclisches oder tricyclisches Ringsystem;
Cycloalkyl kann wahlweise 1, 2, 3, 4 oder 5 Substituenten haben, unabhängig gewählt aus der Gruppe bestehend aus Alkenyl, Alkoxy, Alkoxycarbonyl, Alkoxycarbonylalkyl, Alkyl, Alkylcarbonylalkyl, Alkylcarbonyloxy, Alkylcarbonyloxyalkyl, Alkylsulfonylalkyl, Alkinyl, Amido, Amidoalkyl, Cyanoalkyl, Halo, Haloalkoxy, Haloalkyl, Hydroxy, Hydroxyalkyl, Sulfamylalkyl, -NR^{A}R^{B} und (NR^{A}R^{B})Alkyl, worin R^{A} und R^{B} unabhängig gewählt sind aus Wasserstoff, Alkyl, Alkylcarbonyl und Formyl;
Heterocyclus alleine oder als Teil einer anderen Gruppe ist ein monocyclisches oder bicyclisches Ringsystem, worin (i) das monocyclische Ringsystem ein 5- oder 6-gliedriger Ring ist, enthaltend 1, 2, 3 oder 4 Heteroatome, unabhängig gewählt aus Sauerstoff, Stickstoff und Schwefel, worin der 5-gliedrige Ring von 0-2 Doppelbindungen hat, und der 6-gliedrige Ring hat von 0-3 Doppelbindungen, und (ii) das bicyclische Ringsystem ist irgendeines der oben genannten monocyclischen Ringsysteme, ankondensiert an eine monocyclische Arylgruppe, wie oben definiert, eine monocyclische Cycloalkylgruppe, wie oben definiert, oder ein monocyclisches heterocyclisches Ringsystem, wie oben definiert;
Heterocyclus kann wahlweise 1, 2 oder 3 Substituenten haben, unabhängig gewählt aus der Gruppe bestehend aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Oxo, Sulfamyl, Sulfamylalkyl, -NR^{A}R^{B}, (NR^{A}R^{B})Alkyl, worin R^{A} und R^{B} unabhängig gewählt sind aus Wasserstoff, Alkyl, Alkylcarbonyl und Formyl, und Phenyl hat wahlweise 1 oder 2 Substituenten unabhängig gewählt aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Sulfamyl, Sulfamylalkyl, -NR^{A}R^{B}, (NR^{A}R^{B})Alkyl, worin R^{A} und R^{B} unabhängig gewählt sind aus Wasserstoff, Alkyl, Alkylcarbonyl und Formyl;
Amido alleine oder als Teil einer anderen Gruppe ist -C (O)NR⁹R¹⁰, worin R⁹ und R¹⁰ unabhängig gewählt sind aus der Gruppe bestehend aus Wasserstoff, Alkyl, Alkylcarbonyl, Phenyl, Phenylalkyl, Phenylcarbonyl, Formyl und S(O)₂R¹¹, worin R¹¹ gewählt ist aus Alkyl, Phenyl und Phenylalkyl;
Cycloalkenyl alleine oder als Teil einer anderen Gruppe ist ein cyclischer Kohlenwasserstoff, enthaltend von drei bis acht Kohlenstoffe und enthaltend mindestens eine Kohlenstoff-Kohlenstoff-Doppelbindung;
Cycloalkenyl kann wahlweise 1, 2, 3, 4 oder 5 Substituenten haben, unabhängig gewählt aus Alkenyl, Alkoxy, Alkoxycarbonyl, Alkoxycarbonylalkyl, Alkyl, Alkylcarbonyloxy, Alkylcarbonyloxyalkyl, Alkinyl, Amido, Amidoalkyl, Halo, Haloalkoxy, Haloalkyl, Hydroxy, Hydroxyalkyl, Sulfamylalkyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Sulfamyl ist eine -SO₂NR⁹⁴R⁹⁵ Gruppe, worin R⁹⁴ und R⁹⁵ unabhängig gewählt sind aus Wasserstoff, Alkyl, Aryl und Arylalkyl;
vorausgesetzt, daß, wenn X O ist, R² -CCl₃ ist; R³ Alkyl oder Phenyl ist; und R⁵ und R⁷ Wasserstoff sind; dann ist R¹ nicht Phenyl;
weiter vorausgesetzt, daß
N-[[[(Phenylamino)carbonyl]amino]methyl]-3-pyridincarboxamid,
N-[[[[(2-Methylphenyl)amino)carbonyl]amino]methyl]-3,-pyridincarboxamid;
N-[[[[(4-Methylphenyl)amino)carbonyl]amino]methyl]-3-pyridincarboxamid;
N-[[[[(3-Ethoxyphenyl)amino)carbonyl]amino]methyl]-3-pyridincarboxamid;
N-[[[[(4-Nitrophenyl)amino)carbonyl]amino]methyl]-3-pyridincarboxamid;
und
N-[[[[(3-Methylphenyl)amino)carbonyl]amino]methyl]-3-pyridincarboxamid
ausgeschlossen sind.

84. Verwendung einer Verbindung mit der Formel I: 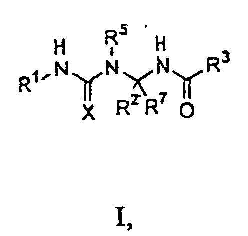 oder eines pharmazeutisch verträglichen Salzes davon, für die Herstellung eines Medikaments zur Behandlung oder Verhinderung einer Erkrankung, welche verhindert oder verbessert wird mit Kaliumkanalöffnern in einem Wirtsäugetier, das eine solche Behandlung benötigt, durch Verabreichen einer therapeutisch wirksamen Menge,
worin
X gewählt ist aus der Gruppe bestehend aus O, S, CHCN, C(CN)₂, CHNO₂ und NR⁸;
R⁸ ist gewählt aus der Gruppe bestehend aus Wasserstoff, Alkoxy, Alkyl, Alkylsulfonyl, Arylalkoxy, Aryloxy, Arylsulfonyl, Cyano, Haloalkylsulfonyl, Heterocyclusalkoxy, Heterocyclusoxy, Hydroxy, Nitro und Sulfamyl;
R¹ ist gewählt aus der Gruppe bestehend aus Aryl, Arylalkyl, Heterocyclus und Heterocyclusalkyl;
R² ist gewählt aus Wasserstoff, Alkenyl, Alkenyloxyalkyl, Alkenyloxy(alkenyloxy)alkyl, Alkoxyalkyl, Alkoxycarbonyl, Alkoxycarbonylalkyl, Alkoxycarbonyl(halo)alkyl, Alkoxy(halo)alkyl, Alkyl, Alkylcarbonyl, Alkylcarbonylalkyl, Alkylcarbonyl(halo)alkyl, Alkylcarbonyloxyalkyl, Alkylsulfinylalkyl, Alkylsulfonylalkyl, Alkylthioalkyl, Alkinyl, Amido, Amidoalkyl, Aryl, Arylalkoxyalkyl, Arylalkoxycarbonyl, Arylalkoxycarbonylalkyl, Arylalkyl, Arylcarbonyl, Arylcarbonylalkyl, Arylcarbonyloxyalkyl, Aryl(halo)alkyl, Aryloxyalkyl, Aryloxycarbonyl, Aryloxycarbonylalkyl, Arylalkylthioalkyl, Arylsulfonylalkyl, Carboxy, Carboxyalkyl, Carboxy(halo)alkyl, Cyanoalkyl, Cyano(halo)alkyl, Cycloalkenyl, Cycloalkenylalkyl, Cycloalkyl, Cycloalkylalkoxyalkyl, Cycloalkylalkyl, Cycloalkylcarbonyl, Cycloalkyloxyalkyl, Cycloalkylalkylthioalkyl, Formyl, Haloalkenyl, haloalkyl, Haloalkylcarbonyl, Haloalkinyl, Heterocyclus, Heterocyclusalkoxyalkyl, Heterocyclusalkyl, Heterocycluscarbonyl, Heterocyclusoxyalkyl, Heterocyclusalkylthioalkyl, Hydroxyalkyl, Mercaptoalkyl, Sulfamylalkyl, Sulfamyl (halo) alkyl, und (NR⁹R¹⁰)Alkyl, worin R⁹ und R¹⁰ unabhängig gewählt sind aus Wasserstoff, Alkyl, Alkylcarbonyl, Aryl, Arylalkyl, Arylcarbonyl, Formyl und S(O)₂R¹¹, worin R¹¹ gewählt ist aus Alkyl, Aryl und Arylalkyl;
R³ ist gewählt aus der Gruppe bestehend aus Alkyl, Aryl, Arylalkyl, Heterocyclus und Heterocyclusalkyl;
R⁵ ist gewählt aus der Gruppe bestehend aus Wasserstoff, Alkyl und OR¹², R¹² ist gewählt aus der Gruppe bestehend aus Wasserstoff, Alkyl und Arylalkyl; und
R⁷ ist gewählt aus der Gruppe bestehend aus Wasserstoff, Haloalkyl und C₁-C₆ Alkyl; oder
R⁷ und R² zusammengenommen mit dem Kohlenstoffatom, an welches sie gebunden sind, bilden einen 5- oder 6-gliedrigen carbocyclischen Ring, worin der 5- oder 6-gliedrige carbocyclische Ring wahlweise substituiert ist mit 1 oder 2 Substituenten unabhängig gewählt aus der Gruppe bestehend aus Alkenyl, Alkoxy, Alkyl, Alkinyl, Halogen, Haloalkoxy und Haloalkyl;
worin, wenn nichts anderes angegeben ist, Alkyl alleine oder als Teil einer anderen Gruppe, von ein bis zehn Kohlenstoffatome enthält;
Alkenyl alleine oder als Teil einer anderen Gruppe, enthält von zwei bis zehn Kohlenstoffatome;
Alkinyl alleine oder als Teil einer anderen Gruppe, enthält von zwei bis zehn Kohlenstoffatome;
Aryl alleine oder als Teil einer anderen Gruppe ist ein monocyclisches carbocyclisches Ringsystem oder ein bicyclisches carbocyclisches ankondensiertes Ringsystem mit einem oder mehreren aromatischen Ringen,
Aryl kann wahlweise 1, 2, 3, 4 oder 5 Substituenten haben, unabhängig gewählt aus der Gruppe bestehend aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Sulfamyl, Sulfamylalkyl, Phenyl, Furyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Cycloalkyl ist ein monocyclisches, bicyclisches oder tricyclisches Ringsystem;
Cycloalkyl kann wahlweise 1, 2, 3, 4 oder 5 Substituenten haben, unabhängig gewählt aus der Gruppe bestehend aus Alkenyl, Alkoxy, Alkoxycarbonyl, Alkoxycarbonylalkyl, Alkyl, Alkylcarbonylalkyl, Alkylcarbonyloxy, Alkylcarbonyloxyalkyl, Alkylsulfonylalkyl, Alkinyl, Amido, Amidoalkyl, Cyanoalkyl, Halo, Haloalkoxy, Haloalkyl, Hydroxy, Hydroxyalkyl, Sulfamylalkyl, -NR^{A}R^{B} und (NR^{A}R^{B}) Alkyl, worin R^{A} und R^{B} unabhängig gewählt sind aus Wasserstoff, Alkyl, Alkylcarbonyl und Formyl;
Heterocyclus alleine oder als Teil einer anderen Gruppe ist ein monocyclisches oder bicyclisches Ringsystem, worin (i) das monocyclische Ringsystem ein 5- oder 6-gliedriger Ring ist, enthaltend 1, 2, 3 oder 4 Heteroatome, unabhängig gewählt aus Sauerstoff, Stickstoff und Schwefel, worin der 5-gliedrige Ring von 0-2 Doppelbindungen hat und der 6-gliedrige Ring von 0-3 Doppelbindungen hat, und (ii) das bicyclische Ringsystem irgendines der obigen monocyclischen Ringsysteme ist, ankondensiert an eine monocyclische Arylgruppe, wie oben definiert, eine monocyclische Cycloalkylgruppe, wie oben definiert, oder ein monocyclisches heterocyclisches Ringsystem, wie oben definiert;
Heterocyclus kann wahlweise 1, 2 oder 3 Substituenten haben, unabhängig gewählt aus der Gruppe bestehend aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Oxo, Sulfamyl, Sulfamylalkyl, -NR^{A}R^{B}, (NR^{A}R^{B}) Alkyl, worin R^{A} und R^{B} unabhängig gewählt sind aus Wasserstoff, Alkyl, Alkylcarbonyl und Formyl, und Phenyl hat wahlweise 1 oder 2 Substituenten unabhängig gewählt aus Alkenyl, Alkoxy, Alkoxycarbonyl, Alkoxycarbonylalkyl, Alkyl, Alkylcarbonyl, Alkylcarbonyloxy, Alkylcarbonyloxyalkyl, Alkylsulfinyl, Alkylsulfonyl, Alkinyl, Amido, Amidoalkyl, Phenylalkoxycarbonyl, Phenylalkoxycarbonylalkyl, Phenylcarbonyloxy, Phenylcarbonyloxyalkyl, Phenyloxycarbonyl, Phenyloxycarbonylalkyl, Phenylsulfonyl, Cyano, Halo, Haloalkyl, Haloalkoxy, Nitro, Sulfamyl, Sulfamylalkyl, -NR^{A}R^{B}, (NR^{A}R^{B}) Alkyl, worin R^{A} und R^{B} unabhängig gewählt sind aus Wasserstoff, Alkyl, Alkylcarbonyl und Formyl;
Amido alleine oder als Teil einer anderen Gruppe ist -C (O)NR⁹R¹⁰, worin R⁹ und R¹⁰ unabhängig gewählt sind aus der Gruppe bestehend aus Wasserstoff, Alkyl, Alkylcarbonyl, Phenyl, Phenylalkyl, Phenylcarbonyl, Formyl und S(O)₂R¹¹, worin R¹¹ gewählt ist aus Alkyl, Phenyl und Phenylalkyl;
Cycloalkenyl alleine oder als Teil einer anderen Gruppe ist ein cyclischer Kohlenwasserstoff, enthaltend von drei bis acht Kohlenstoffe, und enthaltend mindestens eine Kohlenstoff-Kohlenstoff-Doppelbindung;
Cycloalkenyl kann wahlweise 1, 2, 3, 4 oder 5 Substituenten haben, unabhängig gewählt aus Alkenyl, Alkoxy, Alkoxycarbonyl, Alkoxycarbonylalkyl, Alkyl, Alkylcarbonyloxy, Alkylcarbonyloxyalkyl, Alkinyl, Amido, Amidoalkyl, Halo, Haloalkoxy, Haloalkyl, Hydroxy, Hydroxyalkyl, Sulfamylalkyl, -NR^{A}R^{B} und (NR^{A}R^{B})Alkyl, worin R^{A} und R^{B} unabhängig gewählt sind aus Wasserstoff, Alkyl, Alkylcarbonyl und Formyl;
Sulfamyl ist eine -SO₂NR⁹⁴R⁹⁵ Gruppe, worin R⁹⁴ und R⁹⁵ unabhängig gewählt sind aus Wasserstoff, Alkyl, Aryl und Arylalkyl;
vorausgesetzt, daß, wenn X O ist; R² -CCl₃ ist; R³ Alkyl oder Phenyl ist; und R⁵ und R⁷ Wasserstoff sind; dann ist R¹ nicht Phenyl;
weiter vorausgesetzt, daß die Verwendung von
N-[[[(Phenylamino)carbonyl]amino]methyl]-3-pyridincarboxamid;
N-[[[[(2-Methylphenyl)amino)carbonyl]amino]methyl]-3-pyridincarboxamid;
N-[[[[(4-Methylphenylamino)carbonyl]amino]methyl]-3-pyridincarboxamid;
N-[[[[(3-Ethoxyphenyl)amino)carbonyl]amino]methyl]-3-pyridincarboxamid;
N-[[[[(4-Nitrophenyl)amino)carbonyl]amino]methyl]-3-pyridincarboxamid;
oder
N-[[[[(3-Methylphenyl)amino)carbonyl]amino]methyl]-3-pyridincarboxamid
für die Herstellung eines Medikaments für die Behandlung der Parkinson'schen Krankheit ausgeschlossen ist.

85. Die Verwendung von Anspruch 84, worin die Erkrankung gewählt ist aus der Gruppe bestehend aus Asthma, Epilepsie, Raynaud's Syndrom, Claudicatio Intermittens, Migräne, Schmerz, Pollakisurie, Blaseninstabilität, Nykturie, Blasenhyperreflexie, Enurese, Alopezie, Kardioprotektion, Ischämie, Essstörungen, funktionelle Darmerkrankungen und Neurodegeneration.

86. Die Verwendung von Anspruch 84, worin die Erkrankung Blasenüberaktivität ist.

87. Die Verwendung von Anspruch 84, worin die Erkrankung benigne Prostatahyperplasie ist.

88. Die Verwendung von Anspruch 84, worin die Erkrankung Dysmenorrhoe ist.

89. Die Verwendung von Anspruch 84, worin die Erkrankung Frühgeburt ist.

90. Die Verwendung von Anspruch 84, worin die Erkrankung Harninkontinenz ist.

91. Die Verwendung von Anspruch 84, worin die Erkrankung gewählt ist aus der Gruppe bestehend aus männlicher erektiler Dysfunktion und vorzeitigem Samenerguss.

92. Die Verwendung von Anspruch 84, worin die Erkrankung weibliche sexuelle Dysfunktion ist.

93. Verwendung einer Verbindung von Formel VII: 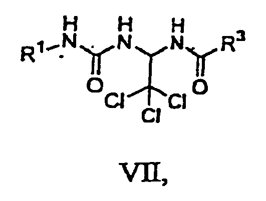 oder eines pharmazeutisch verträglichen Salzes davon, worin
R¹ Phenyl ist;
R³ ist gewählt aus der Gruppe bestehend aus Alkyl und Phenyl, für die Herstellung eines Medikaments zur Behandlung oder Verhinderung einer Erkrankung, welche verhindert oder verbessert wird mit Kaliumkanalöffnern, in einem Wirtsäugetier, das eine solche Behandlung benötigt durch Verabreichen einer therapeutisch wirksamen Menge.

94. Eine Verbindung gemäß Anspruch 93, gewählt aus der Gruppe bestehend aus
N-{1-[(Anilinocarbonyl)amino]-2,2,2-trichlorethyl}-4-methylbenzamid und
4-Methyl-N-(2,2,2-trichlor-1-{[(2-fluoranilino)carbonyl]amino}ethyl)benzamid.

95. Die Verwendung von Anspruch 93, worin die Erkrankung gewählt ist aus der Gruppe bestehend aus Asthma, Epilepsie, Raynaud's Syndrom, Claudicatio Intermittens, Migräne, Schmerz, Pollakisurie, Blaseninstabilität, Nykturie, Blasenhyperreflexie, Enurese, Alopezie, Kardioprotektion, Ischämie, Essstörungen, funktionelle Darmerkrankungen und Neurodegeneration.

96. Die Verwendung von Anspruch 93, worin die Erkrankung Blasenüberaktivität ist.

97. Die Verwendung von Anspruch 93, worin die Erkrankung benigne Prostatahyperplasie ist.

98. Die Verwendung von Anspruch 93, worin die Erkrankung Dysmenorrhoe ist.

99. Die Verwendung von Anspruch 93, worin die Erkrankung Frühgeburt ist.

100. Die Verwendung von Anspruch 93, worin die Erkrankung Harninkontinenz ist.

101. Die Verwendung von Anspruch 93, worin die Erkrankung egwählt ist aus der Gruppe bestehend männlicher erektiler Dysfunktion und vorzeitigem Samenerguss.

102. Die Verwendung von Anspruch 93, worin die Erkrankung weibliche sexuelle Dysfunktion ist.

103. Eine Verbindung von irgendeinem der Ansprüche 1-82 und 94 für die Verwendung als ein therapeutisches Mittel, vorausgesetzt, daß
N-[[[[(2-Methylphenyl)amino]carbonyl]amino]phenylmethyl]-benzamid;
N-[[[[(4-Chlorphenyl)amino]carbonyl]amino]phenylmethyl]-benzamid;
N-[2,2-Dichlor-1-[[phenylamino)thioxomethyl]amino]ethyl]-benzamid;
N-[2,2-Dichlor-1-[[phenylamino)thioxomethyl]amino]ethyl]-4-methyl-benzamid;
N-[2,2-Dichlor-1-[[[4-methylphenyl)amino)thioxomethyl]amino]ethyl]-benzamid;
N-[2,2-Dichlor-1-[[[4-methylphenyl)amino)thioxomethyl]amino]ethyl]-4-methyl-benzamid;
N-[2,2-Dichlor-1-[[[4-chlorphenyl)amino)thioxomethyl]amino]ethyl]-benzamid;
N-[2,2-Dichlor-1-[[[4-chlorphenyl)amino)thioxomethyl]amino]ethyl]-4-methyl-benzamid;
N-[[[(Phenylamino)carbonyl]amino]methyl]-benzamid;
N-[[[(Phenylamino)thioxomethyl]amino]methyl]-benzamid;
N-[[[(Phenylamino)carbonyl]amino]methyl]-4-chlor-benzamid;
N-[[[(Phenylamino)thioxomethyl]amino]methyl]-4-chlor-benzamid;
1-(1-Acetamido-2,2,2-trichlorethyl)-3-phenyl-2-thio-harnstoff;
1-Phenyl-2-thio-3-(2,2,2-trichlor-1-propionamidoethyl)-harnstoff; und
1-(1-Butyramido-2,2,2-trichlorethyl)-3-phenyl-2-thio-harnstoff nicht ausgeschlossen sind.

## Revendications

1. Composé répondant à la formule I : 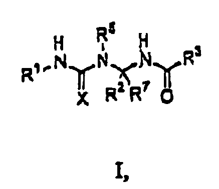 ou un sel pharmaceutiquement acceptable de celui-ci dans lequel,
X est choisi dans le groupe consistant en O, S, CHCN, C(CN)₂, CHNO₂ et NR⁸ ;
R⁸ est choisi dans le groupe consistant en un atome d'hydrogène, un groupe alcoxy, alkyle, alkylsulfonyle, arylalcoxy, aryloxy, arylsulfonyle, cyano, halogénoalkylsulfonyle, hétérocyclealcoxy, hétérocycleoxy, hydroxy, nitro, et sulfamyle ;
R¹ est choisi dans le groupe consistant en un groupe aryle, arylalkyle, hétérocycle, et hétérocyclealkyle ;
R² est choisi parmi un atome d'hydrogène, un groupe alcényle, alcényloxyalkyle, alcényloxy(alcényloxy)alkyle, alcoxyalkyle, alcoxycarbonyle, alcoxycarbonylalkyle, alcoxycarbonyl(halogéno)alkyle, alcoxy(halogéno)alkyle, alkyle, alkylcarbonyle, alkylcarbonylalkyle, alkylcarbonyl(halogéno)alkyle, alkylcarbonyloxyalkyle, alkylsulfinylalkyle, alkylsulfonylalkyle, alkylthioalkyle, alcynyle, amido, amidoalkyle, aryle, arylalcoxyalkyle, arylalcoxycarbonyle, arylalcoxycarbonylalkyle, arylalkyle, arylcarbonyle, arylcarbonylalkyle, arylcarbonyloxyalkyle, aryl(halogéno)alkyle, aryloxyalkyle, aryloxycarbonyle, aryloxycarbonylalkyle, arylalkylthioalkyle, arylsulfonylalkyle, carboxy, carboxyalkyle, carboxy(halogéno)alkyle, cyanoalkyle, cyano(halogéno)alkyle, cycloalcényle, cycloalcénylalkyle, cycloalkyle, cycloalkylalcoxyalkyle, cycloalkylalkyle, cycloalkylcarbonyle, cycloalkyloxyalkyle, cycloalkylalkylthioalkyle, formyle, halogénoalcényle, halogénoalkyle, halogénoalkylcarbonyle, halogénoalcynyle, hétérocycle, hétérocyclealcoxyalkyle, hétérocyclealkyle, hétérocyclecarbonyle, hétérocycleoxyalkyle, hétérocyclealkylthioalkyle, hydroxyalkyle, mercaptoalkyle, sulfamylalkyle, sulfamyl(halogéno)alkyle, et (NR⁹R¹⁰)alkyle dans lesquels R⁹ et R¹⁰ sont indépendamment choisis parmi un atome d'hydrogène, un groupe alkyle, alkylcarbonyle, aryle, arylalkyle, arylcarbonyle, formyle, et S(O)₂R¹¹, dans lequel R¹¹ est choisi parmi un groupe alkyle, aryle, et arylalkyle ;
R³ est choisi dans le groupe consistant en un groupe alkyle, aryle, arylalkyle, hétérocycle, et hétérocyclealkyle ;
R⁵ est choisi dans le groupe consistant en un atome d'hydrogène, un groupe alkyle et
OR¹² ;
R¹² est choisi dans le groupe consistant en un atome d'hydrogène, un groupe alkyle et arylalkyle ; et
R⁷ est choisi dans le groupe consistant en un atome d'hydrogène, un groupe halogénoalkyle ; et alkyle en C₁ à C₆ ; ou
R⁷ et R² pris conjointement avec l'atome de carbone auquel ils sont attachés, forment ensemble un cycle carbocyclique à 5 ou 6 chaînons dans lequel le cycle carbocyclique à 5 ou 6 chaînons est facultativement substitué par 1 ou 2 substituants indépendamment choisis dans le groupe consistant en un groupe alcényle, alcoxy, alkyle, alcynyle, halogéno, halogénoalcoxy, et halogénoalkyle ;
dans lequel sauf indication contraire, alkyle par lui-même ou comme partie d'un autre groupe contient d'un à dix atomes de carbone ;
alcényle par lui-même ou comme partie d'un autre groupe contient de deux à dix atomes de carbone ;
alcynyle par lui-même ou comme partie d'un autre groupe contient de deux à dix atomes de carbone ;
aryle par lui-même ou comme partie d'un autre groupe est un système de cycle carbocyclique monocyclique ou un système de cycle condensé carbocyclique bicyclique comportant un ou plusieurs cycles aromatiques,
aryle peut facultativement comporter 1, 2, 3, 4 ou 5 substituants indépendamment choisis dans le groupe consistant en un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, sulfamyle, sulfamylalkyle, phényle, furyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle ;
cycloalkyle est un système de cycle monocyclique, bicyclique ou tricyclique ;
cycloalkyle peut facultativement comporter 1, 2, 3, 4 ou 5 substituants indépendamment choisis dans le groupe consistant en un groupe alcényle, alcoxy, alcoxycarbonyle, alcoxycarbonylalkyle, alkyle, alkylcarbonylalkyle, alkylcarbonyloxy, alkylcarbonyloxyalkyle, alkylsulfonylalkyle, alcynyle, amido, amidoalkyle, cyanoalkyle, halogéno, halogénoalcoxy, halogénoalkyle, hydroxy, hydroxyalkyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle ;
hétérocycle par lui-même ou comme partie d'un autre groupe est un système de cycle monocyclique ou bicyclique dans lequel (i) le système de cycle monocyclique est un cycle à 5 ou 6 chaînons contenant 1, 2, 3 ou 4 hétéroatomes indépendamment choisis parmi l'oxygène, l'azote et le soufre dans lequel le cycle à 5 chaînons comporte de 0 à 2 doubles liaisons et le cycle à 6 chaînons comporte de 0 à 3 doubles liaisons et (ii) le système de cycle bicyclique est l'un quelconque des systèmes de cycle monocycliques ci-dessus condensés à un groupe aryle monocyclique tel que défini ci-dessus, un groupe cycloalkyle monocyclique tel que défini ci-dessus ou un système de cycle hétérocyclique monocyclique tel que défini ci-dessus ;
hétérocycle peut facultativement comporter 1, 2 ou 3 substituants indépendamment choisis dans le groupe consistant en un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, oxo, sulfamyle, sulfamylalkyle, (-NR^{A}R^{B}) et (NR^{A}R^{B})alkyle dans lequel R^{A} et R^{B} sont indépendamment choisis parmi un atome d'hydrogène, un groupe alkyle, alkylcarbonyle et formyle, et phényle comportant facultativement 1 ou 2 substituants indépendamment choisis parmi un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, sulfamyle, sulfamylalkyle, -NR^{A}R^{B}, (NR^{A}R^{B})alkyle dans lequel R^{A} et R^{B} sont indépendamment choisis parmi un atome d'hydrogène, un groupe aikyie, alkylcarbonyle et formyle ;
amido par lui-même ou comme partie d'un autre groupe est -C(O)NR⁹R¹⁰ dans lequel R⁹ et R¹⁰ sont indépendamment choisis dans le groupe consistant en un atome d'hydrogène, un groupe alkyle, alkylcarbonyle, phényle, phénylalkyle, phénylcarbonyle, formyle et S(O)₂R¹¹ dans lequel R¹¹ est choisi parmi un groupe alkyle, phényle et phénylalkyle ;
cycloalcényle par lui-même ou comme partie d'un autre groupe est un hydrocarbure cyclique contenant de trois à huit atomes de carbone et contenant au moins une double liaison carbone-carbone ;
cycloalcényle peut facultativement comporter 1, 2, 3, 4 ou 5 substituants indépendamment choisis parmi un groupe alcényle, alcoxy, alcoxycarbonyle, alcoxycarbonylalkyle, alkyle, alkylcarbonyloxy, alkylcarbonyloxyalkyle, alcynyle, amido, amidoalkyle, halogéno, halogénoalcoxy, halogénoalkyle, hydroxy, hydroxyalkyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle;
sulfamyle est un groupe -SO₂NR⁹⁴R⁹⁵ dans lequel R⁹⁴ et R⁹⁵ sont indépendamment choisis parmi un atome d'hydrogène, un groupe alkyle, aryle et arylalkyle ;
à condition que lorsque X est O ; R² est -CCl₃ ; R³ est un groupe alkyle ou phényle ; et R⁵ et R⁷ sont un atome d'hydrogène ; alors R¹ est autre qu'un groupe phényle ;
à condition en outre que
le N-[[[[(2-méthylphényl)amino]carbonyl]amino]phénylméthyl]-benzamide ;
le N-[[[[(4-chlorophényl)amino]carbonyl]amino]phénylméthyl]-benzamide ;
le N-[2,2-dichloro-1-[[phénylamino)thioxométhyl]amino]éthyl)-benzamide ;
le N-[2,2-dichloro-1-[[phénylamino)thioxométhyl]amino]éthyl]-4-méthyl-benzamide ;
le N-[2,2-dichloro-1-[[[4-méthylphényl)amino)thioxométhyl]amino]éthyl]-benzamide ;
le N-[2,2-dichloro-1-[[[4-méthylphényl)amino)thioxométhyl]amino]éthyl]-4-méthyl-benzamide ;
le N-[2,2-dichloro-1-[[[4-chlorophényl)amino)thioxométhyl]amino]éthyl]-benzamide ;
le N-[2,2-dichloro-1-[[[4-chlorophényl)amino)thioxométhyl]amino]éthyl]-4-méthyl-benzamide ;
le N-[[[(phénylamino)carbonyl]amino]méthyl]-3-pyridinecarboxamide ;
le N-[[[[(2-méthylphényl)amino)carbonyl]amino]méthyl]-3-pyridinecarboxamide ;
le N-[[[[(4-méthylphényl)amino)carbonyl]amino]méthyl]-3-pyridinecarboxamide ;
le N-[[[[(3-éthoxyphényl)amino)carbonyl]amino]méthyl]-3-pyridinecarboxamide ;
le N-[[[((4-nitrophényl)amino)carbonyl]amino]méthyl]-3-pyridinecarboxamide ;
le N-[[[[(3-méthyiphényl)amino)carbonyl]amino]méthyl]-3-pyridinecarboxamide ;
le N-[[[(phénylamino)carbonyl]amino]méthyl]-benzamide ;
le N-[[[(phénylamino)thioxométhyl]amino]méthyl]-benzamide ;
le N-[[[(phénylamino)carbonyl]amino]méthyl]-4-chloro-benzamide ;
le N-[[[(phénylamino)thioxométhyl]amino]méthyl]-4-chloro-benzamide ;
la 1-(1-acétamido-2,2,2-trichloroéthyl)-3-phényl-2-thio-urée ;
la 1-phényl-2-thio-3-(2,2,2-trichloro-1-propionamidoéthyl)-urée ; et
la 1-(1-butyramido-2,2,2-trichloroéthyl)-3-phényl-2-thio-urée soient exclus.

2. Composé selon la revendication 1, dans lequel
R⁸ est choisi dans le groupe consistant en un groupe alcoxy, alkylsulfonyle, arylalcoxy, arylsulfonyle, cyano, halogénoalkylsulfonyle, hydroxy, et nitro ;
R² est choisi dans le groupe consistant en un atome d'hydrogène, un groupe alcényle, alcényloxyalkyle, alcényloxy(alcényloxy)alkyle, alcoxyalkyle, alcoxycarbonylalkyle, alkyle, alkylcarbonyle, alkylsulfonylalkyle, alkylthioalkyle, aryle, arylalkyle, arylsulfonylalkyle, cyanoalkyle, cycloalcényle, cycloalcénylalkyle, cycloalkyle, cycloalkylalkyle, halogénoalkyle, halogénoalkylcarbonyle, hétérocycle, hétérocyclealkyle, hydroxyalkyle, sulfamylalkyle, et (NR⁹R¹⁰)alkyle ;
R³ est choisi dans le groupe consistant en un groupe aryle, arylalkyle, et hétérocycle ;
R⁵ est choisi dans le groupe consistant en un atome d'hydrogène et un groupe alkyle ; et
R⁷ est choisi dans le groupe consistant en un atome d'hydrogène, un groupe halogénoalkyle, et alkyle en C₁ à C₆ ;
ou
R⁷ et R² pris conjointement avec l'atome de carbone auquel ils sont attachés, forment ensemble un cycle carbocyclique à 5 ou 6 chaînons dans lequel le cycle carbocyclique à 5 ou 6 chaînons est facultativement substitué par 1 ou 2 substituants indépendamment choisis dans le groupe consistant en un groupe alkyle, halogéno, halogénoalcoxy, et halogénoalkyle.

3. Composé selon la revendication 2, dans lequel
R⁸ est choisi dans le groupe consistant en un groupe alcoxy, alkylsulfonyle, halogénoalkylsulfonyle, cyano, hydroxy, nitro, arylalcoxy dans lesquels la portion aryle du groupe arylalcoxy est un phényle, et arylsulfonyle dans lequel la portion aryle du groupe arylsulfonyle est un phényle ;
R¹ est un groupe pyridine, pyrimidine ou quinoléine facultativement substitué par 1, 2, ou 3 substituants indépendamment choisis dans le groupe consistant en un groupe alcoxy, alkyle, halogéno, halogénoalkyle, nitro, phénylsulfonyle et sulfamyle et aryle dans lequel le groupe aryle est un groupe phényle facultativement substitué par 1, 2, ou 3 substituants indépendamment choisis dans le groupe consistant en un groupe alcoxy, alkyle, halogéno, halogénoalkyle, nitro, phénylsulfonyle, et sulfamyle ;
R² est choisi dans le groupe consistant en un atome d'hydrogène, un groupe alcényle, alcényloxyalkyle, alcényloxy(alcényloxy)alkyle, alcoxyalkyle, alcoxycarbonylalkyle, alkyle, alkylcarbonyle, alkylsulfonylalkyle, aryle dans lequel le groupe aryle est un groupe phényle, arylalkyle dans lequel la portion aryle du groupe arylalkyle est un groupe phényle, arylsulfonylalkyle dans lequel la portion aryle du groupe arylsulfonylalkyle est un groupe phényle, cyanoalkyle, cycloalcénylalkyle, cycloalkyle, halogénoalkyle, halogénoalkylcarbonyle, hydroxyalkyle, sulfamylalkyle, (NR⁹R¹⁰)alkyle et hétérocycle dans lequel l'hétérocycle est choisi dans le groupe consistant en le 1,3-dioxane, la pyrrolidine et le thiophène ; et
R³ est choisi dans le groupe consistant en un groupe aryle dans lequel le groupe aryle est un groupe phényle et arylalkyle dans lequel la portion aryle du groupe arylalkyle est un groupe phényle.

4. Composé selon la revendication 1, dans lequel,
X est choisi dans le groupe consistant en O, S, CHNO₂, C(CN)₂, et NR⁸ ; et
R⁸ est choisi dans le groupe consistant en un groupe arylsulfonyle, cyano, halogénoalkylsulfonyle, nitro et sulfamyle ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ;
R³ est choisi dans le groupe consistant en un groupe aryle et arylalkyle ;
R⁵ est choisi dans le groupe consistant en un atome d'hydrogène et un groupe alkyle ; et
R⁷ est un atome d'hydrogène.

5. Composé selon la revendication 1, dans lequel,
X est choisi dans le groupe consistant en O, S, CHNO₂, C(CN)₂, et
NR⁸;
R⁸ est choisi dans le groupe consistant en un groupe arylsulfonyle dans lequel la portion aryle du groupe arylsulfonyle est un groupe phényle, cyano, halogénoalkylsulfonyle, nitro et sulfamyle ;
R¹ est choisi dans le groupe consistant en un groupe aryle dans lequel aryle est un groupe phényle, arylalkyle dans lequel la portion aryle du groupe arylalkyle est un groupe phényle, hétérocycle dans lequel l'hétérocycle est choisi dans le groupe consistant en la quinoléine, la pyridine et la pyrimidine, et hétérocyclealkyle dans lequel la portion hétérocycle de l'hétérocyclealkyle est un groupe pyridine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est choisi dans le groupe consistant en un groupe aryle dans lequel le groupe aryle est un groupe phényle et arylalkyle dans lequel la portion aryle du groupe arylalkyle est un groupe phényle ;
R⁵ est choisi dans le groupe consistant en un atome d'hydrogène et un groupe alkyle ; et
R⁷ est un atome d'hydrogène.

6. Composé selon la revendication 1, dans lequel,
X est NR⁸;
R⁸ est un groupe cyano ;
R¹ est choisi dans le groupe consistant en un groupe hétérocycle et hétérocyclealkyle ;
R³ est choisi dans le groupe consistant en un groupe hétérocycle et hétérocyclealkyle ; et
R⁵ est un atome d'hydrogène.

7. Composé selon la revendication 1, dans lequel,
X est NR⁸;
R⁸ est un groupe cyano ;
R¹ est choisi dans le groupe consistant en un groupe hétérocycle et hétérocyclealkyle ;
R³ est choisi dans le groupe consistant en un groupe aryle et arylalkyle ; et
R⁵ est un atome d'hydrogène.

8. Composé selon la revendication 7 dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ; et
R⁷ est un atome d'hydrogène.

9. Composé selon la revendication 1, dans lequel,
X est NR⁸ ;
R⁸ est un groupe cyano ;
R¹ est un groupe hétérocyclealkyle dans lequel la portion hétérocycle du groupe hétérocyclealkyle est la pyridine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel aryle est le phényle ; et
R⁵ est un atome d'hydrogène.

10. Composé selon la revendication 9, choisi dans le groupe consistant en
le 4-chloro-N-[1-({(cyanoimino)[(3-pyridinylméthyl)amino]méthyl}amino)-2,2-diméthylpropyl]benzamide ;
le 4-chloro-N-[1-({(cyanoimino)[(4-pyridinylméthyl)amino]méthyl}amino)-2,2-diméthylpropyl]benzamide ; et
le 4-chloro-N-[1-({(cyanoimino)[(2-pyridinylméthyl)amino]méthyl}amino)-2,2-diméthylpropyl]benzamide.

11. Composé selon la revendication 1, dans lequel,
X est NR⁸ ;
R⁸ est un groupe cyano ;
R¹ est un groupe hétérocycle dans lequel l'hétérocycle est choisi dans le groupe consistant en la quinoléine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel aryle est un groupe phényle ;
R⁵ est un atome d'hydrogène ; et
R⁷ est un atome d'hydrogène.

12. Composé selon la revendication 11, qui est choisi dans le groupe consistant en
le 4-méthyl-N-(2,2,2-trichloro-1-{[(cyanoimino)(5-pyrimidinylamino)méthyl]amino}éthyl)benzamide et
le 4-chloro-N-(1-{[(cyanoimino)(3-quinoléinylamino)méthyl]amino}-2,2-diméthylpropyl)benzamide.

13. Composé selon la revendication 1, dans lequel,
X est NR⁸;
R⁸ est un groupe cyano ;
R¹ est un groupe hétérocycle dans lequel l'hétérocycle est la pyridine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel aryle est un groupe phényle ;
R⁵ est un atome d'hydrogène ; et
R⁷ est un atome d'hydrogène.

14. Composé selon la revendication 13, choisi dans le groupe consistant en
le N-(1-{[(cyanoimino)(3-pyridinylamino)méthyl]amino}-2,2-diméthylpropyl)-4-méthylbenzamide ;
le 4-chloro-N-(1-{[(cyanoimino)(3-pyridinylamino)méthyl]amino}-2,2-diméthylpropyl)benzamide ;
le 4-chloro-N-[{[(cyanoimino)(3-pyridinylamino)méthyl]amino}(cyclopropyl)méthyl]benzamide ;
le N-(1-{[[(6-chloro-3-pyridinyl)amino](cyanoimino)méthyl]amino}-2,2-diméthylpropyl)-4-méthylbenzamide ;
le (-)-N-(1-{[(cyanoimino)(3-pyridinylamino)méthyl]amino}-2,2-diméthylpropyl)-4-méthylbenzamide ;
le (+)-N-(1-{[(cyanoimino)(3-pyridinylamino)méthyl]amino}-2,2-diméthylpropyl)-4-méthylbenzamide ;
le 4-chloro-N-(1-{[(cyanoimino)(3-pyridinylamino)méthyl]amino }-2-éthylbutyl)benzamide ;
le 4-chloro-N-(1- {[(cyanoimino)(3-pyridinylamino)méthyl]amino }-3-méthylbutyl)benzamide ;
le 4-chloro-N-[{[(cyanoimino)(3-pyridinylamino)méthyl]amino}(cyclohexyl)méthyl]benzamide ;
le 4-chloro-N-(1- {[(cyanoimino)(3-pyridinylamino)méthyl]amino }-3,3-diméthylbutyl)benzamide ;
le 4-chloro-N-(1-{[(cyanoimino)(3-pyridinylamino)méthyl]amino}-2-méthylpropyl)benzamide ;
le 4-chloro-N-(4-cyano- 1 - {[(cyanoimino)(3-pyridinylamino)méthyl]amino}-2,2-diéthylbutyl)benzamide ;
le 4-chloro-N-[1-{[(cyanoimino)(3-pyridinylamino)méthyl]amino}-2-(2,6,6-triméthyl-1-cyclohexén-1-yl)éthyl]benzamide ;
le 4-chloro-N-(1-{[(cyanoimino)(3-pyridinylamino)méthyl]amino}-2,2-diméthyl-4-pentényl)benzamide ;
le 4-chloro-N-(1-{[(cyanoimino)(3-pyridinylamino)méthyl]amino}propyl)benzamide;
le 4-chloro-N-({[cyanoimino)(3-pyridinylamino)méthyl]amino}méthyl)benzamide;
le (-) 4-chloro-N-(4-cyano-1-{[(cyanoimino)(3-pyridinylamino)méthyl]amino}-2,2-diéthylbutyl)benzamide ;
le (+) 4-chloro-N-(4-cyano-1-([(cyanoimino)(3-pyridinylamino)méthyl]amino}-2,2-diéthylbutyl)benzamide ;
le (+) 4-chloro-N-[1-{[(cyanoimino)(3-pyridinylamino)méthyl]amino}-2-(2,6,6-triméthyl-1-cyclohexén-1-yl)éthyl]benzamide ;
le (-) 4-chloro-N-[1-{[(cyanoimino)(3-pyridinylamino)méthyl]amino}-2-(2,6,6-triméthyl-1-cyclohexén-1-yl)éthyl]benzamide ;
le (-)4-chloro-N-(1-{[(cyanoimino)(3-pyridinylamino)méthyl]amino}-2,2-diméthyl-4-pentényl)benzamide ;
le (+) 4-chloro-N-(1-{[(cyanoimino)(3-pyridinylamino)méthyl]amino}-2,2-diméthyl-4-pentényl)benzamide ;
le 4-chloro-N-(1- {[(cyanoimino)(3-pyridinylamino)méthyl]amino)-3,3-diméthyl-4-pentényl)benzamide ;
le 4-chloro-N-(1- {[(cyanoimino)(3-pyridinylamino)méthyl]amino)-2-cyclohexyl-2-méthylpropyl)benzamide ;
le 4-chloro-N-(1-{ [(cyanoimino)(3-pyridinylamino)méthyl]amino }-2,2-diméthylhexyl)benzamide ;
le N-(2-(1-adamantyl)-1- {([(cyanoimino)(3-pyridinylamino)méthyl]amino}éthyl)-4-chlorobenzamide ;
le N-(2,2-bis[(allyloxy)méthyl]-1- {[(cyanoimino)(3-pyridinylamino)méthyl]amino}butyl)-4-chlorobenzamide ;
le 4-chloro-N-[1-{[(cyanoimino)(3-pyridinylamino)méthyl]amino}-3-(diméthylamino)-2,2-diméthylpropyl]benzamide ;
le (2R)-2-((R)-[(4-chlorobenzoyl)amino] {[(cyanoimino)(3-pyridinylamino)méthyl]amino}méthyl)-1-pyrrolidinecarboxylate de tert-butyle ;
le 4-chloro-N-[1-{[(cyanoimino)(3-pyridinylamino)méthyl]amino}-3-(méthylsulfanyl)propyl]benzamide ;
le N-(1-adamantyl{[(cyanoimino)(3-pyridinylamino)méthyl]amino}méthyl)-4-chlorobenzamide ;
le 4-chloro-N-[{[(cyanoimino)(3-pyridinylamino)méthyl]amino}(5-éthyl-1,3-dioxan-5-yl)méthyl]benzamide ;
le 4-chloro-N-(1-{[(cyanoimino)(3-pyridinylamino)méthyl]amino}-2,2-diméthyl-3-phénylpropyl)benzamide ;
le N-(1-{[(cyanoimino)(3-pyridinylamino)méthyl]amino}-2,2-diméthylpropyl)-4-iodobenzamide ;
le N-(1-{[(cyanoimino)(3-pyridinylamino)méthyl]amino}-2,2-diméthylpropyl)-4-(2-furyl)benzamide ;
le 4-bromo-N-(1-{[(cyanoimino)(3-pyridinylamino)méthyl]amino}-2,2-diméthylpropyl)benzamide ;
le 4-chloro-N-(1-{[(cyanoimino)(3-pyridinylamino)méthyl]amino)-2,2-diméthylpropyl)-2-fluorobenzamide ;
le N-(1-{[(cyanoimino)(3-pyridinylamino)méthyl]amino}-2,2-diméthylpropyl)-4-fluorobenzamide ;
le N-(1-{[(cyanoimino)(3-pyridinylamino)méthyl]amino} -2,2-diméthylpropyl)-3-méthylbenzamide ;
le N-(1-{[(cyanoimino)(3-pyridinylamino)méthyl]amino}-2,2-diméthylpropyl)-2-méthylbenzamide ; et
le N-(1-{[(cyanoimino)(3-pyridinylamino)méthyl]amino}-2,2-diméthylpropyl)-3,5-difluorobenzamide.

15. Composé selon la revendication 1, dans lequel,
X est NR⁸ ;
R⁸ est un groupe cyano ;
R¹ est un groupe hétérocycle ;
R² est un groupe halogénoalkyle ;
R³ est un groupe aryle ;
R⁵ est un atome d'hydrogène ; et
R⁷ est un atome d'hydrogène.

16. Composé selon la revendication 1, dans lequel,
X est NR⁸ ;
R⁸ est un groupe cyano ;
R¹ est un groupe hétérocycle dans lequel l'hétérocycle est la pyridine ;
R² est un groupe halogénoalkyle ;
R³ est un groupe aryle dans lequel aryle est un groupe phényle ;
R⁵ est un atome d'hydrogène ; et
R⁷ est un atome d'hydrogène.

17. Composé selon la revendication 16, choisi dans le groupe consistant en
le 4-méthyl-N-(2,2,2-trichloro-1-{[(cyanoimino)(3-pyridinylamino)méthyl]amino}éthyl)benzamide ;
le 4-chloro-N-(2,2,2-trichloro-1-{[(cyanoimino)(3-pyridinylamino)méthyl]amino}éthyl)benzamide ;
le N-(2,2,2-trichloro-1-{[(cyanoimino)(3-pyridinylamino)méthyl]amino}éthyl)benzamide ;
le 2-méthyl-N-(2,2,2-trichloro-1-{[(cyanoimino)(3-pyridinylamino)méthyl]amino}éthyl)benzamide ;
le 4-chloro-N-(1-{[(cyanoimino)(3-pyridinylamino)méthyl]amino}-2,2,2-trifluoroéthyl)benzamide ;
le (-) 4-chloro-N-(2,2,2-trichloro-1-{[(cyanoimino)(3-pyridinylamino)méthyl]amino}éthyl)benzamide ;
le (+) 4-chloro-N-(2,2,2-trichloro-1-{[(cyanoimino)(3-pyridinylamino)méthyl]amino}éthyl)benzamide ;
le 4-iodo-N-(2,2,2-trichloro-1-([(cyanoimino)(3-pyridinylamino)méthyl]amino}éthyl)benzamide ;
le 4-chloro-N-(2,2-dichloro-1-{[(cyanoimino)(3-pyridinylamino)méthyl]amino}pentyl)benzamide ;
le 4-chloro-N-(2,2-dichloro-1-{ [(cyanoimino)(3-pyridinylamino)méthyl]amino}propyl)benzamide ;
le (-) 4-chloro-N-(2,2-dichloro-1-{[(cyanoimino)(3-pyridinylamino)méthyl]amino}propyl)benzamide ;
le (+) 4-chloro-N-(2,2-dichloro-1-{[(cyanoimino)(3-pyridinylamino)méthyl]amino}propyl)benzamide ;
le 3-chloro-N-(2,2-dichloro-1-{[(cyanoimino)(3-pyridinylamino)méthyl]amino}propyl)benzamide ;
le N-(2,2-dichloro-1-{[(cyanoimino)(3-pyridinylamino)méthyl]amino}propyl)-3,5-difluorobenzamide ;
le 4-chloro-N-(1-{[(cyanoimino)(3-pyridinylamino)méthyl]amino}-2,2,3,3,3-pentafluoropropyl)benzamide ; et
le 3-chloro-N-(1-{[(cyanoimino)(3-pyridinylamino)méthyl]amino}-2,2,3,3,3-pentafluoropropyl)benzamide.

18. Composé selon la revendication 1, dans lequel,
X est NR⁸ ;
R⁸ est un groupe cyano ;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel aryle est un groupe phényle ;
R⁵ est un groupe alkyle ; et
R⁷ est un atome d'hydrogène.

19. Composé selon la revendication 18, qui est le 4-chloro-N-{1-[[(cyanoimino)(3-pyridinylamino)méthyl](méthyl)amino]-2,2-diméthylpropyl}benzamide.

20. Composé selon la revendication 1, dans lequel,
X est NR⁸ ;
R⁸ est un groupe cyano ;
R¹ est choisi dans le groupe consistant en un groupe hétérocycle et hétérocyclealkyle ;
R³ est un groupe alkyle ; et
R⁵ est un atome d'hydrogène.

21. Composé selon la revendication 1, dans lequel,
X est NR⁸ ;
R⁸ est un groupe cyano ;
R¹ est choisi dans le groupe consistant en un groupe aryle et arylalkyle ;
R³ est choisi dans le groupe consistant en un groupe hétérocycle et hétérocyclealkyle ; et
R⁵ est un atome d'hydrogène.

22. Composé selon la revendication 1, dans lequel,
X est NR⁸ ;
R⁸ est un groupe cyano ;
R¹ est choisi dans le groupe consistant en un groupe aryle et arylalkyle ;
R³ est choisi dans le groupe consistant en un groupe aryle et arylalkyle ; et
R⁵ est un atome d'hydrogène.

23. Composé selon la revendication 22, dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ;
et
R⁷ est un atome d'hydrogène.

24. Composé selon la revendication 1, dans lequel,
X est NR⁸ ;
R⁸ est un groupe cyano ;
R¹ est un groupe arylalkyle dans lequel la portion aryle du groupe arylalkyle est un groupe phényle ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel aryle est un groupe phényle ;
R⁵ est un atome d'hydrogène ; et
R⁷ est un atome d'hydrogène.

25. Composé selon la revendication 24, choisi dans le groupe consistant en
le 4-chloro-N-(1-{[[(2-chlorobenzyl)amino](cyanoimino)méthyl]amino}-2,2-diméthylpropyl)benzamide ;
le 4-chloro-N-(1-([[(3-chlorobenzyl)amino](cyanoimino)méthyl]amino}-2,2-diméthylpropyl)benzamide ; et
le 4-chloro-N-(1-{[[(4-chlorobenzyl)amino](cyanoimino)méthyl]amino}-2,2-diméthylpropyl)benzamide.

26. Composé selon la revendication 1, dans lequel,
X est NR⁸ ;
R⁸ est un groupe cyano ;
R¹ est un groupe aryle dans lequel aryle est un groupe phényle ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel aryle est un groupe phényle ;
R⁵ est un atome d'hydrogène ; et
R⁷ est un atome d'hydrogène.

27. Composé selon la revendication 26, qui est choisi dans le groupe consistant en
le N-(1-{[anilino(cyanoimino)méthyl]amino}-2,2,2-trichloroéthyl)-4-méthylbenzamide ;
le 4-méthyl-N-(2,2,2-trichloro-1-{ [(cyanoimino)(2-fluoroanilino)méthyl]amino)éthyl)benzamide ;
le N-(1-{[(cyanoimino)(3-fluoroanilino)méthyl]amino}-2,2-diméthylpropyl)-4-méthylbenzamide ;
le 4-chloro-N[{[(cyanoimino)(3-fluoroanilino)méthyl]amino} (3-thiényl)méthyl]benzamide ;
le 4-chloro-N-(1-{ [(cyanoimino)(2-fluoroanilino)méthyl]amino }-2,2-diméthylpropyl)benzamide ;
le 4-chloro-N-(1-{[(cyanoimino)(3-fluoroanilino)méthyl]amino }-2,2-diméthylpropyl)benzamide ;
le 4-chloro-N-[1-({(cyanoimino)[3-(trifluorométhyl)anilino]méthyl}amino)-2,2-diméthylpropyl]benzamide ;
le 4-chloro-N-(1-{[(cyanoimino)(3,5-difluoroanilino)méthyl}amino}-2,2-diméthylpropyl)benzamide ;
le 4-chloro-N-(1-{[(cyanoimino)(2,5-difluoroanilino)méthyl]amino -2,2-diméthylpropyl)benzamide ;
le 4-chloro-N-(1-{[(cyanoimino)(2,6-difluoroanilino)méthyl]amino -2,2-diméthylpropyl)benzamide ;
le 4-chloro-N-(1-{ [(cyanoimino)(3-chloroanilino)méthyl]amino} -2,2-diméthylpropyl)benzamide ; et
le 4-chloro-N-(1-{[(cyanoimino)(3-méthoxyanilino)méthyl]amino}-2,2-diméthylpropyl)benzamide.

28. Composé selon la revendication 1, dans lequel,
X est NR⁸ ;
R⁸ est un groupe cyano ;
R¹ est choisi dans le groupe consistant en un groupe aryle et arylalkyle ;
R³ est un groupe alkyle ; et
R⁵ est un atome d'hydrogène.

29. Composé selon la revendication 1, dans lequel,
X est NR⁸ ;
R⁸ est choisi dans le groupe consistant en un atome d'hydrogène, un groupe alcoxy, alkyle, alkylsulfonyle, arylalcoxy, aryloxy, arylsulfonyle, halogénoalkylsulfonyle, hétérocyclealcoxy, hétérocycleoxy, hydroxy, nitro, et sulfamyle ; et
R⁵ est un atome d'hydrogène.

30. Composé selon la revendication 1, dans lequel,
X est NR⁸;
R⁸ est un groupe nitro ; et
R⁵ est un atome d'hydrogène.

31. Composé selon la revendication 1, dans lequel,
X est NR⁸ ;
R⁸ est un groupe nitro ;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ; et
R⁵ est un atome d'hydrogène.

32. Composé selon la revendication 31, choisi dans le groupe consistant en
le 4-chloro-N-(1-{[(nitroimino)(3-pyridinylamino)méthyl]amino }-2,2-diméthylpropyl)benzamide ;
le 4-chloro-N-(1-([(nitroimino)(3-pyridinylamino)méthyl]amino}-3,3-diméthylbutyl)benzamide ;
le (+) 4-chloro-N-(1-{[(nitroimino)(3-pyridinylamino)méthyl]amino}-3,3-diméthylbutyl)benzamide ;
le (-) 4-chloro-N-(1-{[(nitroimino)(3-pyridinylamino)méthyl]amino}-3,3-diméthylbutyl)benzamide ;
le 4-chloro-N-(1-{[(nitroimino)(3-pyridinylamino)méthyl]amino}-2,2,diméthyl-4-pentényl)benzamide ;
le 4-chloro-N-(1-{[(nitroimino)(3-pyridinylamino)méthyl]amino}-2,2-diméthyl-3-phénylpropyl)benzamide ;
le 4-chloro-N-[1-{[(nitroimino)(3-pyridinylamino)méthyl]amino}-2-(2,6,6-triméthyl-1-cyclohexén-1-yl)éthyl]benzamide ;
le 4-chloro-N-(1-{[(nitroimino)(3-pyridinylamino)méthyl]amino}-2-cyclohexyl-2-méthylpropyl)benzamide ;
le N-(2,2-bis[(allyloxy)méthyl]-1-{[(nitroimino)(3-pyridinylamino)méthyl]amino}butyl)-4-chlorobenzamide ;
le 4-chloro-N-(4-cyano-1-{[(nitroimino)(3-pyridinylamino}méthyl]amino}-2,2-diéthylbutyl)benzamide ;
le 4-chloro-N-(1-{[(nitroimino)(3-pyridinylamino)méthyl]amino}-3,3-diméthyl-4-pentényl)benzamide ;
le N-(2-(1-adamantyl)-1-{[(nitroimino)(3-pyridinylamino)méthyl]amino)éthyl)-4-chlorobenzamide ;
le N-(1-{[(nitroimino)(3-pyridinylamino)méthyl]amino) -2,2-diméthylpropyl)-4-phénylbenzamide ;
le 4-chloro-N-(2,2-dichloro-1-{(nitroimino)(3-pyridinylamino)méthyl]amino}pentyl)benzamide ;
le 4-chloro-N-(2,2-dichloro-1- {[(nitroimino)(3-pyridinylamino)méthyl]amino}propyl)benzamide ; et
le 3-chloro-N-(2,2-dichloro-1-{[(nitroimino)(3-pyridinylamino)méthyl]amino}propyl)benzamide.

33. Composé selon la revendication 1, dans lequel,
X est NR⁸ ;
R⁸ est choisi dans le groupe consistant en un groupe arylsulfonyle, halogénoalkylsulfonyle et sulfamyle ; et
R⁵ est un atome d'hydrogène.

34. Composé selon la revendication 1, dans lequel,
X est NR⁸ ;
R⁸ est choisi dans le groupe consistant en un groupe arylsulfonyle dans lequel la portion aryle du groupe arylsulfonyle est un groupe phényle, halogénoalkylsulfonyle et sulfamyle ;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ; et
R⁵ est un atome d'hydrogène.

35. Composé selon la revendication 34, choisi dans le groupe consistant en
le 4-chloro-N-(2,2-diméthyl-1- {[[(phénylsulfonyl)imino](3-pyridinylamino)méthyl]amino}propyl)benzamide ;
le 4-chloro-N-(3,3-diméthyl-1-{ [[(phénylsulfonyl)imino](3-pyridinylamino)méthyl]amino}butyl)benzamide;
le 4-chloro-N-{2,2-diméthyl-1-[((3-pyridinylamino){[(trifluorométhyl)sulfonyl]imino}méthyl)amino]propyl}benzamide ;
le 4-chloro-N-{3,3-diméthyl-1-[((3-pyridinylamino){[(trifluorométhyl)sulfonyl]imino}méthyl)amino]butyl}benzamide ;
le N-(1-{ [[(aminosulfonyl)imino](3-pyridinylamino)méthyl]amino}-2,2-diméthylpropyl)-4-chlorobenzamide ;
le N-(1 - {[[(aminosulfonyl)imino](3-pyridinylamino)méthyl]amino}-3,3-diméthylbutyl)-4-chlorobenzamide ;
le 4-chloro-N-(1-{[{[(diméthylamino)sulfonyl]imino}(3-pyridinylamino)méthyl]amino}-2,2-diméthylpropyl)benzamide ; et
le 4-chloro-N-(1-{[{[(diméthylamino)sulfonyl]imino}(3-pyridinylamino)méthyl]amino} -3,3-diméthylbutyl)benzamide.

36. Composé selon la revendication 1, dans lequel,
X est S ; et
R⁵ est un atome d'hydrogène.

37. Composé selon la revendication 1, dans lequel,
X est S ;
R¹ est choisi dans le groupe consistant en un groupe hétérocycle et hétérocyclealkyle ;
R³ est choisi dans le groupe consistant en un groupe aryle et arylalkyle ; et
R⁵ est un atome d'hydrogène.

38. Composé selon la revendication 37, dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle;
et
R⁷ est un atome d'hydrogène.

39. Composé selon la revendication 1, dans lequel,
X est S;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ;
R⁵ est un atome d'hydrogène ; et
R⁷ est un atome d'hydrogène.

40. Composé selon la revendication 39, qui est choisi dans le groupe consistant en
le N-(2,2-diméthyl-1-([(3-pyridinylamino)carbothioyl]amino}propyl)-4-méthylbenzamide ;
le N-((1R)-2,2-diméthyl-1-{[(3-pyridinylamino)carbothioyl]amino}propyl)-4-méthylbenzamide ;
le N-((1S)-2,2-diméthyl-1-{[(3-pyridinylamino)carbothioyl]amino}propyl)-4-méthylbenzamide ;
le N-(2,2-diméthyl-1-{[(3-pyridinylamino)carbothioyl]amino}propyl)-2-méthylbenzamide ;
le 4-chloro-N-(2,2-diméthyl-1-{[(3-pyridinylamino)carbothioyl]amino}propyl)benzamide ;
le N-(2,2-diméthyl-1-{[(3-pyridinylamino)carbothioyl]amino}propyl)benzamide;
le 4-méthyl-N-(phényl{[(3-pyridinylamino)carbothioyl]amino}méthyl)benzamide ;
le 4-méthyl-N-(2-méthyl-1-{[{3-pyridinylamino)carbothioyl]amino}propyl)benzamide ; et
le 4-méthyl-N-((1R,2S)-2-méthyl-1-{[(3-pyridinylamino)carbothioyl]amino}butyl)benzamide.

41. Composé selon la revendication 1, dans lequel,
X est S ;
R¹ est un groupe hétérocycle ;
R² est un groupe halogénoalkyle ;
R³ est un groupe aryle ;
R⁵ est un atome d'hydrogène ; et
R⁷ est un atome d'hydrogène.

42. Composé selon la revendication 1, dans lequel,
X est S ;
R¹ est un groupe hétérocycle dans lequel l'hétérocycle est la pyridine ;
R² est un groupe halogénoalkyle ;
R³ est un groupe aryle dans lequel le groupe aryle est un groupe phényle ;
R⁵ est un atome d'hydrogène ; et
R⁷ est un atome d'hydrogène.

43. Composé selon la revendication 42, qui est choisi dans le groupe consistant en
le 4-méthyl-N-(2,2,2-trifluoro-1-{[(3-pyridinylamino)carbothioyl]amino}éthyl)benzamide ;
le N-[1-({[(6-chloro-3-pyridinyl)amino]carbothioyl)amino)-2,2,2-trifluoroéthyl]-4-méthylbenzamide ;
le 4-chloro-N-(2,2,2-trifluoro-1-{[(3-pyridinylamino)carbothioyl]amino}éthyl)benzamide ; et
le 4-iodo-N-(2,2,2-trichloro-1-{[(3-pyridinylamino)carbothioyl]amino)éthyl)benzamide.

44. Composé selon la revendication 1, dans lequel,
X est S ;
R¹ est choisi dans le groupe consistant en un groupe aryle et arylalkyle ;
R³ est choisi dans le groupe consistant en un groupe aryle et arylalkyle ; et
R⁵ est un atome d'hydrogène.

45. Composé selon la revendication 44, dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ;
et
R⁷ est un atome d'hydrogène.

46. Composé selon la revendication 1, dans lequel,
X est S ;
R¹ est un groupe aryle dans lequel aryle est un groupe phényle ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ;
R⁵ est un atome d'hydrogène ; et
R⁷ est un atome d'hydrogène.

47. Composé selon la revendication 46, qui est choisi dans le groupe consistant en
le 4-méthyl-N-{2,2,2-trifluoro-1-[(2-toluidinocarbothioyl)amino]éthyl}benzamide ;
le 4-méthyl-N-(2,2,2-trifluoro-1- {[(4-fluoroanilino)carbothioyl]amino}éthyl)benzamide ;
le 4-méthyl-N-(2,2,2-trifluoro-1- {[(3-nitroanilino)carbothioyl]amino}éthyl)benzamide ;
le 4-méthyl-N-[2,2,2-trifluoro-1-({[2-fluoro-3-(trifluorométhyl)anilino]carbothioyl}amino)éthyl]benzamide ;
le 4-méthyl-N-(2,2,2-trifluoro-1- {[(4-méthoxyanilino)carbothioyl]amino}éthyl)benzamide ;
le 4-méthyl-N-(2,2,2-trifluoro-1-{[(2-méthoxyanilino)carbothioyl]amino}éthyl)benzamide ;
le N-{1-[(anilinocarbothioyl)amino]-2,2,2-trifluoroéthyl}-4-méthylbenzamide ;
le 4-méthyl-N-{2,2,2-trifluoro-1-[(4-toluidinocarbothioyl)amino]éthyl}benzamide;
le 4-méthyl-N-(2,2,2-trifluoro-1- {[(2-fluoroanilino)carbothioyl]amino}éthyl)benzamide ;
le 4-méthyl-N-(2,2,2-trifluoro-1-{[(3-méthoxyanilino)carbothioyl]amino}éthyl)benzamide ;
le 4-méthyl-N-(2,2,2-trifluoro-1-{[(3-fluoroanilino)carbothioyl]amino}éthyl)benzamide ;
le N-(1-{[(2,5-difluoroanilino)carbothioyl]amino}-2,2,2-trifluoroéthyl)-4-méthylbenzamide ;
le N-(1-{[(2,4-difluoroanilino)carbothioyl]amino}-2,2,2-trifluoroéthyl)-4-méthylbenzamide ;
le 4-méthyl-N-{2,2,2-trifluoro-1-[(3-toluidinocarbothioyl)amino]éthyl}benzamide ;
le N-(1- {[(2,6-difluoroanilino)carbothioyl]amino -2,2,2-trifluoroéthyl)-4-méthylbenzamide ;
le N-(1-{[(2,3-difluoroanilino)carbothioyl]amino)-2,2,2-trifluoroéthyl)-4-méthylbenzamide ;
le N-{1-[(anilinocarbothioyl)amino]-2,2,2-trifluoroéthyl}-4-chlorobenzamide ;
le 4-chloro-N-(2,2,2-trifluoro-1-{[(2-fluoroanilino)carbothioyl]amino}éthyl)benzamide ;
le N-(2,2-diméthyl-1-{[(3-nitroanilino)carbothioyl]amino}propyl)-4-méthylbenzamide ;
le 4-méthyl-N-(1-{[(3-nitroanilino)carbothioyl]amino)éthyl)benzamide;
le 4-méthyl-N-(1-{[(3-nitroanilino)carbothioyl]amino}-2-phényléthyl)benzamide;
le N-((1R)-2-(tert-butoxy)-1-{[(3-nitroanilino)carbothioyl]amino}éthyl)-4-méthylbenzamide ;
le N-(2-fluoro-1-{[(3-nitroanilino)carbothioyl]amino}éthyl)-4-méthylbenzamide ; et
le 4-méthyl-N-[{[(3-nitroanilino)carbothioyl]amino}(phényl)méthyl]benzamide.

48. Composé selon la revendication 1, dans lequel,
X est S ;
R¹ est un groupe aryle dans lequel aryle est un groupe phényle ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle ;
R³ est un groupe aryle dans lequel aryle est un groupe phényle ;
R⁵ est un atome d'hydrogène ; et
R⁷ est un atome d'hydrogène.

49. Composé selon la revendication 48, qui est le 3-phényl-N-(2,2,2-trichloro-1-{[(3-nitroanilino)carbothioyl]amino}éthyl)propanamide.

50. Composé selon la revendication 1, dans lequel,
X est O;
R¹ est choisi dans le groupe consistant en un groupe hétérocycle et hétérocyclealkyle ;
R³ est choisi dans le groupe consistant en un groupe aryle et arylalkyle ; et
R⁵ est un atome d'hydrogène.

51. Composé selon la revendication 50, dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ;
et
R⁷ est un atome d'hydrogène.

52. Composé selon la revendication 1, dans lequel,
X est O ;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ;
R⁵ est un atome d'hydrogène ; et
R⁷ est un atome d'hydrogène.

53. Composé selon la revendication 1, dans lequel,
X est O ;
R¹ est un groupe hétérocycle;
R² est un groupe halogénoalkyle;
R³ est un groupe aryle ;
R⁵ est un atome d'hydrogène ; et
R⁷ est un atome d'hydrogène.

54. Composé selon la revendication 1, dans lequel,
X est O ;
R¹ est un groupe hétérocycle dans lequel l'hétérocycle est la pyridine ;
R² est un groupe halogénoalkyle ;
R³ est un groupe aryle dans lequel le groupe aryle est un groupe phényle ;
R⁵ est un atome d'hydrogène ; et
R⁷ est un atome d'hydrogène.

55. Composé selon la revendication 54 qui est choisi dans le groupe consistant en
le 4-méthyl-N-(2,2,2-trichloro-1-{[(3-pyridinylamino)carbonyl]amino}éthyl)benzamide ;
le 2-méthyl-N-(2,2,2-trichloro-1-{[(3-pyridinylamino)carbonyl]amino}éthyl)benzamide ;
le N-(2,2,2-trichloro-1-{[(3-pyridinylamino)carbonyl]amino}éthyl)benzamide; et
le 4-chloro-N-(2,2,2-trichloro-1-{R(3 - pyridinylamino)carbonyl]amino}éthyl)benzamide.

56. Composé selon la revendication 1, dans lequel,
X est O ;
R¹ est choisi dans le groupe consistant en un groupe hétérocycle et hétérocyclealkyle ;
R³ est choisi dans le groupe consistant en un groupe hétérocycle et hétérocyclealkyle ; et
R⁵ est un atome d'hydrogène.

57. Composé selon la revendication 1, dans lequel,
X est O ;
R¹ est choisi dans le groupe consistant en un groupe aryle et arylalkyle ;
R³ est choisi dans le groupe consistant en un groupe aryle et arylalkyle ; et
R⁵ est un atome d'hydrogène.

58. Composé selon la revendication 57, dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ; et
R⁷ est un atome d'hydrogène.

59. Composé selon la revendication 1, dans lequel,
X est O ;
R¹ est un groupe aryle dans lequel le groupe aryle est un groupe phényle ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ; et
R⁵ est un atome d'hydrogène.

60. Composé selon la revendication 59, qui est le 4-chloro-N-(1-{[(2-fluoroanilino)carbonyl]amino}-2,2-diméthylpropyl)benzamide.

61. Composé selon la revendication 1, dans lequel,
X est choisi dans le groupe consistant en CHCN et CHNO₂ ;
R¹ est choisi dans le groupe consistant en un groupe hétérocycle et hétérocyclealkyle ;
R³ est choisi dans le groupe consistant en un groupe hétérocycle et hétérocyclealkyle ; et
**R**⁵ est un atome d'hydrogène.

62. Composé selon la revendication 1, dans lequel,
X est choisi dans le groupe consistant en CHCN et CHNO₂ ;
R¹ est choisi dans le groupe consistant en un groupe hétérocycle et hétérocyclealkyle ;
R³ est choisi dans le groupe consistant en un groupe aryle et arylalkyle ; et
R⁵ est un atome d'hydrogène.

63. Composé selon la revendication 62, dans lequel,
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle, arylalkyle, cyanoalkyle, cycloalcénylalkyle, cycloalkyle, cycloalkylalkyle, halogénoalkyle, hétérocycle et (NR⁹R¹⁰)alkyle ;
et
R⁷ est un atome d'hydrogène.

64. Composé selon la revendication 1, dans lequel,
X est choisi dans le groupe consistant en CHCN et CHNO₂ ;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ;
R⁵ est un atome d'hydrogène ; et
R⁷ est un atome d'hydrogène.

65. Composé selon la revendication 64, choisi dans le groupe consistant en
le 4-chloro-N-(2-éthyl-1-{[2-nitro-1-(3-pyridinylamino)éthényl]amino}butyl)benzamide ; et
le 4-chloro-N-(2,2-diméthyl-1-{ [2-nitro-1-(3-pyridinylamino)éthényl]amino}propyl)benzamide.

66. Composé selon la revendication 1, dans lequel,
X est choisi dans le groupe consistant en CHCN et CHNO₂;
R¹ est un groupe hétérocycle ;
R² est un groupe halogénoalkyle;
R³ est un groupe aryle ;
R⁵ est un atome d'hydrogène ; et
R⁷ est un atome d'hydrogène.

67. Composé selon la revendication 1, dans lequel,
X est choisi dans le groupe consistant en CHCN et CHNO₂ ;
R¹ est un groupe hétérocycle dans lequel l'hétérocycle est la pyridine ;
R² est un groupe halogénoalkyle ;
R³ est un groupe aryle dans lequel le groupe aryle est un groupe phényle ;
R⁵ est un atome d'hydrogène ; et
R⁷ est un atome d'hydrogène.

68. Composé selon la revendication 67, qui est le 4-chloro-N-(2,2-dichloro-1-{[2-nitro-1-(3-pyridinylamino)éthényl]amino} pentyl)benzamide.

69. Composé selon la revendication 1, dans lequel,
X est choisi dans le groupe consistant en CHCN et CHNO₂;
R¹ est choisi dans le groupe consistant en un groupe hétérocycle et hétérocyclealkyle ;
R³ est un groupe alkyle ; et
R⁵ est un atome d'hydrogène.

70. Composé selon la revendication 1, dans lequel,
X est choisi dans le groupe consistant en CHCN et CHNO₂ ;
R¹ est choisi dans le groupe consistant en un groupe aryle et arylalkyle ;
R³ est choisi dans le groupe consistant en un groupe hétérocycle et hétérocyclealkyle ; et
R⁵ est un atome d'hydrogène.

71. Composé selon la revendication 1, dans lequel,
X est choisi dans le groupe consistant en CHCN et CHNO₂;
R¹ est choisi dans le groupe consistant en un groupe aryle et arylalkyle ;
R³ est choisi dans le groupe consistant en un groupe aryle et arylalkyle ; et
R⁵ est un atome d'hydrogène.

72. Composé selon la revendication 1, dans lequel,
X est choisi dans le groupe consistant en CHCN et CHNO₂;
R¹ est choisi dans le groupe consistant en un groupe aryle et arylalkyle ;
R³ est un groupe alkyle ; et
R⁵ est un atome d'hydrogène.

73. Composé selon la revendication 1, dans lequel,
X est C(CN)₂ ;
R¹ est choisi dans le groupe consistant en un groupe hétérocycle et hétérocyclealkyle ;
R³ est choisi dans le groupe consistant en un groupe aryle et arylalkyle ; et
R⁵ est un atome d'hydrogène.

74. Composé selon la revendication 1, dans lequel,
X est C(CN)₂ ;
R¹ est un groupe hétérocycle dans lequel l'hétérocycle est choisi dans le groupe consistant en la quinoléine, la pyridine et la pyrimidine ;
R² est choisi dans le groupe consistant en un atome d'hydrogène, un groupe alcényle, alcényloxyalkyle, alcényloxy(alcényloxy)alkyle, alcoxyalkyle, alkyle, alkylthioalkyle, aryle dans lequel le groupe aryle est un groupe phényle, arylalkyle dans lequel la portion aryle du groupe arylalkyle est un groupe phényle, cyanoalkyle, cycloalcénylalkyle, cycloalkyle, cycloalkylalkyle, halogénoalkyle, (NR⁹R¹⁰)alkyle et hétérocycle dans lequel l'hétérocycle est choisi dans le groupe consistant en le 1,3-dioxane, la pyrrolidine et le thiophène ;
R³ est un groupe aryle dans lequel le groupe aryle est un groupe phényle ; et
R⁵ est un atome d'hydrogène.

75. Composé selon la revendication 74, qui est le 4-chloro-N-(1-{[2,2-dicyano-1-(3-pyridinylamino)vinyl]amino}-2,2-diméthylpropyl)benzamide.

76. Composé selon la revendication 1 de formule VI : 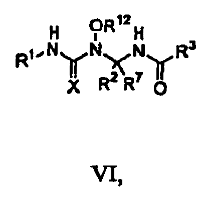 ou un sel pharmaceutiquement acceptable de celui-ci.

77. Composé selon la revendication 76, dans lequel,
X est NR⁸ ; et
R⁸ est un groupe cyano.

78. Composé selon la revendication 76, dans lequel,
X est NR⁸ ; et
R⁸ est choisi dans le groupe consistant en un atome d'hydrogène, un groupe alcoxy, alkyle, alkylsulfonyle, arylalcoxy, aryloxy, arylsulfonyle, halogénoalkylsulfonyle, hétérocyclealcoxy, hydroxy, nitro, et sulfamyle.

79. Composé selon la revendication 76, dans lequel X est S.

80. Composé selon la revendication 76, dans lequel X est O.

81. Composé selon la revendication 76, dans lequel X est choisi dans le groupe consistant en CHCN et CHNO₂.

82. Composé choisi dans le groupe consistant en
le 4-chloro-N-(1-{[(hydroxyimino)(3-pyridinylamino)méthyl]amino}-2,2-diméthylpropyl)benzamide ;
le 4-chloro-N-(1-{[(méthoxyimino)(3-pyridinylamino)méthyl]amino}-2,2-diméthylpropyl)benzamide ;
le 4-chloro-N-(1-{[{[(4-fluorobenzyl)oxy]imino}(3-pyridinylamino)méthyl]amino}-2,2-diméthylpropyl)benzamide ;
le 4-chloro-N-(2,2-diméthyl-1-{[[(méthylsulfonyl)imino](3-pyridinylamino)méthyl]amino}propyl)benzamide ;
le 3-(4-chlorophényl)-N-(1-{[(cyanoimino)(3-pyridinylamino)méthyl]amino}-2,2-diméthylpropyl)propanamide ;
le N-(1-{[(cyanoimino)(3-pyridinylamino)méthyl]amino}-2,2-diméthylpropyl)-3-phénylpropanamide ;
le N-(1-{[(cyanoimino)(3-pyridinylamino)méthyl]amino}-2,2-diméthylpropyl)-2-phénylacétamide ;
le 4-(aminosulfonyl)-N-(1-{[(cyanoimino)(3-pyridinylamino)méthyl]amino}-2,2-diméthylpropyl)-2-fluorobenzamide ;
le 4-chloro-N-[1-({(cyanoimino)[(4-éthyl-3-pyridinyl)amino]méthyl}amino)-2,2-diméthylpropyl]benzamide ;
le N-(1-{[(cyanoimino)(3-pyridinylamino)méthyl]amino}-2,2-diméthylpropyl)-4-(trifluorométhoxy)benzamide ;
le 4-chloro-N-[1-({(cyanoimino)[(4-éthyl-3-pyridinyl)amino]méthyl}amino)-2,2-diméthylpropyl]-2-fluorobenzamide ;
le 4-chloro-N-(1-{[(cyanoimino)(5-pyrimidinylamino)méthyl]amino}-2,2-diméthylpropyl)benzamide ;
le 4-chloro-N-(1-{[(cyanoimino)(5-pyrimidinylamino)méthyl]amino}-2,2-diméthylpropyl)-2-fluorobenzamide ;
le N-(1-{[[(4-bromo-3-pyridinyl)amino](cyanoimino)méthyl]amino}2,2-diméthylpropyl)-4-chlorobenzamide ;
le 4-chloro-2-fluoro-N-[2,2,2-trichloro-1-({(cyanoimino)[(4-éthyl-3-pyridinyl)amino]méthyl}amino)éthyl]benzamide ;
le 4-chloro-N-(2,2,2-trichloro-1-{[(cyanoimino)(5-pyrimidinylamino)méthyl]amino}éthyl)benzamide ;
le 4-chloro-2-fluoro-N-(2,2,2-trichloro-1-{[(cyanoimino)(5-pyrimidinylamino)méthyl]amino}éthyl)benzamide ;
le N-(1-{[[(4-bromo-3-pyridinyl)amino](cyanoimino)méthyl]amino}-2,2,2-trichloroéthyl)-4-chlorobenzamide ;
le N-(1-{[[(2-bromo-3-pyridinyl)amino](cyanoimino)méthyl]amino}-2,2-diméthylpropyl)-4-chlorobenzamide ;
le 4-chloro-N-[1-({(cyanoimino)[(2-éthyl-3-pyridinyl)amino]méthyl}amino)-2,2-diméthylpropyl]benzamide ;
le N-(1-{[[(5-bromo-4-éthyl-3-pyridinyl)amino](cyanoimino)méthyl]amino}-2,2-diméthylpropyl)-4-chlorobenzamide ;
le 4-chloro-N-[1-({(cyanoimino)[(4,5-dibromo-3-pyridinyl)amino]méthyl}amino)-2,2-diméthylpropyl]benzamide ;
le 4-chloro-N-(1-{[[(5-chloro-3-pyridinyl)amino](cyanoimino)méthyl]amino}-2,2-diméthylpropyl)benzamide ;
le N-(1-{[[(5-bromo-6-chloro-3-pyridinyl)amino](cyanoimino)méthyl]amino}-2,2-diméthylpropyl)-4-chlorobenzamide ;
le N-(1-{[[(5-bromo-3-pyridinyl)amino](cyanoimino)méthyl]amino}-2,2-diméthylpropyl)-4-chlorobenzamide ;
le N-(1-{[[(6-bromo-3-pyridinyl)amino](cyanoimino)méthyl]amino}-2,2-diméthylpropyl)-4-chlorobenzamide ;
le 4-chloro-N-(1- {[(cyanoimino)([5-[(4-fluorophényl)sulfonyl]-3-pyridinyl}amino)méthyl]amino}-2,2-diméthylpropyl)benzamide ;
le N-(1-{(({5-[(aminoperoxy)sulfanyl]-3-pyridinyl}amino)(cyanoimino)méthyl]amino}-2,2-diméthylpropyl)-4-chlorobenzamide ;
le N-(1-{[[(6-bromo-4-fluoro-3-pyridinyl)amino](cyanoimino)méthyl]amino}-2,2-diméthylpropyl)-4-chlorobenzamide ;
le 4-chloro-N-[1-{[(cyanoimino)(3-pyridinylamino)méthyl]amino}-2,2,2-trifluoro-1-(trifluorométhyl)éthyl]benzamide;
le 4-chloro-N-(1-([(cyanoimino)(3-pyridinylamino)méthyl]amino}cyclopentyl)benzamide;
le 4-chloro-N-(1- {[(cyanoimino)(3-pyridinylamino)méthyl]amino}cyclohexyl)benzamide;
le 4-chloro-N-[{[(cyanoimino)(3-pyridinylamino)méthyl]amino}(2,6-diméthylphényl)méthyl]benzamide ;
le 4-chloro-N-[{[(cyanoimino)(3-pyridinylamino)méthyl]amino}(3-pyridinyl)méthyl]benzamide ;
le 4-chloro-N-[{[(cyanoimino)(3-pyridinylamino)méthyl]amino}(2-pyridinyl)méthyl]benzamide ;
le 4-chloro-N-(1-{[(cyanoimino)(3-pyridinylamino)méthyl]amino}-2-méthyl-2-phénylpropyl)benzamide;
le 4-chloro-N-(1-{[(cyanoimino)(3-pyridinylamino)méthyl]amino} -3,3-diméthyl-2-oxobutyl)benzamide ;
le 4-chloro-N-(1-{[(cyanoimino)(3-pyridinylamino)méthyl]amino}-3,3,3-trifluoro-2-oxopropyl)benzamide ;
le 4-chloro-N-[1-{[(cyanoimino)(3-pyridinylamino)méthyl]amino}-3,3,3-trifluoro-2-méthyl-2-(trifluorométhyl)propyl]benzamide ;
le 4-[(4-chlorobenzoyl)amino]-4-{[(cyanoimino)(3-pyridinylamino)méthyl]amino}-3,3-diméthylbutanoate de méthyle ;
le 4-chloro-N-[ 1-{ [(cyanoimino)(3-pyridinylamino)méthyl]amino}-4-(diméthylamino)-2,2-diméthylbutyl]benzamide ;
le 4-chloro-N-(4-cyano-1-{[(cyanoimino)(3-pyridinylamino)méthyl]amino}-2,2-diméthylbutyl)benzamide ;
le 4-chloro-N-(1-{[(cyanoimino)(3-pyridinylamino)méthyl]amino}-4-méthoxy-2,2-diméthylbutyl)benzamide ;
le 4-chloro-N-(1-{[(cyanoimino)(3-pyridinylamino)méthyl]amino}-4-hydroxy-2,2-diméthylbutyl)benzamide ;
le N-(4-(aminosulfonyl)-1-{[(cyanoimino)(3-pyridinylamino)méthyl]amino}-2,2-diméthylbutyl)-4-chlorobenzamide ;
le 4-chloro-N-[1-{[(cyanoimino)(3-pyridinylamino)méthyl]amino}-2,2-diméthyl-4-(phénylsulfonyl)butyl]benzamide ;
le 4-chloro-N-(1-{[(cyanoimino)(3-pyridinylamino)méthyl]amino}-3-hydroxy-2,2-diméthylpropyl)benzamide ;
le N-(4-(aminosulfonyl)-2,2-dichloro-1-{[(cyanoimino)(3-pyridinylamino)méthyl]amino}butyl)-4-chlorobenzamide ;
le 4-chloro-N-[4-cyano-1-{[(cyanoimino)(3-pyridinylamino)méthyl]amino}-2,2-bis(trifluorométhyl)butyl]benzamide ;
le 4-chloro-N-(1-{[(cyanoimino)(3-pyridinylamino)méthyl]amino}-2,2-difluoro-4-oxopentyl)benzamide ;
le 4-chloro-N-(1-{[2-cyano-1-(3-pyridinylamino)éthényl]amino}-2,2-diméthylpropyl)benzamide ;
le 4-chloro-N-{1-[[(cyanoimino)(3-pyridinylamino)méthyl](hydroxy)amino]-2,2-diméthylpropyl}benzamide ;
le 4-chloro-N-(2,2,2-trichloro-1-{[2-nitro-1-(3-pyridinylamino)éthényl]amino)éthyl)benzamide ; et
le 4-chloro-N-(2,2,2-trichloro-1-{ [2-cyano-1-(3-pyridinylamino)éthényl]amino}éthyl)benzamide.

83. Composition pharmaceutique comprenant une quantité thérapeutiquement efficace d'un composé répondant à la formule 1 : 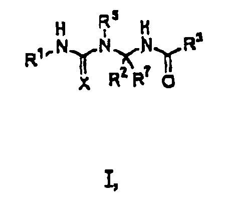 ou d'un sel pharmaceutiquement acceptable en combinaison avec un support pharmaceutiquement acceptable, dans lequel
X est choisi dans le groupe consistant en O, S, CHCN, C(CN)₂, CHNO₂, et NR⁸ ;
R⁸ est choisi dans le groupe consistant en un atome d'hydrogène, un groupe alcoxy, alkyle, alkylsulfonyle, arylalcoxy, aryloxy, arylsulfonyle, cyano, halogénoalkylsulfonyle, hétérocyclealcoxy, hétérocycleoxy, hydroxy, nitro, et sulfamyle ;
R¹ est choisi dans le groupe consistant en un groupe aryle, arylalkyle, hétérocycle, et hétérocyclealkyle ;
R² est choisi parmi un atome d'hydrogène, un groupe alcényle, alcényloxyalkyle, alcényloxy(alcényloxy)alkyle, alcoxyalkyle, alcoxycarbonyle, alcoxycarbonylalkyle, alcoxycarbonyl(halogéno)alkyle, alcoxy(halogéno)alkyle, alkyle, alkylcarbonyle, alkylcarbonylalkyle, alkylcarbonyl(halogéno)alkyle, alkylcarbonyloxyalkyle, alkylsulfinylalkyle, alkylsulfonylalkyle, alkylthioalkyle, alcynyle, amido, amidoalkyle, aryle, arylalcoxyalkyle, arylalcoxycarbonyle, arylalcoxycarbonylalkyle, arylalkyle, arylcarbonyle, arylcarbonylalkyle, arylcarbonyloxyalkyle, aryl(halogéno)alkyle, aryloxyalkyle, aryloxycarbonyle, aryloxycarbonylalkyle, arylalkylthioalkyle, arylsulfonylalkyle, carboxy, carboxyalkyle, carboxy(halogéno)alkyle, cyanoalkyle, cyano(halogéno)alkyle, cycloalcényle, cycloalcénylalkyle, cycloalkyle, cycloalkylalcoxyalkyle, cycloalkylalkyle, cycloalkylcarbonyle, cycloalkyloxyalkyle, cycloalkylalkylthioalkyle, formyle, halogénoalcényle, halogénoalkyle, halogénoalkylcarbonyle, halogénoalcynyle, hétérocycle, hétérocyclealcoxyalkyle, hétérocyclealkyle, hétérocyclecarbonyle, hétérocycleoxyalkyle, hétérocyclealkylthioalkyle, hydroxyalkyle, mercaptoalkyle, sulfamylalkyle, sulfamyl(halogéno)alkyle, et (NR⁹R¹⁰)alkyle dans lesquels R⁹ et R¹⁰ sont indépendamment choisis parmi un atome d'hydrogène, un groupe alkyle, alkylcarbonyle, aryle, arylalkyle, arylcarbonyle, formyle, et S(O)₂R¹¹, dans lequel R¹¹ est choisi parmi un groupe alkyle, aryle, et arylalkyle ;
R³ est choisi dans le groupe consistant en un groupe alkyle, aryle, arylalkyle, hétérocycle, et hétérocyclealkyle ;
R⁵ est choisi dans le groupe consistant en un atome d'hydrogène, un groupe alkyle et OR¹²;
R¹² est choisi dans le groupe consistant en un atome d'hydrogène, un groupe alkyle et arylalkyle ; et
R⁷ est choisi dans le groupe consistant en un atome d'hydrogène, un groupe halogénoalkyle et alkyle en C₁ à C₆ ; ou
R⁷ et R² pris conjointement avec l'atome de carbone auquel ils sont attachés, forment ensemble un cycle carbocyclique à 5 ou 6 chaînons dans lequel le cycle carbocyclique à 5 ou 6 chaînons est facultativement substitué par 1 ou 2 substituants indépendamment choisi dans le groupe consistant en un groupe alcényle, alcoxy, alkyle, alcynyle, halogéno, halogénoalcoxy, et halogénoalkyle ;
dans lequel sauf indication contraire, alkyle par lui-même ou comme partie d'un autre groupe contient de un à dix atomes de carbone ;
alcényle par lui-même ou comme partie d'un autre groupe contient de deux à dix atomes de carbone ;
alcynyle par lui-même ou comme partie d'un autre groupe contient de deux à dix atomes de carbone ;
aryle par lui-même ou comme partie d'un autre groupe est un système de cycle carbocyclique monocyclique ou un système de cycle condensé carbocyclique bicyclique comportant un ou plusieurs cycles aromatiques,
aryle peut facultativement comporter 1, 2, 3, 4 ou 5 substituants indépendamment choisis dans le groupe consistant en un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, sulfamyle, sulfamylalkyle, phényle, furyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle ;
cycloalkyle est un système de cycle monocyclique, bicyclique ou tricyclique ;
cycloalkyle peut facultativement comporter 1, 2, 3, 4 ou 5 substituants indépendamment choisis dans le groupe consistant en un groupe alcényle, alcoxy, alcoxycarbonyle, alcoxycarbonylalkyle, alkyle, alkylcarbonylalkyle, alkylcarbonyloxy, alkylcarbonyloxyalkyle, alkylsulfonylalkyle, alcynyle, amido, amidoalkyle, cyanoalkyle, halogéno, halogénoalcoxy, halogénoalkyle, hydroxy, hydroxyalkyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle ;
hétérocycle par lui-même ou comme partie d'un autre groupe est un système de cycle monocyclique ou bicyclique dans lequel (i) le système de cycle monocyclique est un cycle à 5 ou 6 chaînons contenant 1, 2, 3 ou 4 hétéroatomes indépendamment choisis parmi l'oxygène, l'azote et le soufre dans lequel le cycle à 5 chaînons comporte de 0 à 2 doubles liaisons et le cycle à 6 chaînons comporte de 0 à 3 doubles liaisons et (ii) le système de cycle bicyclique est l'un quelconque des systèmes de cycle monocycliques ci-dessus condensés à un groupe aryle monocyclique tel que défini ci-dessus, un groupe cycloalkyle monocyclique tel que défini ci-dessus ou un système de cycle hétérocyclique monocyclique tel que défini ci-dessus ;
hétérocycle peut facultativement comporter 1, 2 ou 3 substituants indépendamment choisis dans le groupe consistant en un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, oxo, sulfamyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle, et phényle comportant facultativement 1 ou 2 substituants indépendamment choisis parmi un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, sulfamyle, sulfamylalkyle, -NR^{A}R^{B}, (NR^{A}R^{B})alkyle dans lequel R^{A} et R^{B} sont indépendamment choisis parmi un atome d'hydrogène, un groupe alkyle, alkylcarbonyle et formyle ;
amido par lui-même ou comme partie d'un autre groupe est -C(O)NR⁹R¹⁰ dans lequel R⁹ et R¹⁰ sont indépendamment choisis dans le groupe consistant en un atome d'hydrogène, un groupe alkyle, alkylcarbonyle, phényle, phénylalkyle, phénylcarbonyle, formyle et S(O)₂R¹¹ dans lequel R¹¹ est choisi parmi un groupe alkyle, phényle et phénylalkyle ;
cycloalcényle par lui-même ou comme partie d'un autre groupe est un hydrocarbure cyclique contenant de trois à huit atomes de carbone et contenant au moins une double liaison carbone-carbone ;
cycloalcényle peut facultativement comporter 1, 2, 3, 4 ou 5 substituants indépendamment choisis parmi un groupe alcényle, alcoxy, alcoxycarbonyle, alcoxycarbonylalkyle, alkyle, alkylcarbonyloxy, alkylcarbonyloxyalkyle, alcynyle, amido, amidoalkyle, halogéno, halogénoalcoxy, halogénoalkyle, hydroxy, hydroxyalkyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle ; .
sulfamyle est un groupe -SO₂NR⁹⁴R⁹⁵ dans lequel R⁹⁴ et R⁹⁵ sont indépendamment choisis parmi un atome d'hydrogène, un groupe alkyle, aryle et arylalkyle ;
à condition que lorsque X est O ; R² est -CCl₃ ; R³ est un groupe alkyle ou phényle ; et R⁵ et R⁷ sont un atome d'hydrogène ; alors R¹ est autre qu'un groupe phényle ; à condition en outre que
le N-[[[(phénylamino)carbonyl]amino]méthyl]-3-pyridinecarboxamide ;
le N-[[[[(2-méthylphényl)amino)carbonyl]amino]méthyl]-3-pyridinecarboxamide ;
le N-[[[[(4-méthylphényl)amino)carbonyl]amino]méthyl]-3-pyridinecarboxamide ;
le N-[[[[(3-éthoxyphényl)amino)carbonyl]amino]méthyl]-3-pyridinecarboxamide ;
le N-[[[[(4-nitrophényl)amino)carbonyl]amino]méthyl]-3-pyridinecarboxamide ;
et
le N-[[[[(3-méthylphényl)amino)carbonyl]amino]méthyl]-3-pyridinecarboxamide soient exclus.

84. Utilisation d'un composé répondant à la formule I : 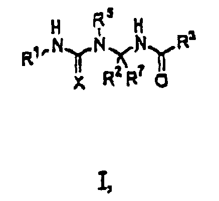 ou d'un sel pharmaceutiquement acceptable de celui-ci pour la fabrication d'un médicament destiné à traiter ou prévenir une maladie qui est prévenue ou améliorée avec des agents d'ouverture des canaux potassiques chez un hôte mammifère en besoin d'un tel traitement par administration d'une quantité thérapeutiquement efficace, dans laquelle
X est choisi dans le groupe consistant en O, S, CHCN, C(CN)₂, CHNO₂, et NR⁸ ;
R⁸ est choisi dans le groupe consistant en un atome d'hydrogène, un groupe alcoxy, alkyle, alkylsulfonyle, arylalcoxy, aryloxy, arylsulfonyle, cyano, halogénoalkylsulfonyle, hétérocyclealcoxy, hétérocycleoxy, hydroxy, nitro, et sulfamyle ;
R¹ est choisi dans le groupe consistant en un groupe aryle, arylalkyle, hétérocycle, et hétérocyclealkyle ;
R² est choisi parmi un atome d'hydrogène, un groupe alcényle, alcényloxyalkyle, alcényloxy(alcényloxy)alkyle, alcoxyalkyle, alcoxycarbonyle, alcoxycarbonylalkyle, alcoxycarbonyl(halogéno)alkyle, alcoxy(halogéno)alkyle, alkyle, alkylcarbonyle, alkylcarbonylalkyle, alkylcarbonyl(halogéno)alkyle, alkylcarbonyloxyalkyle, alkylsulfinylalkyle, alkylsulfonylalkyle, alkylthioalkyle, alcynyle, amido, amidoalkyle, aryle, arylalcoxyalkyle, arylalcoxycarbonyle, arylalcoxycarbonylalkyle, arylalkyle, arylcarbonyle, arylcarbonylalkyle, arylcarbonyloxyalkyle, aryl(halogéno)alkyle, aryloxyalkyle, aryloxycarbonyle, aryloxycarbonylalkyle, arylalkylthioalkyle, arylsulfonylalkyle, carboxy, carboxyalkyle, carboxy(halogéno)alkyle, cyanoalkyle, cyano(halogéno)alkyle, cycloalcényle, cycloalcénylalkyle, cycloalkyle, cycloalkylalcoxyalkyle, cycloalkylalkyle, cycloalkylcarbonyle, cycloalkyloxyalkyle, cycloalkylalkylthioalkyle, formyle, halogénoalcényle, halogénoalkyle, halogénoalkylcarbonyle, halogénoalcynyle, hétérocycle, hétérocyclealcoxyalkyle, hétérocyclealkyle, hétérocyclecarbonyle, hétérocycleoxyalkyle, hétérocyclealkylthioalkyle, hydroxyalkyle, mercaptoalkyle, sulfamylalkyle, sulfamyl(halogéno)alkyle, et (NR⁹R¹⁰)alkyle dans lesquels R⁹ et R¹⁰ sont indépendamment choisis parmi un atome d'hydrogène, un groupe alkyle, alkylcarbonyle, aryle, arylalkyle, arylcarbonyle, formyle, et S(O)₂R¹¹, dans lequel R¹¹ est choisi parmi un groupe alkyle, aryle, et arylalkyle ;
R³ est choisi dans le groupe consistant en un groupe alkyle, aryle, arylalkyle, hétérocycle, et hétérocyclealkyle ;
R⁵ est choisi dans le groupe consistant en un atome d'hydrogène, un groupe alkyle et OR¹²;
R¹² est choisi dans le groupe consistant en un atome d'hydrogène, un groupe alkyle et arylalkyle ; et
R⁷ est choisi dans le groupe consistant en un atome d'hydrogène, un groupe halogénoalkyle ; et alkyle en C₁ à C₆ ; ou
R⁷ et R² pris conjointement avec l'atome de carbone auquel ils sont attachés, forment ensemble un cycle carbocyclique à 5 ou 6 chaînons dans lequel le cycle carboxylique à 5 ou 6 chaînons est facultativement substitué par 1 ou 2 substituants indépendamment choisis dans le groupe consistant en un groupe alcényle, alcoxy, alkyle, alcynyle, halogéno, halogénoalcoxy, et halogénoalkyle ;
dans lequel sauf indication contraire, alkyle par lui-même ou comme partie d'un autre groupe contient de un à dix atomes de carbone ;
alcényle par lui-même ou comme partie d'un autre groupe contient de deux à dix atomes de carbone ;
alcynyle par lui-même ou comme partie d'un autre groupe contient de deux à dix atomes de carbone ;
aryle par lui-même ou comme partie d'un autre groupe est un système de cycle carbocyclique monocyclique ou un système de cycle condensé carbocyclique bicyclique comportant un ou plusieurs cycles aromatiques,
aryle peut facultativement comporter 1, 2, 3, 4 ou 5 substituants indépendamment choisis dans le groupe consistant en un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, sulfamyle, sulfamylalkyle, phényle, furyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle ;
cycloalkyle est un système de cycle monocyclique, bicyclique ou tricyclique ;
cycloalkyle peut facultativement comporter 1, 2, 3, 4 ou 5 substituants indépendamment choisis dans le groupe consistant en un groupe alcényle, alcoxy, alcoxycarbonyle, alcoxycarbonylalkyle, alkyle, alkylcarbonylalkyle, alkylcarbonyloxy, alkylcarbonyloxyalkyle, alkylsulfonylalkyle, alcynyle, amido, amidoalkyle, cyanoalkyle, halogéno, halogénoalcoxy, halogénoalkyle, hydroxy, hydroxyalkyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle;
hétérocycle par lui-même ou comme partie d'un autre groupe est un système de cycle monocyclique ou bicyclique dans lequel (i) le système de cycle monocyclique est un cycle à 5 ou 6 chaînons contenant 1, 2, 3 ou 4 hétéroatomes indépendamment choisis parmi l'oxygène, l'azote et le soufre dans lequel le cycle à 5 chaînons comporte de 0 à 2 doubles liaisons et le cycle à 6 chaînons comporte de 0 à 3 doubles liaisons et (ii) le système de cycle bicyclique est l'un quelconque des systèmes de cycle monocycliques ci-dessus condensés à un groupe aryle monocyclique tel que défini ci-dessus, un groupe cycloalkyle monocyclique tel que défini ci-dessus ou un système de cycle hétérocyclique monocyclique tel que défini ci-dessus ;
hétérocycle peut facultativement comporter 1, 2 ou 3 substituants indépendamment choisis dans le groupe consistant en un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, oxo, sulfamyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle, et phényle comportant facultativement 1 ou 2 substituants indépendamment choisis parmi un groupe alcényle, alcoxy, alcoxycarbonyle, alcoxycarbonylalkyle, alkyle, alkylcarbonyle, alkylcarbonyloxy, alkylcarbonyloxyalkyle, alkylsulfinyle, alkylsulfonyle, alcynyle, amido, amidoalkyle, phénylalcoxycarbonyle, phénylalcoxycarbonylalkyle, phénylcarbonyloxy, phénylcarbonyloxyalkyle, phényloxycarbonyle, phényloxycarbonylalkyle, phénylsulfonyle, cyano, halogéno, halogénoalkyle, halogénoalcoxy, nitro, sulfamyle, sulfamylalkyle, -NR^{A}R^{B}, (NR^{A}R^{B})alkyle dans lequel R^{A} et R^{B} sont indépendamment choisis parmi un atome d'hydrogène, un groupe alkyle, alkylcarbonyle et formyle ;
amido par lui-même ou comme partie d'un autre groupe est -C(O)NR⁹R¹⁰ dans lequel R⁹ et R¹⁰ sont indépendamment choisis dans le groupe consistant en un atome d'hydrogène, un groupe alkyle, alkylcarbonyle, phényle, phénylalkyle, phénylcarbonyle, formyle et S(O)₂R¹¹ dans lequel R¹¹ est choisi parmi un groupe alkyle, phényle et phénylalkyle ;
cycloalcényle par lui-même ou comme partie d'un autre groupe est un hydrocarbure cyclique contenant de trois à huit atomes de carbone et contenant au moins une double liaison carbone-carbone ;
cycloalcényle peut facultativement comporter 1, 2, 3, 4 ou 5 substituants indépendamment choisis parmi un groupe alcényle, alcoxy, alcoxycarbonyle, alcoxycarbonylalkyle, alkyle, alkylcarbonyloxy, alkylcarbonyloxyalkyle, alcynyle, amido, amidoalkyle, halogéno, halogénoalcoxy, halogénoalkyle, hydroxy, hydroxyalkyle, sulfamylalkyle, -NR^{A}R^{B} et (NR^{A}R^{B})alkyle dans lequel R^{A} et R^{B} sont indépendamment choisis parmi un atome d'hydrogène, un groupe alkyle, alkylcarbonyle et formyle;
sulfamyle est un groupe -SO₂NR⁹⁴R⁹⁵ dans lequel R⁹⁴ et R⁹⁵ sont indépendamment choisis parmi un atome d'hydrogène, un groupe alkyle, aryle et arylalkyle ;
à condition que lorsque X est O ; R² est -CCl₃ ; R³ est un groupe alkyle ou phényle ; et R⁵ et R⁷ sont un atome d'hydrogène ; alors R¹ est autre qu'un groupe phényle ;
à condition en outre que l'utilisation de
N-[[[(phénylamino)carbonyl]amino]méthyl)-3-pyridinecarboxamide ;
N[[[[(2-méthylphényl)amino)carbonyl]amino]méthyl]-3-pyridinecarboxamide ;
N-[[[[(4-méthylphényl)amino)carbonyl]amino]méthyl]-3-pyridinecarboxamide ;
N-[[[[(3-éthoxyphényl)amino)carbonyl]amino]méthyl]-3-pyridinecarboxamide ;
N-[[[[(4-nitrophényl)amino)carbonyl]amino]méthyl]-3-pyridinecarboxamide ;
ou de
N-[[[[(3-méthylphényl)amino)carbonyl]amino]méthyl]-3-pyridinecarboxamide
pour la fabrication d'un médicament destiné au traitement de la maladie de Parkinson est exclue.

85. Utilisation selon la revendication 84, dans laquelle le trouble est choisi dans le groupe consistant en l'asthme, l'épilepsie, le syndrome de Raynaud's, la claudication intermittente, la migraine, la douleur, la pollakiurie, l'instabilité de la vessie, la nycturie, l'hyperréflectivité de la vessie, l'énurésie, l'alopécie, la cardioprotection, l'ischémie, les troubles de l'alimentation, les troubles fonctionnels de l'intestin et la neurodégénérescence.

86. Utilisation selon la revendication 84, dans laquelle le trouble est l'hyperactivité de la vessie.

87. Utilisation selon la revendication 84, dans laquelle le trouble est l'hyperplasie bénigne de la prostate.

88. Utilisation selon la revendication 84, dans laquelle le trouble est la dysménorrhée.

89. Utilisation selon la revendication 84, dans laquelle le trouble est l'accouchement prématuré.

90. Utilisation selon la revendication 84, dans lequel le trouble est l'incontinence urinaire.

91. Utilisation selon la revendication 84, dans laquelle le trouble est choisi dans le groupe consistant en le dysfonctionnement érectile et l'éjaculation précoce.

92. Utilisation selon la revendication 84, dans laquelle le trouble est un dysfonctionnement sexuel chez la femme.

93. Utilisation d'un composé de formule VII : 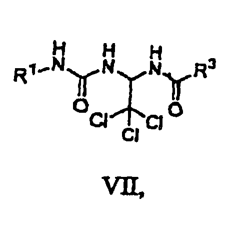
ou d'un sel pharmaceutiquement acceptable de celui-ci dans lequel,
R¹ est un groupe phényle ;
R³ est choisi dans le groupe consistant en un groupe alkyle et phényle,
pour la fabrication d'un médicament destiné à traiter ou prévenir une maladie qui est prévenue ou améliorée avec des agents d'ouverture des canaux potassiques chez un hôte mammifère en besoin d'un tel traitement par administration d'une quantité thérapeutiquement efficace.

94. Composé selon la revendication 93, choisi dans le groupe consistant en
le N-{ 1-[(anilinocarbonyl)amino]-2,2,2-trichloroéthyl}-4-méthylbenzamide et
le 4-méthyl-N-(2,2,2-trichloro-1-([(2-fluoroanilino)carbonyl]aminoléthyl)benzamide.

95. Utilisation selon la revendication 93, dans laquelle le trouble est choisi dans le groupe consistant en l'asthme, l'épilepsie, le syndrome de Raynaud's, la claudication intermittente, la migraine, la douleur, la pollakiurie, l'instabilité de la vessie, la nycturie, l'hyperréflectivité de la vessie, l'énurésie, l'alopécie, la cardiaprotection, l'ischémie, les troubles de l'alimentation, les troubles fonctionnels de l'intestin et la neurodégénérescence.

96. Utilisation selon la revendication 93, dans laquelle le trouble est l'hyperactivité de la vessie.

97. Utilisation selon la revendication 93, dans laquelle le trouble est l'hyperplasie bénigne de la prostate.

98. Utilisation selon la revendication 93, dans laquelle le trouble est la dysménorrhée.

99. Utilisation selon la revendication 93, dans laquelle le trouble est l'accouchement prématuré.

100. Utilisation selon la revendication 93, dans laquelle le trouble est l'incontinence urinaire.

101. Utilisation selon la revendication 93, dans laquelle le trouble est choisi dans le groupe consistant en e dysfonctionnement érectile et l'éjaculation précoce.

102. Utilisation selon la revendication 93, dans laquelle le trouble est un dysfonctionnement sexuel chez la femme.

103. Composé selon l'une quelconque des revendications 1 à 82 et 94, pour une utilisation en tant qu'agent thérapeutique, à condition que
le N-[[[[(2-méthylphényl)amino]carbonyl]amino]phénylméthyl]-benzamide ;
le N-[[[[(4-chlorophényl)amino]carbonyl]amino]phénylméthyl]-benzamide;
le N-[2,2-dichloro-1-[[phénylamino)thioxométhyl]amino]éthyl]-benzamide ;
le N-[2,2-dichloro-1-[[phénylamino)thioxométhyl]amino)éthyl]-4-méthyl-benzamide ;
le N-[2,2-dichloro-1-[[[4-méthylphényl)amino)thioxométhyl)amino)éthyl)-benzamide ;
le N-[2,2-dichloro-1 -[[[4-méthylphényl)amino)thioxométhyl]amino]éthyl]-4-méthyl-benzamide ;
le N-[2,2-dichloro-l -[[[4-chlorophényl)amino)thioxométhyl]amino]éthyl]-benzamide ;
le N-[2,2-dichloro-1 -[[[4-chlorophényl)amino)thioxométhyl]amino]éthyl]-4-méthyl-benzamide ;
le N-[[[(phénylamino)carbonyl]amino]méthyl]-benzamide ;
le N-[[[(phénylamino)thioxométhyl]amino]méthyl]benzamide ;
le N-[[[(phénylamino)carbonyl]amino]méthyl]-4-chloro-benzamide ;
le N-[[[(phénylamino)thioxométhyl]amino]méthyl]-4-chloro-benzamide;
la 1-(1-acétamido-2,2,2-trichloroéthyl)-3-phényl-2-thio-urée ;
la 1-phényl-2-thio-3-(2,2,2-trichloro-1-propionamidoéthyl)-urée ; et
la 1-(1-butyramido-2,2,2-trichloroéthyl)-3-phényl-2-thio-urée ne soient pas exclus.
